# EUROPEAN PATENT SPECIFICATION

(11) **EP 2 690 178 B1**
(45) Date of publication and mention of the grant of the patent: **22.12.2021**
(21) Application number: 13189930.4
(22) Date of filing: 03.10.2007
(51) Int. Cl.: C12P 19/18, C12P 19/26, C12P 19/28, C08B 37/00, C08B 37/08

(54) **Targeted glycosaminoglycan polymers by polymer grafting and methods of making and using same**
Gezielte Glycosaminoglycanpolymere durch Polymerpfropfung und Verfahren zur Herstellung und Verwendung davon
Polymères de glycosaminoglycane ciblés par greffage polymère et leurs procédés de fabrication et d'utilisation

(30) Priority: 03.10.2006 US 849034 P; 30.03.2007 US 921296 P
(43) Date of publication of application: 29.01.2014
(62) Divisional of application: 07874512.2
(73) Proprietor: The Board Of Regents Of The University Of Oklahoma, Oklahoma City, OK 73019 (US)
(72) Inventor: Deangelis, Paul, L, Edmond, OK 73034 (US); Sismey-Ragatz, Alison, Cassville, WI 53806 (US)
(74) Representative: Carpmaels & Ransford LLP

(56) References cited:
- WO-A2-2006/033693
- KANE ET AL: "Functional characterization of PmHS1, a Pasteurella multocida heparosan synthase", THE JOURNAL OF BIOLOGICAL CHEMISTRY, vol. 281, 7 September 2006 (2006-09-07), pages 33192-33197, XP002699111,
- WILLIAMS ET AL: "Critical elements of oligosaccharide acceptor substrates for the Pasteurella multocida hyaluronan synthase", THE JOURNAL OF BIOLOGICAL CHEMISTRY, vol. 281, 16 December 2005 (2005-12-16), pages 5391-5397, XP002482219,
- JING ET AL: "Defined megadalton hyaluronan polymer standards", ANALYTICAL BIOCHEMISTRY, vol. 355, 23 June 2006 (2006-06-23), pages 183-188, XP024942078,
- DEANGELIS ET AL: "Chemoenzymatic synthesis of glycosaminoglycans with Pasteurella synthases", Conference Abstracts / Annual Conference of the Society for Glycobiology; December 2003, San Diego, CA, 2003, pages 838-839, XP002716695, Retrieved from the Internet: URL:http://www.glycobiology.org/files/2003 _-_san_diego_full.pdf [retrieved on 2013-11-20]
- DEANGELIS ET AL: "Identification of a distinct, cryptic heparosan synthase from Pasteurella multocida types A, D, and F", JOURNAL OF BACTERIOLOGY, vol. 186, 2004, pages 8529-8532, XP002716696,
- PUMMILL ET AL: "The functional molecular mass of the Pasteurella hyaluronan synthase is a monomer", BIOCHIMICA ET BIOPHYSICA ACTA, vol. 1770, 4 October 2006 (2006-10-04), pages 286-290, XP005818421,
- TRACY ET AL: "Acceptor specificity of the Pasteurella hyaluronan and chondroitin synthases and production of chimeric glycosaminoglycans", THE JOURNAL OF BIOLOGICAL CHEMISTRY, vol. 282, 10 November 2006 (2006-11-10), pages 337-344, XP002699112,
- AVCI ET AL: "Enzymatic synthesis of glycosaminoglycans: Improving on nature" In: Demchenko (Editor): "Fronteirs in Modern Carbohydrate Chemistry / ACS Symposium Series", 13 March 2007 (2007-03-13), ACS, XP002699118, vol. 960, pages 253-284, * See pages 264-269 (Section 3); published in print after the first priority date *
- SISMEY-RAGATZ ET AL: "Chemoenzymatic synthesis with distinct Pasteurella heparosan synthases", THE JOURNAL OF BIOLOGICAL CHEMISTRY, vol. 282, 11 July 2007 (2007-07-11), pages 28321-28327, XP002699113,
- LINHARDT ET AL: "Enzymatic synthesis of glycosaminoglycan heparin", SEMINARS IN THROMBOSIS AND HEMOSTASIS, vol. 33, July 2007 (2007-07), pages 453-465, XP008163151,
- DEANGELIS: "Hyaluronan biosynthesis systems from microbes to man" In: Kamerling (Editor-in-chief): "Comprehensive Glycoscience", 2007, Elsevier, XP002699207, ISBN: 978-0-4445-2746-2 vol. 3, pages 325-341, * See pages 332-333 (section 3.18.4.3) and pages 335-337 (section 3.18.6); the date of publication is not yet available *
- DEANGELIS ET AL: "Identification and molecular cloning of a heparosan synthase from Pasteurella multocida type D", THE JOURNAL OF BIOLOGICAL CHEMISTRY, vol. 277, 2002, pages 7209-7213, XP001100687,

### SEQUENCE LISTING

<110> DeAngelis, Paul Sismey Ragatz, Alison
<120> TARGETED GLYCOSAMINOGLYCAN POLYMERS BY POLYMER GRAFTING AND METHODS OF MAKING AND USING SAME
<130> P062779EP
<150> 60/849,034
   <151> 2006-10-03
<160> 71
<170> PatentIn version 3.4
<210> 1
   <211> 2920
   <212> DNA
   <213> Pasteurella multocida
<400> 1
   atgaatacat tatcacaagc aataaaagca tataacagca atgactatca attagcactc     60
   aaattatttg aaaagtcggc ggaaatctat ggacggaaaa ttgttgaatt tcaaattacc    120
   aaatgcaaag aaaaactctc agcacatcct tctgttaatt cagcacatct ttctgtaaat    180
   aaagaagaaa aagtcaatgt ttgcgatagt ccgttagata ttgcaacaca actgttactt    240
   tccaacgtaa aaaaattagt actttctgac tcggaaaaaa acacgttaaa aaataaatgg    300
   aaattgctca ctgagaagaa atctgaaaat gcggaggtaa gagcggtcgc ccttgtacca    360
   aaagattttc ccaaagatct ggttttagcg cctttacctg atcatgttaa tgattttaca    420
   tggtacaaaa agcgaaagaa aagacttggc ataaaacctg aacatcaaca tgttggtctt    480
   tctattatcg ttacaacatt caatcgacca gcaattttat cgattacatt agcctgttta    540
   gtaaaccaaa aaacacatta cccgtttgaa gttatcgtga cagatgatgg tagtcaggaa    600
   gatctatcac cgatcattcg ccaatatgaa aataaattgg atattcgcta cgtcagacaa    660
   aaagataacg gttttcaagc cagtgccgct cggaatatgg gattacgctt agcaaaatat    720
   gactttattg gcttactcga ctgtgatatg gcgccaaatc cattatgggt tcattcttat    780
   gttgcagagc tattagaaga tgatgattta acaatcattg gtccaagaaa atacatcgat    840
   acacaacata ttgacccaaa agacttctta aataacgcga gtttgcttga atcattacca    900
   gaagtgaaaa ccaataatag tgttgccgca aaaggggaag gaacagtttc tctggattgg    960
   cgcttagaac aattcgaaaa aacagaaaat ctccgcttat ccgattcgcc tttccgtttt   1020
   tttgcggcgg gtaatgttgc tttcgctaaa aaatggctaa ataaatccgg tttctttgat   1080
   gaggaattta atcactgggg tggagaagat gtggaatttg gatatcgctt attccgttac   1140
   ggtagtttct ttaaaactat tgatggcatt atggcctacc atcaagagcc accaggtaaa   1200
   gaaaatgaaa ccgatcgtga agcgggaaaa aatattacgc tcgatattat gagagaaaag 1260
   gtcccttata tctatagaaa acttttacca atagaagatt cgcatatcaa tagagtacct 1320
   ttagtttcaa tttatatccc agcttataac tgtgcaaact atattcaacg ttgcgtagat 1380
   agtgcactga atcagactgt tgttgatctc gaggtttgta tttgtaacga tggttcaaca 1440
   gataatacct tagaagtgat caataagctt tatggtaata atcctagggt acgcatcatg 1500
   tctaaaccaa atggcggaat agcctcagca tcaaatgcag ccgtttcttt tgctaaaggt 1560
   tattacattg ggcagttaga ttcagatgat tatcttgagc ctgatgcagt tgaactgtgt 1620
   ttaaaagaat ttttaaaaga taaaacgcta gcttgtgttt ataccactaa tagaaacgtc 1680
   aatccggatg gtagcttaat cgctaatggt tacaattggc cagaattttc acgagaaaaa 1740
   ctcacaacgg ctatgattgc tcaccacttt agaatgttca cgattagagc ttggcattta 1800
   actgatggat tcaatgaaaa aattgaaaat gccgtagact atgacatgtt cctcaaactc 1860
   agtgaagttg gaaaatttaa acatcttaat aaaatctgct ataaccgtgt attacatggt 1920
   gataacacat caattaagaa acttggcatt caaaagaaaa accattttgt tgtagtcaat 1980
   cagtcattaa atagacaagg cataacttat tataattatg acgaatttga tgatttagat 2040
   gaaagtagaa agtatatttt caataaaacc gctgaatatc aagaagagat tgatatctta 2100
   aaagatatta aaatcatcca gaataaagat gccaaaatcg cagtcagtat tttttatccc 2160
   aatacattaa acggcttagt gaaaaaacta aacaatatta ttgaatataa taaaaatata 2220
   ttcgttattg ttctacatgt tgataagaat catcttacac cagatatcaa aaaagaaata 2280
   ctagccttct atcataaaca tcaagtgaat attttactaa ataatgatat ctcatattac 2340
   acgagtaata gattaataaa aactgaggcg catttaagta atattaataa attaagtcag 2400
   ttaaatctaa attgtgaata catcattttt gataatcatg acagcctatt cgttaaaaat 2460
   gacagctatg cttatatgaa aaaatatgat gtcggcatga atttctcagc attaacacat 2520
   gattggatcg agaaaatcaa tgcgcatcca ccatttaaaa agctcattaa aacttatttt 2580
   aatgacaatg acttaaaaag tatgaatgtg aaaggggcat cacaaggtat gtttatgacg 2640
   tatgcgctag cgcatgagct tctgacgatt attaaagaag tcatcacatc ttgccagtca 2700
   attgatagtg tgccagaata taacactgag gatatttggt tccaatttgc acttttaatc 2760
   ttagaaaaga aaaccggcca tgtatttaat aaaacatcga ccctgactta tatgccttgg 2820
   gaacgaaaat tacaatggac aaatgaacaa attgaaagtg caaaaagagg agaaaatata 2880
   cctgttaaca agttcattat taatagtata actctataaa 2920
<210> 2
   <211> 972
   <212> PRT
   <213> Pasteurella multocida
<400> 2
   Met Asn Thr Leu Ser Gln Ala Ile Lys Ala Tyr Asn Ser Asn Asp Tyr
   1               5                   10                  15
   Gln Leu Ala Leu Lys Leu Phe Glu Lys Ser Ala Glu Ile Tyr Gly Arg
               20                  25                  30
   Lys Ile Val Glu Phe Gln Ile Thr Lys Cys Lys Glu Lys Leu Ser Ala
           35                  40                  45
   His Pro Ser Val Asn Ser Ala His Leu Ser Val Asn Lys Glu Glu Lys
       50                  55                  60
   Val Asn Val Cys Asp Ser Pro Leu Asp Ile Ala Thr Gln Leu Leu Leu
   65                  70                  75                  80
   Ser Asn Val Lys Lys Leu Val Leu Ser Asp Ser Glu Lys Asn Thr Leu
                   85                  90                  95
   Lys Asn Lys Trp Lys Leu Leu Thr Glu Lys Lys Ser Glu Asn Ala Glu
               100                 105                 110
   Val Arg Ala Val Ala Leu Val Pro Lys Asp Phe Pro Lys Asp Leu Val
           115                 120                 125
   Leu Ala Pro Leu Pro Asp His Val Asn Asp Phe Thr Trp Tyr Lys Lys
       130                 135                 140
   Arg Lys Lys Arg Leu Gly Ile Lys Pro Glu His Gln His Val Gly Leu
   145                 150                 155                 160
   Ser Ile Ile Val Thr Thr Phe Asn Arg Pro Ala Ile Leu Ser Ile Thr
                   165                 170                 175
   Leu Ala Cys Leu Val Asn Gln Lys Thr His Tyr Pro Phe Glu Val Ile
               180                 185                 190
   Val Thr Asp Asp Gly Ser Gln Glu Asp Leu Ser Pro Ile Ile Arg Gln
           195                 200                 205
   Tyr Glu Asn Lys Leu Asp Ile Arg Tyr Val Arg Gln Lys Asp Asn Gly
       210                 215                 220
   Phe Gln Ala Ser Ala Ala Arg Asn Met Gly Leu Arg Leu Ala Lys Tyr
   225                 230                 235                 240
   Asp Phe Ile Gly Leu Leu Asp Cys Asp Met Ala Pro Asn Pro Leu Trp
                   245                 250                 255
   Val His Ser Tyr Val Ala Glu Leu Leu Glu Asp Asp Asp Leu Thr Ile
               260                 265                 270
   Ile Gly Pro Arg Lys Tyr Ile Asp Thr Gln His Ile Asp Pro Lys Asp
           275                 280                 285
   Phe Leu Asn Asn Ala Ser Leu Leu Glu Ser Leu Pro Glu Val Lys Thr
       290                 295                 300
   Asn Asn Ser Val Ala Ala Lys Gly Glu Gly Thr Val Ser Leu Asp Trp
   305                 310                 315                 320
   Arg Leu Glu Gln Phe Glu Lys Thr Glu Asn Leu Arg Leu Ser Asp Ser
                   325                 330                 335
   Pro Phe Arg Phe Phe Ala Ala Gly Asn Val Ala Phe Ala Lys Lys Trp
               340                 345                 350
   Leu Asn Lys Ser Gly Phe Phe Asp Glu Glu Phe Asn His Trp Gly Gly
           355                 360                 365
   Glu Asp Val Glu Phe Gly Tyr Arg Leu Phe Arg Tyr Gly Ser Phe Phe
       370                 375                 380
   Lys Thr Ile Asp Gly Ile Met Ala Tyr His Gln Glu Pro Pro Gly Lys
   385                 390                 395                 400
   Glu Asn Glu Thr Asp Arg Glu Ala Gly Lys Asn Ile Thr Leu Asp Ile
                   405                 410                 415
   Met Arg Glu Lys Val Pro Tyr Ile Tyr Arg Lys Leu Leu Pro Ile Glu
               420                 425                 430
   Asp Ser His Ile Asn Arg Val Pro Leu Val Ser Ile Tyr Ile Pro Ala
           435                 440                 445
   Tyr Asn Cys Ala Asn Tyr Ile Gln Arg Cys Val Asp Ser Ala Leu Asn
       450                 455                 460
   Gln Thr Val Val Asp Leu Glu Val Cys Ile Cys Asn Asp Gly Ser Thr
   465                 470                 475                 480
   Asp Asn Thr Leu Glu Val Ile Asn Lys Leu Tyr Gly Asn Asn Pro Arg
                   485                 490                 495
   Val Arg Ile Met Ser Lys Pro Asn Gly Gly Ile Ala Ser Ala Ser Asn
   500 505 510
   Ala Ala Val Ser Phe Ala Lys Gly Tyr Tyr Ile Gly Gln Leu Asp Ser
   515 520 525
   Asp Asp Tyr Leu Glu Pro Asp Ala Val Glu Leu Cys Leu Lys Glu Phe
   530 535 540
   Leu Lys Asp Lys Thr Leu Ala Cys Val Tyr Thr Thr Asn Arg Asn Val
   545 550 555 560
   Asn Pro Asp Gly Ser Leu Ile Ala Asn Gly Tyr Asn Trp Pro Glu Phe
   565 570 575
   Ser Arg Glu Lys Leu Thr Thr Ala Met Ile Ala His His Phe Arg Met
   580 585 590
   Phe Thr Ile Arg Ala Trp His Leu Thr Asp Gly Phe Asn Glu Lys Ile
   595 600 605
   Glu Asn Ala Val Asp Tyr Asp Met Phe Leu Lys Leu Ser Glu Val Gly
   610 615 620
   Lys Phe Lys His Leu Asn Lys Ile Cys Tyr Asn Arg Val Leu His Gly
   625 630 635 640
   Asp Asn Thr Ser Ile Lys Lys Leu Gly Ile Gln Lys Lys Asn His Phe
   645 650 655
   Val Val Val Asn Gln Ser Leu Asn Arg Gln Gly Ile Thr Tyr Tyr Asn
   660 665 670
   Tyr Asp Glu Phe Asp Asp Leu Asp Glu Ser Arg Lys Tyr Ile Phe Asn
   675 680 685
   Lys Thr Ala Glu Tyr Gln Glu Glu Ile Asp Ile Leu Lys Asp Ile Lys
   690 695 700
   Ile Ile Gln Asn Lys Asp Ala Lys Ile Ala Val Ser Ile Phe Tyr Pro
   705 710 715 720
   Asn Thr Leu Asn Gly Leu Val Lys Lys Leu Asn Asn Ile Ile Glu Tyr
   725 730 735
   Asn Lys Asn Ile Phe Val Ile Val Leu His Val Asp Lys Asn His Leu
   740 745 750
   Thr Pro Asp Ile Lys Lys Glu Ile Leu Ala Phe Tyr His Lys His Gln
   755 760 765
   Val Asn Ile Leu Leu Asn Asn Asp Ile Ser Tyr Tyr Thr Ser Asn Arg
   770 775 780
   Leu Ile Lys Thr Glu Ala His Leu Ser Asn Ile Asn Lys Leu Ser Gln
   785 790 795 800
   Leu Asn Leu Asn Cys Glu Tyr Ile Ile Phe Asp Asn His Asp Ser Leu
   805 810 815
   Phe Val Lys Asn Asp Ser Tyr Ala Tyr Met Lys Lys Tyr Asp Val Gly
   820 825 830
   Met Asn Phe Ser Ala Leu Thr His Asp Trp Ile Glu Lys Ile Asn Ala
   835 840 845
   His Pro Pro Phe Lys Lys Leu Ile Lys Thr Tyr Phe Asn Asp Asn Asp
   850 855 860
   Leu Lys Ser Met Asn Val Lys Gly Ala Ser Gln Gly Met Phe Met Thr
   865 870 875 880
   Tyr Ala Leu Ala His Glu Leu Leu Thr Ile Ile Lys Glu Val Ile Thr
   885 890 895
   Ser Cys Gln Ser Ile Asp Ser Val Pro Glu Tyr Asn Thr Glu Asp Ile
   900 905 910
   Trp Phe Gln Phe Ala Leu Leu Ile Leu Glu Lys Lys Thr Gly His Val
   915 920 925
   Phe Asn Lys Thr Ser Thr Leu Thr Tyr Met Pro Trp Glu Arg Lys Leu
   930 935 940
   Gln Trp Thr Asn Glu Gln Ile Glu Ser Ala Lys Arg Gly Glu Asn Ile
   945 950 955 960
   Pro Val Asn Lys Phe Ile Ile Asn Ser Ile Thr Leu
   965 970
<210> 3
   <211> 2979
   <212> DNA
   <213> Pasteurella multocida
<400> 3
   ttataaactg attaaagaag gtaaacgatt caagcaaggt taatttttaa aggaaagaaa 60
   atgaatacat tatcacaagc aataaaagca tataacagca atgactatga attagcactc 120
   aaattatttg agaagtctgc tgaaacctac gggcgaaaaa tcgttgaatt ccaaattatc 180
   aaatgtaaag aaaaactctc gaccaattct tatgtaagtg aagataaaaa aaacagtgtt 240
   tgcgatagct cattagatat cgcaacacag ctcttacttt ccaacgtaaa aaaattaact 300
   ctatccgaat cagaaaaaaa cagtttaaaa aataaatgga aatctatcac tgggaaaaaa 360
   tcggagaacg cagaaatcag aaaggtggaa ctagtaccca aagattttcc taaagatctt 420
   gttcttgctc cattgccaga tcatgttaat gattttacat ggtacaaaaa tcgaaaaaaa 480
   agcttaggta taaagcctgt aaataagaat atcggtcttt ctattattat tcctacattt 540
   aatcgtagcc gtattttaga tataacgtta gcctgtttgg tcaatcagaa aacaaactac 600
   ccatttgaag tcgttgttgc agatgatggt agtaaggaaa acttacttac cattgtgcaa 660
   aaatacgaac aaaaacttga cataaagtat gtaagacaaa aagattatgg atatcaattg 720
   tgtgcagtca gaaacttagg tttacgtaca gcaaagtatg attttgtctc gattctagac 780
   tgcgatatgg caccacaaca attatgggtt cattcttatc ttacagaact attagaagac 840
   aatgatattg ttttaattgg acctagaaaa tatgtggata ctcataatat taccgcagaa 900
   caattcctta acgatccata tttaatagaa tcactacctg aaaccgctac aaataacaat 960
   ccttcgatta catcaaaagg aaatatatcg ttggattgga gattagaaca tttcaaaaaa 1020
   accgataatc tacgtctatg tgattctccg tttcgttatt ttagttgcgg taatgttgca 1080
   ttttctaaag aatggctaaa taaagtaggt tggttcgatg aagaatttaa tcattggggg 1140
   ggcgaagatg tagaatttgg ttacagatta tttgccaaag gctgtttttt cagagtaatt 1200
   gacggcggaa tggcatacca tcaagaacca cctggtaaag aaaatgaaac agaccgcgaa 1260
   gctggtaaaa gtattacgct taaaattgtg aaagaaaagg taccttacat ctatagaaag 1320
   cttttaccaa tagaagattc acatattcat agaatacctt tagtttctat ttatatcccc 1380
   gcttataact gtgcaaatta tattcaaaga tgtgtagata gtgctcttaa tcaaactgtt 1440
   gtcgatctcg aggtttgtat ttgtaacgat ggttcaacag ataatacctt agaagtgatc 1500
   aataagcttt atggtaataa tcctagggta cgcatcatgt ctaaaccaaa tggcggaata 1560
   gcctcagcat caaatgcagc cgtttctttt gctaaaggtt attacattgg gcagttagat 1620
   tcagatgatt atcttgagcc tgatgcagtt gaactgtgtt taaaagaatt tttaaaagat 1680
   aaaacgctag cttgtgttta taccactaat agaaacgtca atccggatgg tagcttaatc 1740
   gctaatggtt acaattggcc agaattttca cgagaaaaac tcacaacggc tatgattgct 1800
   caccatttta gaatgtttac gattagagct tggcatttaa cggatggatt taacgaaaat 1860
   attgaaaacg ccgtggatta tgacatgttc cttaaactca gtgaagttgg aaaatttaaa   1920
   catcttaata aaatctgcta taaccgcgta ttacatggtg ataacacatc cattaagaaa   1980
   ctcggcattc aaaagaaaaa ccattttgtt gtagtcaatc agtcattaaa tagacaaggc   2040
   atcaattatt ataattatga caaatttgat gatttagatg aaagtagaaa gtatatcttc   2100
   aataaaaccg ctgaatatca agaagaaatg gatattttaa aagatcttaa actcattcaa   2160
   aataaagatg ccaaaatcgc agtcagtatt ttctatccca atacattaaa cggcttagtg   2220
   aaaaaactaa acaatattat tgaatataat aaaaatatat tcgttattat tctacatgtt   2280
   gataagaatc atcttacacc agacatcaaa aaagaaatat tggctttcta tcataagcac   2340
   caagtgaata ttttactaaa taatgacatc tcatattaca cgagtaatag actaataaaa   2400
   actgaggcac atttaagtaa tattaataaa ttaagtcagt taaatctaaa ttgtgaatac   2460
   atcatttttg ataatcatga cagcctattc gttaaaaatg acagctatgc ttatatgaaa   2520
   aaatatgatg tcggcatgaa tttctcagca ttaacacatg attggatcga gaaaatcaat   2580
   gcgcatccac catttaaaaa gctgattaaa acctatttta atgacaatga cttaagaagt   2640
   atgaatgtga aaggggcatc acaaggtatg tttatgaagt atgcgctacc gcatgagctt   2700
   ctgacgatta ttaaagaagt catcacatcc tgccaatcaa ttgatagtgt gccagaatat   2760
   aacactgagg atatttggtt ccaatttgca cttttaatct tagaaaagaa aaccggccat   2820
   gtatttaata aaacatcgac cctgacttat atgccttggg aacgaaaatt acaatggaca   2880
   aatgaacaaa ttcaaagtgc aaaaaaaggc gaaaatatcc ccgttaacaa gttcattatt   2940
   aatagtataa cgctataaaa catttgcatt ttattaaaa                          2979
<210> 4
   <211> 965
   <212> PRT
   <213> Pasteurella multocida
<400> 4
   Met Asn Thr Leu Ser Gln Ala Ile Lys Ala Tyr Asn Ser Asn Asp Tyr
   1               5                   10                  15
   Glu Leu Ala Leu Lys Leu Phe Glu Lys Ser Ala Glu Thr Tyr Gly Arg
               20                  25                  30
   Lys Ile Val Glu Phe Gln Ile Ile Lys Cys Lys Glu Lys Leu Ser Thr
           35                  40                  45
   Asn Ser Tyr Val Ser Glu Asp Lys Lys Asn Ser Val Cys Asp Ser Ser
       50                  55                  60
   Leu Asp Ile Ala Thr Gln Leu Leu Leu Ser Asn Val Lys Lys Leu Thr
   65                  70                  75                  80
   Leu Ser Glu Ser Glu Lys Asn Ser Leu Lys Asn Lys Trp Lys Ser Ile
                   85                  90                  95
   Thr Gly Lys Lys Ser Glu Asn Ala Glu Ile Arg Lys Val Glu Leu Val
               100                 105                 110
   Pro Lys Asp Phe Pro Lys Asp Leu Val Leu Ala Pro Leu Pro Asp His
           115                 120                 125
   Val Asn Asp Phe Thr Trp Tyr Lys Asn Arg Lys Lys Ser Leu Gly Ile
       130                 135                 140
   Lys Pro Val Asn Lys Asn Ile Gly Leu Ser Ile Ile Ile Pro Thr Phe
   145                 150                 155                 160
   Asn Arg Ser Arg Ile Leu Asp Ile Thr Leu Ala Cys Leu Val Asn Gln
                   165                 170                 175
   Lys Thr Asn Tyr Pro Phe Glu Val Val Val Ala Asp Asp Gly Ser Lys
               180                 185                 190
   Glu Asn Leu Leu Thr Ile Val Gln Lys Tyr Glu Gln Lys Leu Asp Ile
           195                 200                 205
   Lys Tyr Val Arg Gln Lys Asp Tyr Gly Tyr Gln Leu Cys Ala Val Arg
       210                 215                 220
   Asn Leu Gly Leu Arg Thr Ala Lys Tyr Asp Phe Val Ser Ile Leu Asp
   225                 230                 235                 240
   Cys Asp Met Ala Pro Gln Gln Leu Trp Val His Ser Tyr Leu Thr Glu
                   245                 250                 255
   Leu Leu Glu Asp Asn Asp Ile Val Leu Ile Gly Pro Arg Lys Tyr Val
               260                 265                 270
   Asp Thr His Asn Ile Thr Ala Glu Gln Phe Leu Asn Asp Pro Tyr Leu
           275                 280                 285
   Ile Glu Ser Leu Pro Glu Thr Ala Thr Asn Asn Asn Pro Ser Ile Thr
       290                 295                 300
   Ser Lys Gly Asn Ile Ser Leu Asp Trp Arg Leu Glu His Phe Lys Lys
   305                 310                 315                 320
   Thr Asp Asn Leu Arg Leu Cys Asp Ser Pro Phe Arg Tyr Phe Ser Cys
                   325                 330                 335
   Gly Asn Val Ala Phe Ser Lys Glu Trp Leu Asn Lys Val Gly Trp Phe
               340                 345                 350
   Asp Glu Glu Phe Asn His Trp Gly Gly Glu Asp Val Glu Phe Gly Tyr
           355                 360                 365
   Arg Leu Phe Ala Lys Gly Cys Phe Phe Arg Val Ile Asp Gly Gly Met
       370                 375                 380
   Ala Tyr His Gln Glu Pro Pro Gly Lys Glu Asn Glu Thr Asp Arg Glu
   385                 390                 395                 400
   Ala Gly Lys Ser Ile Thr Leu Lys Ile Val Lys Glu Lys Val Pro Tyr
                   405                 410                 415
   Ile Tyr Arg Lys Leu Leu Pro Ile Glu Asp Ser His Ile His Arg Ile
               420                 425                 430
   Pro Leu Val Ser Ile Tyr Ile Pro Ala Tyr Asn Cys Ala Asn Tyr Ile
           435                 440                 445
   Gln Arg Cys Val Asp Ser Ala Leu Asn Gln Thr Val Val Asp Leu Glu
       450                 455                 460
   Val Cys Ile Cys Asn Asp Gly Ser Thr Asp Asn Thr Leu Glu Val Ile
   465                 470                 475                 480
   Asn Lys Leu Tyr Gly Asn Asn Pro Arg Val Arg Ile Met Ser Lys Pro
                   485                 490                 495
   Asn Gly Gly Ile Ala Ser Ala Ser Asn Ala Ala Val Ser Phe Ala Lys
               500                 505                 510
   Gly Tyr Tyr Ile Gly Gln Leu Asp Ser Asp Asp Tyr Leu Glu Pro Asp
           515                 520                 525
   Ala Val Glu Leu Cys Leu Lys Glu Phe Leu Lys Asp Lys Thr Leu Ala
       530                 535                 540
   Cys Val Tyr Thr Thr Asn Arg Asn Val Asn Pro Asp Gly Ser Leu Ile
   545                 550                 555                 560
   Ala Asn Gly Tyr Asn Trp Pro Glu Phe Ser Arg Glu Lys Leu Thr Thr
                   565                 570                 575
   Ala Met Ile Ala His His Phe Arg Met Phe Thr Ile Arg Ala Trp His
   580 585 590
   Leu Thr Asp Gly Phe Asn Glu Asn Ile Glu Asn Ala Val Asp Tyr Asp
   595 600 605
   Met Phe Leu Lys Leu Ser Glu Val Gly Lys Phe Lys His Leu Asn Lys
   610 615 620
   Ile Cys Tyr Asn Arg Val Leu His Gly Asp Asn Thr Ser Ile Lys Lys
   625 630 635 640
   Leu Gly Ile Gln Lys Lys Asn His Phe Val Val Val Asn Gln Ser Leu
   645 650 655
   Asn Arg Gln Gly Ile Asn Tyr Tyr Asn Tyr Asp Lys Phe Asp Asp Leu
   660 665 670
   Asp Glu Ser Arg Lys Tyr Ile Phe Asn Lys Thr Ala Glu Tyr Gln Glu
   675 680 685
   Glu Met Asp Ile Leu Lys Asp Leu Lys Leu Ile Gln Asn Lys Asp Ala
   690 695 700
   Lys Ile Ala Val Ser Ile Phe Tyr Pro Asn Thr Leu Asn Gly Leu Val
   705 710 715 720
   Lys Lys Leu Asn Asn Ile Ile Glu Tyr Asn Lys Asn Ile Phe Val Ile
   725 730 735
   Ile Leu His Val Asp Lys Asn His Leu Thr Pro Asp Ile Lys Lys Glu
   740 745 750
   Ile Leu Ala Phe Tyr His Lys His Gln Val Asn Ile Leu Leu Asn Asn
   755 760 765
   Asp Ile Ser Tyr Tyr Thr Ser Asn Arg Leu Ile Lys Thr Glu Ala His
   770 775 780
   Leu Ser Asn Ile Asn Lys Leu Ser Gln Leu Asn Leu Asn Cys Glu Tyr
   785 790 795 800
   Ile Ile Phe Asp Asn His Asp Ser Leu Phe Val Lys Asn Asp Ser Tyr
   805 810 815
   Ala Tyr Met Lys Lys Tyr Asp Val Gly Met Asn Phe Ser Ala Leu Thr
   820 825 830
   His Asp Trp Ile Glu Lys Ile Asn Ala His Pro Pro Phe Lys Lys Leu
   835 840 845
   Ile Lys Thr Tyr Phe Asn Asp Asn Asp Leu Arg Ser Met Asn Val Lys
   850 855 860
   Gly Ala Ser Gln Gly Met Phe Met Lys Tyr Ala Leu Pro His Glu Leu
   865 870 875 880
   Leu Thr Ile Ile Lys Glu Val Ile Thr Ser Cys Gln Ser Ile Asp Ser
   885 890 895
   Val Pro Glu Tyr Asn Thr Glu Asp Ile Trp Phe Gln Phe Ala Leu Leu
   900 905 910
   Ile Leu Glu Lys Lys Thr Gly His Val Phe Asn Lys Thr Ser Thr Leu
   915 920 925
   Thr Tyr Met Pro Trp Glu Arg Lys Leu Gln Trp Thr Asn Glu Gln Ile
   930 935 940
   Gln Ser Ala Lys Lys Gly Glu Asn Ile Pro Val Asn Lys Phe Ile Ile
   945 950 955 960
   Asn Ser Ile Thr Leu
   965
<210> 5
   <211> 1851
   <212> DNA
   <213> Pasteurella multocida
<400> 5
   atgagcttat ttaaacgtgc tactgagcta tttaagtcag gaaactataa agatgcacta 60
   actctatatg aaaatatagc taaaatttat ggttcagaaa gccttgttaa atataatatt 120
   gatatatgta aaaaaaatat aacacaatca aaaagtaata aaatagaaga agataatatt 180
   tctggagaaa acaaattttc agtatcaata aaagatctat ataacgaaat aagcaatagt 240
   gaattaggga ttacaaaaga aagactagga gccccccctc tagtcagtat tataatgact 300
   tctcataata cagaaaaatt cattgaagcc tcaattaatt cactattatt gcaaacatac 360
   aataacttag aagttatcgt tgtagatgat tatagcacag ataaaacatt tcagatcgca 420
   tccagaatag caaactctac aagtaaagta aaaacattcc gattaaactc aaatctaggg 480
   acatactttg cgaaaaatac aggaatttta aagtctaaag gagatattat tttctttcag 540
   gatagcgatg atgtatgtca ccatgaaaga atcgaaagat gtgttaatgc attattatcg 600
   aataaagata atatagctgt tagatgtgca tattctagaa taaatctaga aacacaaaat     660
   ataataaaag ttaatgataa taaatacaaa ttaggattaa taactttagg cgtttataga     720
   aaagtattta atgaaattgg tttttttaac tgcacaacca aagcatcgga tgatgaattt      780
   tatcatagaa taattaaata ctatggtaaa aataggataa ataacttatt tctaccactg     840
   tattataaca caatgcgtga agattcatta ttttctgata tggttgagtg ggtagatgaa     900
   aataatataa agcaaaaaac ctctgatgct agacaaaatt atctccatga attccaaaaa     960
   atacacaatg aaaggaaatt aaatgaatta aaagagattt ttagctttcc tagaattcat    1020
   gacgccttac ctatatcaaa agaaatgagt aagctcagca accctaaaat tcctgtttat    1080
   ataaatatat gctcaatacc ttcaagaata aaacaacttc aatacactat tggagtacta    1140
   aaaaaccaat gcgatcattt tcatatttat cttgatggat atccagaagt acctgatttt    1200
   ataaaaaaac tagggaataa agcgaccgtt attaattgtc aaaacaaaaa tgagtctatt    1260
   agagataatg gaaagtttat tctattagaa aaacttataa aggaaaataa agatggatat    1320
   tatataactt gtgatgatga tatccggtat cctgctgact acacaaacac tatgataaaa    1380
   aaaattaata aatacaatga taaagcagca attggattac atggtgttat attcccaagt    1440
   agagtcaaca agtatttttc atcagacaga attgtctata attttcaaaa acctttagaa    1500
   aatgatactg ctgtaaatat attaggaact ggaactgttg cctttagagt atctattttt    1560
   aataaatttt ctctatctga ttttgagcat cctggcatgg tagatatcta tttttctata    1620
   ctatgtaaga aaaacaatat actccaagtt tgtatatcac gaccatcgaa ttggctaaca    1680
   gaagataaca aaaacactga gaccttattt catgaattcc aaaatagaga tgaaatacaa    1740
   agtaaactca ttatttcaaa caacccttgg ggatactcaa gtatatatcc actattaaat    1800
   aataatgcta attattctga acttattccg tgtttatctt tttataacga g             1851
<210> 6
   <211> 615
   <212> PRT
   <213> Pasteurella multocida
<400> 6
   Met Ser Leu Phe Lys Arg Ala Thr Glu Leu Phe Lys Ser Gly Asn Tyr
   1               5                   10                  15
   Lys Asp Ala Leu Thr Leu Tyr Glu Asn Ile Ala Lys Ile Tyr Gly Ser
               20                  25                  30
   Glu Ser Leu Val Lys Tyr Asn Ile Asp Ile Cys Lys Lys Asn Ile Thr
           35                  40                  45
   Gln Ser Lys Ser Asn Lys Ile Glu Glu Asp Asn Ile Ser Gly Glu Asn
       50                  55                  60
   Lys Phe Ser Val Ser Ile Lys Asp Leu Tyr Asn Glu Ile Ser Asn Ser
   65                  70                  75                  80
   Glu Leu Gly Ile Thr Lys Glu Arg Leu Gly Ala Pro Pro Leu Val Ser
                   85                  90                  95
   Ile Ile Met Thr Ser His Asn Thr Glu Lys Phe Ile Glu Ala Ser Ile
               100                 105                 110
   Asn Ser Leu Leu Leu Gln Thr Tyr Asn Leu Glu Val Ile Val Val Asp
           115                 120                 125
   Asp Tyr Ser Thr Asp Lys Thr Phe Gln Ile Ala Ser Arg Ile Ala Asn
       130                 135                 140
   Ser Thr Ser Lys Val Lys Thr Phe Arg Leu Asn Ser Asn Leu Gly Thr
   145                 150                 155                 160
   Tyr Phe Ala Lys Asn Thr Gly Ile Leu Lys Ser Lys Gly Asp Ile Ile
                   165                 170                 175
   Phe Phe Gln Ser Asp Asp Val Cys His His Glu Arg Ile Glu Arg Cys
               180                 185                 190
   Val Asn Ala Leu Leu Ser Asn Lys Asp Asn Ile Ala Val Arg Cys Ala
           195                 200                 205
   Tyr Ser Arg Ile Asn Leu Glu Thr Gln Asn Ile Ile Lys Val Asn Asp
       210                 215                 220
   Asn Lys Tyr Lys Leu Gly Leu Ile Thr Leu Gly Val Tyr Arg Lys Val
   225                 230                 235                 240
   Phe Asn Glu Ile Gly Phe Phe Asn Cys Thr Thr Lys Ala Ser Asp Asp
                   245                 250                 255
   Glu Phe Tyr His Arg Ile Ile Lys Tyr Tyr Gly Lys Asn Arg Ile Asn
               260                 265                 270
   Asn Leu Phe Leu Pro Leu Tyr Tyr Asn Thr Met Arg Glu Asp Ser Leu
           275                 280                 285
   Phe Ser Asp Met Val Glu Trp Val Asp Glu Asn Asn Ile Lys Gln Lys
       290                 295                 300
   Thr Ser Asp Ala Arg Gln Asn Tyr Leu His Glu Phe Gln Lys Ile His
   305                 310                 315                 320
   Asn Glu Arg Lys Leu Asn Glu Leu Lys Glu Ile Phe Ser Phe Pro Arg
                   325                 330                 335
   Ile His Asp Ala Leu Pro Ile Ser Lys Glu Met Ser Lys Leu Ser Asn
               340                 345                 350
   Pro Lys Ile Pro Val Tyr Ile Asn Ile Cys Ser Ile Pro Ser Arg Ile
           355                 360                 365
   Lys Gln Leu Gln Tyr Thr Ile Gly Val Leu Lys Asn Gln Cys Asp His
       370                 375                 380
   Phe His Ile Tyr Leu Asp Gly Tyr Pro Glu Val Pro Asp Phe Ile Lys
   385                 390                 395                 400
   Lys Leu Gly Asn Lys Ala Thr Val Ile Asn Cys Gln Asn Lys Asn Glu
                   405                 410                 415
   Ser Ile Arg Asp Asn Gly Lys Phe Ile Leu Leu Glu Lys Leu Ile Lys
               420                 425                 430
   Glu Asn Lys Asp Gly Tyr Tyr Ile Thr Cys Asp Asp Asp Ile Arg Tyr
           435                 440                 445
   Pro Ala Asp Tyr Thr Asn Thr Met Ile Lys Lys Ile Asn Lys Tyr Asn
       450                 455                 460
   Asp Lys Ala Ala Ile Gly Leu His Gly Val Ile Phe Pro Ser Arg Val
   465                 470                 475                 480
   Asn Lys Tyr Phe Ser Ser Asp Arg Ile Val Tyr Asn Phe Gln Lys Pro
                   485                 490                 495
   Leu Glu Asn Asp Thr Ala Val Asn Ile Leu Gly Thr Gly Thr Val Ala
               500                 505                 510
   Phe Arg Val Ser Ile Phe Asn Lys Phe Ser Leu Ser Asp Phe Glu His
           515                 520                 525
   Pro Gly Met Val Asp Ile Tyr Phe Ser Ile Leu Cys Lys Lys Asn Asn
       530                 535                 540
   Ile Leu Gln Val Cys Ile Ser Arg Pro Ser Asn Trp Leu Thr Glu Asp
   545                 550                 555                 560
   Asn Lys Asn Thr Glu Thr Leu Phe His Glu Phe Gln Asn Arg Asp Glu
                   565                 570                 575
   Ile Gln Ser Lys Leu Ile Ile Ser Asn Asn Pro Trp Gly Tyr Ser Ser
               580                 585                 590
   Ile Tyr Pro Leu Leu Asn Asn Asn Ala Asn Tyr Ser Glu Leu Ile Pro
           595                 600                 605
   Cys Leu Ser Phe Tyr Asn Glu
       610                 615
<210> 7
   <211> 1940
   <212> DNA
   <213> Pasteurella multocida
<400> 7
   aacaggggat aaggtcagta aatttaggat gatttttgac taatggataa atacttgaat      60
   atccccatgg accgttttcc atgatcagct gagtttgttg ctcatcattg tctcgatatt     120
   gatgatagag tgtttcgctg tctctattat cttccgttag ccagtttgct ggtcttgaaa     180
   tacaaatctg aagaatatta tttttcttac acaagagaga gaaatagata tcagccatgc     240
   ctgaatgggt aaagtcagaa agagaaaatt gattaaagag actgactcta aagctaacag     300
   ttcctgtacc taatacattg accgctttgt ctttttccag aggtttatag aagctatata     360
   ccagtctatc cgccgaaaaa tatttggtca ttctacttgg aaagagaatg ccgtgtaaac     420
   caataaccgc tttatcatcg tattcattca gcttcttgat catcgtattg atgtaatcgc     480
   ttggatagat aatgtcatca tcacaggtta tataatatcc atcttgattt ttttcaatca     540
   actcttccag taaaatgaat ttgccattat ctctaatgga gttatcttta tctttgcaat     600
   gaacaacggt tgctttatta cctaaatttt ttatgaagtc agggatttct acatagccat     660
   caagataaat atgaaaatga tcacattgat tttttagtat gccgataata cgtcgtaatt     720
   gcgctattct tgagggaata gaacaaatat tgatataaac aggaatctta ggattggaca     780
   acttactcat ttcttgtggt actggtaagg catcgtaaat acgagggaat tgaaaaagat     840
   ttttgaaatc atgtgaggca gtttcgttat gcatcgcttg aaacagggtt gcataatgtt     900
   gtctggtatc agacattttc tgtattatgt tatgattgtc tatccattca accatatcag     960
   taaataaaga gttttctctc attgtgttgt agtataacgg caagagtaaa ttttttattt   1020
   tttcttttcc ataatatttc gcaattctat gaaaaaactc atcatctgag cctttagtcg    1080
   tacaattgaa gaaaccaatt tcttgaaata cttttctgtg catacccaag gttataaaac    1140
   ctaatctata atccatatta ttgactttaa tgatatgttg tgtttctggt gctagtcttg    1200
   agtatgcaca acgaacagca atagtttctt tattagctaa taatatattt acacatcttt   1260
   ctattctttc atgatgacat acatcatcac tatcttgaaa gaaaataatg tcacctttag   1320
   attttaatat gcctgtattt ttcgcaaagt aagttcctag gtttgaattt aatctaaata   1380
   ctctgacttt gcttgttgta ttcgctattc tcgaggcaat ttcaaatgta ttatccgagc   1440
   tatcatcatc tacaataata atttctatgt ttttatatgt ttgtaacaat aatgaattaa   1500
   tagaagcttc gataaattgc gctgtattgt gagatgtcat gataatactg actaatggat   1560
   ttacgctgtt ggtttctttg actaacccta aatcactttt agcgacttca ttatataaat   1620
   ctgttattga tgttgtttgc ttatcttttt ctagctttgc ttctaatgct tgattatagg   1680
   tatatatttt ttcaaattct tgcagaacca attggagttg ttttaataaa agtttatttt   1740
   cgttttcaag ggatgcggat agcggatgtt tactgtcctg ttttgccaat aaagtttgtt   1800
   gagaaataat gtctttgttt aaagttgttt ttagactatc aattttattt tgaaaggtgt   1860
   tgagttcatt ttctttttca tgttgggggg gatttttagt catttgtttt tgagtcatct   1920
   ctttttttct cttcatttca                                              1940
<210> 8
   <211> 651
   <212> PRT
   <213> Pasteurella multocida
<400> 8
   Met Lys Arg Lys Lys Glu Met Thr Gln Lys Gln Met Thr Lys Asn Pro
   1               5                   10                  15
   Pro Gln His Glu Lys Glu Asn Glu Leu Asn Thr Phe Gln Asn Lys Ile
               20                  25                  30
   Asp Ser Leu Lys Thr Thr Leu Asn Lys Asp Ile Ile Ser Gln Gln Thr
           35                  40                  45
   Leu Leu Ala Lys Gln Asp Ser Lys His Pro Leu Ser Ala Ser Leu Glu
       50                  55                  60
   Asn Glu Asn Lys Leu Leu Leu Lys Gln Leu Gln Leu Val Leu Gln Glu
   65                  70                  75                  80
   Phe Glu Lys Ile Tyr Thr Tyr Asn Gln Ala Leu Glu Ala Lys Leu Glu
                   85                  90                  95
   Lys Asp Lys Gln Thr Thr Ser Ile Thr Asp Leu Tyr Asn Glu Val Ala
               100                 105                 110
   Lys Ser Asp Leu Gly Leu Val Lys Glu Thr Asn Ser Val Asn Pro Leu
           115                 120                 125
   Val Ser Ile Ile Met Thr Ser His Asn Thr Ala Gln Phe Ile Glu Ala
       130                 135                 140
   Ser Ile Asn Ser Leu Leu Leu Gln Thr Tyr Lys Asn Ile Glu Ile Ile
   145                 150                 155                 160
   Ile Val Asp Asp Asp Ser Ser Asp Asn Thr Phe Glu Ile Ala Ser Arg
                   165                 170                 175
   Ile Ala Asn Thr Thr Ser Lys Val Arg Val Phe Arg Leu Asn Ser Asn
               180                 185                 190
   Leu Gly Thr Tyr Phe Ala Lys Asn Thr Gly Ile Leu Lys Ser Lys Gly
           195                 200                 205
   Asp Ile Ile Phe Phe Gln Asp Ser Asp Asp Val Cys His His Glu Arg
       210                 215                 220
   Ile Glu Arg Cys Val Asn Ile Leu Leu Ala Asn Lys Glu Thr Ile Ala
   225                 230                 235                 240
   Val Arg Cys Ala Tyr Ser Arg Leu Ala Pro Glu Thr Gln His Ile Ile
                   245                 250                 255
   Lys Val Asn Asn Met Asp Tyr Arg Leu Gly Phe Ile Thr Leu Gly Met
               260                 265                 270
   His Arg Lys Val Phe Gln Glu Ile Gly Phe Phe Asn Cys Thr Thr Lys
           275                 280                 285
   Gly Ser Asp Asp Glu Phe Phe His Arg Ile Ala Lys Tyr Tyr Gly Lys
       290                 295                 300
   Glu Lys Ile Lys Asn Leu Leu Leu Pro Leu Tyr Tyr Asn Thr Met Arg
   305                 310                 315                 320
   Glu Asn Ser Leu Phe Thr Asp Met Val Glu Trp Ile Asp Asn His Asn
                   325                 330                 335
   Ile Ile Gln Lys Met Ser Asp Thr Arg Gln His Tyr Ala Thr Leu Phe
               340                 345                 350
   Gln Ala Met His Asn Glu Thr Ala Ser His Asp Phe Lys Asn Leu Phe
           355                 360                 365
   Gln Phe Pro Arg Ile Tyr Asp Ala Leu Pro Val Pro Gln Glu Met Ser
       370                 375                 380
   Lys Leu Ser Asn Pro Lys Ile Pro Val Tyr Ile Asn Ile Cys Ser Ile
   385                 390                 395                 400
   Pro Ser Arg Ile Ala Gln Leu Arg Arg Ile Ile Gly Ile Leu Lys Asn
                   405                 410                 415
   Gln Cys Asp His Phe His Ile Tyr Leu Asp Gly Tyr Val Glu Ile Pro
               420                 425                 430
   Asp Phe Ile Lys Asn Leu Gly Asn Lys Ala Thr Val Val His Cys Lys
           435                 440                 445
   Asp Lys Asp Asn Ser Ile Arg Asp Asn Gly Lys Phe Ile Leu Leu Glu
       450                 455                 460
   Glu Leu Ile Glu Lys Asn Gln Asp Gly Tyr Tyr Ile Thr Cys Asp Asp
   465                 470                 475                 480
   Asp Ile Ile Tyr Pro Ser Asp Tyr Ile Asn Thr Met Ile Lys Lys Leu
                   485                 490                 495
   Asn Glu Tyr Asp Asp Lys Ala Val Ile Gly Leu His Gly Ile Leu Phe
               500                 505                 510
   Pro Ser Arg Met Thr Lys Tyr Phe Ser Ala Asp Arg Leu Val Tyr Ser
           515                 520                 525
   Phe Tyr Lys Pro Leu Glu Lys Asp Lys Ala Val Asn Val Leu Gly Thr
       530                 535                 540
   Gly Thr Val Ser Phe Arg Val Ser Leu Phe Asn Gln Phe Ser Leu Ser
   545                 550                 555                 560
   Asp Phe Thr His Ser Gly Met Ala Asp Ile Tyr Phe Ser Leu Leu Cys
                   565                 570                 575
   Lys Lys Asn Asn Ile Leu Gln Ile Cys Ile Ser Arg Pro Ala Asn Trp
               580                 585                 590
   Leu Thr Glu Asp Asn Arg Asp Ser Glu Thr Leu Tyr His Gln Tyr Arg
           595                 600                 605
   Asp Asn Asp Glu Gln Gln Thr Gln Leu Ile Met Glu Asn Gly Pro Trp
       610                 615                 620
   Gly Tyr Ser Ser Ile Tyr Pro Leu Val Lys Asn His Pro Lys Phe Thr
   625 630 635 640
   Asp Leu Ile Pro Cys Leu Pro Phe Tyr Phe Leu
   645 650
<210> 9
   <211> 703
   <212> PRT
   <213> Pasteurella multocida
<400> 9
   Met Asn Thr Leu Ser Gln Ala Ile Lys Ala Tyr Asn Ser Asn Asp Tyr
   1 5 10 15
   Gln Leu Ala Leu Lys Leu Phe Glu Lys Ser Ala Glu Ile Tyr Gly Arg
   20 25 30
   Lys Ile Val Glu Phe Gln Ile Thr Lys Cys Lys Glu Lys Leu Ser Ala
   35 40 45
   His Pro Ser Val Asn Ser Ala His Leu Ser Val Asn Lys Glu Glu Lys
   50 55 60
   Val Asn Val Cys Asp Ser Pro Leu Asp Ile Ala Thr Gln Leu Leu Leu
   65 70 75 80
   Ser Asn Val Lys Lys Leu Val Leu Ser Asp Ser Glu Lys Asn Thr Leu
   85 90 95
   Lys Asn Lys Trp Lys Leu Leu Thr Glu Lys Lys Ser Glu Asn Ala Glu
   100 105 110
   Val Arg Ala Val Ala Leu Val Pro Lys Asp Phe Pro Lys Asp Leu Val
   115 120 125
   Leu Ala Pro Leu Pro Asp His Val Asn Asp Phe Thr Trp Tyr Lys Lys
   130 135 140
   Arg Lys Lys Arg Leu Gly Ile Lys Pro Glu His Gln His Val Gly Leu
   145 150 155 160
   Ser Ile Ile Val Thr Thr Phe Asn Arg Pro Ala Ile Leu Ser Ile Thr
   165 170 175
   Leu Ala Cys Leu Val Asn Gln Lys Thr His Tyr Pro Phe Glu Val Ile
   180 185 190
   Val Thr Asp Asp Gly Ser Gln Glu Asp Leu Ser Pro Ile Ile Arg Gln
   195 200 205
   Tyr Glu Asn Lys Leu Asp Ile Arg Tyr Val Arg Gln Lys Asp Asn Gly
   210 215 220
   Phe Gln Ala Ser Ala Ala Arg Asn Met Gly Leu Arg Leu Ala Lys Tyr
   225 230 235 240
   Asp Phe Ile Gly Leu Leu Asp Cys Asp Met Ala Pro Asn Pro Leu Trp
   245 250 255
   Val His Ser Tyr Val Ala Glu Leu Leu Glu Asp Asp Asp Leu Thr Ile
   260 265 270
   Ile Gly Pro Arg Lys Tyr Ile Asp Thr Gln His Ile Asp Pro Lys Asp
   275 280 285
   Phe Leu Asn Asn Ala Ser Leu Leu Glu Ser Leu Pro Glu Val Lys Thr
   290 295 300
   Asn Asn Ser Val Ala Ala Lys Gly Glu Gly Thr Val Ser Leu Asp Trp
   305 310 315 320
   Arg Leu Glu Gln Phe Glu Lys Thr Glu Asn Leu Arg Leu Ser Asp Ser
   325 330 335
   Pro Phe Arg Phe Phe Ala Ala Gly Asn Val Ala Phe Ala Lys Lys Trp
   340 345 350
   Leu Asn Lys Ser Gly Phe Phe Asp Glu Glu Phe Asn His Trp Gly Gly
   355 360 365
   Glu Asp Val Glu Phe Gly Tyr Arg Leu Phe Arg Tyr Gly Ser Phe Phe
   370 375 380
   Lys Thr Ile Asp Gly Ile Met Ala Tyr His Gln Glu Pro Pro Gly Lys
   385 390 395 400
   Glu Asn Glu Thr Asp Arg Glu Ala Gly Lys Asn Ile Thr Leu Asp Ile
   405 410 415
   Met Arg Glu Lys Val Pro Tyr Ile Tyr Arg Lys Leu Leu Pro Ile Glu
   420 425 430
   Asp Ser His Ile Asn Arg Val Pro Leu Val Ser Ile Tyr Ile Pro Ala
           435                 440                 445
   Tyr Asn Cys Ala Asn Tyr Ile Gln Arg Cys Val Asp Ser Ala Leu Asn
       450                 455                 460
   Gln Thr Val Val Asp Leu Glu Val Cys Ile Cys Asn Asp Gly Ser Thr
   465                 470                 475                 480
   Asp Asn Thr Leu Glu Val Ile Asn Lys Leu Tyr Gly Asn Asn Pro Arg
                   485                 490                 495
   Val Arg Ile Met Ser Lys Pro Asn Gly Gly Ile Ala Ser Ala Ser Asn
               500                 505                 510
   Ala Ala Val Ser Phe Ala Lys Gly Tyr Tyr Ile Gly Gln Leu Asp Ser
           515                 520                 525
   Asp Asp Tyr Leu Glu Pro Asp Ala Val Glu Leu Cys Leu Lys Glu Phe
       530                 535                 540
   Leu Lys Asp Lys Thr Leu Ala Cys Val Tyr Thr Thr Asn Arg Asn Val
   545                 550                 555                 560
   Asn Pro Asp Gly Ser Leu Ile Ala Asn Gly Tyr Asn Trp Pro Glu Phe
                   565                 570                 575
   Ser Arg Glu Lys Leu Thr Thr Ala Met Ile Ala His His Phe Arg Met
               580                 585                 590
   Phe Thr Ile Arg Ala Trp His Leu Thr Asp Gly Phe Asn Glu Lys Ile
           595                 600                 605
   Glu Asn Ala Val Asp Tyr Asp Met Phe Leu Lys Leu Ser Glu Val Gly
       610                 615                 620
   Lys Phe Lys His Leu Asn Lys Ile Cys Tyr Asn Arg Val Leu His Gly
   625                 630                 635                 640
   Asp Asn Thr Ser Ile Lys Lys Leu Gly Ile Gln Lys Lys Asn His Phe
                   645                 650                 655
   Val Val Val Asn Gln Ser Leu Asn Arg Gln Gly Ile Thr Tyr Tyr Asn
               660                 665                 670
   Tyr Asp Glu Phe Asp Asp Leu Asp Glu Ser Arg Lys Tyr Ile Phe Asn
           675                 680                 685
   Lys Thr Ala Glu Tyr Gln Glu Glu Ile Asp Ile Leu Lys Asp Ile
   690 695 700
<210> 10
   <211> 1953
   <212> DNA
   <213> Pasteurella multocida
<400> 10
   atgaatacat tatcacaagc aataaaagca tataacagca atgactatca attagcactc 60
   aaattatttg aaaagtcggc ggaaatctat ggacggaaaa ttgttgaatt tcaaattacc 120
   aaatgcaaag aaaaactctc agcacatcct tctgttaatt cagcacatct ttctgtaaat 180
   aaagaagaaa aagtcaatgt ttgcgatagt ccgttagata ttgcaacaca actgttactt 240
   tccaacgtaa aaaaattagt actttctgac tcggaaaaaa acacgttaaa aaataaatgg 300
   aaattgctca ctgagaagaa atctgaaaat gcggaggtaa gagcggtcgc ccttgtacca 360
   aaagattttc ccaaagatct ggttttagcg cctttacctg atcatgttaa tgattttaca 420
   tggtacaaaa agcgaaagaa aagacttggc ataaaacctg aacatcaaca tgttggtctt 480
   tctattatcg ttacaacatt caatcgacca gcaattttat cgattacatt agcctgttta 540
   gtaaaccaaa aaacacatta cccgtttgaa gttatcgtga cagatgatgg tagtcaggaa 600
   gatctatcac cgatcattcg ccaatatgaa aataaattgg atattcgcta cgtcagacaa 660
   aaagataacg gttttcaagc cagtgccgct cggaatatgg gattacgctt agcaaaatat 720
   gactttattg gcttactcga ctgtgatatg gcgccaaatc cattatgggt tcattcttat 780
   gttgcagagc tattagaaga tgatgattta acaatcattg gtccaagaaa atacatcgat 840
   acacaacata ttgacccaaa agacttctta aataacgcga gtttgcttga atcattacca 900
   gaagtgaaaa ccaataatag tgttgccgca aaaggggaag gaacagtttc tctggattgg 960
   cgcttagaac aattcgaaaa aacagaaaat ctccgcttat ccgattcgcc tttccgtttt 1020
   tttgcggcgg gtaatgttgc tttcgctaaa aaatggctaa ataaatccgg tttctttgat 1080
   gaggaattta atcactgggg tggagaagat gtggaatttg gatatcgctt attccgttac 1140
   ggtagtttct ttaaaactat tgatggcatt atggcctacc atcaagagcc accaggtaaa 1200
   gaaaatgaaa ccgatcgtga agcgggaaaa aatattacgc tcgatattat gagagaaaag 1260
   gtcccttata tctatagaaa acttttacca atagaagatt cgcatatcaa tagagtacct 1320
   ttagtttcaa tttatatccc agcttataac tgtgcaaact atattcaacg ttgcgtagat 1380
   agtgcactga atcagactgt tgttgatctc gaggtttgta tttgtaacga tggttcaaca 1440
   gataatacct tagaagtgat caataagctt tatggtaata atcctagggt acgcatcatg 1500
   tctaaaccaa atggcggaat agcctcagca tcaaatgcag ccgtttcttt tgctaaaggt 1560
   tattacattg ggcagttaga ttcagatgat tatcttgagc ctgatgcagt tgaactgtgt 1620
   ttaaaagaat ttttaaaaga taaaacgcta gcttgtgttt ataccactaa tagaaacgtc   1680
   aatccggatg gtagcttaat cgctaatggt tacaattggc cagaattttc acgagaaaaa   1740
   ctcacaacgg ctatgattgc tcaccacttt agaatgttca cgattagagc ttggcattta   1800
   actgatggat tcaatgaaaa aattgaaaat gccgtagact atgacatgtt cctcaaactc   1860
   agtgaagttg gaaaatttaa acatcttaat aaaatctgct ataaccgtgt attacatggt   1920
   gataacacat caattaagaa acttggcatt caa                                1953
<210> 11
   <211> 2112
   <212> DNA
   <213> Pasteurella multocida
<400> 11
   atgaatacat tatcacaagc aataaaagca tataacagca atgactatca attagcactc      60
   aaattatttg aaaagtcggc ggaaatctat ggacggaaaa ttgttgaatt tcaaattacc     120
   aaatgcaaag aaaaactctc agcacatcct tctgttaatt cagcacatct ttctgtaaat     180
   aaagaagaaa aagtcaatgt ttgcgatagt ccgttagata ttgcaacaca actgttactt     240
   tccaacgtaa aaaaattagt actttctgac tcggaaaaaa acacgttaaa aaataaatgg     300
   aaattgctca ctgagaagaa atctgaaaat gcggaggtaa gagcggtcgc ccttgtacca     360
   aaagattttc ccaaagatct ggttttagcg cctttacctg atcatgttaa tgattttaca     420
   tggtacaaaa agcgaaagaa aagacttggc ataaaacctg aacatcaaca tgttggtctt     480
   tctattatcg ttacaacatt caatcgacca gcaattttat cgattacatt agcctgttta     540
   gtaaaccaaa aaacacatta cccgtttgaa gttatcgtga cagatgatgg tagtcaggaa     600
   gatctatcac cgatcattcg ccaatatgaa aataaattgg atattcgcta cgtcagacaa     660
   aaagataacg gttttcaagc cagtgccgct cggaatatgg gattacgctt agcaaaatat     720
   gactttattg gcttactcga ctgtgatatg gcgccaaatc cattatgggt tcattcttat     780
   gttgcagagc tattagaaga tgatgattta acaatcattg gtccaagaaa atacatcgat     840
   acacaacata ttgacccaaa agacttctta aataacgcga gtttgcttga atcattacca     900
   gaagtgaaaa ccaataatag tgttgccgca aaaggggaag gaacagtttc tctggattgg     960
   cgcttagaac aattcgaaaa aacagaaaat ctccgcttat ccgattcgcc tttccgtttt    1020
   tttgcggcgg gtaatgttgc tttcgctaaa aaatggctaa ataaatccgg tttctttgat    1080
   gaggaattta atcactgggg tggagaagat gtggaatttg gatatcgctt attccgttac    1140
   ggtagtttct ttaaaactat tgatggcatt atggcctacc atcaagagcc accaggtaaa    1200
   gaaaatgaaa ccgatcgtga agcgggaaaa aatattacgc tcgatattat gagagaaaag    1260
   gtcccttata tctatagaaa acttttacca atagaagatt cgcatatcaa tagagtacct    1320
   ttagtttcaa tttatatccc agcttataac tgtgcaaact atattcaacg ttgcgtagat   1380
   agtgcactga atcagactgt tgttgatctc gaggtttgta tttgtaacaa tggttcaaca   1440
   gataatacct tagaagtgat caataagctt tatggtaata atcctagggt acgcatcatg   1500
   tctaaaccaa atggcggaat agcctcagca tcaaatgcag ccgtttcttt tgctaaaggt   1560
   tattacattg ggcagttaga ttcagatgat tatcttgagc ctgatgcagt tgaactgtgt   1620
   ttaaaagaat ttttaaaaga taaaacgcta gcttgtgttt ataccactaa tagaaacgtc   1680
   aatccggatg gtagcttaat cgctaatggt tacaattggc cagaattttc acgagaaaaa   1740
   ctcacaacgg ctatgattgc tcaccacttt agaatgttca cgattagagc ttggcattta   1800
   actgatggat tcaatgaaaa aattgaaaat gccgtagact atgacatgtt cctcaaactc   1860
   agtgaagttg gaaaatttaa acatcttaat aaaatctgct ataaccgtgt attacatggt   1920
   gataacacat caattaagaa acttggcatt caaaagaaaa accattttgt tgtagtcaat   1980
   cagtcattaa atagacaagg cataacttat tataattatg acgaatttga tgatttagat   2040
   gaaagtagaa agtatatttt caataaaacc gctgaatatc aagaagagat tgatatctta   2100
   aaagatattt aa                                                       2112
<210> 12
   <211> 2112
   <212> DNA
   <213> Pasteurella multocida
<400> 12
   atgaatacat tatcacaagc aataaaagca tataacagca atgactatca attagcactc      60
   aaattatttg aaaagtcggc ggaaatctat ggacggaaaa ttgttgaatt tcaaattacc     120
   aaatgcaaag aaaaactctc agcacatcct tctgttaatt cagcacatct ttctgtaaat     180
   aaagaagaaa aagtcaatgt ttgcgatagt ccgttagata ttgcaacaca actgttactt     240
   tccaacgtaa aaaaattagt actttctgac tcggaaaaaa acacgttaaa aaataaatgg     300
   aaattgctca ctgagaagaa atctgaaaat gcggaggtaa gagcggtcgc ccttgtacca     360
   aaagattttc ccaaagatct ggttttagcg cctttacctg atcatgttaa tgattttaca     420
   tggtacaaaa agcgaaagaa aagacttggc ataaaacctg aacatcaaca tgttggtctt     480
   tctattatcg ttacaacatt caatcgacca gcaattttat cgattacatt agcctgttta     540
   gtaaaccaaa aaacacatta cccgtttgaa gttatcgtga cagataatgg tagtcaggaa     600
   gatctatcac cgatcattcg ccaatatgaa aataaattgg atattcgcta cgtcagacaa     660
   aaagataacg gttttcaagc cagtgccgct cggaatatgg gattacgctt agcaaaatat     720
   gactttattg gcttactcga ctgtgatatg gcgccaaatc cattatgggt tcattcttat     780
   gttgcagagc tattagaaga tgatgattta acaatcattg gtccaagaaa atacatcgat     840
   acacaacata ttgacccaaa agacttctta aataacgcga gtttgcttga atcattacca 900
   gaagtgaaaa ccaataatag tgttgccgca aaaggggaag gaacagtttc tctggattgg 960
   cgcttagaac aattcgaaaa aacagaaaat ctccgcttat ccgattcgcc tttccgtttt 1020
   tttgcggcgg gtaatgttgc tttcgctaaa aaatggctaa ataaatccgg tttctttgat 1080
   gaggaattta atcactgggg tggagaagat gtggaatttg gatatcgctt attccgttac 1140
   ggtagtttct ttaaaactat tgatggcatt atggcctacc atcaagagcc accaggtaaa 1200
   gaaaatgaaa ccgatcgtga agcgggaaaa aatattacgc tcgatattat gagagaaaag 1260
   gtcccttata tctatagaaa acttttacca atagaagatt cgcatatcaa tagagtacct 1320
   ttagtttcaa tttatatccc agcttataac tgtgcaaact atattcaacg ttgcgtagat 1380
   agtgcactga atcagactgt tgttgatctc gaggtttgta tttgtaacga tggttcaaca 1440
   gataatacct tagaagtgat caataagctt tatggtaata atcctagggt acgcatcatg 1500
   tctaaaccaa atggcggaat agcctcagca tcaaatgcag ccgtttcttt tgctaaaggt 1560
   tattacattg ggcagttaga ttcagatgat tatcttgagc ctgatgcagt tgaactgtgt 1620
   ttaaaagaat ttttaaaaga taaaacgcta gcttgtgttt ataccactaa tagaaacgtc 1680
   aatccggatg gtagcttaat cgctaatggt tacaattggc cagaattttc acgagaaaaa 1740
   ctcacaacgg ctatgattgc tcaccacttt agaatgttca cgattagagc ttggcattta 1800
   actgatggat tcaatgaaaa aattgaaaat gccgtagact atgacatgtt cctcaaactc 1860
   agtgaagttg gaaaatttaa acatcttaat aaaatctgct ataaccgtgt attacatggt 1920
   gataacacat caattaagaa acttggcatt caaaagaaaa accattttgt tgtagtcaat 1980
   cagtcattaa atagacaagg cataacttat tataattatg acgaatttga tgatttagat 2040
   gaaagtagaa agtatatttt caataaaacc gctgaatatc aagaagagat tgatatctta 2100
   aaagatattt aa 2112
<210> 13
   <211> 1614
   <212> DNA
   <213> Pasteurella multocida
<400> 13
   atcaatagag tacctttagt ttcaatttat atcccagctt ataactgtgc aaactatatt 60
   caacgttgcg tagatagtgc actgaatcag actgttgttg atctcgaggt ttgtatttgt 120
   aacgatggtt caacagataa taccttagaa gtgatcaata agctttatgg taataatcct 180
   agggtacgca tcatgtctaa accaaatggc ggaatagcct cagcatcaaa tgcagccgtt 240
   tcttttgcta aaggttatta cattgggcag ttagattcag atgattatct tgagcctgat 300
   gcagttgaac tgtgtttaaa agaattttta aaagataaaa cgctagcttg tgtttatacc 360
   actaatagaa acgtcaatcc ggatggtagc ttaatcgcta atggttacaa ttggccagaa 420
   ttttcacgag aaaaactcac aacggctatg attgctcacc actttagaat gttcacgatt 480
   agagcttggc atttaactga tggattcaat gaaaaaattg aaaatgccgt agactatgac 540
   atgttcctca aactcagtga agttggaaaa tttaaacatc ttaataaaat ctgctataac 600
   cgtgtattac atggtgataa cacatcaatt aagaaacttg gcattcaaaa gaaaaaccat 660
   tttgttgtag tcaatcagtc attaaataga caaggcataa cttattataa ttatgacgaa 720
   tttgatgatt tagatgaaag tagaaagtat attttcaata aaaccgctga atatcaagaa 780
   gagattgata tcttaaaaga tattaaaatc atccagaata aagatgccaa aatcgcagtc 840
   agtatttttt atcccaatac attaaacggc ttagtgaaaa aactaaacaa tattattgaa 900
   tataataaaa atatattcgt tattgttcta catgttgata agaatcatct tacaccagat 960
   atcaaaaaag aaatactagc cttctatcat aaacatcaag tgaatatttt actaaataat 1020
   gatatctcat attacacgag taatagatta ataaaaactg aggcgcattt aagtaatatt 1080
   aataaattaa gtcagttaaa tctaaattgt gaatacatca tttttgataa tcatgacagc 1140
   ctattcgtta aaaatgacag ctatgcttat atgaaaaaat atgatgtcgg catgaatttc 1200
   tcagcattaa cacatgattg gatcgagaaa atcaatgcgc atccaccatt taaaaagctc 1260
   attaaaactt attttaatga caatgactta aaaagtatga atgtgaaagg ggcatcacaa 1320
   ggtatgttta tgacgtatgc gctagcgcat gagcttctga cgattattaa agaagtcatc 1380
   acatcttgcc agtcaattga tagtgtgcca gaatataaca ctgaggatat ttggttccaa 1440
   tttgcacttt taatcttaga aaagaaaacc ggccatgtat ttaataaaac atcgaccctg 1500
   acttatatgc cttgggaacg aaaattacaa tggacaaatg aacaaattga aagtgcaaaa 1560
   agaggagaaa atatacctgt taacaagttc attattaata gtataactct ataa 1614
<210> 14
   <211> 966
   <212> DNA
   <213> Pasteurella multocida
<400> 14
   atcaatagag tacctttagt ttcaatttat atcccagctt ataactgtgc aaactatatt     60
   caacgttgcg tagatagtgc actgaatcag actgttgttg atctcgaggt ttgtatttgt    120
   aacgatggtt caacagataa taccttagaa gtgatcaata agctttatgg taataatcct    180
   agggtacgca tcatgtctaa accaaatggc ggaatagcct cagcatcaaa tgcagccgtt    240
   tcttttgcta aaggttatta cattgggcag ttagattcag atgattatct tgagcctgat    300
   gcagttgaac tgtgtttaaa agaattttta aaagataaaa cgctagcttg tgtttatacc    360
   actaatagaa acgtcaatcc ggatggtagc ttaatcgcta atggttacaa ttggccagaa    420
   ttttcacgag aaaaactcac aacggctatg attgctcacc actttagaat gttcacgatt    480
   agagcttggc atttaactga tggattcaat gaaaaaattg aaaatgccgt agactatgac    540
   atgttcctca aactcagtga agttggaaaa tttaaacatc ttaataaaat ctgctataac    600
   cgtgtattac atggtgataa cacatcaatt aagaaacttg gcattcaaaa gaaaaaccat    660
   tttgttgtag tcaatcagtc attaaataga caaggcataa cttattataa ttatgacgaa    720
   tttgatgatt tagatgaaag tagaaagtat attttcaata aaaccgctga atatcaagaa    780
   gagattgata tcttaaaaga tattaaaatc atccagaata aagatgccaa aatcgcagtc    840
   agtatttttt atcccaatac attaaacggc ttagtgaaaa aactaaacaa tattattgaa    900
   tataataaaa atatattcgt tattgttcta catgttgata agaatcatct tacaccagat    960
   atctaa                                                               966
<210> 15
   <211> 1821
   <212> DNA
   <213> Pasteurella multocida
<400> 15
   atgaaacctg aacatcaaca tgttggtctt tctattatcg ttacaacatt caatcgacca 60
   gcaattttat cgattacatt agcctgttta gtaaaccaaa aaacacatta cccgtttgaa 120
   gttatcgtga cagatgatgg tagtcaggaa gatctatcac cgatcattcg ccaatatgaa 180
   aataaattgg atattcgcta cgtcagacaa aaagataacg gttttcaagc cagtgccgct 240
   cggaatatgg gattacgctt agcaaaatat gactttattg gcttactcga ctgtgatatg 300
   gcgccaaatc cattatgggt tcattcttat gttgcagagc tattagaaga tgatgattta 360
   acaatcattg gtccaagaaa atacatcgat acacaacata ttgacccaaa agacttctta 420
   aataacgcga gtttgcttga atcattacca gaagtgaaaa ccaataatag tgttgccgca 480
   aaaggggaag gaacagtttc tctggattgg cgcttagaac aattcgaaaa aacagaaaat 540
   ctccgcttat ccgattcgcc tttccgtttt tttgcggcgg gtaatgttgc tttcgctaaa 600
   aaatggctaa ataaatccgg tttctttgat gaggaattta atcactgggg tggagaagat 660
   gtggaatttg gatatcgctt attccgttac ggtagtttct ttaaaactat tgatggcatt 720
   atggcctacc atcaagagcc accaggtaaa gaaaatgaaa ccgatcgtga agcgggaaaa 780
   aatattacgc tcgatattat gagagaaaag gtcccttata tctatagaaa acttttacca 840
   atagaagatt cgcatatcaa tagagtacct ttagtttcaa tttatatccc agcttataac 900
   tgtgcaaact atattcaacg ttgcgtagat agtgcactga atcagactgt tgttgatctc 960
   gaggtttgta tttgtaacga tggttcaaca gataatacct tagaagtgat caataagctt 1020
   tatggtaata atcctagggt acgcatcatg tctaaaccaa atggcggaat agcctcagca 1080
   tcaaatgcag ccgtttcttt tgctaaaggt tattacattg ggcagttaga ttcagatgat 1140
   tatcttgagc ctgatgcagt tgaactgtgt ttaaaagaat ttttaaaaga taaaacgcta 1200
   gcttgtgttt ataccactaa tagaaacgtc aatccggatg gtagcttaat cgctaatggt 1260
   tacaattggc cagaattttc acgagaaaaa ctcacaacgg ctatgattgc tcaccacttt 1320
   agaatgttca cgattagagc ttggcattta actgatggat tcaatgaaaa aattgaaaat 1380
   gccgtagact atgacatgtt cctcaaactc agtgaagttg gaaaatttaa acatcttaat 1440
   aaaatctgct ataaccgtgt attacatggt gataacacat caattaagaa acttggcatt 1500
   caaaagaaaa accattttgt tgtagtcaat cagtcattaa atagacaagg cataacttat 1560
   tataattatg acgaatttga tgatttagat gaaagtagaa agtatatttt caataaaacc 1620
   gctgaatatc aagaagagat tgatatctta aaagatatta aaatcatcca gaataaagat 1680
   gccaaaatcg cagtcagtat tttttatccc aatacattaa acggcttagt gaaaaaacta 1740
   aacaatatta ttgaatataa taaaaatata ttcgttattg ttctacatgt tgataagaat 1800
   catcttacac cagatatcta a 1821
<210> 16
   <211> 2112
   <212> DNA
   <213> Pasteurella multocida
<400> 16
   atgaatacat tatcacaagc aataaaagca tataacagca atgactatca attagcactc      60
   aaattatttg aaaagtcggc ggaaatctat ggacggaaaa ttgttgaatt tcaaattacc     120
   aaatgcaaag aaaaactctc agcacatcct tctgttaatt cagcacatct ttctgtaaat     180
   aaagaagaaa aagtcaatgt ttgcgatagt ccgttagata ttgcaacaca actgttactt     240
   tccaacgtaa aaaaattagt actttctgac tcggaaaaaa acacgttaaa aaataaatgg     300
   aaattgctca ctgagaagaa atctgaaaat gcggaggtaa gagcggtcgc ccttgtacca     360
   aaagattttc ccaaagatct ggttttagcg cctttacctg atcatgttaa tgattttaca     420
   tggtacaaaa agcgaaagaa aagacttggc ataaaacctg aacatcaaca tgttggtctt     480
   tctattatcg ttacaacatt caatcgacca gcaattttat cgattacatt agcctgttta     540
   gtaaaccaaa aaacacatta cccgtttgaa gttatcgtga cagatgaagg tagtcaggaa     600
   gatctatcac cgatcattcg ccaatatgaa aataaattgg atattcgcta cgtcagacaa     660
   aaagataacg gttttcaagc cagtgccgct cggaatatgg gattacgctt agcaaaatat     720
   gactttattg gcttactcga ctgtgatatg gcgccaaatc cattatgggt tcattcttat     780
   gttgcagagc tattagaaga tgatgattta acaatcattg gtccaagaaa atacatcgat     840
   acacaacata ttgacccaaa agacttctta aataacgcga gtttgcttga atcattacca     900
   gaagtgaaaa ccaataatag tgttgccgca aaaggggaag gaacagtttc tctggattgg 960
   cgcttagaac aattcgaaaa aacagaaaat ctccgcttat ccgattcgcc tttccgtttt 1020
   tttgcggcgg gtaatgttgc tttcgctaaa aaatggctaa ataaatccgg tttctttgat 1080
   gaggaattta atcactgggg tggagaagat gtggaatttg gatatcgctt attccgttac 1140
   ggtagtttct ttaaaactat tgatggcatt atggcctacc atcaagagcc accaggtaaa 1200
   gaaaatgaaa ccgatcgtga agcgggaaaa aatattacgc tcgatattat gagagaaaag 1260
   gtcccttata tctatagaaa acttttacca atagaagatt cgcatatcaa tagagtacct 1320
   ttagtttcaa tttatatccc agcttataac tgtgcaaact atattcaacg ttgcgtagat 1380
   agtgcactga atcagactgt tgttgatctc gaggtttgta tttgtaacga tggttcaaca 1440
   gataatacct tagaagtgat caataagctt tatggtaata atcctagggt acgcatcatg 1500
   tctaaaccaa atggcggaat agcctcagca tcaaatgcag ccgtttcttt tgctaaaggt 1560
   tattacattg ggcagttaga ttcagatgat tatcttgagc ctgatgcagt tgaactgtgt 1620
   ttaaaagaat ttttaaaaga taaaacgcta gcttgtgttt ataccactaa tagaaacgtc 1680
   aatccggatg gtagcttaat cgctaatggt tacaattggc cagaattttc acgagaaaaa 1740
   ctcacaacgg ctatgattgc tcaccacttt agaatgttca cgattagagc ttggcattta 1800
   actgatggat tcaatgaaaa aattgaaaat gccgtagact atgacatgtt cctcaaactc 1860
   agtgaagttg gaaaatttaa acatcttaat aaaatctgct ataaccgtgt attacatggt 1920
   gataacacat caattaagaa acttggcatt caaaagaaaa accattttgt tgtagtcaat 1980
   cagtcattaa atagacaagg cataacttat tataattatg acgaatttga tgatttagat 2040
   gaaagtagaa agtatatttt caataaaacc gctgaatatc aagaagagat tgatatctta 2100
   aaagatattt aa 2112
<210> 17
   <211> 2112
   <212> DNA
   <213> Pasteurella multocida
<400> 17
   atgaatacat tatcacaagc aataaaagca tataacagca atgactatca attagcactc     60
   aaattatttg aaaagtcggc ggaaatctat ggacggaaaa ttgttgaatt tcaaattacc    120
   aaatgcaaag aaaaactctc agcacatcct tctgttaatt cagcacatct ttctgtaaat    180
   aaagaagaaa aagtcaatgt ttgcgatagt ccgttagata ttgcaacaca actgttactt    240
   tccaacgtaa aaaaattagt actttctgac tcggaaaaaa acacgttaaa aaataaatgg    300
   aaattgctca ctgagaagaa atctgaaaat gcggaggtaa gagcggtcgc ccttgtacca    360
   aaagattttc ccaaagatct ggttttagcg cctttacctg atcatgttaa tgattttaca    420
   tggtacaaaa agcgaaagaa aagacttggc ataaaacctg aacatcaaca tgttggtctt    480
   tctattatcg ttacaacatt caatcgacca gcaattttat cgattacatt agcctgttta    540
   gtaaaccaaa aaacacatta cccgtttgaa gttatcgtga cagataaagg tagtcaggaa    600
   gatctatcac cgatcattcg ccaatatgaa aataaattgg atattcgcta cgtcagacaa    660
   aaagataacg gttttcaagc cagtgccgct cggaatatgg gattacgctt agcaaaatat    720
   gactttattg gcttactcga ctgtgatatg gcgccaaatc cattatgggt tcattcttat    780
   gttgcagagc tattagaaga tgatgattta acaatcattg gtccaagaaa atacatcgat    840
   acacaacata ttgacccaaa agacttctta aataacgcga gtttgcttga atcattacca    900
   gaagtgaaaa ccaataatag tgttgccgca aaaggggaag gaacagtttc tctggattgg    960
   cgcttagaac aattcgaaaa aacagaaaat ctccgcttat ccgattcgcc tttccgtttt   1020
   tttgcggcgg gtaatgttgc tttcgctaaa aaatggctaa ataaatccgg tttctttgat   1080
   gaggaattta atcactgggg tggagaagat gtggaatttg gatatcgctt attccgttac   1140
   ggtagtttct ttaaaactat tgatggcatt atggcctacc atcaagagcc accaggtaaa   1200
   gaaaatgaaa ccgatcgtga agcgggaaaa aatattacgc tcgatattat gagagaaaag   1260
   gtcccttata tctatagaaa acttttacca atagaagatt cgcatatcaa tagagtacct   1320
   ttagtttcaa tttatatccc agcttataac tgtgcaaact atattcaacg ttgcgtagat   1380
   agtgcactga atcagactgt tgttgatctc gaggtttgta tttgtaacga tggttcaaca   1440
   gataatacct tagaagtgat caataagctt tatggtaata atcctagggt acgcatcatg   1500
   tctaaaccaa atggcggaat agcctcagca tcaaatgcag ccgtttcttt tgctaaaggt   1560
   tattacattg ggcagttaga ttcagatgat tatcttgagc ctgatgcagt tgaactgtgt   1620
   ttaaaagaat ttttaaaaga taaaacgcta gcttgtgttt ataccactaa tagaaacgtc   1680
   aatccggatg gtagcttaat cgctaatggt tacaattggc cagaattttc acgagaaaaa   1740
   ctcacaacgg ctatgattgc tcaccacttt agaatgttca cgattagagc ttggcattta   1800
   actgatggat tcaatgaaaa aattgaaaat gccgtagact atgacatgtt cctcaaactc   1860
   agtgaagttg gaaaatttaa acatcttaat aaaatctgct ataaccgtgt attacatggt   1920
   gataacacat caattaagaa acttggcatt caaaagaaaa accattttgt tgtagtcaat   1980
   cagtcattaa atagacaagg cataacttat tataattatg acgaatttga tgatttagat   2040
   gaaagtagaa agtatatttt caataaaacc gctgaatatc aagaagagat tgatatctta   2100
   aaagatattt aa                                                       2112
<210> 18
   <211> 2112
   <212> DNA
   <213> Pasteurella multocida
<400> 18
   atgaatacat tatcacaagc aataaaagca tataacagca atgactatca attagcactc 60
   aaattatttg aaaagtcggc ggaaatctat ggacggaaaa ttgttgaatt tcaaattacc 120
   aaatgcaaag aaaaactctc agcacatcct tctgttaatt cagcacatct ttctgtaaat 180
   aaagaagaaa aagtcaatgt ttgcgatagt ccgttagata ttgcaacaca actgttactt 240
   tccaacgtaa aaaaattagt actttctgac tcggaaaaaa acacgttaaa aaataaatgg 300
   aaattgctca ctgagaagaa atctgaaaat gcggaggtaa gagcggtcgc ccttgtacca 360
   aaagattttc ccaaagatct ggttttagcg cctttacctg atcatgttaa tgattttaca 420
   tggtacaaaa agcgaaagaa aagacttggc ataaaacctg aacatcaaca tgttggtctt 480
   tctattatcg ttacaacatt caatcgacca gcaattttat cgattacatt agcctgttta 540
   gtaaaccaaa aaacacatta cccgtttgaa gttatcgtga cagatgatgg tagtcaggaa 600
   gatctatcac cgatcattcg ccaatatgaa aataaattgg atattcgcta cgtcagacaa 660
   aaagataacg gttttcaagc cagtgccgct cggaatatgg gattacgctt agcaaaatat 720
   gactttattg gcttactcga ctgtgatatg gcgccaaatc cattatgggt tcattcttat 780
   gttgcagagc tattagaaga tgatgattta acaatcattg gtccaagaaa atacatcgat 840
   acacaacata ttgacccaaa agacttctta aataacgcga gtttgcttga atcattacca 900
   gaagtgaaaa ccaataatag tgttgccgca aaaggggaag gaacagtttc tctggattgg 960
   cgcttagaac aattcgaaaa aacagaaaat ctccgcttat ccgattcgcc tttccgtttt 1020
   tttgcggcgg gtaatgttgc tttcgctaaa aaatggctaa ataaatccgg tttctttgat 1080
   gaggaattta atcactgggg tggagaagat gtggaatttg gatatcgctt attccgttac 1140
   ggtagtttct ttaaaactat tgatggcatt atggcctacc atcaagagcc accaggtaaa 1200
   gaaaatgaaa ccgatcgtga agcgggaaaa aatattacgc tcgatattat gagagaaaag 1260
   gtcccttata tctatagaaa acttttacca atagaagatt cgcatatcaa tagagtacct 1320
   ttagtttcaa tttatatccc agcttataac tgtgcaaact atattcaacg ttgcgtagat 1380
   agtgcactga atcagactgt tgttgatctc gaggtttgta tttgtaacga aggttcaaca 1440
   gataatacct tagaagtgat caataagctt tatggtaata atcctagggt acgcatcatg 1500
   tctaaaccaa atggcggaat agcctcagca tcaaatgcag ccgtttcttt tgctaaaggt 1560
   tattacattg ggcagttaga ttcagatgat tatcttgagc ctgatgcagt tgaactgtgt 1620
   ttaaaagaat ttttaaaaga taaaacgcta gcttgtgttt ataccactaa tagaaacgtc 1680
   aatccggatg gtagcttaat cgctaatggt tacaattggc cagaattttc acgagaaaaa 1740
   ctcacaacgg ctatgattgc tcaccacttt agaatgttca cgattagagc ttggcattta 1800
   actgatggat tcaatgaaaa aattgaaaat gccgtagact atgacatgtt cctcaaactc   1860
   agtgaagttg gaaaatttaa acatcttaat aaaatctgct ataaccgtgt attacatggt   1920
   gataacacat caattaagaa acttggcatt caaaagaaaa accattttgt tgtagtcaat   1980
   cagtcattaa atagacaagg cataacttat tataattatg acgaatttga tgatttagat   2040
   gaaagtagaa agtatatttt caataaaacc gctgaatatc aagaagagat tgatatctta   2100
   aaagatattt aa                                                       2112
<210> 19
   <211> 2112
   <212> DNA
   <213> Pasteurella multocida
<400> 19
   atgaatacat tatcacaagc aataaaagca tataacagca atgactatca attagcactc     60
   aaattatttg aaaagtcggc ggaaatctat ggacggaaaa ttgttgaatt tcaaattacc    120
   aaatgcaaag aaaaactctc agcacatcct tctgttaatt cagcacatct ttctgtaaat    180
   aaagaagaaa aagtcaatgt ttgcgatagt ccgttagata ttgcaacaca actgttactt    240
   tccaacgtaa aaaaattagt actttctgac tcggaaaaaa acacgttaaa aaataaatgg    300
   aaattgctca ctgagaagaa atctgaaaat gcggaggtaa gagcggtcgc ccttgtacca    360
   aaagattttc ccaaagatct ggttttagcg cctttacctg atcatgttaa tgattttaca    420
   tggtacaaaa agcgaaagaa aagacttggc ataaaacctg aacatcaaca tgttggtctt    480
   tctattatcg ttacaacatt caatcgacca gcaattttat cgattacatt agcctgttta    540
   gtaaaccaaa aaacacatta cccgtttgaa gttatcgtga cagatgatgg tagtcaggaa    600
   gatctatcac cgatcattcg ccaatatgaa aataaattgg atattcgcta cgtcagacaa    660
   aaagataacg gttttcaagc cagtgccgct cggaatatgg gattacgctt agcaaaatat    720
   gactttattg gcttactcga ctgtgatatg gcgccaaatc cattatgggt tcattcttat    780
   gttgcagagc tattagaaga tgatgattta acaatcattg gtccaagaaa atacatcgat    840
   acacaacata ttgacccaaa agacttctta aataacgcga gtttgcttga atcattacca    900
   gaagtgaaaa ccaataatag tgttgccgca aaaggggaag gaacagtttc tctggattgg    960
   cgcttagaac aattcgaaaa aacagaaaat ctccgcttat ccgattcgcc tttccgtttt   1020
   tttgcggcgg gtaatgttgc tttcgctaaa aaatggctaa ataaatccgg tttctttgat   1080
   gaggaattta atcactgggg tggagaagat gtggaatttg gatatcgctt attccgttac   1140
   ggtagtttct ttaaaactat tgatggcatt atggcctacc atcaagagcc accaggtaaa   1200
   gaaaatgaaa ccgatcgtga agcgggaaaa aatattacgc tcgatattat gagagaaaag   1260
   gtcccttata tctatagaaa acttttacca atagaagatt cgcatatcaa tagagtacct   1320
   ttagtttcaa tttatatccc agcttataac tgtgcaaact atattcaacg ttgcgtagat 1380
   agtgcactga atcagactgt tgttgatctc gaggtttgta tttgtaacaa aggttcaaca 1440
   gataatacct tagaagtgat caataagctt tatggtaata atcctagggt acgcatcatg 1500
   tctaaaccaa atggcggaat agcctcagca tcaaatgcag ccgtttcttt tgctaaaggt 1560
   tattacattg ggcagttaga ttcagatgat tatcttgagc ctgatgcagt tgaactgtgt 1620
   ttaaaagaat ttttaaaaga taaaacgcta gcttgtgttt ataccactaa tagaaacgtc 1680
   aatccggatg gtagcttaat cgctaatggt tacaattggc cagaattttc acgagaaaaa 1740
   ctcacaacgg ctatgattgc tcaccacttt agaatgttca cgattagagc ttggcattta 1800
   actgatggat tcaatgaaaa aattgaaaat gccgtagact atgacatgtt cctcaaactc 1860
   agtgaagttg gaaaatttaa acatcttaat aaaatctgct ataaccgtgt attacatggt 1920
   gataacacat caattaagaa acttggcatt caaaagaaaa accattttgt tgtagtcaat 1980
   cagtcattaa atagacaagg cataacttat tataattatg acgaatttga tgatttagat 2040
   gaaagtagaa agtatatttt caataaaacc gctgaatatc aagaagagat tgatatctta 2100
   aaagatattt aa 2112
<210> 20
   <211> 2271
   <212> DNA
   <213> Pasteurella multocida
<400> 20
   atgaatacat tatcacaagc aataaaagca tataacagca atgactatca attagcactc     60
   aaattatttg aaaagtcggc ggaaatctat ggacggaaaa ttgttgaatt tcaaattacc    120
   aaatgcaaag aaaaactctc agcacatcct tctgttaatt cagcacatct ttctgtaaat    180
   aaagaagaaa aagtcaatgt ttgcgatagt ccgttagata ttgcaacaca actgttactt    240
   tccaacgtaa aaaaattagt actttctgac tcggaaaaaa acacgttaaa aaataaatgg    300
   aaattgctca ctgagaagaa atctgaaaat gcggaggtaa gagcggtcgc ccttgtacca    360
   aaagattttc ccaaagatct ggttttagcg cctttacctg atcatgttaa tgattttaca    420
   tggtacaaaa agcgaaagaa aagacttggc ataaaacctg aacatcaaca tgttggtctt    480
   tctattatcg ttacaacatt caatcgacca gcaattttat cgattacatt agcctgttta    540
   gtaaaccaaa aaacacatta cccgtttgaa gttatcgtga cagatgatgg tagtcaggaa    600
   gatctatcac cgatcattcg ccaatatgaa aataaattgg atattcgcta cgtcagacaa    660
   aaagataacg gttttcaagc cagtgccgct cggaatatgg gattacgctt agcaaaatat    720
   gactttattg gcttactcga ctgtgatatg gcgccaaatc cattatgggt tcattcttat    780
   gttgcagagc tattagaaga tgatgattta acaatcattg gtccaagaaa atacatcgat    840
   acacaacata ttgacccaaa agacttctta aataacgcga gtttgcttga atcattacca 900
   gaagtgaaaa ccaataatag tgttgccgca aaaggggaag gaacagtttc tctggattgg 960
   cgcttagaac aattcgaaaa aacagaaaat ctccgcttat ccgattcgcc tttccgtttt 1020
   tttgcggcgg gtaatgttgc tttcgctaaa aaatggctaa ataaatccgg tttctttgat 1080
   gaggaattta atcactgggg tggagaagat gtggaatttg gatatcgctt attccgttac 1140
   ggtagtttct ttaaaactat tgatggcatt atggcctacc atcaagagcc accaggtaaa 1200
   gaaaatgaaa ccgatcgtga agcgggaaaa aatattacgc tcgatattat gagagaaaag 1260
   gtcccttata tctatagaaa acttttacca atagaagatt cgcatatcaa tagagtacct 1320
   ttagtttcaa tttatatccc agcttataac tgtgcaaact atattcaacg ttgcgtagat 1380
   agtgcactga atcagactgt tgttgatctc gaggtttgta tttgtaacga tggttcaaca 1440
   gataatacct tagaagtgat caataagctt tatggtaata atcctagggt acgcatcatg 1500
   tctaaaccaa atggcggaat agcctcagca tcaaatgcag ccgtttcttt tgctaaaggt 1560
   tattacattg ggcagttaga ttcagatgat tatcttgagc ctgatgcagt tgaactgtgt 1620
   ttaaaagaat ttttaaaaga taaaacgcta gcttgtgttt ataccactaa tagaaacgtc 1680
   aatccggatg gtagcttaat cgctaatggt tacaattggc cagaattttc acgagaaaaa 1740
   ctcacaacgg ctatgattgc tcaccacttt agaatgttca cgattagagc ttggcattta 1800
   actgatggat tcaatgaaaa aattgaaaat gccgtagact atgacatgtt cctcaaactc 1860
   agtgaagttg gaaaatttaa acatcttaat aaaatctgct ataaccgtgt attacatggt 1920
   gataacacat caattaagaa acttggcatt caaaagaaaa accattttgt tgtagtcaat 1980
   cagtcattaa atagacaagg cataacttat tataattatg acgaatttga tgatttagat 2040
   gaaagtagaa agtatatttt caataaaacc gctgaatatc aagaagagat tgatatctta 2100
   aaagatatta aaatcatcca gaataaagat gccaaaatcg cagtcagtat tttttatccc 2160
   aatacattaa acggcttagt gaaaaaacta aacaatatta ttgaatataa taaaaatata 2220
   ttcgttattg ttctacatgt tgataagaat catcttacac cagatatcta a 2271
<210> 21
   <211> 1704
   <212> DNA
   <213> Pasteurella multocida
<400> 21
   atgaatacat tatcacaagc aataaaagca tataacagca atgactatca attagcactc      60
   aaattatttg aaaagtcggc ggaaatctat ggacggaaaa ttgttgaatt tcaaattacc     120
   aaatgcaaag aaaaactctc agcacatcct tctgttaatt cagcacatct ttctgtaaat     180
   aaagaagaaa aagtcaatgt ttgcgatagt ccgttagata ttgcaacaca actgttactt     240
   tccaacgtaa aaaaattagt actttctgac tcggaaaaaa acacgttaaa aaataaatgg     300
   aaattgctca ctgagaagaa atctgaaaat gcggaggtaa gagcggtcgc ccttgtacca     360
   aaagattttc ccaaagatct ggttttagcg cctttacctg atcatgttaa tgattttaca     420
   tggtacaaaa agcgaaagaa aagacttggc ataaaacctg aacatcaaca tgttggtctt     480
   tctattatcg ttacaacatt caatcgacca gcaattttat cgattacatt agcctgttta     540
   gtaaaccaaa aaacacatta cccgtttgaa gttatcgtga cagatgatgg tagtcaggaa     600
   gatctatcac cgatcattcg ccaatatgaa aataaattgg atattcgcta cgtcagacaa     660
   aaagataacg gttttcaagc cagtgccgct cggaatatgg gattacgctt agcaaaatat     720
   gactttattg gcttactcga ctgtgatatg gcgccaaatc cattatgggt tcattcttat     780
   gttgcagagc tattagaaga tgatgattta acaatcattg gtccaagaaa atacatcgat     840
   acacaacata ttgacccaaa agacttctta aataacgcga gtttgcttga atcattacca     900
   gaagtgaaaa ccaataatag tgttgccgca aaaggggaag gaacagtttc tctggattgg     960
   cgcttagaac aattcgaaaa aacagaaaat ctccgcttat ccgattcgcc tttccgtttt    1020
   tttgcggcgg gtaatgttgc tttcgctaaa aaatggctaa ataaatccgg tttctttgat    1080
   gaggaattta atcactgggg tggagaagat gtggaatttg gatatcgctt attccgttac    1140
   ggtagtttct ttaaaactat tgatggcatt atggcctacc atcaagagcc accaggtaaa    1200
   gaaaatgaaa ccgatcgtga agcgggaaaa aatattacgc tcgatattat gagagaaaag    1260
   gtcccttata tctatagaaa acttttacca atagaagatt cgcatatcaa tagagtacct    1320
   ttagtttcaa tttatatccc agcttataac tgtgcaaact atattcaacg ttgcgtagat    1380
   agtgcactga atcagactgt tgttgatctc gaggtttgta tttgtaacga tggttcaaca    1440
   gataatacct tagaagtgat caataagctt tatggtaata atcctagggt acgcatcatg    1500
   tctaaaccaa atggcggaat agcctcagca tcaaatgcag ccgtttcttt tgctaaaggt    1560
   tattacattg ggcagttaga ttcagatgat tatcttgagc ctgatgcagt tgaactgtgt    1620
   ttaaaagaat ttttaaaaga taaaacgcta gcttgtgttt ataccactaa tagaaacgtc    1680
   aatccggatg gtagcttaat ctaa                                           1704
<210> 22
   <211> 2115
   <212> DNA
   <213> Pasteurella multocida
<400> 22
   atgaatacat tatcacaagc aataaaagca tataacagca atgactatga attagcactc     60
   aaattatttg agaagtctgc tgaaacctac gggcgaaaaa tcgttgaatt ccaaattatc    120
   aaatgtaaag aaaaactctc gaccaattct tatgtaagtg aagataaaaa aaacagtgtt    180
   tgcgatagct cattagatat cgcaacacag ctcttacttt ccaacgtaaa aaaattaact 240
   ctatccgaat cagaaaaaaa cagtttaaaa aataaatgga aatctatcac tgggaaaaaa 300
   tcggagaacg cagaaatcag aaaggtggaa ctagtaccca aagattttcc taaagatctt 360
   gttcttgctc cattgccaga tcatgttaat gattttacat ggtacaaaaa tcgaaaaaaa 420
   agcttaggta taaagcctgt aaataagaat atcggtcttt ctattattat tcctacattt 480
   aatcgtagcc gtattttaga tataacgtta gcctgtttgg tcaatcagaa aacaaactac 540
   ccatttgaag tcgttgttgc agatgatggt agtaaggaaa acttacttac cattgtgcaa 600
   aaatacgaac aaaaacttga cataaagtat gtaagacaaa aagattatgg atatcaattg 660
   tgtgcagtca gaaacttagg tttacgtaca gcaaagtatg attttgtctc gattctagac 720
   tgcgatatgg caccacaaca attatgggtt cattcttatc ttacagaact attagaagac 780
   aatgatattg ttttaattgg acctagaaaa tatgtggata ctcataatat taccgcagaa 840
   caattcctta acgatccata tttaatagaa tcactacctg aaaccgctac aaataacaat 900
   ccttcgatta catcaaaagg aaatatatcg ttggattgga gattagaaca tttcaaaaaa 960
   accgataatc tacgtctatg tgattctccg tttcgttatt ttagttgcgg taatgttgca 1020
   ttttctaaag aatggctaaa taaagtaggt tggttcgatg aagaatttaa tcattggggg 1080
   ggcgaagatg tagaatttgg ttacagatta tttgccaaag gctgtttttt cagagtaatt 1140
   gacggcggaa tggcatacca tcaagaacca cctggtaaag aaaatgaaac agaccgcgaa 1200
   gctggtaaaa gtattacgct taaaattgtg aaagaaaagg taccttacat ctatagaaag 1260
   cttttaccaa tagaagattc acatattcat agaatacctt tagtttctat ttatatcccc 1320
   gcttataact gtgcaaatta tattcaaaga tgtgtagata gtgctcttaa tcaaactgtt 1380
   gtcgatctcg aggtttgtat ttgtaacgat ggttcaacag ataatacctt agaagtgatc 1440
   aataagcttt atggtaataa tcctagggta cgcatcatgt ctaaaccaaa tggcggaata 1500
   gcctcagcat caaatgcagc cgtttctttt gctaaaggtt attacattgg gcagttagat 1560
   tcagatgatt atcttgagcc tgatgcagtt gaactgtgtt taaaagaatt tttaaaagat 1620
   aaaacgctag cttgtgttta taccactaat agaaacgtca atccggatgg tagcttaatc 1680
   gctaatggtt acaattggcc agaattttca cgagaaaaac tcacaacggc tatgattgct 1740
   caccatttta gaatgtttac gattagagct tggcatttaa cggatggatt taacgaaaat 1800
   attgaaaacg ccgtggatta tgacatgttc cttaaactca gtgaagttgg aaaatttaaa 1860
   catcttaata aaatctgcta taaccgcgta ttacatggtg ataacacatc cattaagaaa 1920
   ctcggcattc aaaagaaaaa ccattttgtt gtagtcaatc agtcattaaa tagacaaggc 1980
   atcaattatt ataattatga caaatttgat gatttagatg aaagtagaaa gtatatcttc 2040
   aataaaaccg ctgaatatca agaagaaatg gatattttaa aagatcttaa actcattcaa 2100
   aataaagatg cctaa 2115
<210> 23
   <211> 1980
   <212> DNA
   <213> Pasteurella multocida
<400> 23
   atgctctcag cacatccttc tgttaattca gcacatcttt ctgtaaataa agaagaaaaa     60
   gtcaatgttt gcgatagtcc gttagatatt gcaacacaac tgttactttc caacgtaaaa    120
   aaattagtac tttctgactc ggaaaaaaac acgttaaaaa ataaatggaa attgctcact    180
   gagaagaaat ctgaaaatgc ggaggtaaga gcggtcgccc ttgtaccaaa agattttccc    240
   aaagatctgg ttttagcgcc tttacctgat catgttaatg attttacatg gtacaaaaag    300
   cgaaagaaaa gacttggcat aaaacctgaa catcaacatg ttggtctttc tattatcgtt    360
   acaacattca atcgaccagc aattttatcg attacattag cctgtttagt aaaccaaaaa    420
   acacattacc cgtttgaagt tatcgtgaca gatgatggta gtcaggaaga tctatcaccg    480
   atcattcgcc aatatgaaaa taaattggat attcgctacg tcagacaaaa agataacggt    540
   tttcaagcca gtgccgctcg gaatatggga ttacgcttag caaaatatga ctttattggc    600
   ttactcgact gtgatatggc gccaaatcca ttatgggttc attcttatgt tgcagagcta    660
   ttagaagatg atgatttaac aatcattggt ccaagaaaat acatcgatac acaacatatt    720
   gacccaaaag acttcttaaa taacgcgagt ttgcttgaat cattaccaga agtgaaaacc    780
   aataatagtg ttgccgcaaa aggggaagga acagtttctc tggattggcg cttagaacaa    840
   ttcgaaaaaa cagaaaatct ccgcttatcc gattcgcctt tccgtttttt tgcggcgggt    900
   aatgttgctt tcgctaaaaa atggctaaat aaatccggtt tctttgatga ggaatttaat    960
   cactggggtg gagaagatgt ggaatttgga tatcgcttat tccgttacgg tagtttcttt   1020
   aaaactattg atggcattat ggcctaccat caagagccac caggtaaaga aaatgaaacc   1080
   gatcgtgaag cgggaaaaaa tattacgctc gatattatga gagaaaaggt cccttatatc   1140
   tatagaaaac ttttaccaat agaagattcg catatcaata gagtaccttt agtttcaatt   1200
   tatatcccag cttataactg tgcaaactat attcaacgtt gcgtagatag tgcactgaat   1260
   cagactgttg ttgatctcga ggtttgtatt tgtaacgatg gttcaacaga taatacctta   1320
   gaagtgatca ataagcttta tggtaataat cctagggtac gcatcatgtc taaaccaaat   1380
   ggcggaatag cctcagcatc aaatgcagcc gtttcttttg ctaaaggtta ttacattggg   1440
   cagttagatt cagatgatta tcttgagcct gatgcagttg aactgtgttt aaaagaattt   1500
   ttaaaagata aaacgctagc ttgtgtttat accactaata gaaacgtcaa tccggatggt   1560
   agcttaatcg ctaatggtta caattggcca gaattttcac gagaaaaact cacaacggct   1620
   atgattgctc accactttag aatgttcacg attagagctt ggcatttaac tgatggattc 1680
   aatgaaaaaa ttgaaaatgc cgtagactat gacatgttcc tcaaactcag tgaagttgga 1740
   aaatttaaac atcttaataa aatctgctat aaccgtgtat tacatggtga taacacatca 1800
   attaagaaac ttggcattca aaagaaaaac cattttgttg tagtcaatca gtcattaaat 1860
   agacaaggca taacttatta taattatgac gaatttgatg atttagatga aagtagaaag 1920
   tatattttca ataaaaccgc tgaatatcaa gaagagattg atatcttaaa agatatttaa 1980
<210> 24
   <211> 1902
   <212> DNA
   <213> Pasteurella multocida
<400> 24
   atgttagata ttgcaacaca actgttactt tccaacgtaa aaaaattagt actttctgac 60
   tcggaaaaaa acacgttaaa aaataaatgg aaattgctca ctgagaagaa atctgaaaat 120
   gcggaggtaa gagcggtcgc ccttgtacca aaagattttc ccaaagatct ggttttagcg 180
   cctttacctg atcatgttaa tgattttaca tggtacaaaa agcgaaagaa aagacttggc 240
   ataaaacctg aacatcaaca tgttggtctt tctattatcg ttacaacatt caatcgacca 300
   gcaattttat cgattacatt agcctgttta gtaaaccaaa aaacacatta cccgtttgaa 360
   gttatcgtga cagatgatgg tagtcaggaa gatctatcac cgatcattcg ccaatatgaa 420
   aataaattgg atattcgcta cgtcagacaa aaagataacg gttttcaagc cagtgccgct 480
   cggaatatgg gattacgctt agcaaaatat gactttattg gcttactcga ctgtgatatg 540
   gcgccaaatc cattatgggt tcattcttat gttgcagagc tattagaaga tgatgattta 600
   acaatcattg gtccaagaaa atacatcgat acacaacata ttgacccaaa agacttctta 660
   aataacgcga gtttgcttga atcattacca gaagtgaaaa ccaataatag tgttgccgca 720
   aaaggggaag gaacagtttc tctggattgg cgcttagaac aattcgaaaa aacagaaaat 780
   ctccgcttat ccgattcgcc tttccgtttt tttgcggcgg gtaatgttgc tttcgctaaa 840
   aaatggctaa ataaatccgg tttctttgat gaggaattta atcactgggg tggagaagat 900
   gtggaatttg gatatcgctt attccgttac ggtagtttct ttaaaactat tgatggcatt 960
   atggcctacc atcaagagcc accaggtaaa gaaaatgaaa ccgatcgtga agcgggaaaa 1020
   aatattacgc tcgatattat gagagaaaag gtcccttata tctatagaaa acttttacca 1080
   atagaagatt cgcatatcaa tagagtacct ttagtttcaa tttatatccc agcttataac 1140
   tgtgcaaact atattcaacg ttgcgtagat agtgcactga atcagactgt tgttgatctc 1200
   gaggtttgta tttgtaacga tggttcaaca gataatacct tagaagtgat caataagctt 1260
   tatggtaata atcctagggt acgcatcatg tctaaaccaa atggcggaat agcctcagca 1320
   tcaaatgcag ccgtttcttt tgctaaaggt tattacattg ggcagttaga ttcagatgat   1380
   tatcttgagc ctgatgcagt tgaactgtgt ttaaaagaat ttttaaaaga taaaacgcta   1440
   gcttgtgttt ataccactaa tagaaacgtc aatccggatg gtagcttaat cgctaatggt   1500
   tacaattggc cagaattttc acgagaaaaa ctcacaacgg ctatgattgc tcaccacttt   1560
   agaatgttca cgattagagc ttggcattta actgatggat tcaatgaaaa aattgaaaat   1620
   gccgtagact atgacatgtt cctcaaactc agtgaagttg gaaaatttaa acatcttaat   1680
   aaaatctgct ataaccgtgt attacatggt gataacacat caattaagaa acttggcatt   1740
   caaaagaaaa accattttgt tgtagtcaat cagtcattaa atagacaagg cataacttat   1800
   tataattatg acgaatttga tgatttagat gaaagtagaa agtatatttt caataaaacc   1860
   gctgaatatc aagaagagat tgatatctta aaagatattt aa                      1902
<210> 25
   <211> 1830
   <212> DNA
   <213> Pasteurella multocida
<400> 25
   atgttaaaaa ataaatggaa attgctcact gagaagaaat ctgaaaatgc ggaggtaaga     60
   gcggtcgccc ttgtaccaaa agattttccc aaagatctgg ttttagcgcc tttacctgat    120
   catgttaatg attttacatg gtacaaaaag cgaaagaaaa gacttggcat aaaacctgaa    180
   catcaacatg ttggtctttc tattatcgtt acaacattca atcgaccagc aattttatcg    240
   attacattag cctgtttagt aaaccaaaaa acacattacc cgtttgaagt tatcgtgaca    300
   gatgatggta gtcaggaaga tctatcaccg atcattcgcc aatatgaaaa taaattggat    360
   attcgctacg tcagacaaaa agataacggt tttcaagcca gtgccgctcg gaatatggga    420
   ttacgcttag caaaatatga ctttattggc ttactcgact gtgatatggc gccaaatcca    480
   ttatgggttc attcttatgt tgcagagcta ttagaagatg atgatttaac aatcattggt    540
   ccaagaaaat acatcgatac acaacatatt gacccaaaag acttcttaaa taacgcgagt    600
   ttgcttgaat cattaccaga agtgaaaacc aataatagtg ttgccgcaaa aggggaagga    660
   acagtttctc tggattggcg cttagaacaa ttcgaaaaaa cagaaaatct ccgcttatcc    720
   gattcgcctt tccgtttttt tgcggcgggt aatgttgctt tcgctaaaaa atggctaaat    780
   aaatccggtt tctttgatga ggaatttaat cactggggtg gagaagatgt ggaatttgga    840
   tatcgcttat tccgttacgg tagtttcttt aaaactattg atggcattat ggcctaccat    900
   caagagccac caggtaaaga aaatgaaacc gatcgtgaag cgggaaaaaa tattacgctc    960
   gatattatga gagaaaaggt cccttatatc tatagaaaac ttttaccaat agaagattcg   1020
   catatcaata gagtaccttt agtttcaatt tatatcccag cttataactg tgcaaactat   1080
   attcaacgtt gcgtagatag tgcactgaat cagactgttg ttgatctcga ggtttgtatt   1140
   tgtaacgatg gttcaacaga taatacctta gaagtgatca ataagcttta tggtaataat   1200
   cctagggtac gcatcatgtc taaaccaaat ggcggaatag cctcagcatc aaatgcagcc   1260
   gtttcttttg ctaaaggtta ttacattggg cagttagatt cagatgatta tcttgagcct   1320
   gatgcagttg aactgtgttt aaaagaattt ttaaaagata aaacgctagc ttgtgtttat   1380
   accactaata gaaacgtcaa tccggatggt agcttaatcg ctaatggtta caattggcca   1440
   gaattttcac gagaaaaact cacaacggct atgattgctc accactttag aatgttcacg   1500
   attagagctt ggcatttaac tgatggattc aatgaaaaaa ttgaaaatgc cgtagactat   1560
   gacatgttcc tcaaactcag tgaagttgga aaatttaaac atcttaataa aatctgctat   1620
   aaccgtgtat tacatggtga taacacatca attaagaaac ttggcattca aaagaaaaac   1680
   cattttgttg tagtcaatca gtcattaaat agacaaggca taacttatta taattatgac   1740
   gaatttgatg atttagatga aagtagaaag tatattttca ataaaaccgc tgaatatcaa   1800
   gaagagattg atatcttaaa agatatttaa                                    1830
<210> 26
   <211> 1764
   <212> DNA
   <213> Pasteurella multocida
<400> 26
   atgcttgtac caaaagattt tcccaaagat ctggttttag cgcctttacc tgatcatgtt      60
   aatgatttta catggtacaa aaagcgaaag aaaagacttg gcataaaacc tgaacatcaa     120
   catgttggtc tttctattat cgttacaaca ttcaatcgac cagcaatttt atcgattaca     180
   ttagcctgtt tagtaaacca aaaaacacat tacccgtttg aagttatcgt gacagatgat     240
   ggtagtcagg aagatctatc accgatcatt cgccaatatg aaaataaatt ggatattcgc     300
   tacgtcagac aaaaagataa cggttttcaa gccagtgccg ctcggaatat gggattacgc     360
   ttagcaaaat atgactttat tggcttactc gactgtgata tggcgccaaa tccattatgg     420
   gttcattctt atgttgcaga gctattagaa gatgatgatt taacaatcat tggtccaaga     480
   aaatacatcg atacacaaca tattgaccca aaagacttct taaataacgc gagtttgctt     540
   gaatcattac cagaagtgaa aaccaataat agtgttgccg caaaagggga aggaacagtt     600
   tctctggatt ggcgcttaga acaattcgaa aaaacagaaa atctccgctt atccgattcg     660
   cctttccgtt tttttgcggc gggtaatgtt gctttcgcta aaaaatggct aaataaatcc     720
   ggtttctttg atgaggaatt taatcactgg ggtggagaag atgtggaatt tggatatcgc     780
   ttattccgtt acggtagttt ctttaaaact attgatggca ttatggccta ccatcaagag     840
   ccaccaggta aagaaaatga aaccgatcgt gaagcgggaa aaaatattac gctcgatatt     900
   atgagagaaa aggtccctta tatctataga aaacttttac caatagaaga ttcgcatatc 960
   aatagagtac ctttagtttc aatttatatc ccagcttata actgtgcaaa ctatattcaa 1020
   cgttgcgtag atagtgcact gaatcagact gttgttgatc tcgaggtttg tatttgtaac 1080
   gatggttcaa cagataatac cttagaagtg atcaataagc tttatggtaa taatcctagg 1140
   gtacgcatca tgtctaaacc aaatggcgga atagcctcag catcaaatgc agccgtttct 1200
   tttgctaaag gttattacat tgggcagtta gattcagatg attatcttga gcctgatgca 1260
   gttgaactgt gtttaaaaga atttttaaaa gataaaacgc tagcttgtgt ttataccact 1320
   aatagaaacg tcaatccgga tggtagctta atcgctaatg gttacaattg gccagaattt 1380
   tcacgagaaa aactcacaac ggctatgatt gctcaccact ttagaatgtt cacgattaga 1440
   gcttggcatt taactgatgg attcaatgaa aaaattgaaa atgccgtaga ctatgacatg 1500
   ttcctcaaac tcagtgaagt tggaaaattt aaacatctta ataaaatctg ctataaccgt 1560
   gtattacatg gtgataacac atcaattaag aaacttggca ttcaaaagaa aaaccatttt 1620
   gttgtagtca atcagtcatt aaatagacaa ggcataactt attataatta tgacgaattt 1680
   gatgatttag atgaaagtag aaagtatatt ttcaataaaa ccgctgaata tcaagaagag 1740
   attgatatct taaaagatat ttaa 1764
<210> 27
   <211> 2007
   <212> DNA
   <213> Pasteurella multocida
<400> 27
   atgaatacat tatcacaagc aataaaagca tataacagca atgactatca attagcactc    60
   aaattatttg aaaagtcggc ggaaatctat ggacggaaaa ttgttgaatt tcaaattacc   120
   aaatgcaaag aaaaactctc agcacatcct tctgttaatt cagcacatct ttctgtaaat   180
   aaagaagaaa aagtcaatgt ttgcgatagt ccgttagata ttgcaacaca actgttactt   240
   tccaacgtaa aaaaattagt actttctgac tcggaaaaaa acacgttaaa aaataaatgg   300
   aaattgctca ctgagaagaa atctgaaaat gcggaggtaa gagcggtcgc ccttgtacca   360
   aaagattttc ccaaagatct ggttttagcg cctttacctg atcatgttaa tgattttaca   420
   tggtacaaaa agcgaaagaa aagacttggc ataaaacctg aacatcaaca tgttggtctt   480
   tctattatcg ttacaacatt caatcgacca gcaattttat cgattacatt agcctgttta   540
   gtaaaccaaa aaacacatta cccgtttgaa gttatcgtga cagatgatgg tagtcaggaa   600
   gatctatcac cgatcattcg ccaatatgaa aataaattgg atattcgcta cgtcagacaa   660
   aaagataacg gttttcaagc cagtgccgct cggaatatgg gattacgctt agcaaaatat   720
   gactttattg gcttactcga ctgtgatatg gcgccaaatc cattatgggt tcattcttat   780
   gttgcagagc tattagaaga tgatgattta acaatcattg gtccaagaaa atacatcgat 840
   acacaacata ttgacccaaa agacttctta aataacgcga gtttgcttga atcattacca 900
   gaagtgaaaa ccaataatag tgttgccgca aaaggggaag gaacagtttc tctggattgg 960
   cgcttagaac aattcgaaaa aacagaaaat ctccgcttat ccgattcgcc tttccgtttt 1020
   tttgcggcgg gtaatgttgc tttcgctaaa aaatggctaa ataaatccgg tttctttgat 1080
   gaggaattta atcactgggg tggagaagat gtggaatttg gatatcgctt attccgttac 1140
   ggtagtttct ttaaaactat tgatggcatt atggcctacc atcaagagcc accaggtaaa 1200
   gaaaatgaaa ccgatcgtga agcgggaaaa aatattacgc tcgatattat gagagaaaag 1260
   gtcccttata tctatagaaa acttttacca atagaagatt cgcatatcaa tagagtacct 1320
   ttagtttcaa tttatatccc agcttataac tgtgcaaact atattcaacg ttgcgtagat 1380
   agtgcactga atcagactgt tgttgatctc gaggtttgta tttgtaacga tggttcaaca 1440
   gataatacct tagaagtgat caataagctt tatggtaata atcctagggt acgcatcatg 1500
   tctaaaccaa atggcggaat agcctcagca tcaaatgcag ccgtttcttt tgctaaaggt 1560
   tattacattg ggcagttaga ttcagatgat tatcttgagc ctgatgcagt tgaactgtgt 1620
   ttaaaagaat ttttaaaaga taaaacgcta gcttgtgttt ataccactaa tagaaacgtc 1680
   aatccggatg gtagcttaat cgctaatggt tacaattggc cagaattttc acgagaaaaa 1740
   ctcacaacgg ctatgattgc tcaccacttt agaatgttca cgattagagc ttggcattta 1800
   actgatggat tcaatgaaaa aattgaaaat gccgtagact atgacatgtt cctcaaactc 1860
   agtgaagttg gaaaatttaa acatcttaat aaaatctgct ataaccgtgt attacatggt 1920
   gataacacat caattaagaa acttggcatt caaaagaaaa accattttgt tgtagtcaat 1980
   cagtcattaa atagacaagg catataa 2007
<210> 28
   <211> 2061
   <212> DNA
   <213> Pasteurella multocida
<400> 28
   atgaatacat tatcacaagc aataaaagca tataacagca atgactatca attagcactc     60
   aaattatttg aaaagtcggc ggaaatctat ggacggaaaa ttgttgaatt tcaaattacc    120
   aaatgcaaag aaaaactctc agcacatcct tctgttaatt cagcacatct ttctgtaaat    180
   aaagaagaaa aagtcaatgt ttgcgatagt ccgttagata ttgcaacaca actgttactt    240
   tccaacgtaa aaaaattagt actttctgac tcggaaaaaa acacgttaaa aaataaatgg    300
   aaattgctca ctgagaagaa atctgaaaat gcggaggtaa gagcggtcgc ccttgtacca    360
   aaagattttc ccaaagatct ggttttagcg cctttacctg atcatgttaa tgattttaca    420
   tggtacaaaa agcgaaagaa aagacttggc ataaaacctg aacatcaaca tgttggtctt     480
   tctattatcg ttacaacatt caatcgacca gcaattttat cgattacatt agcctgttta     540
   gtaaaccaaa aaacacatta cccgtttgaa gttatcgtga cagatgatgg tagtcaggaa     600
   gatctatcac cgatcattcg ccaatatgaa aataaattgg atattcgcta cgtcagacaa     660
   aaagataacg gttttcaagc cagtgccgct cggaatatgg gattacgctt agcaaaatat     720
   gactttattg gcttactcga ctgtgatatg gcgccaaatc cattatgggt tcattcttat     780
   gttgcagagc tattagaaga tgatgattta acaatcattg gtccaagaaa atacatcgat     840
   acacaacata ttgacccaaa agacttctta aataacgcga gtttgcttga atcattacca     900
   gaagtgaaaa ccaataatag tgttgccgca aaaggggaag gaacagtttc tctggattgg     960
   cgcttagaac aattcgaaaa aacagaaaat ctccgcttat ccgattcgcc tttccgtttt    1020
   tttgcggcgg gtaatgttgc tttcgctaaa aaatggctaa ataaatccgg tttctttgat    1080
   gaggaattta atcactgggg tggagaagat gtggaatttg gatatcgctt attccgttac    1140
   ggtagtttct ttaaaactat tgatggcatt atggcctacc atcaagagcc accaggtaaa    1200
   gaaaatgaaa ccgatcgtga agcgggaaaa aatattacgc tcgatattat gagagaaaag    1260
   gtcccttata tctatagaaa acttttacca atagaagatt cgcatatcaa tagagtacct    1320
   ttagtttcaa tttatatccc agcttataac tgtgcaaact atattcaacg ttgcgtagat    1380
   agtgcactga atcagactgt tgttgatctc gaggtttgta tttgtaacga tggttcaaca    1440
   gataatacct tagaagtgat caataagctt tatggtaata atcctagggt acgcatcatg    1500
   tctaaaccaa atggcggaat agcctcagca tcaaatgcag ccgtttcttt tgctaaaggt    1560
   tattacattg ggcagttaga ttcagatgat tatcttgagc ctgatgcagt tgaactgtgt    1620
   ttaaaagaat ttttaaaaga taaaacgcta gcttgtgttt ataccactaa tagaaacgtc    1680
   aatccggatg gtagcttaat cgctaatggt tacaattggc cagaattttc acgagaaaaa    1740
   ctcacaacgg ctatgattgc tcaccacttt agaatgttca cgattagagc ttggcattta    1800
   actgatggat tcaatgaaaa aattgaaaat gccgtagact atgacatgtt cctcaaactc    1860
   agtgaagttg gaaaatttaa acatcttaat aaaatctgct ataaccgtgt attacatggt    1920
   gataacacat caattaagaa acttggcatt caaaagaaaa accattttgt tgtagtcaat    1980
   cagtcattaa atagacaagg cataacttat tataattatg acgaatttga tgatttagat    2040
   gaaagtagaa agtatattta a                                              2061
<210> 29
   <211> 2112
   <212> DNA
   <213> Pasteurella multocida
<400> 29
   atgaatacat tatcacaagc aataaaagca tataacagca atgactatca attagcactc 60
   aaattatttg aaaagtcggc ggaaatctat ggacggaaaa ttgttgaatt tcaaattacc 120
   aaatgcaaag aaaaactctc agcacatcct tctgttaatt cagcacatct ttctgtaaat 180
   aaagaagaaa aagtcaatgt ttgcgatagt ccgttagata ttgcaacaca actgttactt 240
   tccaacgtaa aaaaattagt actttctgac tcggaaaaaa acacgttaaa aaataaatgg 300
   aaattgctca ctgagaagaa atctgaaaat gcggaggtaa gagcggtcgc ccttgtacca 360
   aaagattttc ccaaagatct ggttttagcg cctttacctg atcatgttaa tgattttaca 420
   tggtacaaaa agcgaaagaa aagacttggc ataaaacctg aacatcaaca tgttggtctt 480
   tctattatcg ttacaacatt caatcgacca gcaattttat cgattacatt agcctgttta 540
   gtaaaccaaa aaacacatta cccgtttgaa gttatcgtga cagatgatgg tagtcaggaa 600
   gatctatcac cgatcattcg ccaatatgaa aataaattgg atattcgcta cgtcagacaa 660
   aaagataacg gttttcaagc cagtgccgct cggaatatgg gattacgctt agcaaaatat 720
   gactttattg gcttactcga atgtgatatg gcgccaaatc cattatgggt tcattcttat 780
   gttgcagagc tattagaaga tgatgattta acaatcattg gtccaagaaa atacatcgat 840
   acacaacata ttgacccaaa agacttctta aataacgcga gtttgcttga atcattacca 900
   gaagtgaaaa ccaataatag tgttgccgca aaaggggaag gaacagtttc tctggattgg 960
   cgcttagaac aattcgaaaa aacagaaaat ctccgcttat ccgattcgcc tttccgtttt 1020
   tttgcggcgg gtaatgttgc tttcgctaaa aaatggctaa ataaatccgg tttctttgat 1080
   gaggaattta atcactgggg tggagaagat gtggaatttg gatatcgctt attccgttac 1140
   ggtagtttct ttaaaactat tgatggcatt atggcctacc atcaagagcc accaggtaaa 1200
   gaaaatgaaa ccgatcgtga agcgggaaaa aatattacgc tcgatattat gagagaaaag 1260
   gtcccttata tctatagaaa acttttacca atagaagatt cgcatatcaa tagagtacct 1320
   ttagtttcaa tttatatccc agcttataac tgtgcaaact atattcaacg ttgcgtagat 1380
   agtgcactga atcagactgt tgttgatctc gaggtttgta tttgtaacga tggttcaaca 1440
   gataatacct tagaagtgat caataagctt tatggtaata atcctagggt acgcatcatg 1500
   tctaaaccaa atggcggaat agcctcagca tcaaatgcag ccgtttcttt tgctaaaggt 1560
   tattacattg ggcagttaga ttcagatgat tatcttgagc ctgatgcagt tgaactgtgt 1620
   ttaaaagaat ttttaaaaga taaaacgcta gcttgtgttt ataccactaa tagaaacgtc 1680
   aatccggatg gtagcttaat cgctaatggt tacaattggc cagaattttc acgagaaaaa 1740
   ctcacaacgg ctatgattgc tcaccacttt agaatgttca cgattagagc ttggcattta 1800
   actgatggat tcaatgaaaa aattgaaaat gccgtagact atgacatgtt cctcaaactc 1860
   agtgaagttg gaaaatttaa acatcttaat aaaatctgct ataaccgtgt attacatggt 1920
   gataacacat caattaagaa acttggcatt caaaagaaaa accattttgt tgtagtcaat 1980
   cagtcattaa atagacaagg cataacttat tataattatg acgaatttga tgatttagat 2040
   gaaagtagaa agtatatttt caataaaacc gctgaatatc aagaagagat tgatatctta 2100
   aaagatattt aa 2112
<210> 30
   <211> 2112
   <212> DNA
   <213> Pasteurella multocida
<400> 30
   atgaatacat tatcacaagc aataaaagca tataacagca atgactatca attagcactc     60
   aaattatttg aaaagtcggc ggaaatctat ggacggaaaa ttgttgaatt tcaaattacc    120
   aaatgcaaag aaaaactctc agcacatcct tctgttaatt cagcacatct ttctgtaaat    180
   aaagaagaaa aagtcaatgt ttgcgatagt ccgttagata ttgcaacaca actgttactt    240
   tccaacgtaa aaaaattagt actttctgac tcggaaaaaa acacgttaaa aaataaatgg    300
   aaattgctca ctgagaagaa atctgaaaat gcggaggtaa gagcggtcgc ccttgtacca    360
   aaagattttc ccaaagatct ggttttagcg cctttacctg atcatgttaa tgattttaca    420
   tggtacaaaa agcgaaagaa aagacttggc ataaaacctg aacatcaaca tgttggtctt    480
   tctattatcg ttacaacatt caatcgacca gcaattttat cgattacatt agcctgttta    540
   gtaaaccaaa aaacacatta cccgtttgaa gttatcgtga cagatgatgg tagtcaggaa    600
   gatctatcac cgatcattcg ccaatatgaa aataaattgg atattcgcta cgtcagacaa    660
   aaagataacg gttttcaagc cagtgccgct cggaatatgg gattacgctt agcaaaatat    720
   gactttattg gcttactcaa ctgtgatatg gcgccaaatc cattatgggt tcattcttat    780
   gttgcagagc tattagaaga tgatgattta acaatcattg gtccaagaaa atacatcgat    840
   acacaacata ttgacccaaa agacttctta aataacgcga gtttgcttga atcattacca    900
   gaagtgaaaa ccaataatag tgttgccgca aaaggggaag gaacagtttc tctggattgg    960
   cgcttagaac aattcgaaaa aacagaaaat ctccgcttat ccgattcgcc tttccgtttt   1020
   tttgcggcgg gtaatgttgc tttcgctaaa aaatggctaa ataaatccgg tttctttgat   1080
   gaggaattta atcactgggg tggagaagat gtggaatttg gatatcgctt attccgttac   1140
   ggtagtttct ttaaaactat tgatggcatt atggcctacc atcaagagcc accaggtaaa   1200
   gaaaatgaaa ccgatcgtga agcgggaaaa aatattacgc tcgatattat gagagaaaag   1260
   gtcccttata tctatagaaa acttttacca atagaagatt cgcatatcaa tagagtacct   1320
   ttagtttcaa tttatatccc agcttataac tgtgcaaact atattcaacg ttgcgtagat   1380
   agtgcactga atcagactgt tgttgatctc gaggtttgta tttgtaacga tggttcaaca 1440
   gataatacct tagaagtgat caataagctt tatggtaata atcctagggt acgcatcatg 1500
   tctaaaccaa atggcggaat agcctcagca tcaaatgcag ccgtttcttt tgctaaaggt 1560
   tattacattg ggcagttaga ttcagatgat tatcttgagc ctgatgcagt tgaactgtgt 1620
   ttaaaagaat ttttaaaaga taaaacgcta gcttgtgttt ataccactaa tagaaacgtc 1680
   aatccggatg gtagcttaat cgctaatggt tacaattggc cagaattttc acgagaaaaa 1740
   ctcacaacgg ctatgattgc tcaccacttt agaatgttca cgattagagc ttggcattta 1800
   actgatggat tcaatgaaaa aattgaaaat gccgtagact atgacatgtt cctcaaactc 1860
   agtgaagttg gaaaatttaa acatcttaat aaaatctgct ataaccgtgt attacatggt 1920
   gataacacat caattaagaa acttggcatt caaaagaaaa accattttgt tgtagtcaat 1980
   cagtcattaa atagacaagg cataacttat tataattatg acgaatttga tgatttagat 2040
   gaaagtagaa agtatatttt caataaaacc gctgaatatc aagaagagat tgatatctta 2100
   aaagatattt aa 2112
<210> 31
   <211> 2112
   <212> DNA
   <213> Pasteurella multocida
<400> 31
   atgaatacat tatcacaagc aataaaagca tataacagca atgactatca attagcactc     60
   aaattatttg aaaagtcggc ggaaatctat ggacggaaaa ttgttgaatt tcaaattacc    120
   aaatgcaaag aaaaactctc agcacatcct tctgttaatt cagcacatct ttctgtaaat    180
   aaagaagaaa aagtcaatgt ttgcgatagt ccgttagata ttgcaacaca actgttactt    240
   tccaacgtaa aaaaattagt actttctgac tcggaaaaaa acacgttaaa aaataaatgg    300
   aaattgctca ctgagaagaa atctgaaaat gcggaggtaa gagcggtcgc ccttgtacca    360
   aaagattttc ccaaagatct ggttttagcg cctttacctg atcatgttaa tgattttaca    420
   tggtacaaaa agcgaaagaa aagacttggc ataaaacctg aacatcaaca tgttggtctt    480
   tctattatcg ttacaacatt caatcgacca gcaattttat cgattacatt agcctgttta    540
   gtaaaccaaa aaacacatta cccgtttgaa gttatcgtga cagatgatgg tagtcaggaa    600
   gatctatcac cgatcattcg ccaatatgaa aataaattgg atattcgcta cgtcagacaa    660
   aaagataacg gttttcaagc cagtgccgct cggaatatgg gattacgctt agcaaaatat    720
   gactttattg gcttactcaa atgtgatatg gcgccaaatc cattatgggt tcattcttat    780
   gttgcagagc tattagaaga tgatgattta acaatcattg gtccaagaaa atacatcgat    840
   acacaacata ttgacccaaa agacttctta aataacgcga gtttgcttga atcattacca    900
   gaagtgaaaa ccaataatag tgttgccgca aaaggggaag gaacagtttc tctggattgg 960
   cgcttagaac aattcgaaaa aacagaaaat ctccgcttat ccgattcgcc tttccgtttt 1020
   tttgcggcgg gtaatgttgc tttcgctaaa aaatggctaa ataaatccgg tttctttgat 1080
   gaggaattta atcactgggg tggagaagat gtggaatttg gatatcgctt attccgttac 1140
   ggtagtttct ttaaaactat tgatggcatt atggcctacc atcaagagcc accaggtaaa 1200
   gaaaatgaaa ccgatcgtga agcgggaaaa aatattacgc tcgatattat gagagaaaag 1260
   gtcccttata tctatagaaa acttttacca atagaagatt cgcatatcaa tagagtacct 1320
   ttagtttcaa tttatatccc agcttataac tgtgcaaact atattcaacg ttgcgtagat 1380
   agtgcactga atcagactgt tgttgatctc gaggtttgta tttgtaacga tggttcaaca 1440
   gataatacct tagaagtgat caataagctt tatggtaata atcctagggt acgcatcatg 1500
   tctaaaccaa atggcggaat agcctcagca tcaaatgcag ccgtttcttt tgctaaaggt 1560
   tattacattg ggcagttaga ttcagatgat tatcttgagc ctgatgcagt tgaactgtgt 1620
   ttaaaagaat ttttaaaaga taaaacgcta gcttgtgttt ataccactaa tagaaacgtc 1680
   aatccggatg gtagcttaat cgctaatggt tacaattggc cagaattttc acgagaaaaa 1740
   ctcacaacgg ctatgattgc tcaccacttt agaatgttca cgattagagc ttggcattta 1800
   actgatggat tcaatgaaaa aattgaaaat gccgtagact atgacatgtt cctcaaactc 1860
   agtgaagttg gaaaatttaa acatcttaat aaaatctgct ataaccgtgt attacatggt 1920
   gataacacat caattaagaa acttggcatt caaaagaaaa accattttgt tgtagtcaat 1980
   cagtcattaa atagacaagg cataacttat tataattatg acgaatttga tgatttagat 2040
   gaaagtagaa agtatatttt caataaaacc gctgaatatc aagaagagat tgatatctta 2100
   aaagatattt aa 2112
<210> 32
   <211> 2112
   <212> DNA
   <213> Pasteurella multocida
<400> 32
   atgaatacat tatcacaagc aataaaagca tataacagca atgactatca attagcactc 60
   aaattatttg aaaagtcggc ggaaatctat ggacggaaaa ttgttgaatt tcaaattacc 120
   aaatgcaaag aaaaactctc agcacatcct tctgttaatt cagcacatct ttctgtaaat 180
   aaagaagaaa aagtcaatgt ttgcgatagt ccgttagata ttgcaacaca actgttactt 240
   tccaacgtaa aaaaattagt actttctgac tcggaaaaaa acacgttaaa aaataaatgg 300
   aaattgctca ctgagaagaa atctgaaaat gcggaggtaa gagcggtcgc ccttgtacca 360
   aaagattttc ccaaagatct ggttttagcg cctttacctg atcatgttaa tgattttaca 420
   tggtacaaaa agcgaaagaa aagacttggc ataaaacctg aacatcaaca tgttggtctt     480
   tctattatcg ttacaacatt caatcgacca gcaattttat cgattacatt agcctgttta     540
   gtaaaccaaa aaacacatta cccgtttgaa gttatcgtga cagatgatgg tagtcaggaa     600
   gatctatcac cgatcattcg ccaatatgaa aataaattgg atattcgcta cgtcagacaa     660
   aaagataacg gttttcaagc cagtgccgct cggaatatgg gattacgctt agcaaaatat     720
   gactttattg gcttactcga ctgtgaaatg gcgccaaatc cattatgggt tcattcttat     780
   gttgcagagc tattagaaga tgatgattta acaatcattg gtccaagaaa atacatcgat     840
   acacaacata ttgacccaaa agacttctta aataacgcga gtttgcttga atcattacca     900
   gaagtgaaaa ccaataatag tgttgccgca aaaggggaag gaacagtttc tctggattgg     960
   cgcttagaac aattcgaaaa aacagaaaat ctccgcttat ccgattcgcc tttccgtttt    1020
   tttgcggcgg gtaatgttgc tttcgctaaa aaatggctaa ataaatccgg tttctttgat    1080
   gaggaattta atcactgggg tggagaagat gtggaatttg gatatcgctt attccgttac    1140
   ggtagtttct ttaaaactat tgatggcatt atggcctacc atcaagagcc accaggtaaa    1200
   gaaaatgaaa ccgatcgtga agcgggaaaa aatattacgc tcgatattat gagagaaaag    1260
   gtcccttata tctatagaaa acttttacca atagaagatt cgcatatcaa tagagtacct    1320
   ttagtttcaa tttatatccc agcttataac tgtgcaaact atattcaacg ttgcgtagat    1380
   agtgcactga atcagactgt tgttgatctc gaggtttgta tttgtaacga tggttcaaca    1440
   gataatacct tagaagtgat caataagctt tatggtaata atcctagggt acgcatcatg    1500
   tctaaaccaa atggcggaat agcctcagca tcaaatgcag ccgtttcttt tgctaaaggt    1560
   tattacattg ggcagttaga ttcagatgat tatcttgagc ctgatgcagt tgaactgtgt    1620
   ttaaaagaat ttttaaaaga taaaacgcta gcttgtgttt ataccactaa tagaaacgtc    1680
   aatccggatg gtagcttaat cgctaatggt tacaattggc cagaattttc acgagaaaaa    1740
   ctcacaacgg ctatgattgc tcaccacttt agaatgttca cgattagagc ttggcattta    1800
   actgatggat tcaatgaaaa aattgaaaat gccgtagact atgacatgtt cctcaaactc    1860
   agtgaagttg gaaaatttaa acatcttaat aaaatctgct ataaccgtgt attacatggt    1920
   gataacacat caattaagaa acttggcatt caaaagaaaa accattttgt tgtagtcaat    1980
   cagtcattaa atagacaagg cataacttat tataattatg acgaatttga tgatttagat    2040
   gaaagtagaa agtatatttt caataaaacc gctgaatatc aagaagagat tgatatctta    2100
   aaagatattt aa                                                        2112
<210> 33
   <211> 2112
   <212> DNA
   <213> Pasteurella multocida
<400> 33
   atgaatacat tatcacaagc aataaaagca tataacagca atgactatca attagcactc     60
   aaattatttg aaaagtcggc ggaaatctat ggacggaaaa ttgttgaatt tcaaattacc    120
   aaatgcaaag aaaaactctc agcacatcct tctgttaatt cagcacatct ttctgtaaat    180
   aaagaagaaa aagtcaatgt ttgcgatagt ccgttagata ttgcaacaca actgttactt    240
   tccaacgtaa aaaaattagt actttctgac tcggaaaaaa acacgttaaa aaataaatgg    300
   aaattgctca ctgagaagaa atctgaaaat gcggaggtaa gagcggtcgc ccttgtacca    360
   aaagattttc ccaaagatct ggttttagcg cctttacctg atcatgttaa tgattttaca    420
   tggtacaaaa agcgaaagaa aagacttggc ataaaacctg aacatcaaca tgttggtctt    480
   tctattatcg ttacaacatt caatcgacca gcaattttat cgattacatt agcctgttta    540
   gtaaaccaaa aaacacatta cccgtttgaa gttatcgtga cagatgatgg tagtcaggaa    600
   gatctatcac cgatcattcg ccaatatgaa aataaattgg atattcgcta cgtcagacaa    660
   aaagataacg gttttcaagc cagtgccgct cggaatatgg gattacgctt agcaaaatat    720
   gactttattg gcttactcga ctgtaatatg gcgccaaatc cattatgggt tcattcttat    780
   gttgcagagc tattagaaga tgatgattta acaatcattg gtccaagaaa atacatcgat    840
   acacaacata ttgacccaaa agacttctta aataacgcga gtttgcttga atcattacca    900
   gaagtgaaaa ccaataatag tgttgccgca aaaggggaag gaacagtttc tctggattgg    960
   cgcttagaac aattcgaaaa aacagaaaat ctccgcttat ccgattcgcc tttccgtttt   1020
   tttgcggcgg gtaatgttgc tttcgctaaa aaatggctaa ataaatccgg tttctttgat   1080
   gaggaattta atcactgggg tggagaagat gtggaatttg gatatcgctt attccgttac   1140
   ggtagtttct ttaaaactat tgatggcatt atggcctacc atcaagagcc accaggtaaa   1200
   gaaaatgaaa ccgatcgtga agcgggaaaa aatattacgc tcgatattat gagagaaaag   1260
   gtcccttata tctatagaaa acttttacca atagaagatt cgcatatcaa tagagtacct   1320
   ttagtttcaa tttatatccc agcttataac tgtgcaaact atattcaacg ttgcgtagat   1380
   agtgcactga atcagactgt tgttgatctc gaggtttgta tttgtaacga tggttcaaca   1440
   gataatacct tagaagtgat caataagctt tatggtaata atcctagggt acgcatcatg   1500
   tctaaaccaa atggcggaat agcctcagca tcaaatgcag ccgtttcttt tgctaaaggt   1560
   tattacattg ggcagttaga ttcagatgat tatcttgagc ctgatgcagt tgaactgtgt   1620
   ttaaaagaat ttttaaaaga taaaacgcta gcttgtgttt ataccactaa tagaaacgtc   1680
   aatccggatg gtagcttaat cgctaatggt tacaattggc cagaattttc acgagaaaaa   1740
   ctcacaacgg ctatgattgc tcaccacttt agaatgttca cgattagagc ttggcattta   1800
   actgatggat tcaatgaaaa aattgaaaat gccgtagact atgacatgtt cctcaaactc   1860
   agtgaagttg gaaaatttaa acatcttaat aaaatctgct ataaccgtgt attacatggt   1920
   gataacacat caattaagaa acttggcatt caaaagaaaa accattttgt tgtagtcaat   1980
   cagtcattaa atagacaagg cataacttat tataattatg acgaatttga tgatttagat   2040
   gaaagtagaa agtatatttt caataaaacc gctgaatatc aagaagagat tgatatctta   2100
   aaagatattt aa                                                       2112
<210> 34
   <211> 2112
   <212> DNA
   <213> Pasteurella multocida
<400> 34
   atgaatacat tatcacaagc aataaaagca tataacagca atgactatca attagcactc     60
   aaattatttg aaaagtcggc ggaaatctat ggacggaaaa ttgttgaatt tcaaattacc    120
   aaatgcaaag aaaaactctc agcacatcct tctgttaatt cagcacatct ttctgtaaat    180
   aaagaagaaa aagtcaatgt ttgcgatagt ccgttagata ttgcaacaca actgttactt    240
   tccaacgtaa aaaaattagt actttctgac tcggaaaaaa acacgttaaa aaataaatgg    300
   aaattgctca ctgagaagaa atctgaaaat gcggaggtaa gagcggtcgc ccttgtacca    360
   aaagattttc ccaaagatct ggttttagcg cctttacctg atcatgttaa tgattttaca    420
   tggtacaaaa agcgaaagaa aagacttggc ataaaacctg aacatcaaca tgttggtctt    480
   tctattatcg ttacaacatt caatcgacca gcaattttat cgattacatt agcctgttta    540
   gtaaaccaaa aaacacatta cccgtttgaa gttatcgtga cagatgatgg tagtcaggaa    600
   gatctatcac cgatcattcg ccaatatgaa aataaattgg atattcgcta cgtcagacaa    660
   aaagataacg gttttcaagc cagtgccgct cggaatatgg gattacgctt agcaaaatat    720
   gactttattg gcttactcga ctgtaaaatg gcgccaaatc cattatgggt tcattcttat    780
   gttgcagagc tattagaaga tgatgattta acaatcattg gtccaagaaa atacatcgat    840
   acacaacata ttgacccaaa agacttctta aataacgcga gtttgcttga atcattacca    900
   gaagtgaaaa ccaataatag tgttgccgca aaaggggaag gaacagtttc tctggattgg    960
   cgcttagaac aattcgaaaa aacagaaaat ctccgcttat ccgattcgcc tttccgtttt   1020
   tttgcggcgg gtaatgttgc tttcgctaaa aaatggctaa ataaatccgg tttctttgat   1080
   gaggaattta atcactgggg tggagaagat gtggaatttg gatatcgctt attccgttac   1140
   ggtagtttct ttaaaactat tgatggcatt atggcctacc atcaagagcc accaggtaaa   1200
   gaaaatgaaa ccgatcgtga agcgggaaaa aatattacgc tcgatattat gagagaaaag   1260
   gtcccttata tctatagaaa acttttacca atagaagatt cgcatatcaa tagagtacct   1320
   ttagtttcaa tttatatccc agcttataac tgtgcaaact atattcaacg ttgcgtagat   1380
   agtgcactga atcagactgt tgttgatctc gaggtttgta tttgtaacga tggttcaaca   1440
   gataatacct tagaagtgat caataagctt tatggtaata atcctagggt acgcatcatg   1500
   tctaaaccaa atggcggaat agcctcagca tcaaatgcag ccgtttcttt tgctaaaggt   1560
   tattacattg ggcagttaga ttcagatgat tatcttgagc ctgatgcagt tgaactgtgt   1620
   ttaaaagaat ttttaaaaga taaaacgcta gcttgtgttt ataccactaa tagaaacgtc   1680
   aatccggatg gtagcttaat cgctaatggt tacaattggc cagaattttc acgagaaaaa   1740
   ctcacaacgg ctatgattgc tcaccacttt agaatgttca cgattagagc ttggcattta   1800
   actgatggat tcaatgaaaa aattgaaaat gccgtagact atgacatgtt cctcaaactc   1860
   agtgaagttg gaaaatttaa acatcttaat aaaatctgct ataaccgtgt attacatggt   1920
   gataacacat caattaagaa acttggcatt caaaagaaaa accattttgt tgtagtcaat   1980
   cagtcattaa atagacaagg cataacttat tataattatg acgaatttga tgatttagat   2040
   gaaagtagaa agtatatttt caataaaacc gctgaatatc aagaagagat tgatatctta   2100
   aaagatattt aa                                                        2112
<210> 35
   <211> 2112
   <212> DNA
   <213> Pasteurella multocida
<400> 35
   atgaatacat tatcacaagc aataaaagca tataacagca atgactatca attagcactc      60
   aaattatttg aaaagtcggc ggaaatctat ggacggaaaa ttgttgaatt tcaaattacc     120
   aaatgcaaag aaaaactctc agcacatcct tctgttaatt cagcacatct ttctgtaaat     180
   aaagaagaaa aagtcaatgt ttgcgatagt ccgttagata ttgcaacaca actgttactt     240
   tccaacgtaa aaaaattagt actttctgac tcggaaaaaa acacgttaaa aaataaatgg     300
   aaattgctca ctgagaagaa atctgaaaat gcggaggtaa gagcggtcgc ccttgtacca     360
   aaagattttc ccaaagatct ggttttagcg cctttacctg atcatgttaa tgattttaca     420
   tggtacaaaa agcgaaagaa aagacttggc ataaaacctg aacatcaaca tgttggtctt     480
   tctattatcg ttacaacatt caatcgacca gcaattttat cgattacatt agcctgttta     540
   gtaaaccaaa aaacacatta cccgtttgaa gttatcgtga cagatgatgg tagtcaggaa     600
   gatctatcac cgatcattcg ccaatatgaa aataaattgg atattcgcta cgtcagacaa     660
   aaagataacg gttttcaagc cagtgccgct cggaatatgg gattacgctt agcaaaatat     720
   gactttattg gcttactcga ctgtgatatg gcgccaaatc cattatgggt tcattcttat     780
   gttgcagagc tattagaaga tgatgattta acaatcattg gtccaagaaa atacatcgat     840
   acacaacata ttgacccaaa agacttctta aataacgcga gtttgcttga atcattacca     900
   gaagtgaaaa ccaataatag tgttgccgca aaaggggaag gaacagtttc tctggattgg 960
   cgcttagaac aattcgaaaa aacagaaaat ctccgcttat ccgattcgcc tttccgtttt 1020
   tttgcggcgg gtaatgttgc tttcgctaaa aaatggctaa ataaatccgg tttctttgat 1080
   gaggaattta atcactgggg tggagaagat gtggaatttg gatatcgctt attccgttac 1140
   ggtagtttct ttaaaactat tgatggcatt atggcctacc atcaagagcc accaggtaaa 1200
   gaaaatgaaa ccgatcgtga agcgggaaaa aatattacgc tcgatattat gagagaaaag 1260
   gtcccttata tctatagaaa acttttacca atagaagatt cgcatatcaa tagagtacct 1320
   ttagtttcaa tttatatccc agcttataac tgtgcaaact atattcaacg ttgcgtagat 1380
   agtgcactga atcagactgt tgttgatctc gaggtttgta tttgtaacga tggttcaaca 1440
   gataatacct tagaagtgat caataagctt tatggtaata atcctagggt acgcatcatg 1500
   tctaaaccaa atggcggaat agcctcagca tcaaatgcag ccgtttcttt tgctaaaggt 1560
   tattacattg ggcagttaaa ttcagatgat tatcttgagc ctgatgcagt tgaactgtgt 1620
   ttaaaagaat ttttaaaaga taaaacgcta gcttgtgttt ataccactaa tagaaacgtc 1680
   aatccggatg gtagcttaat cgctaatggt tacaattggc cagaattttc acgagaaaaa 1740
   ctcacaacgg ctatgattgc tcaccacttt agaatgttca cgattagagc ttggcattta 1800
   actgatggat tcaatgaaaa aattgaaaat gccgtagact atgacatgtt cctcaaactc 1860
   agtgaagttg gaaaatttaa acatcttaat aaaatctgct ataaccgtgt attacatggt 1920
   gataacacat caattaagaa acttggcatt caaaagaaaa accattttgt tgtagtcaat 1980
   cagtcattaa atagacaagg cataacttat tataattatg acgaatttga tgatttagat 2040
   gaaagtagaa agtatatttt caataaaacc gctgaatatc aagaagagat tgatatctta 2100
   aaagatattt aa 2112
<210> 36
   <211> 2112
   <212> DNA
   <213> Pasteurella multocida
<400> 36
   atgaatacat tatcacaagc aataaaagca tataacagca atgactatca attagcactc     60
   aaattatttg aaaagtcggc ggaaatctat ggacggaaaa ttgttgaatt tcaaattacc    120
   aaatgcaaag aaaaactctc agcacatcct tctgttaatt cagcacatct ttctgtaaat    180
   aaagaagaaa aagtcaatgt ttgcgatagt ccgttagata ttgcaacaca actgttactt    240
   tccaacgtaa aaaaattagt actttctgac tcggaaaaaa acacgttaaa aaataaatgg    300
   aaattgctca ctgagaagaa atctgaaaat gcggaggtaa gagcggtcgc ccttgtacca    360
   aaagattttc ccaaagatct ggttttagcg cctttacctg atcatgttaa tgattttaca    420
   tggtacaaaa agcgaaagaa aagacttggc ataaaacctg aacatcaaca tgttggtctt 480
   tctattatcg ttacaacatt caatcgacca gcaattttat cgattacatt agcctgttta 540
   gtaaaccaaa aaacacatta cccgtttgaa gttatcgtga cagatgatgg tagtcaggaa 600
   gatctatcac cgatcattcg ccaatatgaa aataaattgg atattcgcta cgtcagacaa 660
   aaagataacg gttttcaagc cagtgccgct cggaatatgg gattacgctt agcaaaatat 720
   gactttattg gcttactcga ctgtgatatg gcgccaaatc cattatgggt tcattcttat 780
   gttgcagagc tattagaaga tgatgattta acaatcattg gtccaagaaa atacatcgat 840
   acacaacata ttgacccaaa agacttctta aataacgcga gtttgcttga atcattacca 900
   gaagtgaaaa ccaataatag tgttgccgca aaaggggaag gaacagtttc tctggattgg 960
   cgcttagaac aattcgaaaa aacagaaaat ctccgcttat ccgattcgcc tttccgtttt 1020
   tttgcggcgg gtaatgttgc tttcgctaaa aaatggctaa ataaatccgg tttctttgat 1080
   gaggaattta atcactgggg tggagaagat gtggaatttg gatatcgctt attccgttac 1140
   ggtagtttct ttaaaactat tgatggcatt atggcctacc atcaagagcc accaggtaaa 1200
   gaaaatgaaa ccgatcgtga agcgggaaaa aatattacgc tcgatattat gagagaaaag 1260
   gtcccttata tctatagaaa acttttacca atagaagatt cgcatatcaa tagagtacct 1320
   ttagtttcaa tttatatccc agcttataac tgtgcaaact atattcaacg ttgcgtagat 1380
   agtgcactga atcagactgt tgttgatctc gaggtttgta tttgtaacga tggttcaaca 1440
   gataatacct tagaagtgat caataagctt tatggtaata atcctagggt acgcatcatg 1500
   tctaaaccaa atggcggaat agcctcagca tcaaatgcag ccgtttcttt tgctaaaggt 1560
   tattacattg ggcagttaga atcagatgat tatcttgagc ctgatgcagt tgaactgtgt 1620
   ttaaaagaat ttttaaaaga taaaacgcta gcttgtgttt ataccactaa tagaaacgtc 1680
   aatccggatg gtagcttaat cgctaatggt tacaattggc cagaattttc acgagaaaaa 1740
   ctcacaacgg ctatgattgc tcaccacttt agaatgttca cgattagagc ttggcattta 1800
   actgatggat tcaatgaaaa aattgaaaat gccgtagact atgacatgtt cctcaaactc 1860
   agtgaagttg gaaaatttaa acatcttaat aaaatctgct ataaccgtgt attacatggt 1920
   gataacacat caattaagaa acttggcatt caaaagaaaa accattttgt tgtagtcaat 1980
   cagtcattaa atagacaagg cataacttat tataattatg acgaatttga tgatttagat 2040
   gaaagtagaa agtatatttt caataaaacc gctgaatatc aagaagagat tgatatctta 2100
   aaagatattt aa 2112
<210> 37
   <211> 2112
   <212> DNA
   <213> Pasteurella multocida
<400> 37
   atgaatacat tatcacaagc aataaaagca tataacagca atgactatca attagcactc     60
   aaattatttg aaaagtcggc ggaaatctat ggacggaaaa ttgttgaatt tcaaattacc    120
   aaatgcaaag aaaaactctc agcacatcct tctgttaatt cagcacatct ttctgtaaat    180
   aaagaagaaa aagtcaatgt ttgcgatagt ccgttagata ttgcaacaca actgttactt    240
   tccaacgtaa aaaaattagt actttctgac tcggaaaaaa acacgttaaa aaataaatgg    300
   aaattgctca ctgagaagaa atctgaaaat gcggaggtaa gagcggtcgc ccttgtacca    360
   aaagattttc ccaaagatct ggttttagcg cctttacctg atcatgttaa tgattttaca    420
   tggtacaaaa agcgaaagaa aagacttggc ataaaacctg aacatcaaca tgttggtctt    480
   tctattatcg ttacaacatt caatcgacca gcaattttat cgattacatt agcctgttta    540
   gtaaaccaaa aaacacatta cccgtttgaa gttatcgtga cagatgatgg tagtcaggaa    600
   gatctatcac cgatcattcg ccaatatgaa aataaattgg atattcgcta cgtcagacaa    660
   aaagataacg gttttcaagc cagtgccgct cggaatatgg gattacgctt agcaaaatat    720
   gactttattg gcttactcga ctgtgatatg gcgccaaatc cattatgggt tcattcttat    780
   gttgcagagc tattagaaga tgatgattta acaatcattg gtccaagaaa atacatcgat    840
   acacaacata ttgacccaaa agacttctta aataacgcga gtttgcttga atcattacca    900
   gaagtgaaaa ccaataatag tgttgccgca aaaggggaag gaacagtttc tctggattgg    960
   cgcttagaac aattcgaaaa aacagaaaat ctccgcttat ccgattcgcc tttccgtttt   1020
   tttgcggcgg gtaatgttgc tttcgctaaa aaatggctaa ataaatccgg tttctttgat   1080
   gaggaattta atcactgggg tggagaagat gtggaatttg gatatcgctt attccgttac   1140
   ggtagtttct ttaaaactat tgatggcatt atggcctacc atcaagagcc accaggtaaa   1200
   gaaaatgaaa ccgatcgtga agcgggaaaa aatattacgc tcgatattat gagagaaaag   1260
   gtcccttata tctatagaaa acttttacca atagaagatt cgcatatcaa tagagtacct   1320
   ttagtttcaa tttatatccc agcttataac tgtgcaaact atattcaacg ttgcgtagat   1380
   agtgcactga atcagactgt tgttgatctc gaggtttgta tttgtaacga tggttcaaca   1440
   gataatacct tagaagtgat caataagctt tatggtaata atcctagggt acgcatcatg   1500
   tctaaaccaa atggcggaat agcctcagca tcaaatgcag ccgtttcttt tgctaaaggt   1560
   tattacattg ggcagttaaa atcagatgat tatcttgagc ctgatgcagt tgaactgtgt   1620
   ttaaaagaat ttttaaaaga taaaacgcta gcttgtgttt ataccactaa tagaaacgtc   1680
   aatccggatg gtagcttaat cgctaatggt tacaattggc cagaattttc acgagaaaaa   1740
   ctcacaacgg ctatgattgc tcaccacttt agaatgttca cgattagagc ttggcattta   1800
   actgatggat tcaatgaaaa aattgaaaat gccgtagact atgacatgtt cctcaaactc   1860
   agtgaagttg gaaaatttaa acatcttaat aaaatctgct ataaccgtgt attacatggt   1920
   gataacacat caattaagaa acttggcatt caaaagaaaa accattttgt tgtagtcaat   1980
   cagtcattaa atagacaagg cataacttat tataattatg acgaatttga tgatttagat   2040
   gaaagtagaa agtatatttt caataaaacc gctgaatatc aagaagagat tgatatctta   2100
   aaagatattt aa                                                       2112
<210> 38
   <211> 2112
   <212> DNA
   <213> Pasteurella multocida
<400> 38
   atgaatacat tatcacaagc aataaaagca tataacagca atgactatca attagcactc     60
   aaattatttg aaaagtcggc ggaaatctat ggacggaaaa ttgttgaatt tcaaattacc    120
   aaatgcaaag aaaaactctc agcacatcct tctgttaatt cagcacatct ttctgtaaat    180
   aaagaagaaa aagtcaatgt ttgcgatagt ccgttagata ttgcaacaca actgttactt    240
   tccaacgtaa aaaaattagt actttctgac tcggaaaaaa acacgttaaa aaataaatgg    300
   aaattgctca ctgagaagaa atctgaaaat gcggaggtaa gagcggtcgc ccttgtacca    360
   aaagattttc ccaaagatct ggttttagcg cctttacctg atcatgttaa tgattttaca    420
   tggtacaaaa agcgaaagaa aagacttggc ataaaacctg aacatcaaca tgttggtctt    480
   tctattatcg ttacaacatt caatcgacca gcaattttat cgattacatt agcctgttta    540
   gtaaaccaaa aaacacatta cccgtttgaa gttatcgtga cagatgatgg tagtcaggaa    600
   gatctatcac cgatcattcg ccaatatgaa aataaattgg atattcgcta cgtcagacaa    660
   aaagataacg gttttcaagc cagtgccgct cggaatatgg gattacgctt agcaaaatat    720
   gactttattg gcttactcga ctgtgatatg gcgccaaatc cattatgggt tcattcttat    780
   gttgcagagc tattagaaga tgatgattta acaatcattg gtccaagaaa atacatcgat    840
   acacaacata ttgacccaaa agacttctta aataacgcga gtttgcttga atcattacca    900
   gaagtgaaaa ccaataatag tgttgccgca aaaggggaag gaacagtttc tctggattgg    960
   cgcttagaac aattcgaaaa aacagaaaat ctccgcttat ccgattcgcc tttccgtttt   1020
   tttgcggcgg gtaatgttgc tttcgctaaa aaatggctaa ataaatccgg tttctttgat   1080
   gaggaattta atcactgggg tggagaagat gtggaatttg gatatcgctt attccgttac   1140
   ggtagtttct ttaaaactat tgatggcatt atggcctacc atcaagagcc accaggtaaa   1200
   gaaaatgaaa ccgatcgtga agcgggaaaa aatattacgc tcgatattat gagagaaaag   1260
   gtcccttata tctatagaaa acttttacca atagaagatt cgcatatcaa tagagtacct   1320
   ttagtttcaa tttatatccc agcttataac tgtgcaaact atattcaacg ttgcgtagat 1380
   agtgcactga atcagactgt tgttgatctc gaggtttgta tttgtaacga tggttcaaca 1440
   gataatacct tagaagtgat caataagctt tatggtaata atcctagggt acgcatcatg 1500
   tctaaaccaa atggcggaat agcctcagca tcaaatgcag ccgtttcttt tgctaaaggt 1560
   tattacattg ggcagttaga ttcagaagat tatcttgagc ctgatgcagt tgaactgtgt 1620
   ttaaaagaat ttttaaaaga taaaacgcta gcttgtgttt ataccactaa tagaaacgtc 1680
   aatccggatg gtagcttaat cgctaatggt tacaattggc cagaattttc acgagaaaaa 1740
   ctcacaacgg ctatgattgc tcaccacttt agaatgttca cgattagagc ttggcattta 1800
   actgatggat tcaatgaaaa aattgaaaat gccgtagact atgacatgtt cctcaaactc 1860
   agtgaagttg gaaaatttaa acatcttaat aaaatctgct ataaccgtgt attacatggt 1920
   gataacacat caattaagaa acttggcatt caaaagaaaa accattttgt tgtagtcaat 1980
   cagtcattaa atagacaagg cataacttat tataattatg acgaatttga tgatttagat 2040
   gaaagtagaa agtatatttt caataaaacc gctgaatatc aagaagagat tgatatctta 2100
   aaagatattt aa 2112
<210> 39
   <211> 2112
   <212> DNA
   <213> Pasteurella multocida
<400> 39
   atgaatacat tatcacaagc aataaaagca tataacagca atgactatca attagcactc 60
   aaattatttg aaaagtcggc ggaaatctat ggacggaaaa ttgttgaatt tcaaattacc 120
   aaatgcaaag aaaaactctc agcacatcct tctgttaatt cagcacatct ttctgtaaat 180
   aaagaagaaa aagtcaatgt ttgcgatagt ccgttagata ttgcaacaca actgttactt 240
   tccaacgtaa aaaaattagt actttctgac tcggaaaaaa acacgttaaa aaataaatgg 300
   aaattgctca ctgagaagaa atctgaaaat gcggaggtaa gagcggtcgc ccttgtacca 360
   aaagattttc ccaaagatct ggttttagcg cctttacctg atcatgttaa tgattttaca 420
   tggtacaaaa agcgaaagaa aagacttggc ataaaacctg aacatcaaca tgttggtctt 480
   tctattatcg ttacaacatt caatcgacca gcaattttat cgattacatt agcctgttta 540
   gtaaaccaaa aaacacatta cccgtttgaa gttatcgtga cagatgatgg tagtcaggaa 600
   gatctatcac cgatcattcg ccaatatgaa aataaattgg atattcgcta cgtcagacaa 660
   aaagataacg gttttcaagc cagtgccgct cggaatatgg gattacgctt agcaaaatat 720
   gactttattg gcttactcga ctgtgatatg gcgccaaatc cattatgggt tcattcttat 780
   gttgcagagc tattagaaga tgatgattta acaatcattg gtccaagaaa atacatcgat 840
   acacaacata ttgacccaaa agacttctta aataacgcga gtttgcttga atcattacca 900
   gaagtgaaaa ccaataatag tgttgccgca aaaggggaag gaacagtttc tctggattgg 960
   cgcttagaac aattcgaaaa aacagaaaat ctccgcttat ccgattcgcc tttccgtttt 1020
   tttgcggcgg gtaatgttgc tttcgctaaa aaatggctaa ataaatccgg tttctttgat 1080
   gaggaattta atcactgggg tggagaagat gtggaatttg gatatcgctt attccgttac 1140
   ggtagtttct ttaaaactat tgatggcatt atggcctacc atcaagagcc accaggtaaa 1200
   gaaaatgaaa ccgatcgtga agcgggaaaa aatattacgc tcgatattat gagagaaaag 1260
   gtcccttata tctatagaaa acttttacca atagaagatt cgcatatcaa tagagtacct 1320
   ttagtttcaa tttatatccc agcttataac tgtgcaaact atattcaacg ttgcgtagat 1380
   agtgcactga atcagactgt tgttgatctc gaggtttgta tttgtaacga tggttcaaca 1440
   gataatacct tagaagtgat caataagctt tatggtaata atcctagggt acgcatcatg 1500
   tctaaaccaa atggcggaat agcctcagca tcaaatgcag ccgtttcttt tgctaaaggt 1560
   tattacattg ggcagttaga ttcaaatgat tatcttgagc ctgatgcagt tgaactgtgt 1620
   ttaaaagaat ttttaaaaga taaaacgcta gcttgtgttt ataccactaa tagaaacgtc 1680
   aatccggatg gtagcttaat cgctaatggt tacaattggc cagaattttc acgagaaaaa 1740
   ctcacaacgg ctatgattgc tcaccacttt agaatgttca cgattagagc ttggcattta 1800
   actgatggat tcaatgaaaa aattgaaaat gccgtagact atgacatgtt cctcaaactc 1860
   agtgaagttg gaaaatttaa acatcttaat aaaatctgct ataaccgtgt attacatggt 1920
   gataacacat caattaagaa acttggcatt caaaagaaaa accattttgt tgtagtcaat 1980
   cagtcattaa atagacaagg cataacttat tataattatg acgaatttga tgatttagat 2040
   gaaagtagaa agtatatttt caataaaacc gctgaatatc aagaagagat tgatatctta 2100
   aaagatattt aa 2112
<210> 40
   <211> 2112
   <212> DNA
   <213> Pasteurella multocida
<400> 40
   atgaatacat tatcacaagc aataaaagca tataacagca atgactatca attagcactc      60
   aaattatttg aaaagtcggc ggaaatctat ggacggaaaa ttgttgaatt tcaaattacc     120
   aaatgcaaag aaaaactctc agcacatcct tctgttaatt cagcacatct ttctgtaaat     180
   aaagaagaaa aagtcaatgt ttgcgatagt ccgttagata ttgcaacaca actgttactt     240
   tccaacgtaa aaaaattagt actttctgac tcggaaaaaa acacgttaaa aaataaatgg     300
   aaattgctca ctgagaagaa atctgaaaat gcggaggtaa gagcggtcgc ccttgtacca     360
   aaagattttc ccaaagatct ggttttagcg cctttacctg atcatgttaa tgattttaca     420
   tggtacaaaa agcgaaagaa aagacttggc ataaaacctg aacatcaaca tgttggtctt     480
   tctattatcg ttacaacatt caatcgacca gcaattttat cgattacatt agcctgttta     540
   gtaaaccaaa aaacacatta cccgtttgaa gttatcgtga cagatgatgg tagtcaggaa     600
   gatctatcac cgatcattcg ccaatatgaa aataaattgg atattcgcta cgtcagacaa     660
   aaagataacg gttttcaagc cagtgccgct cggaatatgg gattacgctt agcaaaatat     720
   gactttattg gcttactcga ctgtgatatg gcgccaaatc cattatgggt tcattcttat     780
   gttgcagagc tattagaaga tgatgattta acaatcattg gtccaagaaa atacatcgat     840
   acacaacata ttgacccaaa agacttctta aataacgcga gtttgcttga atcattacca     900
   gaagtgaaaa ccaataatag tgttgccgca aaaggggaag gaacagtttc tctggattgg     960
   cgcttagaac aattcgaaaa aacagaaaat ctccgcttat ccgattcgcc tttccgtttt    1020
   tttgcggcgg gtaatgttgc tttcgctaaa aaatggctaa ataaatccgg tttctttgat    1080
   gaggaattta atcactgggg tggagaagat gtggaatttg gatatcgctt attccgttac    1140
   ggtagtttct ttaaaactat tgatggcatt atggcctacc atcaagagcc accaggtaaa    1200
   gaaaatgaaa ccgatcgtga agcgggaaaa aatattacgc tcgatattat gagagaaaag    1260
   gtcccttata tctatagaaa acttttacca atagaagatt cgcatatcaa tagagtacct    1320
   ttagtttcaa tttatatccc agcttataac tgtgcaaact atattcaacg ttgcgtagat    1380
   agtgcactga atcagactgt tgttgatctc gaggtttgta tttgtaacga tggttcaaca    1440
   gataatacct tagaagtgat caataagctt tatggtaata atcctagggt acgcatcatg    1500
   tctaaaccaa atggcggaat agcctcagca tcaaatgcag ccgtttcttt tgctaaaggt    1560
   tattacattg ggcagttaga ttcaaaagat tatcttgagc ctgatgcagt tgaactgtgt    1620
   ttaaaagaat ttttaaaaga taaaacgcta gcttgtgttt ataccactaa tagaaacgtc    1680
   aatccggatg gtagcttaat cgctaatggt tacaattggc cagaattttc acgagaaaaa    1740
   ctcacaacgg ctatgattgc tcaccacttt agaatgttca cgattagagc ttggcattta    1800
   actgatggat tcaatgaaaa aattgaaaat gccgtagact atgacatgtt cctcaaactc    1860
   agtgaagttg gaaaatttaa acatcttaat aaaatctgct ataaccgtgt attacatggt    1920
   gataacacat caattaagaa acttggcatt caaaagaaaa accattttgt tgtagtcaat    1980
   cagtcattaa atagacaagg cataacttat tataattatg acgaatttga tgatttagat    2040
   gaaagtagaa agtatatttt caataaaacc gctgaatatc aagaagagat tgatatctta    2100
   aaagatattt aa                                                        2112
<210> 41
   <211> 2112
   <212> DNA
   <213> Pasteurella multocida
<400> 41
   atgaatacat tatcacaagc aataaaagca tataacagca atgactatca attagcactc 60
   aaattatttg aaaagtcggc ggaaatctat ggacggaaaa ttgttgaatt tcaaattacc 120
   aaatgcaaag aaaaactctc agcacatcct tctgttaatt cagcacatct ttctgtaaat 180
   aaagaagaaa aagtcaatgt ttgcgatagt ccgttagata ttgcaacaca actgttactt 240
   tccaacgtaa aaaaattagt actttctgac tcggaaaaaa acacgttaaa aaataaatgg 300
   aaattgctca ctgagaagaa atctgaaaat gcggaggtaa gagcggtcgc ccttgtacca 360
   aaagattttc ccaaagatct ggttttagcg cctttacctg atcatgttaa tgattttaca 420
   tggtacaaaa agcgaaagaa aagacttggc ataaaacctg aacatcaaca tgttggtctt 480
   tctattatcg ttacaacatt caatcgacca gcaattttat cgattacatt agcctgttta 540
   gtaaaccaaa aaacacatta cccgtttgaa gttatcgtga cagatgatgg tagtcaggaa 600
   gatctatcac cgatcattcg ccaatatgaa aataaattgg atattcgcta cgtcagacaa 660
   aaagataacg gttttcaagc cagtgccgct cggaatatgg gattacgctt agcaaaatat 720
   gactttattg gcttactcga ctgtgatatg gcgccaaatc cattatgggt tcattcttat 780
   gttgcagagc tattagaaga tgatgattta acaatcattg gtccaagaaa atacatcgat 840
   acacaacata ttgacccaaa agacttctta aataacgcga gtttgcttga atcattacca 900
   gaagtgaaaa ccaataatag tgttgccgca aaaggggaag gaacagtttc tctggattgg 960
   cgcttagaac aattcgaaaa aacagaaaat ctccgcttat ccgattcgcc tttccgtttt 1020
   tttgcggcgg gtaatgttgc tttcgctaaa aaatggctaa ataaatccgg tttctttgat 1080
   gaggaattta atcactgggg tggagacgat gtggaatttg gatatcgctt attccgttac 1140
   ggtagtttct ttaaaactat tgatggcatt atggcctacc atcaagagcc accaggtaaa 1200
   gaaaatgaaa ccgatcgtga agcgggaaaa aatattacgc tcgatattat gagagaaaag 1260
   gtcccttata tctatagaaa acttttacca atagaagatt cgcatatcaa tagagtacct 1320
   ttagtttcaa tttatatccc agcttataac tgtgcaaact atattcaacg ttgcgtagat 1380
   agtgcactga atcagactgt tgttgatctc gaggtttgta tttgtaacga tggttcaaca 1440
   gataatacct tagaagtgat caataagctt tatggtaata atcctagggt acgcatcatg 1500
   tctaaaccaa atggcggaat agcctcagca tcaaatgcag ccgtttcttt tgctaaaggt 1560
   tattacattg ggcagttaga ttcagatgat tatcttgagc ctgatgcagt tgaactgtgt 1620
   ttaaaagaat ttttaaaaga taaaacgcta gcttgtgttt ataccactaa tagaaacgtc 1680
   aatccggatg gtagcttaat cgctaatggt tacaattggc cagaattttc acgagaaaaa 1740
   ctcacaacgg ctatgattgc tcaccacttt agaatgttca cgattagagc ttggcattta 1800
   actgatggat tcaatgaaaa aattgaaaat gccgtagact atgacatgtt cctcaaactc 1860
   agtgaagttg gaaaatttaa acatcttaat aaaatctgct ataaccgtgt attacatggt 1920
   gataacacat caattaagaa acttggcatt caaaagaaaa accattttgt tgtagtcaat 1980
   cagtcattaa atagacaagg cataacttat tataattatg acgaatttga tgatttagat 2040
   gaaagtagaa agtatatttt caataaaacc gctgaatatc aagaagagat tgatatctta 2100
   aaagatattt aa 2112
<210> 42
   <211> 2112
   <212> DNA
   <213> Pasteurella multocida
<400> 42
   atgaatacat tatcacaagc aataaaagca tataacagca atgactatca attagcactc 60
   aaattatttg aaaagtcggc ggaaatctat ggacggaaaa ttgttgaatt tcaaattacc 120
   aaatgcaaag aaaaactctc agcacatcct tctgttaatt cagcacatct ttctgtaaat 180
   aaagaagaaa aagtcaatgt ttgcgatagt ccgttagata ttgcaacaca actgttactt 240
   tccaacgtaa aaaaattagt actttctgac tcggaaaaaa acacgttaaa aaataaatgg 300
   aaattgctca ctgagaagaa atctgaaaat gcggaggtaa gagcggtcgc ccttgtacca 360
   aaagattttc ccaaagatct ggttttagcg cctttacctg atcatgttaa tgattttaca 420
   tggtacaaaa agcgaaagaa aagacttggc ataaaacctg aacatcaaca tgttggtctt 480
   tctattatcg ttacaacatt caatcgacca gcaattttat cgattacatt agcctgttta 540
   gtaaaccaaa aaacacatta cccgtttgaa gttatcgtga cagatgatgg tagtcaggaa 600
   gatctatcac cgatcattcg ccaatatgaa aataaattgg atattcgcta cgtcagacaa 660
   aaagataacg gttttcaagc cagtgccgct cggaatatgg gattacgctt agcaaaatat 720
   gactttattg gcttactcga ctgtgatatg gcgccaaatc cattatgggt tcattcttat 780
   gttgcagagc tattagaaga tgatgattta acaatcattg gtccaagaaa atacatcgat 840
   acacaacata ttgacccaaa agacttctta aataacgcga gtttgcttga atcattacca 900
   gaagtgaaaa ccaataatag tgttgccgca aaaggggaag gaacagtttc tctggattgg 960
   cgcttagaac aattcgaaaa aacagaaaat ctccgcttat ccgattcgcc tttccgtttt 1020
   tttgcggcgg gtaatgttgc tttcgctaaa aaatggctaa ataaatccgg tttctttgat 1080
   gaggaattta atcactgggg tggacaagat gtggaatttg gatatcgctt attccgttac 1140
   ggtagtttct ttaaaactat tgatggcatt atggcctacc atcaagagcc accaggtaaa 1200
   gaaaatgaaa ccgatcgtga agcgggaaaa aatattacgc tcgatattat gagagaaaag 1260
   gtcccttata tctatagaaa acttttacca atagaagatt cgcatatcaa tagagtacct 1320
   ttagtttcaa tttatatccc agcttataac tgtgcaaact atattcaacg ttgcgtagat   1380
   agtgcactga atcagactgt tgttgatctc gaggtttgta tttgtaacga tggttcaaca   1440
   gataatacct tagaagtgat caataagctt tatggtaata atcctagggt acgcatcatg   1500
   tctaaaccaa atggcggaat agcctcagca tcaaatgcag ccgtttcttt tgctaaaggt   1560
   tattacattg ggcagttaga ttcagatgat tatcttgagc ctgatgcagt tgaactgtgt   1620
   ttaaaagaat ttttaaaaga taaaacgcta gcttgtgttt ataccactaa tagaaacgtc   1680
   aatccggatg gtagcttaat cgctaatggt tacaattggc cagaattttc acgagaaaaa   1740
   ctcacaacgg ctatgattgc tcaccacttt agaatgttca cgattagagc ttggcattta   1800
   actgatggat tcaatgaaaa aattgaaaat gccgtagact atgacatgtt cctcaaactc   1860
   agtgaagttg gaaaatttaa acatcttaat aaaatctgct ataaccgtgt attacatggt   1920
   gataacacat caattaagaa acttggcatt caaaagaaaa accattttgt tgtagtcaat   1980
   cagtcattaa atagacaagg cataacttat tataattatg acgaatttga tgatttagat   2040
   gaaagtagaa agtatatttt caataaaacc gctgaatatc aagaagagat tgatatctta   2100
   aaagatattt aa                                                       2112
<210> 43
   <211> 2112
   <212> DNA
   <213> Pasteurella multocida
<400> 43
   atgaatacat tatcacaagc aataaaagca tataacagca atgactatca attagcactc    60
   aaattatttg aaaagtcggc ggaaatctat ggacggaaaa ttgttgaatt tcaaattacc   120
   aaatgcaaag aaaaactctc agcacatcct tctgttaatt cagcacatct ttctgtaaat   180
   aaagaagaaa aagtcaatgt ttgcgatagt ccgttagata ttgcaacaca actgttactt   240
   tccaacgtaa aaaaattagt actttctgac tcggaaaaaa acacgttaaa aaataaatgg   300
   aaattgctca ctgagaagaa atctgaaaat gcggaggtaa gagcggtcgc ccttgtacca   360
   aaagattttc ccaaagatct ggttttagcg cctttacctg atcatgttaa tgattttaca   420
   tggtacaaaa agcgaaagaa aagacttggc ataaaacctg aacatcaaca tgttggtctt   480
   tctattatcg ttacaacatt caatcgacca gcaattttat cgattacatt agcctgttta   540
   gtaaaccaaa aaacacatta cccgtttgaa gttatcgtga cagatgatgg tagtcaggaa   600
   gatctatcac cgatcattcg ccaatatgaa aataaattgg atattcgcta cgtcagacaa   660
   aaagataacg gttttcaagc cagtgccgct cggaatatgg gattacgctt agcaaaatat   720
   gactttattg gcttactcga ctgtgatatg gcgccaaatc cattatgggt tcattcttat   780
   gttgcagagc tattagaaga tgatgattta acaatcattg gtccaagaaa atacatcgat   840
   acacaacata ttgacccaaa agacttctta aataacgcga gtttgcttga atcattacca 900
   gaagtgaaaa ccaataatag tgttgccgca aaaggggaag gaacagtttc tctggattgg 960
   cgcttagaac aattcgaaaa aacagaaaat ctccgcttat ccgattcgcc tttccgtttt 1020
   tttgcggcgg gtaatgttgc tttcgctaaa aaatggctaa ataaatccgg tttctttgat 1080
   gaggaattta atcactgggg tggacacgat gtggaatttg gatatcgctt attccgttac 1140
   ggtagtttct ttaaaactat tgatggcatt atggcctacc atcaagagcc accaggtaaa 1200
   gaaaatgaaa ccgatcgtga agcgggaaaa aatattacgc tcgatattat gagagaaaag 1260
   gtcccttata tctatagaaa acttttacca atagaagatt cgcatatcaa tagagtacct 1320
   ttagtttcaa tttatatccc agcttataac tgtgcaaact atattcaacg ttgcgtagat 1380
   agtgcactga atcagactgt tgttgatctc gaggtttgta tttgtaacga tggttcaaca 1440
   gataatacct tagaagtgat caataagctt tatggtaata atcctagggt acgcatcatg 1500
   tctaaaccaa atggcggaat agcctcagca tcaaatgcag ccgtttcttt tgctaaaggt 1560
   tattacattg ggcagttaga ttcagatgat tatcttgagc ctgatgcagt tgaactgtgt 1620
   ttaaaagaat ttttaaaaga taaaacgcta gcttgtgttt ataccactaa tagaaacgtc 1680
   aatccggatg gtagcttaat cgctaatggt tacaattggc cagaattttc acgagaaaaa 1740
   ctcacaacgg ctatgattgc tcaccacttt agaatgttca cgattagagc ttggcattta 1800
   actgatggat tcaatgaaaa aattgaaaat gccgtagact atgacatgtt cctcaaactc 1860
   agtgaagttg gaaaatttaa acatcttaat aaaatctgct ataaccgtgt attacatggt 1920
   gataacacat caattaagaa acttggcatt caaaagaaaa accattttgt tgtagtcaat 1980
   cagtcattaa atagacaagg cataacttat tataattatg acgaatttga tgatttagat 2040
   gaaagtagaa agtatatttt caataaaacc gctgaatatc aagaagagat tgatatctta 2100
   aaagatattt aa 2112
<210> 44
   <211> 2112
   <212> DNA
   <213> Pasteurella multocida
<400> 44
   atgaatacat tatcacaagc aataaaagca tataacagca atgactatca attagcactc     60
   aaattatttg aaaagtcggc ggaaatctat ggacggaaaa ttgttgaatt tcaaattacc    120
   aaatgcaaag aaaaactctc agcacatcct tctgttaatt cagcacatct ttctgtaaat    180
   aaagaagaaa aagtcaatgt ttgcgatagt ccgttagata ttgcaacaca actgttactt    240
   tccaacgtaa aaaaattagt actttctgac tcggaaaaaa acacgttaaa aaataaatgg    300
   aaattgctca ctgagaagaa atctgaaaat gcggaggtaa gagcggtcgc ccttgtacca    360
   aaagattttc ccaaagatct ggttttagcg cctttacctg atcatgttaa tgattttaca 420
   tggtacaaaa agcgaaagaa aagacttggc ataaaacctg aacatcaaca tgttggtctt 480
   tctattatcg ttacaacatt caatcgacca gcaattttat cgattacatt agcctgttta 540
   gtaaaccaaa aaacacatta cccgtttgaa gttatcgtga cagatgatgg tagtcaggaa 600
   gatctatcac cgatcattcg ccaatatgaa aataaattgg atattcgcta cgtcagacaa 660
   aaagataacg gttttcaagc cagtgccgct cggaatatgg gattacgctt agcaaaatat 720
   gactttattg gcttactcga ctgtgatatg gcgccaaatc cattatgggt tcattcttat 780
   gttgcagagc tattagaaga tgatgattta acaatcattg gtccaagaaa atacatcgat 840
   acacaacata ttgacccaaa agacttctta aataacgcga gtttgcttga atcattacca 900
   gaagtgaaaa ccaataatag tgttgccgca aaaggggaag gaacagtttc tctggattgg 960
   cgcttagaac aattcgaaaa aacagaaaat ctccgcttat ccgattcgcc tttccgtttt 1020
   tttgcggcgg gtaatgttgc tttcgctaaa aaatggctaa ataaatccgg tttctttgat 1080
   gaggaattta atcactgggg tggagaagaa gtggaatttg gatatcgctt attccgttac 1140
   ggtagtttct ttaaaactat tgatggcatt atggcctacc atcaagagcc accaggtaaa 1200
   gaaaatgaaa ccgatcgtga agcgggaaaa aatattacgc tcgatattat gagagaaaag 1260
   gtcccttata tctatagaaa acttttacca atagaagatt cgcatatcaa tagagtacct 1320
   ttagtttcaa tttatatccc agcttataac tgtgcaaact atattcaacg ttgcgtagat 1380
   agtgcactga atcagactgt tgttgatctc gaggtttgta tttgtaacga tggttcaaca 1440
   gataatacct tagaagtgat caataagctt tatggtaata atcctagggt acgcatcatg 1500
   tctaaaccaa atggcggaat agcctcagca tcaaatgcag ccgtttcttt tgctaaaggt 1560
   tattacattg ggcagttaga ttcagatgat tatcttgagc ctgatgcagt tgaactgtgt 1620
   ttaaaagaat ttttaaaaga taaaacgcta gcttgtgttt ataccactaa tagaaacgtc 1680
   aatccggatg gtagcttaat cgctaatggt tacaattggc cagaattttc acgagaaaaa 1740
   ctcacaacgg ctatgattgc tcaccacttt agaatgttca cgattagagc ttggcattta 1800
   actgatggat tcaatgaaaa aattgaaaat gccgtagact atgacatgtt cctcaaactc 1860
   agtgaagttg gaaaatttaa acatcttaat aaaatctgct ataaccgtgt attacatggt 1920
   gataacacat caattaagaa acttggcatt caaaagaaaa accattttgt tgtagtcaat 1980
   cagtcattaa atagacaagg cataacttat tataattatg acgaatttga tgatttagat 2040
   gaaagtagaa agtatatttt caataaaacc gctgaatatc aagaagagat tgatatctta 2100
   aaagatattt aa 2112
<210> 45
   <211> 2112
   <212> DNA
   <213> Pasteurella multocida
<400> 45
   atgaatacat tatcacaagc aataaaagca tataacagca atgactatca attagcactc 60
   aaattatttg aaaagtcggc ggaaatctat ggacggaaaa ttgttgaatt tcaaattacc 120
   aaatgcaaag aaaaactctc agcacatcct tctgttaatt cagcacatct ttctgtaaat 180
   aaagaagaaa aagtcaatgt ttgcgatagt ccgttagata ttgcaacaca actgttactt 240
   tccaacgtaa aaaaattagt actttctgac tcggaaaaaa acacgttaaa aaataaatgg 300
   aaattgctca ctgagaagaa atctgaaaat gcggaggtaa gagcggtcgc ccttgtacca 360
   aaagattttc ccaaagatct ggttttagcg cctttacctg atcatgttaa tgattttaca 420
   tggtacaaaa agcgaaagaa aagacttggc ataaaacctg aacatcaaca tgttggtctt 480
   tctattatcg ttacaacatt caatcgacca gcaattttat cgattacatt agcctgttta 540
   gtaaaccaaa aaacacatta cccgtttgaa gttatcgtga cagatgatgg tagtcaggaa 600
   gatctatcac cgatcattcg ccaatatgaa aataaattgg atattcgcta cgtcagacaa 660
   aaagataacg gttttcaagc cagtgccgct cggaatatgg gattacgctt agcaaaatat 720
   gactttattg gcttactcga ctgtgatatg gcgccaaatc cattatgggt tcattcttat 780
   gttgcagagc tattagaaga tgatgattta acaatcattg gtccaagaaa atacatcgat 840
   acacaacata ttgacccaaa agacttctta aataacgcga gtttgcttga atcattacca 900
   gaagtgaaaa ccaataatag tgttgccgca aaaggggaag gaacagtttc tctggattgg 960
   cgcttagaac aattcgaaaa aacagaaaat ctccgcttat ccgattcgcc tttccgtttt 1020
   tttgcggcgg gtaatgttgc tttcgctaaa aaatggctaa ataaatccgg tttctttgat 1080
   gaggaattta atcactgggg tggagaaaat gtggaatttg gatatcgctt attccgttac 1140
   ggtagtttct ttaaaactat tgatggcatt atggcctacc atcaagagcc accaggtaaa 1200
   gaaaatgaaa ccgatcgtga agcgggaaaa aatattacgc tcgatattat gagagaaaag 1260
   gtcccttata tctatagaaa acttttacca atagaagatt cgcatatcaa tagagtacct 1320
   ttagtttcaa tttatatccc agcttataac tgtgcaaact atattcaacg ttgcgtagat 1380
   agtgcactga atcagactgt tgttgatctc gaggtttgta tttgtaacga tggttcaaca 1440
   gataatacct tagaagtgat caataagctt tatggtaata atcctagggt acgcatcatg 1500
   tctaaaccaa atggcggaat agcctcagca tcaaatgcag ccgtttcttt tgctaaaggt 1560
   tattacattg ggcagttaga ttcagatgat tatcttgagc ctgatgcagt tgaactgtgt 1620
   ttaaaagaat ttttaaaaga taaaacgcta gcttgtgttt ataccactaa tagaaacgtc 1680
   aatccggatg gtagcttaat cgctaatggt tacaattggc cagaattttc acgagaaaaa 1740
   ctcacaacgg ctatgattgc tcaccacttt agaatgttca cgattagagc ttggcattta   1800
   actgatggat tcaatgaaaa aattgaaaat gccgtagact atgacatgtt cctcaaactc   1860
   agtgaagttg gaaaatttaa acatcttaat aaaatctgct ataaccgtgt attacatggt   1920
   gataacacat caattaagaa acttggcatt caaaagaaaa accattttgt tgtagtcaat   1980
   cagtcattaa atagacaagg cataacttat tataattatg acgaatttga tgatttagat   2040
   gaaagtagaa agtatatttt caataaaacc gctgaatatc aagaagagat tgatatctta   2100
   aaagatattt aa                                                       2112
<210> 46
   <211> 2112
   <212> DNA
   <213> Pasteurella multocida
<400> 46
   atgaatacat tatcacaagc aataaaagca tataacagca atgactatca attagcactc 60
   aaattatttg aaaagtcggc ggaaatctat ggacggaaaa ttgttgaatt tcaaattacc 120
   aaatgcaaag aaaaactctc agcacatcct tctgttaatt cagcacatct ttctgtaaat 180
   aaagaagaaa aagtcaatgt ttgcgatagt ccgttagata ttgcaacaca actgttactt 240
   tccaacgtaa aaaaattagt actttctgac tcggaaaaaa acacgttaaa aaataaatgg 300
   aaattgctca ctgagaagaa atctgaaaat gcggaggtaa gagcggtcgc ccttgtacca 360
   aaagattttc ccaaagatct ggttttagcg cctttacctg atcatgttaa tgattttaca 420
   tggtacaaaa agcgaaagaa aagacttggc ataaaacctg aacatcaaca tgttggtctt 480
   tctattatcg ttacaacatt caatcgacca gcaattttat cgattacatt agcctgttta 540
   gtaaaccaaa aaacacatta cccgtttgaa gttatcgtga cagatgatgg tagtcaggaa 600
   gatctatcac cgatcattcg ccaatatgaa aataaattgg atattcgcta cgtcagacaa 660
   aaagataacg gttttcaagc cagtgccgct cggaatatgg gattacgctt agcaaaatat 720
   gactttattg gcttactcga ctgtgatatg gcgccaaatc cattatgggt tcattcttat 780
   gttgcagagc tattagaaga tgatgattta acaatcattg gtccaagaaa atacatcgat 840
   acacaacata ttgacccaaa agacttctta aataacgcga gtttgcttga atcattacca 900
   gaagtgaaaa ccaataatag tgttgccgca aaaggggaag gaacagtttc tctggattgg 960
   cgcttagaac aattcgaaaa aacagaaaat ctccgcttat ccgattcgcc tttccgtttt 1020
   tttgcggcgg gtaatgttgc tttcgctaaa aaatggctaa ataaatccgg tttctttgat 1080
   gaggaattta atcactgggg tggagaaaaa gtggaatttg gatatcgctt attccgttac 1140
   ggtagtttct ttaaaactat tgatggcatt atggcctacc atcaagagcc accaggtaaa 1200
   gaaaatgaaa ccgatcgtga agcgggaaaa aatattacgc tcgatattat gagagaaaag 1260
   gtcccttata tctatagaaa acttttacca atagaagatt cgcatatcaa tagagtacct 1320
   ttagtttcaa tttatatccc agcttataac tgtgcaaact atattcaacg ttgcgtagat 1380
   agtgcactga atcagactgt tgttgatctc gaggtttgta tttgtaacga tggttcaaca 1440
   gataatacct tagaagtgat caataagctt tatggtaata atcctagggt acgcatcatg 1500
   tctaaaccaa atggcggaat agcctcagca tcaaatgcag ccgtttcttt tgctaaaggt 1560
   tattacattg ggcagttaga ttcagatgat tatcttgagc ctgatgcagt tgaactgtgt 1620
   ttaaaagaat ttttaaaaga taaaacgcta gcttgtgttt ataccactaa tagaaacgtc 1680
   aatccggatg gtagcttaat cgctaatggt tacaattggc cagaattttc acgagaaaaa 1740
   ctcacaacgg ctatgattgc tcaccacttt agaatgttca cgattagagc ttggcattta 1800
   actgatggat tcaatgaaaa aattgaaaat gccgtagact atgacatgtt cctcaaactc 1860
   agtgaagttg gaaaatttaa acatcttaat aaaatctgct ataaccgtgt attacatggt 1920
   gataacacat caattaagaa acttggcatt caaaagaaaa accattttgt tgtagtcaat 1980
   cagtcattaa atagacaagg cataacttat tataattatg acgaatttga tgatttagat 2040
   gaaagtagaa agtatatttt caataaaacc gctgaatatc aagaagagat tgatatctta 2100
   aaagatattt aa 2112
<210> 47
   <211> 2136
   <212> DNA
   <213> Pasteurella multocida
<400> 47
   atgaacacat tatcacaagc aataaaagca tataacagca atgactatca attagcactc     60
   aaattatttg aaaagtcggc ggaaatctat ggacggaaaa ttgttgaatt tcaaattacc    120
   aaatgccaag aaaaactctc agcacatcct tctgttaatt cagcacatct ttctgtaaat    180
   aaagaagaaa aagtcaatgt ttgcgatagt ccgttagata ttgcaacaca actgttactt    240
   tccaacgtaa aaaaattagt actttctgac tcggaaaaaa acacgttaaa aaataaatgg    300
   aaattgctca ctgagaagaa atctgaaaat gcggaggtaa gagcggtcgc ccttgtacca    360
   aaagattttc ccaaagatct ggttttagcg cctttacctg atcatgttaa tgattttaca    420
   tggtacaaaa agcgaaagaa aagacttggc ataaaacctg aacatcaaca tgttggtctt    480
   tctattatcg ttacaacatt caatcgacca gcaattttat cgattacatt agcctgttta    540
   gtaaaccaaa aaacacatta cccgtttgaa gttatcgtga cagatgatgg tagtcaggaa    600
   gatctatcac cgatcattcg ccaatatgaa aataaattgg atattcgcta cgtcagacaa    660
   aaagataacg gttttcaagc cagtgccgct cggaatatgg gattacgctt agcaaaatat    720
   gactttattg gcttactcga ctgtgatatg gcgccaaatc cattatgggt tcattcttat    780
   gttgcagagc tattagaaga tgatgattta acaatcattg gtccaagaaa atacatcgat    840
   acacaacata ttgacccaaa agacttctta aataacgcga gtttgcttga atcattacca    900
   gaagtgaaaa ccaataatag tgttgccgca aaaggggaag gaacagtttc tctggattgg    960
   cgcttagaac aattcgaaaa aacagaaaat ctccgcttat ccgattcgcc tttccgtttt   1020
   tttgcggcgg gtaatgttgc tttcgctaaa aaatggctaa ataaatccgg tttctttgat   1080
   gaggaattta atcactgggg tggagaagat gtggaatttg gatatcgctt attccgttac   1140
   ggtagtttct ttaaaactat tgatggcatt atggcctacc atcaagagcc accaggtaaa   1200
   gaaaatgaaa ccgatcgtga agcgggaaaa aatattacgc tcgatattat gagagaaaag   1260
   gtcccttata tctatagaaa acttttacca atagaagatt cgcatattca tagaatacct   1320
   ttagtttcta tttatatccc cgcttataac tgtgcaaatt atattcaaag atgtgtagat   1380
   agtgctctta atcaaactgt tgtcgatctc gaggtttgta tttgtaacga tggttcaaca   1440
   gataatacct tagaagtgat caataagctt tatggtaata atcctagggt acgcatcatg   1500
   tctaaaccaa atggcggaat agcctcagca tcaaatgcag ccgtttcttt tgctaaaggt   1560
   tattacattg ggcagttaga ttcagatgat tatcttgagc ctgatgcagt tgaactgtgt   1620
   ttaaaagaat ttttaaaaga taaaacgcta gcttgtgttt ataccactaa tagaaacgtc   1680
   aatccggatg gtagcttaat cgctaatggt tacaattggc cagaattttc acgagaaaaa   1740
   ctcacaacgg ctatgattgc tcaccatttt agaatgttta cgattagagc ttggcattta   1800
   acggatggat ttaacgaaaa tattgaaaac gccgtggatt atgacatgtt ccttaaactc   1860
   agtgaagttg gaaaatttaa acatcttaat aaaatctgct ataaccgcgt attacatggt   1920
   gataacacat ccattaagaa actcggcatt caaaagaaaa accattttgt tgtagtcaat   1980
   cagtcattaa atagacaagg catcaattat tataattatg acaaatttga tgatttagat   2040
   gaaagtagaa agtatatctt caataaaacc gctgaatatc aagaagaaat ggatatttta   2100
   aaagatctta aactcattca gaataaagat gcctaa                             2136
<210> 48
   <211> 2091
   <212> DNA
   <213> Pasteurella multocida
<400> 48
   atgaatacat tatcacaagc aataaaagca tataacagca atgactatga attagcactc     60
   aaattatttg agaagtctgc tgaaacctac gggcgaaaaa tcgttgaatt ccaaattatc    120
   aaatgtaaag aaaaactctc gaccaattct tatgtaagtg aagataaaaa aaacagtgtt    180
   tgcgatagct cattagatat cgcaacacag ctcttacttt ccaacgtaaa aaaattaact    240
   ctatccgaat cagaaaaaaa cagtttaaaa aataaatgga aatctatcac tgggaaaaaa    300
   tcggagaacg cagaaatcag aaaggtggaa ctagtaccca aagattttcc taaagatctt 360
   gttcttgctc cattgccaga tcatgttaat gattttacat ggtacaaaaa tcgaaaaaaa 420
   agcttaggta taaagcctgt aaataagaat atcggtcttt ctattattat tcctacattt 480
   aatcgtagcc gtattttaga tataacgtta gcctgtttgg tcaatcagaa aacaaactac 540
   ccatttgaag tcgttgttgc agatgatggt agtaaggaaa acttacttac cattgtgcaa 600
   aaatacgaac aaaaacttga cataaagtat gtaagacaaa aagattatgg atatcaattg 660
   tgtgcagtca gaaacttagg tttacgtaca gcaaagtatg attttgtctc gattctagac 720
   tgcgatatgg caccacaaca attatgggtt cattcttatc ttacagaact attagaagac 780
   aatgatattg ttttaattgg acctagaaaa tatgtggata ctcataatat taccgcagaa 840
   caattcctta acgatccata tttaatagaa tcactacctg aaaccgctac aaataacaat 900
   ccttcgatta catcaaaagg aaatatatcg ttggattgga gattagaaca tttcaaaaaa 960
   accgataatc tacgtctatg tgattcaccg tttcgttatt ttagttgcgg taatgttgca 1020
   ttttctaaag aatggctaaa taaagtaggt tggttcgatg aagaatttaa tcattggggg 1080
   ggcgaagatg tagaatttgg ttacagatta tttgccaaag gctgtttttt cagagtaatt 1140
   gacggcggaa tggcatacca tcaagaacca cctggtaaag aaaatgaaac agaccgcgaa 1200
   gctggtaaaa gtattacgct taaaattgtg aaagaaaagg taccttacat ctatagaaaa 1260
   cttttaccaa tagaagattc gcatatcaat agagtacctt tagtttcaat ttatatccca 1320
   gcttataact gtgcaaacta tattcaacgt tgcgtagata gtgcactgaa tcagactgtt 1380
   gttgatctcg aggtttgtat ttgtaacgat ggttcaacag ataatacctt agaagtgatc 1440
   aataagcttt atggtaataa tcctagggta cgcatcatgt ctaaaccaaa tggcggaata 1500
   gcctcagcat caaatgcagc cgtttctttt gctaaaggtt attacattgg gcagttagat 1560
   tcagatgatt atcttgagcc tgatgcagtt gaactgtgtt taaaagaatt tttaaaagat 1620
   aaaacgctag cttgtgttta taccactaat agaaacgtca atccggatgg tagcttaatc 1680
   gctaatggtt acaattggcc agaattttca cgagaaaaac tcacaacggc tatgattgct 1740
   caccacttta gaatgttcac gattagagct tggcatttaa ctgatggatt caatgaaaaa 1800
   attgaaaatg ccgtagacta tgacatgttc ctcaaactca gtgaagttgg aaaatttaaa 1860
   catcttaata aaatctgcta taaccgtgta ttacatggtg ataacacatc aattaagaaa 1920
   cttggcattc aaaagaaaaa ccattttgtt gtagtcaatc agtcattaaa tagacaaggc 1980
   ataacttatt ataattatga cgaatttgat gatttagatg aaagtagaaa gtatattttc 2040
   aataaaaccg ctgaatatca agaagagatt gatatcttaa aagatattta a 2091
<210> 49
   <211> 29
   <212> DNA
   <213> Artificial Sequence
<220>
   <223> primer
<400> 49
   atgaacacat tatcacaagc aataaaagc 29
<210> 50
   <211> 27
   <212> DNA
   <213> Artificial Sequence
<220>
   <223> primer
<400> 50
   gcgaatcttc tattggtaaa agytttc 27
<210> 51
   <211> 26
   <212> DNA
   <213> Artificial sequence
<220>
   <223> primer
<400> 51
   cttttaccaa tagaagattc gcatat 26
<210> 52
   <211> 33
   <212> DNA
   <213> Artificial Sequence
<220>
   <223> primer
<400> 52
   gaagacgtct taggcatctt tattctgaat gag 33
<210> 53
   <211> 43
   <212> DNA
   <213> Artificial Sequence
<220>
   <223> primer
<400> 53
   gggaattctg cagttaaata tcttttaaga tatcaatctc ttc 43
<210> 54
   <211> 33
   <212> DNA
   <213> Artificial Sequence
<220>
   <223> primer
<220>
   <221> misc_feature
   <222> (9)..(9)
   <223> inosine
<220>
   <221> misc_feature
   <222> (12)..(12)
   <223> inosine
<220>
   <221> misc_feature
   <222> (18)..(18)
   <223> inosine
<220>
   <221> misc_feature
   <222> (24)..(24)
   <223> inosine
<220>
   <221> misc_feature
   <222> (27)..(27)
   <223> inosine
<400> 54
   garttybtnm rngarggnaa rgcnytntay gay 33
<210> 55
   <211> 39
   <212> DNA
   <213> Artificial sequence
<220>
   <223> primer
<220>
   <221> misc_feature
   <222> (7)..(7)
   <223> inosine
<220>
   <221> misc_feature
   <222> (10)..(10)
   <223> inosine
<220>
   <221> misc_feature
   <222> (16)..(16)
   <223> inosine
<220>
   <221> misc_feature
   <222> (22)..(22)
   <223> inosine
<220>
   <221> misc_feature
   <222> (25)..(25)
   <223> A, C, G or T
<400> 55
   rcartanccn ccrtanccra answnggrtt rttrtartg 39
<210> 56
   <211> 30
   <212> DNA
   <213> Artificial Sequence
<220>
   <223> primer
<400> 56
   tatatttaca gcagtatcat tttctaaagg 30
<210> 57
   <211> 501
   <212> PRT
   <213> Pasteurella multocida
<400> 57
   Met Ser Leu Phe Lys Arg Ala Thr Glu Leu Phe Lys Ser Gly Asn Tyr
   1               5                   10                  15
   Lys Asp Ala Leu Thr Leu Tyr Glu Asn Ile Ala Lys Ile Tyr Gly Ser
               20                  25                  30
   Glu Ser Leu Val Lys Tyr Asn Ile Asp Ile Cys Lys Lys Asn Ile Thr
           35                  40                  45
   Gln Ser Lys Ser Asn Lys Ile Glu Glu Asp Asn Ile Ser Gly Glu Asn
       50                  55                  60
   Glu Phe Ser Val Ser Ile Lys Asp Leu Tyr Asn Glu Ile Ser Asn Ser
   65                  70                  75                  80
   Glu Leu Gly Ile Thr Lys Glu Arg Leu Gly Ala Pro Pro Leu Val Ser
                   85                  90                  95
   Ile Ile Met Thr Ser His Asn Thr Glu Lys Phe Ile Glu Ala Ser Ile
               100                 105                 110
   Asn Ser Leu Leu Leu Gln Thr Tyr Asn Asn Leu Glu Val Ile Val Val
           115                 120                 125
   Asp Asp Tyr Ser Thr Asp Lys Thr Phe Gln Ile Ala Ser Arg Ile Ala
       130                 135                 140
   Asn Ser Thr Ser Lys Val Lys Thr Phe Arg Leu Asn Ser Asn Leu Gly
   145                 150                 155                 160
   Thr Tyr Phe Ala Lys Asn Thr Gly Ile Leu Lys Ser Lys Gly Asp Ile
                   165                 170                 175
   Ile Phe Phe Gln Asp Ser Asp Asp Val Cys His His Glu Arg Ile Glu
               180                 185                 190
   Arg Cys Val Asn Ala Leu Leu Ser Asn Lys Asp Asn Ile Ala Val Arg
           195                 200                 205
   Cys Ala Tyr Ser Arg Ile Asn Leu Glu Thr Gln Asn Ile Ile Lys Val
       210                 215                 220
   Asn Asp Asn Lys Tyr Lys Leu Gly Leu Ile Thr Leu Gly Val Tyr Arg
   225                 230                 235                 240
   Lys Val Phe Asn Glu Ile Gly Phe Phe Asn Cys Thr Thr Lys Ala Ser
                   245                 250                 255
   Asp Asp Glu Phe Tyr His Arg Ile Ile Lys Tyr Tyr Gly Lys Asn Arg
               260                 265                 270
   Ile Asn Asn Leu Phe Leu Pro Leu Tyr Tyr Asn Thr Met Arg Glu Asp
           275                 280                 285
   Ser Leu Phe Ser Asp Met Val Glu Trp Val Asp Glu Asn Asn Ile Lys
       290                 295                 300
   Gln Lys Thr Ser Asp Ala Arg Gln Asn Tyr Leu His Glu Phe Gln Lys
   305                 310                 315                 320
   Ile His Asn Glu Arg Lys Phe Asn Glu Leu Lys Glu Ile Phe Ser Phe
                   325                 330                 335
   Pro Arg Ile His Asp Ala Leu Pro Ile Ser Lys Glu Met Ser Lys Leu
               340                 345                 350
   Ser Asn Pro Lys Ile Pro Val Tyr Ile Asn Ile Cys Ser Ile Pro Ser
           355                 360                 365
   Arg Ile Lys Gln Leu Gln Tyr Thr Ile Gly Val Leu Lys Asn Gln Cys
       370                 375                 380
   Asp His Phe His Ile Tyr Leu Asp Gly Tyr Pro Glu Val Pro Asp Phe
   385                 390                 395                 400
   Ile Lys Lys Leu Gly Asn Lys Ala Thr Val Ile Asn Cys Gln Asn Lys
   405 410 415
   Asn Glu Ser Ile Arg Asp Asn Gly Lys Phe Ile Leu Leu Glu Lys Leu
   420 425 430
   Ile Lys Glu Asn Lys Asp Gly Tyr Tyr Ile Thr Cys Asp Asp Asp Ile
   435 440 445
   Arg Tyr Pro Ala Asp Tyr Ile Asn Thr Met Ile Lys Lys Ile Asn Lys
   450 455 460
   Tyr Asn Asp Lys Ala Ala Ile Gly Leu His Gly Val Ile Phe Pro Ser
   465 470 475 480
   Arg Val Asn Lys Tyr Phe Ser Ser Asp Arg Ile Val Tyr Asn Phe Gln
   485 490 495
   Lys Thr Phe Arg Lys
   500
<210> 58
   <211> 1510
   <212> DNA
   <213> Pasteurella multocida
<400> 58
   aatgagctta tttaaacgtg ctactgagct atttaagtca ggaaactata aagatgcact 60
   aactctatat gaaaatatag ctaaaattta tggttcagaa agccttgtta aatataatat 120
   tgatatatgt aaaaaaaata taacacaatc aaaaagtaat aaaatagaag aagataatat 180
   ttctggagaa aacgaatttt cagtatcaat aaaagatcta tataacgaaa taagcaatag 240
   tgaattaggg attacaaaag aaagactagg agccccccct ctagtcagta ttataatgac 300
   ttctcataat acagaaaaat tcattgaagc ctcaattaat tcactattat tgcaaacata 360
   caataactta gaagttatcg ttgtagatga ttatagcaca gataaaacat ttcagatcgc 420
   atccagaata gcaaactcta caagtaaagt aaaaacattc cgattaaact caaatctagg 480
   gacatacttt gcgaaaaata caggaatttt aaagtctaaa ggagatatta ttttctttca 540
   ggatagcgat gatgtatgtc accatgaaag aatcgaaaga tgtgttaatg cattattatc 600
   gaataaagat aatatagctg ttagatgtgc atattctaga ataaatctag aaacacaaaa 660
   tataataaaa gttaatgata ataaatacaa attaggatta ataactttag gcgtttatag 720
   aaaagtattt aatgaaattg gtttttttaa ctgcacaacc aaagcatcgg atgatgaatt 780
   ttatcataga ataattaaat actatggtaa aaataggata aataacttat ttctaccact 840
   gtattataac acaatgcgtg aagattcatt attttctgat atggttgagt gggtagatga 900
   aaataatata aagcaaaaaa cctctgatgc tagacaaaat tatctccatg aattccaaaa 960
   aatacacaat gaaaggaaat ttaatgaatt aaaagagatt tttagctttc ctagaattca 1020
   tgacgcctta cctatatcaa aagaaatgag taagctcagc aaccctaaaa ttcctgttta 1080
   tataaatata tgctcaatac cttcaagaat aaaacaactt caatacacta ttggagtact 1140
   aaaaaaccaa tgcgatcatt ttcatattta tcttgatgga tatccagaag tacctgattt 1200
   tataaaaaaa ctagggaata aagcgaccgt tattaattgt caaaacaaaa atgagtctat 1260
   tagagataat ggaaagttta ttctattaga aaaacttata aaggaaaata aagatggata 1320
   ttatataact tgtgatgatg atatccggta tcctgctgac tacataaaca ctatgataaa 1380
   aaaaattaat aaatacaatg ataaagcagc aattggatta catggtgtta tattcccaag 1440
   tagagtcaac aagtattttt catcagacag aattgtctat aattttcaaa aaacctttag 1500
   aaaatgatac 1510
<210> 59
   <211> 238
   <212> PRT
   <213> Escherichia coli
<400> 59
   Met Ile Val Ala Asn Met Ser Ser Tyr Pro Pro Arg Lys Lys Glu Leu
   1               5                   10                  15
   Val His Ser Ile Gln Ser Leu His Ala Gln Val Asp Lys Ile Asn Leu
               20                  25                  30
   Cys Leu Asn Glu Phe Glu Glu Ile Pro Glu Glu Leu Asp Gly Phe Ser
           35                  40                  45
   Lys Leu Asn Pro Val Ile Pro Asp Lys Asp Tyr Lys Asp Val Gly Lys
       50                  55                  60
   Phe Ile Phe Pro Cys Ala Lys Asn Asp Met Ile Val Leu Thr Asp Asp
   65                  70                  75                  80
   Asp Ile Ile Tyr Pro Pro Asp Tyr Val Glu Lys Met Leu Asn Phe Tyr
                   85                  90                  95
   Asn Ser Phe Ala Ile Phe Asn Cys Ile Val Gly Ile His Gly Cys Ile
               100                 105                 110
   Tyr Ile Asp Ala Phe Asp Gly Asp Gln Ser Lys Arg Lys Val Phe Ser
           115                 120                 125
   Phe Thr Gln Gly Leu Leu Arg Pro Arg Val Val Asn Gln Leu Gly Thr
       130                 135                 140
   Gly Thr Val Phe Leu Lys Ala Asp Gln Leu Pro Ser Leu Lys Tyr Met
   145                 150                 155                 160
   Asp Gly Ser Gln Arg Phe Val Asp Val Arg Phe Ser Arg Tyr Met Leu
                   165                 170                 175
   Glu Asn Glu Ile Gly Met Ile Cys Val Pro Arg Glu Lys Asn Trp Leu
               180                 185                 190
   Arg Glu Val Ser Ser Gly Ser Met Glu Gly Leu Trp Asn Thr Phe Thr
           195                 200                 205
   Lys Lys Trp Pro Leu Asp Ile Ile Lys Glu Thr Gln Ala Ile Ala Gly
       210                 215                 220
   Tyr Ser Lys Leu Asn Leu Glu Leu Val Tyr Asn Val Glu Gly
   225                 230                 235
<210> 60
   <211> 520
   <212> PRT
   <213> Escherichia coli
<400> 60
   Met Asn Ala Glu Tyr Ile Asn Leu Val Glu Arg Lys Lys Lys Leu Gly
   1               5                   10                  15
   Thr Asn Ile Gly Ala Leu Asp Phe Leu Leu Ser Ile His Lys Glu Lys
               20                  25                  30
   Val Asp Leu Gln His Lys Asn Ser Pro Leu Lys Gly Asn Asp Asn Leu
           35                  40                  45
   Ile His Lys Arg Ile Asn Glu Tyr Asp Asn Val Leu Glu Leu Ser Lys
       50                  55                  60
   Asn Val Ser Ala Gln Asn Ser Gly Asn Glu Phe Ser Tyr Leu Leu Gly
   65                  70                  75                  80
   Tyr Ala Asp Ser Leu Arg Lys Val Gly Met Leu Asp Thr Tyr Ile Lys
                   85                  90                  95
   Ile Val Cys Tyr Leu Thr Ile Gln Ser Arg Tyr Phe Lys Asn Gly Glu
               100                 105                 110
   Arg Val Lys Leu Phe Glu His Ile Ser Asn Ala Leu Arg Tyr Ser Arg
   115 120 125
   Ser Asp Phe Leu Ile Asn Leu Ile Phe Glu Arg Tyr Ile Glu Tyr Ile
   130 135 140
   Asn His Leu Lys Leu Ser Pro Lys Gln Lys Asp Phe Tyr Phe Cys Thr
   145 150 155 160
   Lys Phe Ser Lys Phe His Asp Tyr Thr Lys Asn Gly Tyr Lys Tyr Leu
   165 170 175
   Ala Phe Asp Asn Gln Ala Asp Ala Gly Tyr Gly Leu Thr Leu Leu Leu
   180 185 190
   Asn Ala Asn Asp Asp Met Gln Asp Ser Tyr Asn Leu Leu Pro Glu Gln
   195 200 205
   Glu Leu Phe Ile Cys Asn Ala Val Ile Asp Asn Met Asn Ile Tyr Arg
   210 215 220
   Ser Gln Phe Asn Lys Cys Leu Arg Lys Tyr Asp Leu Ser Glu Ile Thr
   225 230 235 240
   Asp Ile Tyr Pro Asn Lys Ile Ile Leu Gln Gly Ile Lys Phe Asp Lys
   245 250 255
   Lys Lys Asn Val Tyr Gly Lys Asp Leu Val Ser Ile Ile Met Ser Val
   260 265 270
   Phe Asn Ser Glu Asp Thr Ile Ala Tyr Ser Leu His Ser Leu Leu Asn
   275 280 285
   Gln Thr Tyr Glu Asn Ile Glu Ile Leu Val Cys Asp Asp Cys Ser Ser
   290 295 300
   Asp Lys Ser Leu Glu Ile Ile Lys Ser Ile Ala Tyr Ser Ser Ser Arg
   305 310 315 320
   Val Lys Val Tyr Ser Ser Arg Lys Asn Gln Gly Pro Tyr Asn Ile Arg
   325 330 335
   Asn Glu Leu Ile Lys Lys Ala His Gly Asn Phe Ile Thr Phe Gln Asp
   340 345 350
   Ala Asp Asp Leu Ser His Pro Glu Arg Ile Gln Arg Gln Val Glu Val
   355 360 365
   Leu Arg Asn Asn Lys Ala Val Ile Cys Met Ala Asn Trp Ile Arg Val
   370 375 380
   Ala Ser Asn Gly Lys Ile Gln Phe Phe Tyr Asp Asp Lys Ala Thr Arg
   385 390 395 400
   Met Ser Val Val Ser Ser Met Ile Lys Lys Asp Ile Phe Ala Thr Val
   405 410 415
   Gly Gly Tyr Arg Gln Ser Leu Ile Gly Ala Asp Thr Glu Phe Tyr Glu
   420 425 430
   Thr Val Ile Met Arg Tyr Gly Arg Glu Ser Ile Val Arg Leu Leu Gln
   435 440 445
   Pro Leu Ile Leu Gly Leu Trp Gly Asp Ser Gly Leu Thr Arg Asn Lys
   450 455 460
   Gly Thr Glu Ala Leu Pro Asp Gly Tyr Ile Ser Gln Ser Arg Arg Glu
   465 470 475 480
   Tyr Ser Asp Ile Ala Ala Arg Gln Arg Val Leu Gly Lys Ser Ile Val
   485 490 495
   Ser Asp Lys Asp Val Arg Gly Leu Leu Ser Arg Tyr Gly Leu Phe Lys
   500 505 510
   Asp Val Ser Gly Ile Ile Glu Gln
   515 520
<210> 61
   <211> 746
   <212> PRT
   <213> Mus musculus
<400> 61
   Met Gln Ala Lys Lys Arg Tyr Phe Ile Leu Leu Ser Ala Gly Ser Cys
   1 5 10 15
   Leu Ala Leu Leu Phe Tyr Phe Gly Gly Val Gln Phe Arg Ala Ser Arg
   20 25 30
   Ser His Ser Arg Arg Glu Glu His Ser Gly Arg Asn Gly Leu His Gln
   35 40 45
   Pro Ser Pro Asp His Phe Trp Pro Arg Phe Pro Asp Ala Leu Arg Pro
   50 55 60
   Phe Phe Pro Trp Asp Gln Leu Glu Asn Glu Asp Ser Ser Val His Ile
   65                  70                  75                  80
   Ser Pro Arg Gln Lys Arg Asp Ala Asn Ser Ser Ile Tyr Lys Gly Lys
                   85                  90                  95
   Lys Cys Arg Met Glu Ser Cys Phe Asp Phe Thr Leu Cys Lys Lys Asn
               100                 105                 110
   Gly Phe Lys Val Tyr Val Tyr Pro Gln Gln Lys Gly Glu Lys Ile Ala
           115                 120                 125
   Glu Ser Tyr Gln Asn Ile Leu Ala Ala Ile Glu Gly Ser Arg Phe Tyr
       130                 135                 140
   Thr Ser Asp Pro Ser Gln Ala Cys Leu Phe Val Leu Ser Leu Asp Thr
   145                 150                 155                 160
   Leu Asp Arg Asp Gln Leu Ser Pro Gln Tyr Val His Asn Leu Arg Ser
                   165                 170                 175
   Lys Val Gln Ser Leu His Leu Trp Asn Asn Gly Arg Asn His Leu Ile
               180                 185                 190
   Phe Asn Leu Tyr Ser Gly Thr Trp Pro Asp Tyr Thr Glu Asp Val Gly
           195                 200                 205
   Phe Asp Ile Gly Gln Ala Met Leu Ala Lys Ala Ser Ile Ser Thr Glu
       210                 215                 220
   Asn Phe Arg Pro Asn Phe Asp Val Ser Ile Pro Leu Phe Ser Lys Asp
   225                 230                 235                 240
   His Pro Arg Thr Gly Gly Glu Arg Gly Phe Leu Lys Phe Asn Thr Ile
                   245                 250                 255
   Pro Pro Leu Arg Lys Tyr Met Leu Val Phe Lys Gly Lys Arg Tyr Leu
               260                 265                 270
   Thr Gly Ile Gly Ser Asp Thr Arg Asn Ala Leu Tyr His Val His Asn
           275                 280                 285
   Gly Glu Asp Val Leu Leu Leu Thr Thr Cys Lys His Gly Lys Asp Trp
       290                 295                 300
   Gln Lys His Lys Asp Ser Arg Cys Asp Arg Asp Asn Thr Glu Tyr Glu
   305                 310                 315                 320
   Lys Tyr Asp Tyr Arg Glu Met Leu His Asn Ala Thr Phe Cys Leu Val
                   325                 330                 335
   Pro Arg Gly Arg Arg Leu Gly Ser Phe Arg Phe Leu Glu Ala Leu Gln
               340                 345                 350
   Ala Ala Cys Val Pro Val Met Leu Ser Asn Gly Trp Glu Leu Pro Phe
           355                 360                 365
   Ser Glu Val Ile Asn Trp Asn Gln Ala Ala Val Ile Gly Asp Glu Arg
       370                 375                 380
   Leu Leu Leu Gln Ile Pro Ser Thr Ile Arg Ser Ile His Gln Asp Lys
   385                 390                 395                 400
   Ile Leu Ala Leu Arg Gln Gln Thr Gln Phe Leu Trp Glu Ala Tyr Phe
                   405                 410                 415
   Ser Ser Val Glu Lys Ile Val Leu Thr Thr Leu Glu Ile Ile Gln Asp
               420                 425                 430
   Arg Ile Phe Lys His Ile Ser Arg Asn Ser Leu Ile Trp Asn Lys His
           435                 440                 445
   Pro Gly Gly Leu Phe Val Leu Pro Gln Tyr Ser Ser Tyr Leu Gly Asp
       450                 455                 460
   Phe Pro Tyr Tyr Tyr Ala Asn Leu Gly Leu Lys Pro Pro Ser Lys Phe
   465                 470                 475                 480
   Thr Ala Val Ile His Ala Val Thr Pro Leu Val Ser Gln Ser Gln Pro
                   485                 490                 495
   Val Leu Lys Leu Leu Val Ala Ala Ala Lys Ser Gln Tyr Cys Ala Gln
               500                 505                 510
   Ile Ile Val Leu Trp Asn Cys Asp Lys Pro Leu Pro Ala Lys His Arg
           515                 520                 525
   Trp Pro Ala Thr Ala Val Pro Val Ile Val Ile Glu Gly Glu Ser Lys
       530                 535                 540
   Val Met Ser Ser Arg Phe Leu Pro Tyr Asp Asn Ile Ile Thr Asp Ala
   545                 550                 555                 560
   Val Leu Ser Leu Asp Glu Asp Thr Val Leu Ser Thr Thr Glu Val Asp
   565 570 575
   Phe Ala Phe Thr Val Trp Gln Ser Phe Pro Glu Arg Ile Val Gly Tyr
   580 585 590
   Pro Ala Arg Ser His Phe Trp Asp Asn Ser Lys Glu Arg Trp Gly Tyr
   595 600 605
   Thr Ser Lys Trp Thr Asn Asp Tyr Ser Met Val Leu Thr Gly Ala Ala
   610 615 620
   Ile Tyr His Lys Tyr Tyr His Tyr Leu Tyr Ser His Tyr Leu Pro Ala
   625 630 635 640
   Ser Leu Lys Asn Met Val Asp Gln Leu Ala Asn Cys Glu Asp Ile Leu
   645 650 655
   Met Asn Phe Leu Val Ser Ala Val Thr Lys Leu Pro Pro Ile Lys Val
   660 665 670
   Thr Gln Lys Lys Gln Tyr Lys Glu Thr Met Met Gly Gln Thr Ser Arg
   675 680 685
   Ala Ser Arg Trp Ala Asp Pro Asp His Phe Ala Gln Arg Gln Ser Cys
   690 695 700
   Met Asn Thr Phe Ala Ser Trp Phe Gly Tyr Met Pro Leu Ile His Ser
   705 710 715 720
   Gln Met Arg Leu Asp Pro Val Leu Phe Lys Asp Gln Val Ser Ile Leu
   725 730 735
   Arg Lys Lys Tyr Arg Asp Ile Glu Arg Leu
   740 745
<210> 62
   <211> 718
   <212> PRT
   <213> Mus musculus
<400> 62
   Met Cys Ala Ser Val Lys Ser Asn Ile Arg Gly Pro Ala Leu Ile Pro
   1 5 10 15
   Arg Met Lys Thr Lys His Arg Ile Tyr Tyr Val Thr Leu Phe Ser Ile
   20 25 30
   Val Leu Leu Gly Leu Ile Ala Thr Gly Met Phe Gln Phe Trp Pro His
           35                  40                  45
   Ser Ile Glu Ser Ser Ser Asp Gly Gly Val Glu Lys Arg Ser Ile Arg
       50                  55                  60
   Glu Val Pro Val Val Arg Leu Pro Thr Asp Ser Pro Ile Pro Glu Arg
   65                  70                  75                  80
   Gly Asp Leu Ser Cys Arg Met His Thr Cys Phe Asp Val Tyr Arg Cys
                   85                  90                  95
   Gly Phe Asn Pro Lys Asn Lys Ile Lys Val Tyr Ile Tyr Pro Leu Lys
               100                 105                 110
   Lys Tyr Val Asp Asp Ala Gly Val Pro Val Ser Ser Ala Ile Ser Arg
           115                 120                 125
   Glu Tyr Asn Glu Leu Leu Thr Ala Ile Ser Asp Ser Asp Tyr Tyr Thr
       130                 135                 140
   Asp Asp Ile Asn Arg Ala Cys Leu Phe Val Pro Ser Ile Asp Val Leu
   145                 150                 155                 160
   Asn Gln Asn Pro Leu Arg Ile Lys Glu Thr Ala Gln Ala Leu Ala Gln
                   165                 170                 175
   Leu Ser Arg Trp Asp Arg Gly Thr Asn His Leu Leu Phe Asn Met Leu
               180                 185                 190
   Pro Gly Ala Pro Pro Asp Tyr Asn Thr Ala Leu Asp Val Pro Arg Asp
           195                 200                 205
   Arg Ala Leu Leu Ala Gly Gly Gly Phe Ser Thr Trp Thr Tyr Arg Gln
       210                 215                 220
   Gly Tyr Asp Val Ser Ile Pro Val Phe Ser Pro Leu Ser Ala Glu Met
   225                 230                 235                 240
   Ala Leu Pro Glu Lys Ala Pro Gly Pro Arg Arg Tyr Phe Leu Leu Ser
                   245                 250                 255
   Ser Gln Met Ala Ile His Pro Glu Tyr Arg Glu Glu Leu Glu Ala Leu
               260                 265                 270
   Gln Ala Lys His Gln Glu Ser Val Leu Val Leu Asp Lys Cys Thr Asn
           275                 280                 285
   Leu Ser Glu Gly Val Leu Ser Val Arg Lys Arg Cys His Gln His Gln
       290                 295                 300
   Val Phe Asp Tyr Pro Gln Val Leu Gln Glu Ala Thr Phe Cys Thr Val
   305                 310                 315                 320
   Leu Arg Arg Ala Arg Leu Gly Gln Ala Val Leu Ser Asp Val Leu Gln
                   325                 330                 335
   Ala Gly Cys Val Pro Val Val Ile Ala Asp Ser Tyr Ile Leu Pro Phe
               340                 345                 350
   Ser Glu Val Leu Asp Trp Lys Lys Ala Ser Val Val Val Pro Glu Glu
           355                 360                 365
   Lys Met Ser Asp Val Tyr Ser Ile Leu Gln Asn Ile Pro Gln Arg Gln
       370                 375                 380
   Ile Glu Glu Met Gln Arg Gln Ala Arg Trp Phe Trp Glu Ala Tyr Phe
   385                 390                 395                 400
   Gln Ser Ile Lys Ala Ile Ala Leu Ala Thr Leu Gln Ile Ile Asn Asp
                   405                 410                 415
   Arg Ile Tyr Pro Tyr Ala Ala Ile Ser Tyr Glu Glu Trp Asn Asp Pro
               420                 425                 430
   Pro Ala Val Lys Trp Ala Ser Val Ser Asn Pro Leu Phe Leu Pro Leu
           435                 440                 445
   Ile Pro Pro Gln Ser Gln Gly Phe Thr Ala Ile Val Leu Thr Tyr Asp
       450                 455                 460
   Arg Val Glu Ser Leu Phe Arg Val Ile Thr Glu Val Ser Lys Val Pro
   465                 470                 475                 480
   Ser Leu Ser Lys Leu Leu Val Val Trp Asn Asn Gln Asn Lys Asn Pro
                   485                 490                 495
   Pro Glu Glu Ser Leu Trp Pro Lys Ile Arg Val Pro Leu Lys Val Val
               500                 505                 510
   Arg Thr Ala Glu Asn Lys Leu Ser Asn Arg Phe Phe Pro Tyr Asp Glu
           515                 520                 525
   Ile Glu Thr Glu Ala Val Leu Ala Ile Asp Asp Asp Ile Ile Met Leu
       530                 535                 540
   Thr Ser Asp Glu Leu Gln Phe Gly Tyr Glu Val Trp Arg Glu Phe Pro
   545                 550                 555                 560
   Asp Arg Leu Val Gly Tyr Pro Gly Arg Leu His Leu Trp Asp His Glu
                   565                 570                 575
   Met Asn Lys Trp Lys Tyr Glu Ser Glu Trp Thr Asn Glu Val Ser Met
               580                 585                 590
   Val Leu Thr Gly Ala Ala Phe Tyr His Lys Tyr Phe Asn Tyr Leu Tyr
           595                 600                 605
   Thr Tyr Lys Met Pro Gly Asp Ile Lys Asn Trp Val Asp Ala His Met
       610                 615                 620
   Asn Cys Glu Asp Ile Ala Met Asn Phe Leu Val Ala Asn Val Thr Gly
   625                 630                 635                 640
   Lys Ala Val Ile Lys Val Thr Pro Arg Lys Lys Phe Lys Cys Pro Glu
                   645                 650                 655
   Cys Thr Ala Ile Asp Gly Leu Ser Leu Asp Gln Thr His Met Val Glu
               660                 665                 670
   Arg Ser Glu Cys Ile Asn Lys Phe Ala Ser Val Phe Gly Thr Met Pro
           675                 680                 685
   Leu Lys Val Val Glu His Arg Ala Asp Pro Val Leu Tyr Lys Asp Asp
       690                 695                 700
   Phe Pro Glu Lys Leu Lys Ser Phe Pro Asn Ile Gly Ser Leu
   705                 710                 715
<210> 63
   <211> 76
   <212> PRT
   <213> Artificial Sequence
<220>
   <223> motif
<220>
   <221> MISC_FEATURE
   <222> (4)..(4)
   <223> any amino acid
<220>
   <221> MISC_FEATURE
   <222> (6)..(6)
   <223> Leu or Ile
<220>
   <221> MISC_FEATURE
   <222> (8)..(11)
   <223> Any amino acid
<220>
   <221> MISC_FEATURE
   <222> (14)..(14)
   <223> Any amino acid
<220>
   <221> MISC_FEATURE
   <222> (15)..(15)
   <223> Ser or Thr
<220>
   <221> MISC_FEATURE
   <222> (16)..(16)
   <223> Ser or Thr
<220>
   <221> MISC_FEATURE
   <222> (18)..(18)
   <223> Lys or Asn
<220>
   <221> MISC_FEATURE
   <222> (19)..(19)
   <223> Thr or Ser
<220>
   <221> MISC_FEATURE
   <222> (20)..(25)
   <223> any amino acid
<220>
   <221> MISC_FEATURE
   <222> (28)..(28)
   <223> any amino acid
<220>
   <221> MISC_FEATURE
   <222> (29)..(31)
   <223> Ser or Thr
<220>
   <221> MISC_FEATURE
   <222> (32)..(32)
   <223> Lys or Arg
<220>
   <221> MISC_FEATURE
   <222> (34)..(34)
   <223> Lys or Arg
<220>
   <221> MISC_FEATURE
   <222> (35)..(40)
   <223> any amino acid
<220>
   <221> MISC_FEATURE
   <222> (42)..(42)
   <223> any amino acid
<220>
   <221> MISC_FEATURE
   <222> (44)..(44)
   <223> any amino acid
<220>
   <221> MISC_FEATURE
   <222> (46)..(61)
   <223> any amino acid
<220>
   <221> MISC_FEATURE
   <222> (65)..(65)
   <223> any amino acid
<220>
   <221> MISC_FEATURE
   <222> (68)..(68)
   <223> any amino acid
<220>
   <221> MISC_FEATURE
   <222> (69)..(69)
   <223> Cys or Ser
<220>
   <221> MISC_FEATURE
   <222> (71)..(71)
   <223> His or Pro
<220>
   <221> MISC_FEATURE
   <222> (75)..(75)
   <223> any amino acid
<400> 63
   Gln Thr Tyr Xaa Asn Xaa Glu Xaa Xaa Xaa Xaa Asp Asp Xaa Xaa Xaa
   1               5                   10                  15
   Asp Xaa Xaa Xaa Xaa Xaa Xaa Xaa Xaa Ile Ala Xaa Xaa Xaa Xaa Xaa
               20                  25                  30
   Val Xaa Xaa Xaa Xaa Xaa Xaa Xaa Asn Xaa Gly Xaa Tyr Xaa Xaa Xaa
           35                  40                  45
   Xaa Xaa Xaa Xaa Xaa Xaa Xaa Xaa Xaa Xaa Xaa Xaa Xaa Phe Gln Asp
       50                  55                  60
   Xaa Asp Asp Xaa Xaa His Xaa Glu Arg Ile Xaa Arg
   65                  70                  75
<210> 64
   <211> 102
   <212> PRT
   <213> Artificial Sequence
<220>
   <223> motif
<220>
   <221> MISC_FEATURE
   <222> (1)..(1)
   <223> Lys or Arg
<220>
   <221> MISC_FEATURE
   <222> (3)..(3)
   <223> any amino acid
<220>
   <221> MISC_FEATURE
   <222> (8)..(19)
   <223> any amino acid
<220>
   <221> MISC_FEATURE
   <222> (20)..(24)
   <223> may be missing from sequence; each portion (is present) may be any amino acid
<220>
   <221> MISC_FEATURE
   <222> (29)..(29)
   <223> Arg or Ile
<220>
   <221> MISC_FEATURE
   <222> (32)..(32)
   <223> any amino acid
<220>
   <221> MISC_FEATURE
   <222> (35)..(37)
   <223> any amino acid
<220>
   <221> MISC_FEATURE
   <222> (39)..(84)
   <223> any amino acid
<220>
   <221> MISC_FEATURE
   <222> (85)..(94)
   <223> all or part of sequence comprising residues 85-94 may be missing; each position may be any amino acid
<220>
   <221> MISC_FEATURE
   <222> (96)..(96)
   <223> any amino acid
<400> 64
   Xaa Asp Xaa Gly Lys Phe Ile Xaa Xaa Xaa Xaa Xaa Xaa Xaa Xaa Xaa
   1               5                   10                  15
   Xaa Xaa Xaa Xaa Xaa Xaa Xaa Xaa Asp Asp Asp Ile Xaa Tyr Pro Xaa
               20                  25                  30
   Asp Tyr Xaa Xaa Xaa Met Xaa Xaa Xaa Xaa Xaa Xaa Xaa Xaa Xaa Xaa
           35                  40                  45
   Xaa Xaa Xaa Xaa Xaa Xaa Xaa Xaa Xaa Xaa Xaa Xaa Xaa Xaa Xaa Xaa
       50                  55                  60
   Xaa Xaa Xaa Xaa Xaa Xaa Xaa Xaa Xaa Xaa Xaa Xaa Xaa Xaa Xaa Xaa
   65                  70                  75                  80
   Xaa Xaa Xaa Xaa Xaa Xaa Xaa Xaa Xaa Xaa Xaa Xaa Xaa Val Asn Xaa
                   85                  90                  95
   Leu Gly Thr Gly Thr Val
               100
<210> 65
   <211> 1854
   <212> **DNA**
   <213> Pasteurella multocida
<400> 65
   atgagcttat ttaaacgtgc tactgagcta tttaagtcag gaaactataa agatgcacta     60
   actctatatg aaaatatagc taaaatttat ggttcagaaa gccttgttaa atataatatt    120
   gatatatgta aaaaaaatat aacacaatca aaaagtaata aaatagaaga agataatatt    180
   tctggagaaa acaaattttc agtatcaata aaagatctat ataacgaaat aagcaatagt    240
   gaattaggga ttacaaaaga aagactagga gccccccctc tagtcagtat tataatgact    300
   tctcataata cagaaaaatt cattgaagcc tcaattaatt cactattatt gcaaacatac    360
   aataacttag aagttatcgt tgtagatgat tatagcacag ataaaacatt tcagatcgca    420
   tccagaatag caaactctac aagtaaagta aaaacattcc gattaaactc aaatctaggg    480
   acatactttg cgaaaaatac aggaatttta aagtctaaag gagatattat tttctttcag    540
   gatagcgatg atgtatgtca ccatgaaaga atcgaaagat gtgttaatgc attattatcg    600
   aataaagata atatagctgt tagatgtgca tattctagaa taaatctaga aacacaaaat    660
   ataataaaag ttaatgataa taaatacaaa ttaggattaa taactttagg cgtttataga    720
   aaagtattta atgaaattgg tttttttaac tgcacaacca aagcatcgga tgatgaattt    780
   tatcatagaa taattaaata ctatggtaaa aataggataa ataacttatt tctaccactg    840
   tattataaca caatgcgtga agattcatta ttttctgata tggttgagtg ggtagatgaa    900
   aataatataa agcaaaaaac ctctgatgct agacaaaatt atctccatga attccaaaaa    960
   atacacaatg aaaggaaatt aaatgaatta aaagagattt ttagctttcc tagaattcat   1020
   gacgccttac ctatatcaaa agaaatgagt aagctcagca accctaaaat tcctgtttat   1080
   ataaatatat gctcaatacc ttcaagaata aaacaacttc aatacactat tggagtacta   1140
   aaaaaccaat gcgatcattt tcatatttat cttgatggat atccagaagt acctgatttt   1200
   ataaaaaaac tagggaataa agcgaccgtt attaattgtc aaaacaaaaa tgagtctatt   1260
   agagataatg gaaagtttat tctattagaa aaacttataa aggaaaataa agatggatat   1320
   tatataactt gtgatgatga tatccggtat cctgctgact acataaacac tatgataaaa   1380
   aaaattaata aatacaatga taaagcagca attggattac atggtgttat attcccaagt   1440
   agagtcaaca agtatttttc atcagacaga attgtctata attttcaaaa acctttagaa   1500
   aatgatactg ctgtaaatat attaggaact ggaactgttg cctttagagt atctattttt   1560
   aataaatttt ctctatctga ttttgagcat cctggcatgg tagatatcta tttttctata   1620
   ctatgtaaga aaaacaatat actccaagtt tgtatatcac gaccatcgaa ttggctaaca   1680
   gaagataaca aaaacactga gaccttattt catgaattcc aaaatagaga tgaaatacaa   1740
   agtaaactca ttatttcaaa caacccttgg ggatactcaa gtatatatcc attattaaat   1800
   aataatgcta attattctga acttattccg tgtttatctt tttataacga gtaa         1854
<210> 66
   <211> 617
   <212> PRT
   <213> Pasteurella multocida
<400> 66
   Met Ser Leu Phe Lys Arg Ala Thr Glu Leu Phe Lys Ser Gly Asn Tyr
   1               5                   10                  15
   Lys Asp Ala Leu Thr Leu Tyr Glu Asn Ile Ala Lys Ile Tyr Gly Ser
               20                  25                  30
   Glu Ser Leu Val Lys Tyr Asn Ile Asp Ile Cys Lys Lys Asn Ile Thr
           35                  40                  45
   Gln Ser Lys Ser Asn Lys Ile Glu Glu Asp Asn Ile Ser Gly Glu Asn
       50                  55                  60
   Lys Phe Ser Val Ser Ile Lys Asp Leu Tyr Asn Glu Ile Ser Asn Ser
   65                  70                  75                  80
   Glu Leu Gly Ile Thr Lys Glu Arg Leu Gly Ala Pro Pro Leu Val Ser
                   85                  90                  95
   Ile Ile Met Thr Ser His Asn Thr Glu Lys Phe Ile Glu Ala Ser Ile
               100                 105                 110
   Asn Ser Leu Leu Leu Gln Thr Tyr Asn Asn Leu Glu Val Ile Val Val
           115                 120                 125
   Asp Asp Tyr Ser Thr Asp Lys Thr Phe Gln Ile Ala Ser Arg Ile Ala
       130                 135                 140
   Asn Ser Thr Ser Lys Val Lys Thr Phe Arg Leu Asn Ser Asn Leu Gly
   145                 150                 155                 160
   Thr Tyr Phe Ala Lys Asn Thr Gly Ile Leu Lys Ser Lys Gly Asp Ile
                   165                 170                 175
   Ile Phe Phe Gln Asp Ser Asp Asp Val Cys His His Glu Arg Ile Glu
               180                 185                 190
   Arg Cys Val Asn Ala Leu Leu Ser Asn Lys Asp Asn Ile Ala Val Arg
           195                 200                 205
   Cys Ala Tyr Ser Arg Ile Asn Leu Glu Thr Gln Asn Ile Ile Lys Val
       210                 215                 220
   Asn Asp Asn Lys Tyr Lys Leu Gly Leu Ile Thr Leu Gly Val Tyr Arg
   225                 230                 235                 240
   Lys Val Phe Asn Glu Ile Gly Phe Phe Asn Cys Thr Thr Lys Ala Ser
                   245                 250                 255
   Asp Asp Glu Phe Tyr His Arg Ile Ile Lys Tyr Tyr Gly Lys Asn Arg
               260                 265                 270
   Ile Asn Asn Leu Phe Leu Pro Leu Tyr Tyr Asn Thr Met Arg Glu Asp
           275                 280                 285
   Ser Leu Phe Ser Asp Met Val Glu Trp Val Asp Glu Asn Asn Ile Lys
       290                 295                 300
   Gln Lys Thr Ser Asp Ala Arg Gln Asn Tyr Leu His Glu Phe Gln Lys
   305                 310                 315                 320
   Ile His Asn Glu Arg Lys Leu Asn Glu Leu Lys Glu Ile Phe Ser Phe
                   325                 330                 335
   Pro Arg Ile His Asp Ala Leu Pro Ile Ser Lys Glu Met Ser Lys Leu
               340                 345                 350
   Ser Asn Pro Lys Ile Pro Val Tyr Ile Asn Ile Cys Ser Ile Pro Ser
           355                 360                 365
   Arg Ile Lys Gln Leu Gln Tyr Thr Ile Gly Val Leu Lys Asn Gln Cys
       370                 375                 380
   Asp His Phe His Ile Tyr Leu Asp Gly Tyr Pro Glu Val Pro Asp Phe
   385                 390                 395                 400
   Ile Lys Lys Leu Gly Asn Lys Ala Thr Val Ile Asn Cys Gln Asn Lys
                   405                 410                 415
   Asn Glu Ser Ile Arg Asp Asn Gly Lys Phe Ile Leu Leu Glu Lys Leu
               420                 425                 430
   Ile Lys Glu Asn Lys Asp Gly Tyr Tyr Ile Thr Cys Asp Asp Asp Ile
           435                 440                 445
   Arg Tyr Pro Ala Asp Tyr Ile Asn Thr Met Ile Lys Lys Ile Asn Lys
       450                 455                 460
   Tyr Asn Asp Lys Ala Ala Ile Gly Leu His Gly Val Ile Phe Pro Ser
   465                 470                 475                 480
   Arg Val Asn Lys Tyr Phe Ser Ser Asp Arg Ile Val Tyr Asn Phe Gln
                   485                 490                 495
   Lys Pro Leu Glu Asn Asp Thr Ala Val Asn Ile Leu Gly Thr Gly Thr
               500                 505                 510
   Val Ala Phe Arg Val Ser Ile Phe Asn Lys Phe Ser Leu Ser Asp Phe
           515                 520                 525
   Glu His Pro Gly Met Val Asp Ile Tyr Phe Ser Ile Leu Cys Lys Lys
       530                 535                 540
   Asn Asn Ile Leu Gln Val Cys Ile Ser Arg Pro Ser Asn Trp Leu Thr
   545                 550                 555                 560
   Glu Asp Asn Lys Asn Thr Glu Thr Leu Phe His Glu Phe Gln Asn Arg
                   565                 570                 575
   Asp Glu Ile Gln Ser Lys Leu Ile Ile Ser Asn Asn Pro Trp Gly Tyr
               580                 585                 590
   Ser Ser Ile Tyr Pro Leu Leu Asn Asn Asn Ala Asn Tyr Ser Glu Leu
           595                 600                 605
   Ile Pro Cys Leu Ser Phe Tyr Asn Glu
   610 615
<210> 67
   <211> 2112
   <212> DNA
   <213> Pasteurella multocida
<400> 67
   atgaatacat tatcacaagc aataaaagca tataacagca atgactatca attagcactc 60
   aaattatttg aaaagtcggc ggaaatctat ggacggaaaa ttgttgaatt tcaaattacc 120
   aaatgcaaag aaaaactctc agcacatcct tctgttaatt cagcacatct ttctgtaaat 180
   aaagaagaaa aagtcaatgt ttgcgatagt ccgttagata ttgcaacaca actgttactt 240
   tccaacgtaa aaaaattagt actttctgac tcggaaaaaa acacgttaaa aaataaatgg 300
   aaattgctca ctgagaagaa atctgaaaat gcggaggtaa gagcggtcgc ccttgtacca 360
   aaagattttc ccaaagatct ggttttagcg cctttacctg atcatgttaa tgattttaca 420
   tggtacaaaa agcgaaagaa aagacttggc ataaaacctg aacatcaaca tgttggtctt 480
   tctattatcg ttacaacatt caatcgacca gcaattttat cgattacatt agcctgttta 540
   gtaaaccaaa aaacacatta cccgtttgaa gttatcgtga cagatgatgg tagtcaggaa 600
   gatctatcac cgatcattcg ccaatatgaa aataaattgg atattcgcta cgtcagacaa 660
   aaagataacg gttttcaagc cagtgccgct cggaatatgg gattacgctt agcaaaatat 720
   gactttattg gcttactcga ctgtgatatg gcgccaaatc cattatgggt tcattcttat 780
   gttgcagagc tattagaaga tgatgattta acaatcattg gtccaagaaa atacatcgat 840
   acacaacata ttgacccaaa agacttctta aataacgcga gtttgcttga atcattacca 900
   gaagtgaaaa ccaataatag tgttgccgca aaaggggaag gaacagtttc tctggattgg 960
   cgcttagaac aattcgaaaa aacagaaaat ctccgcttat ccgattcgcc tttccgtttt 1020
   tttgcggcgg gtaatgttgc tttcgctaaa aaatggctaa ataaatccgg tttctttgat 1080
   gaggaattta atcactgggg tggagaagat gtggaatttg gatatcgctt attccgttac 1140
   ggtagtttct ttaaaactat tgatggcatt atggcctacc atcaagagcc accaggtaaa 1200
   gaaaatgaaa ccgatcgtga agcgggaaaa aatattacgc tcgatattat gagagaaaag 1260
   gtcccttata tctatagaaa acttttacca atagaagatt cgcatatcaa tagagtacct 1320
   ttagtttcaa tttatatccc agcttataac tgtgcaaact atattcaacg ttgcgtagat 1380
   agtgcactga atcagactgt tgttgatctc gaggtttgta tttgtaacga tggttcaaca 1440
   gataatacct tagaagtgat caataagctt tatggtaata atcctagggt acgcatcatg 1500
   tctaaaccaa atggcggaat agcctcagca tcaaatgcag ccgtttcttt tgctaaaggt 1560
   tattacattg ggcagttaga ttcagatgat tatcttgagc ctgatgcagt tgaactgtgt   1620
   ttaaaagaat ttttaaaaga taaaacgcta gcttgtgttt ataccactaa tagaaacgtc   1680
   aatccggatg gtagcttaat cgctaatggt tacaattggc cagaattttc acgagaaaaa   1740
   ctcacaacgg ctatgattgc tcaccacttt agaatgttca cgattagagc ttggcattta   1800
   actgatggat tcaatgaaaa aattgaaaat gccgtagact atgacatgtt cctcaaactc   1860
   agtgaagttg gaaaatttaa acatcttaat aaaatctgct ataaccgtgt attacatggt   1920
   gataacacat caattaagaa acttggcatt caaaagaaaa accattttgt tgtagtcaat   1980
   cagtcattaa atagacaagg cataacttat tataattatg acgaatttga tgatttagat   2040
   gaaagtagaa agtatatttt caataaaacc gctgaatatc aagaagagat tgatatctta   2100
   aaagatattt aa                                                       2112
<210> 68
   <211> 5
   <212> PRT
   <213> Artificial Sequence
<220>
   <223> motif
<220>
   <221> MISC_FEATURE
   <222> (1)..(1)
   <223> Asp or Asn
<220>
   <221> MISC_FEATURE
   <222> (5)..(5)
   <223> Ser or Thr
<400> 68
   Xaa Asp Gly Ser Xaa
   1               5
<210> 69
   <211> 5
   <212> PRT
   <213> Artificial Sequence
<220>
   <223> motif
<220>
   <221> MISC_FEATURE
   <222> (4)..(4)
   <223> Asp or Thr
<400> 69
   Asp Ser Asp Xaa Tyr
   1 5
<210> 70
   <211> 565
   <212> PRT
   <213> Pasteurella multocida
<400> 70
   Met Ser Leu Phe Lys Arg Ala Thr Glu Leu Phe Lys Ser Gly Asn Tyr
   1 5 10 15
   Lys Asp Ala Leu Thr Leu Tyr Glu Asn Ile Ala Lys Ile Tyr Gly Ser
   20 25 30
   Glu Ser Leu Val Lys Tyr Asn Ile Asp Ile Cys Lys Lys Asn Ile Thr
   35 40 45
   Gln Ser Lys Ser Asn Lys Ile Glu Glu Asp Asn Ile Ser Gly Glu Asn
   50 55 60
   Lys Phe Ser Val Ser Ile Lys Asp Leu Tyr Asn Glu Ile Ser Asn Ser
   65 70 75 80
   Glu Leu Gly Ile Thr Lys Glu Arg Leu Gly Ala Pro Pro Leu Val Ser
   85 90 95
   Ile Ile Met Thr Ser His Asn Thr Glu Lys Phe Ile Glu Ala Ser Ile
   100 105 110
   Asn Ser Leu Leu Leu Gln Thr Tyr Asn Leu Glu Val Ile Val Val Asp
   115 120 125
   Asp Tyr Ser Thr Asp Lys Thr Phe Gln Ile Ala Ser Arg Ile Ala Asn
   130 135 140
   Ser Thr Ser Lys Val Lys Thr Phe Arg Leu Asn Ser Asn Leu Gly Thr
   145 150 155 160
   Tyr Phe Ala Lys Asn Thr Gly Ile Leu Lys Ser Lys Gly Asp Ile Ile
   165 170 175
   Phe Phe Gln Ser Asp Asp Val Cys His His Glu Arg Ile Glu Arg Cys
   180 185 190
   Val Asn Ala Leu Leu Ser Asn Lys Asp Asn Ile Ala Val Arg Cys Ala
   195 200 205
   Tyr Ser Arg Ile Asn Leu Glu Thr Gln Asn Ile Ile Lys Val Asn Asp
       210                 215                 220
   Asn Lys Tyr Lys Leu Gly Leu Ile Thr Leu Gly Val Tyr Arg Lys Val
   225                 230                 235                 240
   Phe Asn Glu Ile Gly Phe Phe Asn Cys Thr Thr Lys Ala Ser Asp Asp
                   245                 250                 255
   Glu Phe Tyr His Arg Ile Ile Lys Tyr Tyr Gly Lys Asn Arg Ile Asn
               260                 265                 270
   Asn Leu Phe Leu Pro Leu Tyr Tyr Asn Thr Met Arg Glu Asp Ser Leu
           275                 280                 285
   Phe Ser Asp Met Val Glu Trp Val Asp Glu Asn Asn Ile Lys Gln Lys
       290                 295                 300
   Thr Ser Asp Ala Arg Gln Asn Tyr Leu His Glu Phe Gln Lys Ile His
   305                 310                 315                 320
   Asn Glu Arg Lys Leu Asn Glu Leu Lys Glu Ile Phe Ser Phe Pro Arg
                   325                 330                 335
   Ile His Asp Ala Leu Pro Ile Ser Lys Glu Met Ser Lys Leu Ser Asn
               340                 345                 350
   Pro Lys Ile Pro Val Tyr Ile Asn Ile Cys Ser Ile Pro Ser Arg Ile
           355                 360                 365
   Lys Gln Leu Gln Tyr Thr Ile Gly Val Leu Lys Asn Gln Cys Asp His
       370                 375                 380
   Phe His Ile Tyr Leu Asp Gly Tyr Pro Glu Val Pro Asp Phe Ile Lys
   385                 390                 395                 400
   Lys Leu Gly Asn Lys Ala Thr Val Ile Asn Cys Gln Asn Lys Asn Glu
                   405                 410                 415
   Ser Ile Arg Asp Asn Gly Lys Phe Ile Leu Leu Glu Lys Leu Ile Lys
               420                 425                 430
   Glu Asn Lys Asp Gly Tyr Tyr Ile Thr Cys Asp Asp Asp Ile Arg Tyr
           435                 440                 445
   Pro Ala Asp Tyr Thr Asn Thr Met Ile Lys Lys Ile Asn Lys Tyr Asn
       450                 455                 460
   Asp Lys Ala Ala Ile Gly Leu His Gly Val Ile Phe Pro Ser Arg Val
   465 470 475 480
   Asn Lys Tyr Phe Ser Ser Asp Arg Ile Val Tyr Asn Phe Gln Lys Pro
   485 490 495
   Leu Glu Asn Asp Thr Ala Val Asn Ile Leu Gly Thr Gly Thr Val Ala
   500 505 510
   Phe Arg Val Ser Ile Phe Asn Lys Phe Ser Leu Ser Asp Phe Glu His
   515 520 525
   Pro Gly Met Val Asp Ile Tyr Phe Ser Ile Leu Cys Lys Lys Asn Asn
   530 535 540
   Ile Leu Gln Val Cys Ile Ser Arg Pro Ser Asn Trp Leu Thr Glu Asp
   545 550 555 560
   Asn Lys Asn Thr Glu
   565
<210> 71
   <211> 538
   <212> PRT
   <213> Pasteurella multocida
<400> 71
   Ser Asn Ser Glu Leu Gly Ile Thr Lys Glu Arg Leu Gly Ala Pro Pro
   1 5 10 15
   Leu Val Ser Ile Ile Met Thr Ser His Asn Thr Glu Lys Phe Ile Glu
   20 25 30
   Ala Ser Ile Asn Ser Leu Leu Leu Gln Thr Tyr Asn Leu Glu Val Ile
   35 40 45
   Val Val Asp Asp Tyr Ser Thr Asp Lys Thr Phe Gln Ile Ala Ser Arg
   50 55 60
   Ile Ala Asn Ser Thr Ser Lys Val Lys Thr Phe Arg Leu Asn Ser Asn
   65 70 75 80
   Leu Gly Thr Tyr Phe Ala Lys Asn Thr Gly Ile Leu Lys Ser Lys Gly
   85 90 95
   Asp Ile Ile Phe Phe Gln Ser Asp Asp Val Cys His His Glu Arg Ile
   100 105 110
   Glu Arg Cys Val Asn Ala Leu Leu Ser Asn Lys Asp Asn Ile Ala Val
   115 120 125
   Arg Cys Ala Tyr Ser Arg Ile Asn Leu Glu Thr Gln Asn Ile Ile Lys
   130 135 140
   Val Asn Asp Asn Lys Tyr Lys Leu Gly Leu Ile Thr Leu Gly Val Tyr
   145 150 155 160
   Arg Lys Val Phe Asn Glu Ile Gly Phe Phe Asn Cys Thr Thr Lys Ala
   165 170 175
   Ser Asp Asp Glu Phe Tyr His Arg Ile Ile Lys Tyr Tyr Gly Lys Asn
   180 185 190
   Arg Ile Asn Asn Leu Phe Leu Pro Leu Tyr Tyr Asn Thr Met Arg Glu
   195 200 205
   Asp Ser Leu Phe Ser Asp Met Val Glu Trp Val Asp Glu Asn Asn Ile
   210 215 220
   Lys Gln Lys Thr Ser Asp Ala Arg Gln Asn Tyr Leu His Glu Phe Gln
   225 230 235 240
   Lys Ile His Asn Glu Arg Lys Leu Asn Glu Leu Lys Glu Ile Phe Ser
   245 250 255
   Phe Pro Arg Ile His Asp Ala Leu Pro Ile Ser Lys Glu Met Ser Lys
   260 265 270
   Leu Ser Asn Pro Lys Ile Pro Val Tyr Ile Asn Ile Cys Ser Ile Pro
   275 280 285
   Ser Arg Ile Lys Gln Leu Gln Tyr Thr Ile Gly Val Leu Lys Asn Gln
   290 295 300
   Cys Asp His Phe His Ile Tyr Leu Asp Gly Tyr Pro Glu Val Pro Asp
   305 310 315 320
   Phe Ile Lys Lys Leu Gly Asn Lys Ala Thr Val Ile Asn Cys Gln Asn
   325 330 335
   Lys Asn Glu Ser Ile Arg Asp Asn Gly Lys Phe Ile Leu Leu Glu Lys
   340 345 350
   Leu Ile Lys Glu Asn Lys Asp Gly Tyr Tyr Ile Thr Cys Asp Asp Asp
   355 360 365
   Ile Arg Tyr Pro Ala Asp Tyr Thr Asn Thr Met Ile Lys Lys Ile Asn
       370                 375                 380
   Lys Tyr Asn Asp Lys Ala Ala Ile Gly Leu His Gly Val Ile Phe Pro
   385                 390                 395                 400
   Ser Arg Val Asn Lys Tyr Phe Ser Ser Asp Arg Ile Val Tyr Asn Phe
                   405                 410                 415
   Gln Lys Pro Leu Glu Asn Asp Thr Ala Val Asn Ile Leu Gly Thr Gly
               420                 425                 430
   Thr Val Ala Phe Arg Val Ser Ile Phe Asn Lys Phe Ser Leu Ser Asp
           435                 440                 445
   Phe Glu His Pro Gly Met Val Asp Ile Tyr Phe Ser Ile Leu Cys Lys
       450                 455                 460
   Lys Asn Asn Ile Leu Gln Val Cys Ile Ser Arg Pro Ser Asn Trp Leu
   465                 470                 475                 480
   Thr Glu Asp Asn Lys Asn Thr Glu Thr Leu Phe His Glu Phe Gln Asn
                   485                 490                 495
   Arg Asp Glu Ile Gln Ser Lys Leu Ile Ile Ser Asn Asn Pro Trp Gly
               500                 505                 510
   Tyr Ser Ser Ile Tyr Pro Leu Leu Asn Asn Asn Ala Asn Tyr Ser Glu
           515                 520                 525
   Leu Ile Pro Cys Leu Ser Phe Tyr Asn Glu
       530                 535

## Description

### BACKGROUND

### Field of the Invention

The present invention relates to methodology for the production of substantially monodisperse glycosaminoglycan polymersby recombinant heparosan synthases.

Various glycosaminoglycans show potential as non-toxic therapeutic agents to modulate blood coagulation, cancer metastasis, or cell growth. Complex sugars cause biological effects by binding to target proteins including enzymes and receptors. Methodologies to synthesize many compounds, however, and to test for potency and selectivity are limiting steps in drug discovery. Moreover, glycosaminoglycans of different sizes can have dramatically different biological effects. As such, the presently claimed and disclosed invention relates to a chemoenzymatic synthesis methodology to create , glycosaminoglycan polymers which are substantially monodisperse in size.

In addition, new structures or chemical groups may be incorporated into the glycosaminoglycan chain for forming unnatural glycosaminoglycan polymers.

### Description of the Related Art

Polysaccharides are large carbohydrate molecules comprising from about 25 sugar units to thousands of sugar units. Oligosaccharides are smaller carbohydrate molecules comprising less than about 25 sugar units. Animals, plants, fungi and bacteria produce an enormous variety of polysaccharide structures that are involved in numerous important biological functions such as structural elements, energy storage, and cellular interaction mediation. Often, the polysaccharide's biological function is due to the interaction of the polysaccharide with proteins such as receptors and growth factors. The glycosaminoglycan class of polysaccharides and oligosaccharides, which includes heparin, chondroitin, dermatan, keratan, and hyaluronic acid, plays major roles in determining cellular behavior (e.g., migration, adhesion) as well as the rate of cell proliferation in mammals. These polysaccharides and oligosaccharides are, therefore, essential for the correct formation and maintenance of the organs of the human body.

Several species of pathogenic bacteria and fungi also take advantage of the polysaccharide's role in cellular communication. These pathogenic microbes form polysaccharide surface coatings or capsules that are identical or chemically similar to host molecules. For instance, Group A & C *Streptococcus* and Type A *Pasteurella multocida* produce authentic hyaluronic acid capsules, and other *Pasteurella multocida* (Type F and D) and pathogenic *Escherichia coli* (K4 and K5) are known to make capsules composed of polymers very similar to chondroitin and heparin. The pathogenic microbes form the polysaccharide surface coatings or capsules because such a coating is nonimmunogenic and protects the bacteria from host defenses, thereby providing the equivalent of molecular camouflage.

Enzymes alternatively called synthases, synthetases, or transferases, catalyze the polymerization of polysaccharides found in living organisms. Many of the known enzymes also polymerize activated sugar nucleotides. The most prevalent sugar donors contain UDP, but ADP, GDP, and CMP are also used depending on (1) the particular sugar to be transferred and (2) the organism. Many types of polysaccharides are found at, or outside of, the cell surface. Accordingly, most of the synthase activity is typically associated with either the plasma membrane on the cell periphery or the Golgi apparatus membranes that are involved in secretion. In general, these membrane-bound synthase proteins are difficult to manipulate by typical procedures, and only a few enzymes have been identified after biochemical purification.

A larger number of synthases have been cloned and sequenced at the nucleotide level using reverse genetic approaches in which the gene or the complementary DNA (cDNA) was obtained before the protein was characterized. Despite this sequence information, the molecular details concerning the three-dimensional native structures, the active sites, and the mechanisms of catalytic action of the polysaccharide synthases, in general, are very limited or absent. For example, the catalytic mechanism for glycogen synthesis is not yet known in detail even though the enzyme was discovered decades ago. In another example, it is still a matter of debate whether most of the enzymes that produce heteropolysaccharides utilize one UDP-sugar binding site to transfer both precursors, or alternatively, if there exist two dedicated regions for each substrate.

A wide variety of polysaccharides are commercially harvested from many sources, such as xanthan from bacteria, carrageenans from seaweed, and gums from trees. This substantial industry supplies thousands of tons of these raw materials for a multitude of consumer products ranging from ice cream desserts to skin cream cosmetics. Vertebrate tissues and pathogenic bacteria are the sources of more exotic polysaccharides utilized in the medical field e.g., as surgical aids, vaccines, and anticoagulants. For example, two glycosaminoglycan polysaccharides, heparin from pig intestinal mucosa and hyaluronic acid from rooster combs, are employed in several applications including clot prevention and eye surgery, respectively. Polysaccharides extracted from bacterial capsules (e.g., various *Streptococcus pneumoniae* strains) are utilized to vaccinate both children and adults against disease with varying levels of success. However, for the most part, one must use the existing structures found in the raw materials as obtained from nature. In many of the older industrial processes, chemical modification (e.g., hydrolysis, sulfation, deacetylation) is used to alter the structure and properties of the native polysaccharide. However, the synthetic control and the reproducibility of large-scale reactions are not always successful. Additionally, such polysaccharides are only available having a large molecular weight distribution, and oligosaccharides of the same repeat units are notavailable.

Some of the current methods for designing and constructing carbohydrate polymers *in vitro* utilize: (i) difficult, multistep sugar chemistry, or (ii) reactions driven by transferase enzymes involved in biosynthesis, or (iii) reactions harnessing carbohydrate degrading enzymes catalyzing transglycosylation or hydrolysis. The latter two methods are often restricted by the specificity and the properties of the available naturally occurring enzymes. Many of these enzymes are neither particularly abundant nor stable but are almost always expensive. Overall, the procedures currently employed yield polymers containing between 2 and about 12 sugars. Unfortunately, many of the physical and biological properties of polysaccharides do not become apparent until the polymer contains 25, 100, or even thousands of monomers.

As stated above, polysaccharides are the most abundant biomaterials on earth, yet many of the molecular details of their biosynthesis and function are not clear. Hyaluronic acid or HA is a linear polysaccharide of the glycosaminoglycan class and is composed of up to thousands of β(1,4)GlcUA-β(1,3)GlcNAc repeats. In vertebrates, HA is a major structural element of the extracellular matrix and plays roles in adhesion and recognition. HA has a high negative charge density and numerous hydroxyl groups, therefore, the molecule assumes an extended and hydrated conformation in solution. The viscoelastic properties of cartilage and synovial fluid are, in part, the result of the physical properties of the HA polysaccharide. HA also interacts with proteins such as CD44, RHAMM, and fibrinogen thereby influencing many natural processes such as angiogenesis, cancer, cell motility, wound healing, and cell adhesion.

There are numerous medical applications of HA. For example, HA has been widely used as a viscoelastic replacement for the vitreous humor of the eye in ophthalmic surgery during implantation of intraocular lenses in cataract patients. HA injection directly into joints is also used to alleviate pain associated with arthritis. Chemically cross-linked gels and films are also utilized to prevent deleterious adhesions after abdominal surgery. Other researchers using other methods have demonstrated that adsorbed HA coatings also improve the biocompatibility of medical devices such as catheters and sensors by reducing fouling and tissue abrasion.

HA is also made by certain microbes that cause disease in humans and animals. Some bacterial pathogens, namely Gram-negative *Pasteurella multocida* Type A and Gram-positive *Streptococcus* Group A and C, produce an extracellular HA capsule which protects the microbes from host defenses such as phagocytosis. Mutant bacteria that do not produce HA capsules are 10²- and 10³-fold less virulent in comparison to the encapsulated strains. Furthermore, the *Paramecium bursaria* Chlorella virus (PBCV-1) directs the algal host cells to produce a HA surface coating early in infection.

The various HA synthases ( HAS ), the enzymes that polymerize HA, utilize UDP-GlcUA and UDP-GIcNAc sugar nucleotide precursors in the presence of a divalent Mn, Mg, or Co ion to polymerize long chains of HA. The HA chains can be quite large (n=10² to 10⁴). Jing et al. discuss defined megadalton hyaluronan polymer standards. In particular, the HASs are membrane proteins localized to the lipid bilayer at the cell surface. During HA biosynthesis, the HA polymer is transported across the bilayer into the extracellular space. In all HASs, a single species of polypeptide catalyzes the transfer of two distinct sugars. In contrast, the vast majority of other known glycosyltransferases transfer only one monosaccharide.

HasA (or spHAS) from Group A *Streptococcus pyogenes* was the first HA synthase to be described at the molecular level. The various vertebrate homologs (*Xenopus* DG42 or XIHAS1; murine and human HAS1, HAS2, and HAS3) and the viral enzyme, A98R, are quite similar at the amino acid level to certain regions of the HasA polypeptide chain (-30% identity overall) and were discovered only after the sequence of spHAS was disclosed in 1994. At least 7 short motifs (5-9 residues) interspersed throughout these Class I enzymes are identical or quite conserved. The evolutionary relationship among these HA synthases from such dissimilar sources is not clear at present. The enzymes are predicted to have a similar overall topology in the bilayer: membrane-associated regions at the amino and the carboxyl termini flank a large cytoplasmic central domain (-200 amino acids). The amino terminal region appears to contain two transmembrane segments, while the carboxyl terminal region appears to contain three to five membrane-associated or transmembrane segments, depending on the species. Very little of these HAS polypeptide chains are expected to be exposed to the outside of the cell.

With respect to the reaction pathway utilized by this group of enzymes, mixed findings have been reported from indirect experiments. The Group A streptococcal enzyme was reported to add sugars to the nonreducing terminus of the growing chain as determined by selective labeling and degradation studies. Using a similar approach, however, two laboratories working with the enzyme preparations from mammalian cells concluded that the new sugars were added to the reducing end of the nascent chain. In comparing these various studies, the analysis of the enzymatically-released sugars from the streptococcal system added more rigorous support for their interpretation. In another type of experiment, HA made in mammalian cells was reported to have a covalently attached UDP group as measured by an incorporation of low amounts of radioactivity derived from ³²P-labeled UDP-sugar into an anionic polymer. This data implied that the last sugar was transferred to the reducing end of the polymer. Thus, it remains unclear if these rather similar HAS polypeptides from vertebrates and streptococci actually utilize different reaction pathways.

On the other hand, the Class II HAS, pmHAS, has many useful catalytic properties including the ability to elongate exogenous acceptors at the non-reducing end with HA chains. The chondroitin synthase, pmCS, and the heparosan synthases, pmHS1 and pmHS2, are also useful, but they add chondroitin or heparosan chains to the acceptor's non-reducing terminus, respectively.

Chondroitin is one of the most prevalent glycosaminoglycans (GAGs) in vertebrates as well as part of the capsular polymer of Type F P. *multocida,* a minor fowl cholera pathogen. This bacterium produces unsulfated chondroitin (DeAngelis et al., 2002) but animals possess sulfated chondroitin polymers. The first chondroitin synthase from any source to be molecularly cloned was the *P. multocida* pmCS (DeAngelis and Padgett-McCue, 2000). The pmCS contains 965 amino acid residues and is about 90% identical to pmHAS. A soluble recombinant *Escherichia* coli-derived pmCS¹⁻⁷⁰⁴ catalyzes the repetitive addition of sugars from UDP-GaINAc and UDP-GIcUA to chondroitin oligosaccharide acceptors *in vitro.*

Heparosan [N-acetylheparosan], (- GlcUA-β1,4-GlcNAc-α1,4-), is the repeating sugar backbone of the polysaccharide found in the capsule of certain pathogenic bacteria as well as the biosynthetic precursor of heparin or heparan sulfate found in animals from hydra to vertebrates. In mammals, the sulfated forms bind to a variety of extremely important polypeptides including hemostasis factors (e.g., antithrombin III, thrombin), growth factors (e.g., EGF, VEGF), and chemokines (e.g., IL-8, platelet factor 4) as well as the adhesive proteins for viral pathogens (e.g., herpes, Dengue fever). Currently, heparin is extracted from animal tissue and used as an anticoagulant or antithrombotic drug. In the future, similar polymers and derivatives should also be useful for pharmacological intervention in a variety of pathologic conditions including neoplasia and viral infection.

Several enzyme systems have been identified that synthesize heparosan. In bacteria, either a pair of two separate glycosyltransferases (*Escherichia coli* KfiA and KfiC) or a single glycosyltransferase (*Pasteurella multocida* PmHS1 or PmHS2; DeAngelis & White, 2002, 2004) have been shown to polymerize heparosan; the enzymes from both species are homologous at the protein level. In vertebrates, a pair of enzymes, EXT 1 and EXT 2, that are not similar to the bacterial systems appear to be responsible for producing the repeating units of the polymer chain which is then subsequently modified by sulfation and epimerization.

The heparosan synthases from *P. multocida* possess both a hexosamine and a glucuronic acid transfer site in the same polypeptide chain, as shown by mutagenesis studies (Kane, T.A. et. al, J. Biol. Chem. 2006), and are therefore referred to as "dual-action" or bifunctional glycosyltransferases. These enzymes are complex because they employ both an inverting and a retaining mechanism when transferring the monosaccharide from UDP precursors to the non-reducing terminus of a growing chain. The two *Pasteurella* heparosan synthases, PmHS1 and PmHS2, are approximately 70% identical at the amino acid sequence level. The two genes are found in different regions of the bacterial chromosome: PmHS1 (*hssA*) is associated with the prototypical Gram-negative Type II carbohydrate biosynthesis gene locus but PmHS2 (*hssB*) resides far removed in an unspecialized region. As shown herein, these catalysts have useful catalytic properties that may be harnessed by the hand of man. Deangelis et al. discuss chemoenzymatic synthesis of glycosaminoglycans with Pasteurella synthases. Avci et al. discuss enzymatic synthesis of glycosaminoglycans: improving on nature. Sismey-Ragatz et al. discuss chemoenzymatic synthesis with distinct Pasteurella heparosan synthases.

WO2006/033693 discloses methods of selectively treating diseases with specific glycosaminoglycan polymers. To facilitate the development of biotechnological medical improvements, the present invention provides a method for enzymatically producing glycosaminoglycans polymers which are substantially monodisperse and thus have a defined size distribution.

Specifically, in order to overcome the disadvantages and defects of the prior art, the present invention uses a recombinant heparosan synthase having the amino acid sequence as set forth in SEQ ID NO: 6 or an amino acid sequence at least 98% identical to the sequence of SEQ ID N0:66 that have the ability to produce unnatural polymers. An advantage of these enzymes is that their altered specificity allows new useful groups or units to be added to the polymer. Thus, the present invention results in (1) the targeting of specific, desirable size distributions or size ranges; (2) the synthesis of monodisperse (narrow size distribution) polymers; and (3) the creation of new, unnatural polymers with altered chemical groups.

### BRIEF DESCRIPTION OF THE SEVERAL VIEWS OF THE DRAWINGS

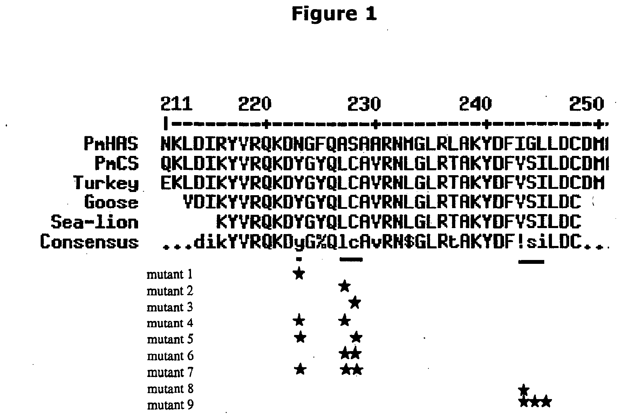

FIG. 1 depicts a comparison of partial primary sequences of pmHAS and different pmCSs. Primary sequences of presumably chondroitin synthases from different Type F *Pasteurella multocida* were obtained by directly sequencing the products of colony-lysis PCR. The MULTALIN alignment indicates that most of the differences between pmHAS and pmCS are conserved among these independent strains. Residues that were substituted in site-mutagenesis studies were underlined. The mutant polypeptides contain a single or combination of different mutations, indicated by *star(s).* None of these mutations changes the specificity of the original enzymes.

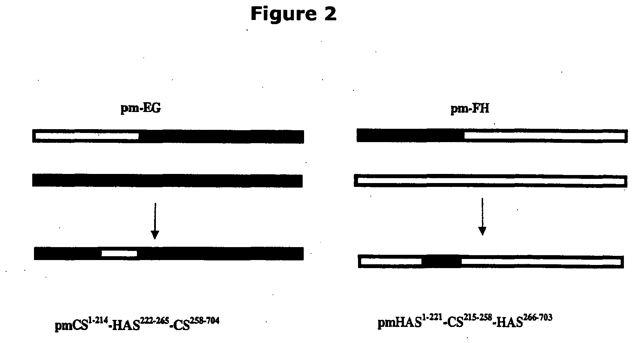

FIG. 2 depicts chimeric constructs of pmHAS¹⁻²²¹-CS²¹⁵⁻²⁵⁸-HAS²⁶⁶⁻⁷⁰³ and pmCS¹⁻²¹⁴-HAS²²²⁻²⁶⁵ -CS²⁵⁸⁻⁷⁰⁴. Pm-FH and pPm7A DNA were used to create pmHAS¹⁻²²¹-CS²¹⁵⁻²⁵⁸-HAS²⁶⁶⁻⁷⁰³. A very interesting result was that pmCS¹⁻²¹⁴ -HAS²²²⁻²⁶⁵ -CS²⁵⁸⁻⁷⁰⁴ can transfer both GalNAc and GlcNAc to HA oligomer acceptor; this enzyme displays relaxed sugar specificity.

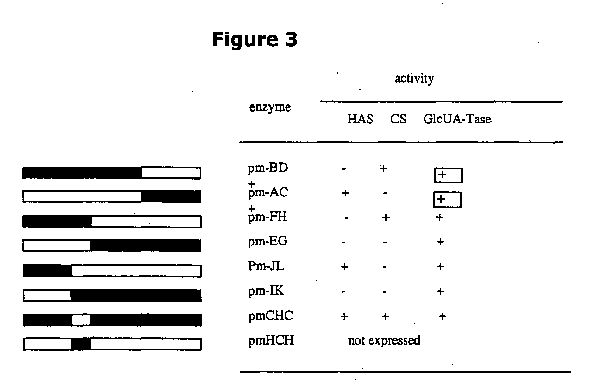

FIG. 3 depicts a summary of enzyme activities of chimeric proteins. The enzymes are drawn as bars. *Black* bars represent pmCS. *White* bars represent pmHAS. +, active; -, inactive. PmCHC represents pmCS¹⁻²¹⁴-HAS²²²⁻²⁶⁵ -CS²⁵⁸⁻⁷⁰⁴. The roles of the two domains are confirmed and we have localized a 44-residue region critical for distinguishing C4 epimers of the hexosamine precursor.

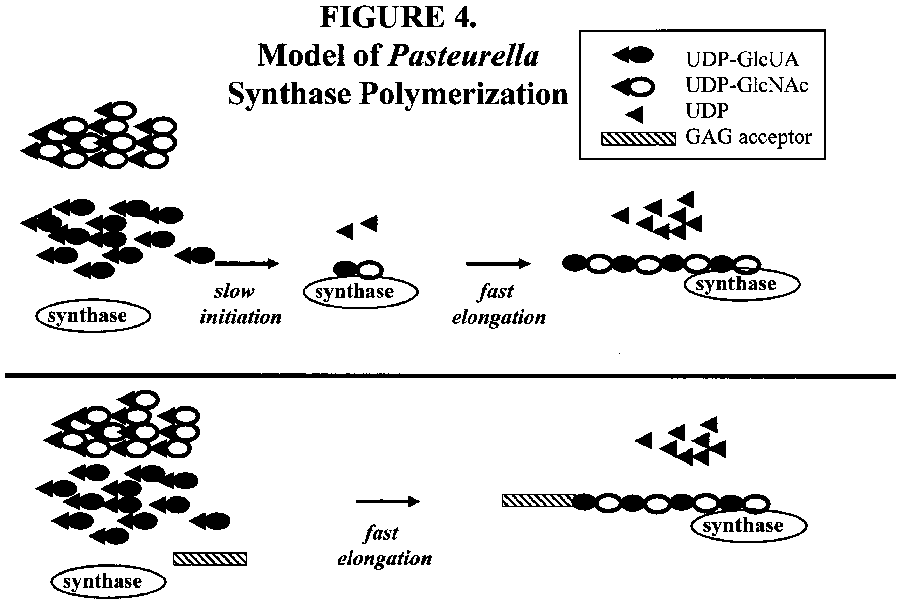

FIG. 4 is a graphical representation of a model of *Pasteurella* synthase polymerization. It is important to note that other uronic acid or hexosamine precursors may be combined or substituted as well. In addition, other acceptor molecules can substitute as the primer for reaction synchronization and sizecontrol.

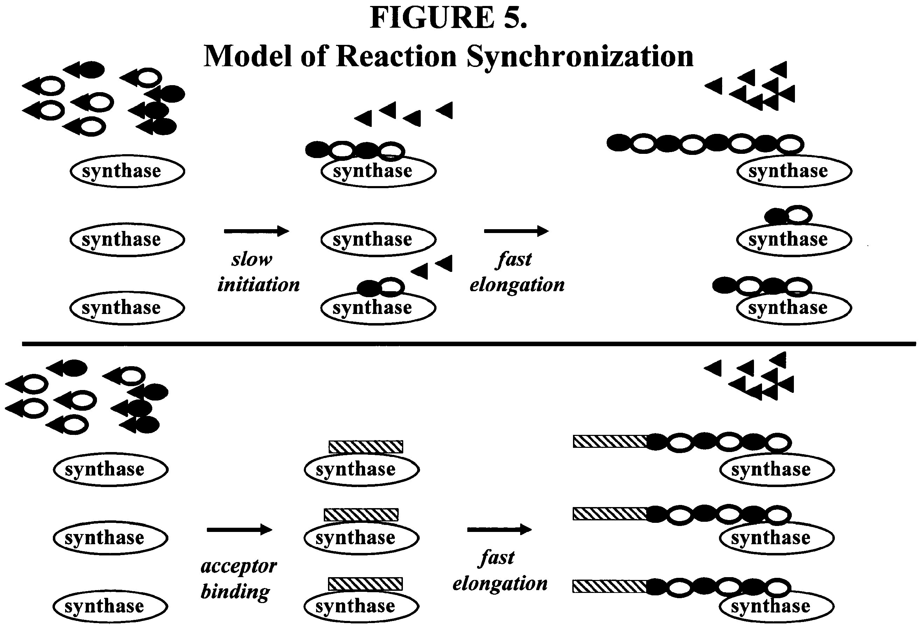

FIG. 5 is a graphical representation of a model of reaction synchronization.

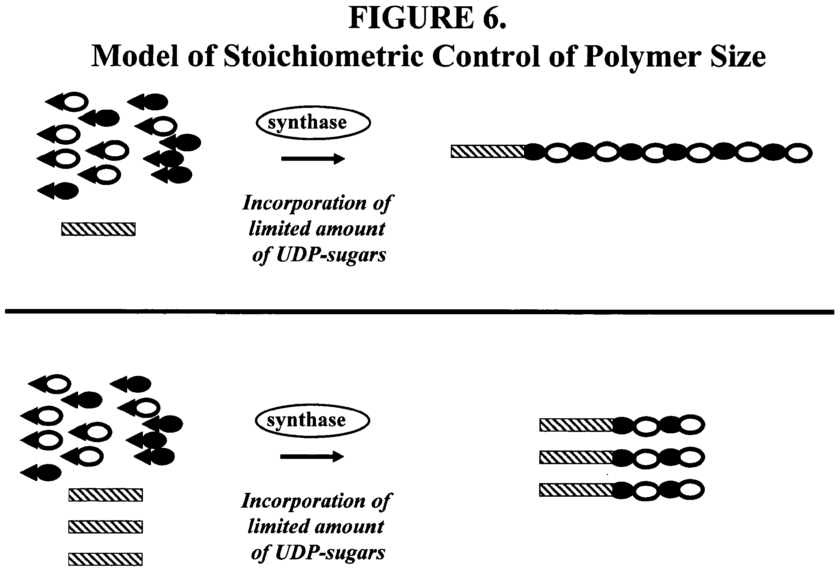

FIG. 6 is a graphical representation of a model of stoichiometric control of polymer size.

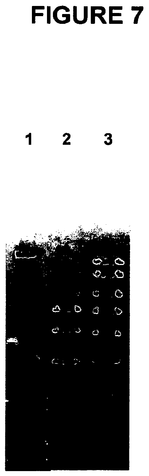

FIG. 7 is an electrophoresis gel illustrating that *in vitro* generated HA can reach the molecular mass of 1.3 MDa. Lane 2, Bio-Rad 1 kilobase DNA ruler with the top band of 15 kb. Lane 3, Bioline DNA hyperLadder with the top band of 10kb.

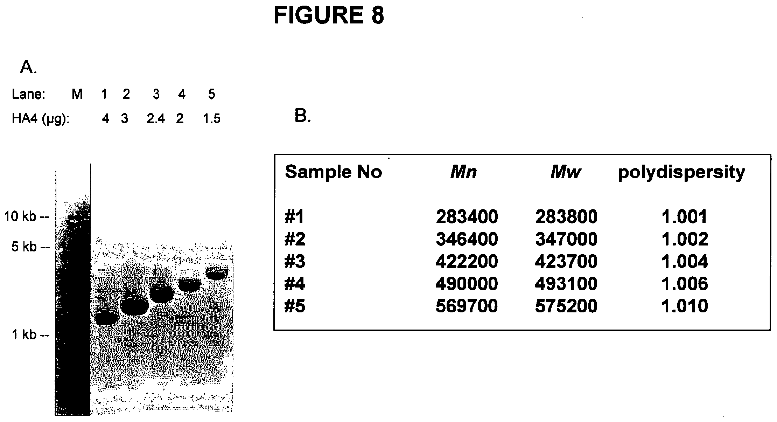

FIG. 8 is a graphical representation illustrating control of HA product size by acceptor concentration. 100 µl of reactions were setup with 0.7 µg/µl of pmHAS, 32mM of UDP-GlcNAc, 32mM of UDP-GIcUA and decreasing amount of HA4. HA were purified, and 1µg of each sample were loaded on a 1.2% agarose gel (A). The molecular mass of HA were determined by MALLS and the results were listed in the table (B). The item numbers in the table correspond to lane number in Panel A. M, Bioline DNA HyperLadder.

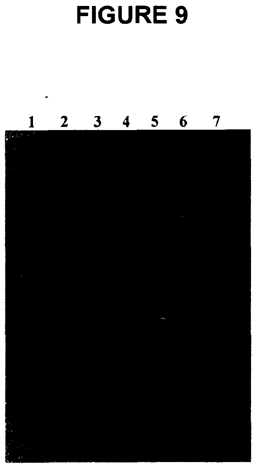

FIG. 9 is an electrophoresis gel illustrating *in vitro* synthesis of fluorescent HA. 20 µl of reactions were setup with 2 µg/µl of pmHAS various amounts of fluorescent HA4 and UDP-sugars. Reaction products were analyzed with 0.8% agarose gel electrophoresis and viewed under UV light.

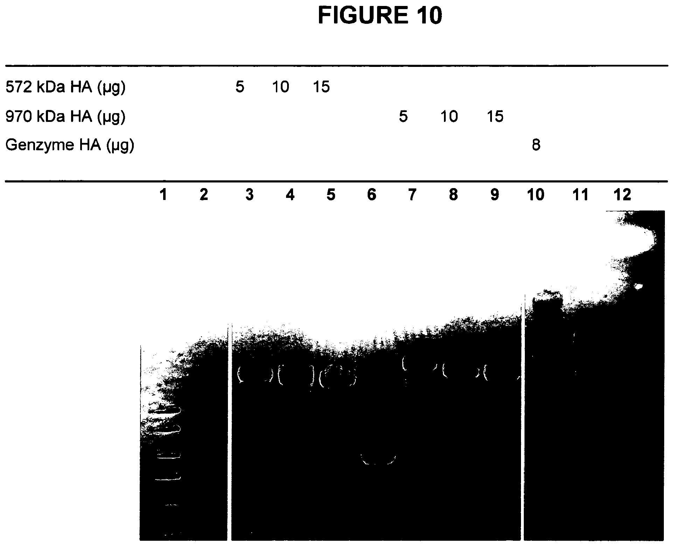

FIG. 10 is an electrophoresis gel illustrating utilization of large HA acceptors. Reactions were carried out at 30°C for 48 hours. The 60 µl reaction contained 0.28 µg/µl of pmHAS, 3.3 mM UDP-GlcNAc, 3.3 mM UDP-GIcUA and without (lane 2) or with various amounts of acceptors (lanes 3-5, 7-9 and 10). 1.0 µl of each reaction was loaded on 0.7% agarose gel and stained with STAINS-ALL. Lane 1, BIORAD kb ladder (top band is 15 kb). Lane 6, 0.5 µg of 970 kDa HA starting acceptor. Lane 11, 3 µg of Genzyme HA starting acceptor. Lane 12, Invitrogen DNA HyperLadder (top band is 48.5 kB).

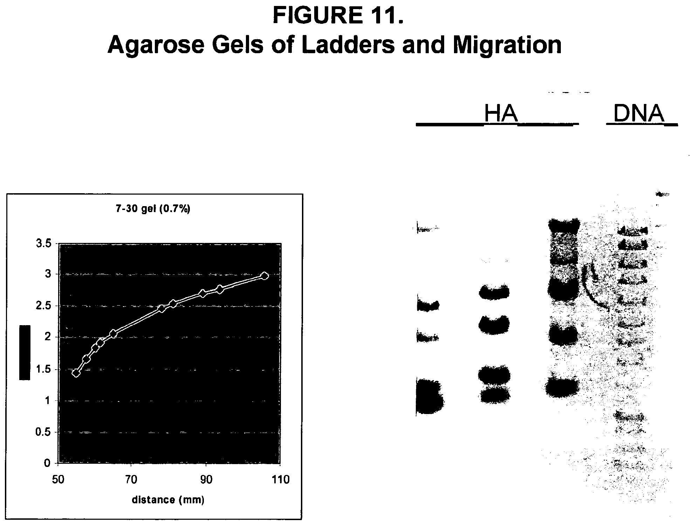

FIG. 11 is an electrophoresis gel that illustrates the migration of a ladder constructed of HA of defined size distribution for use as a standard.

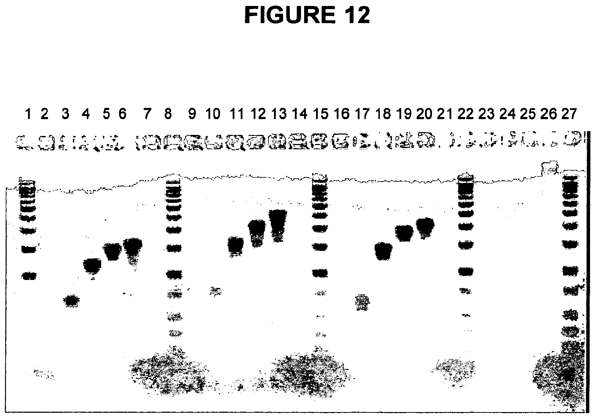

FIG. 12 is an electrophoresis gel illustrating various mondisperse chondroitin sulfate HA hybrid GAGs. The 1% agarose gel stained with STAINS-ALL shows a variety of chondroitin sulfates (either A, B or C) that were elongated with pmHAS, thus adding HA chains. Lanes 1, 8, 15, 22 and 27 contain the Kilobase DNA ladder; lanes 2 and 7 contain starting CSA, while lanes 3-6 contain CSA-HA at 2 hrs, 4 hrs, 6 hrs and O/N, respectively; lanes 9 and 14 contain starting CSB, while lanes 10-13 contain CSB-HA at 2 hrs, 4 hrs, 6 hrs and O/N, respectively; lanes 16 and 21 contain starting CSC, while lanes 17-20 contain CSC-HA at 2 hrs, 4 hrs, 6 hrs and O/N, respectively; lanes 23-26 contain no acceptor at 2 hrs, 4 hrs, 6 hrs and O/N, respectively.

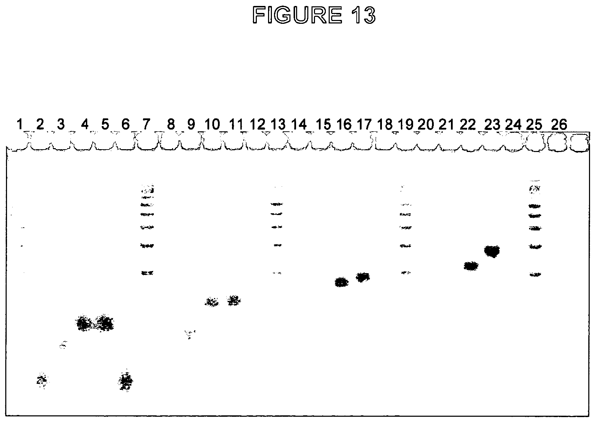

FIG. 13 is an electrophoresis gel illustrating control of hybrid GAG size by stoichiometric control. The 1% agarose gel stained with STAINS-ALL shows chondroitin sulfate A that was elongated with pmHAS, thus adding HA chains. Lanes 1, 7, 13, 19 and 25 contain the Kilobase ladder; lanes 2 and 6 contain 225 µg starting CSA, while lanes 3-5 contain 225 µg CSA-HA at 2 hrs, 6 hrs and O/N, respectively; lanes 8 and 12 contain 75 µg starting CSA, while lanes 9-11 contain 75 µg CSA-HA at 2 hrs, 6 hrs and O/N, respectively; lanes 14 and 18 contain 25 µg starting CSA, while lanes 15-17 contain 25 µg CSA-HA at 2 hrs, 6 hrs and O/N, respectively; lanes 20 and 24 contain 8.3 µg starting CSA, while lanes 21-23 contain 8.3 µg CSA-HA at 2 hrs, 6 hrs and O/N, respectively.

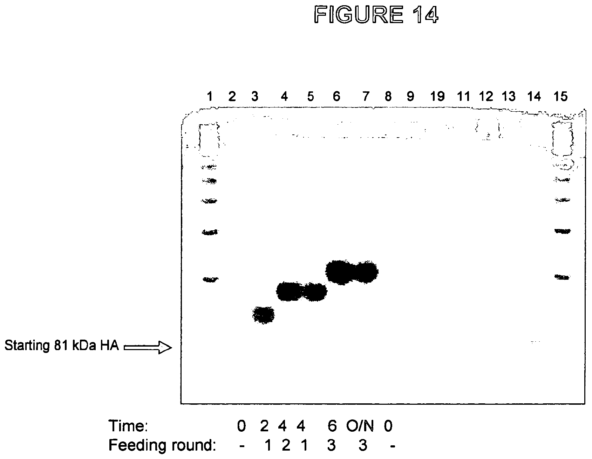

FIG. 14 is an electrophoresis gel illustrating extension of HA with chondroitin chains using pmCS. The 1.2% agarose gel stained with STAINS-ALL shows a reaction with pmCS and UDP-GIcUA, UDP-GaINAc with either an 81 kDa HA acceptor (lanes 3-7) or no acceptor (lanes 9-13). Some reactions were "fed" UDP-sugar during the reaction at various times. Lanes 1 and 15 contain the Kilobase DNA standard. Lanes 2, 8 and 14 contain starting 81 kDa HA. Lanes 3-7: contain HA acceptor +HA-C at 2 hr, 4 hr, 4 hr (set O/N in incubator without 4 hr feeding), 6 hr and O/N, respectively. Lanes 9-13: contain no acceptor (minus) - HA-C at 2 hr, 4 hr, 4 hr (set O/N in incubator without 4 hr feeding), 6 hr and O/N, respectively.

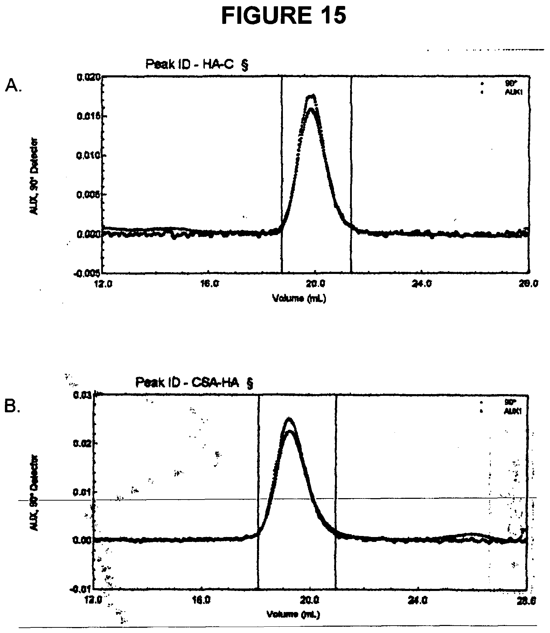

FIG. 15 illustrates size exclusion (or gel filtration) chromatography analysis coupled with multi-angle laser light scattering detection, which confirms the monodisperse nature of polymers. In A, HA (starting MW 81 kDa) extended with chondroitin chains using pmCS (same sample used in Fig 14, lane #7, overnight [O/N] extension) was analyzed; the material was 280,000 Mw and polydispersity (Mw/Mn) was 1.003 +/- 0.024. Chondroitin sulfate extended with HA chains using pmHAS (same sample used in Fig 13, lane #23) was analyzed and shown in the bottom chromatogram; the material was 427,000 Mw and polydispersity (Mw/Mn) was 1.006 +/- 0.024.

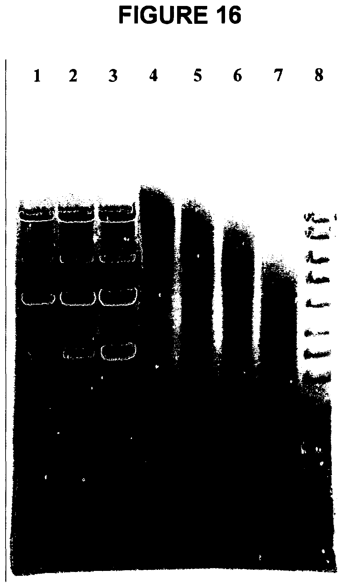

FIG. 16 is an 0.7% agarose gel detected with Stains-all that compares the monodisperse, 'select HA' to commercially produced HA samples. The defined nature of 'selectHA' (the products in lanes 1-3) are evident compared to other extracted commercial HA in lanes 4-7 (DNA standard, lane 8).

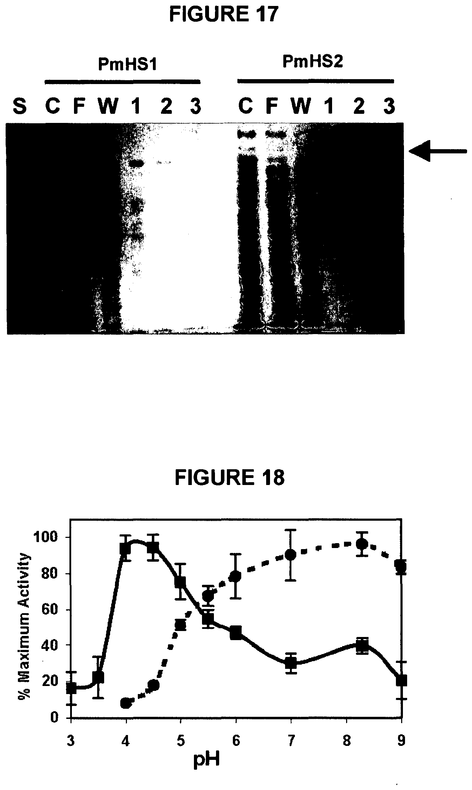

FIG. 17 is a gel analysis of recombinant heparosan synthase proteins (maltose binding protein (MBP) - PmHS fusions). This Coomassie Blue-stained polyacrylamide gel (8%) depicts substantial purification of the two enzymes by affinity chromatography on immobilized amylose. Lanes: S, molecular mass standards (top to bottom 150, 100, 75, 50, 37 kDa); C, starting *E. coli* lysate; F, flow through; W, wash; 1, 2, 3, eluted fractions from amylose column. The bands marked with an arrow are the appropriate molecular weight for the MBP-PmHS fusion constructs (∼113 kDa) and are immunoreactive with anti-PmHS peptide antibody (data not shown). The eluted protein possesses heparosan polymerization activity; the majority of lower molecular weight bands are degradation products that are immunoreactive with anti-heparosan synthase and anti-maltose binding protein antibodies.
FIG. 18 depicts pH dependence of PmHS1 and PmHS2 polymerization activity. The incorporation of [³H]GlcUA into a polysaccharide catalyzed by either PmHS1 or PmHS2 (∼1.5 µg) was measured in polymerization reactions buffered at different pH values. Sodium acetate was used for pH 3-7 and Tris HCI was used for pH 7-9. The assay with the maximal activity was set to 100% to normalize the plot. Three independent reactions were performed; standard deviation is shown. PmHS1 (dotted line, circles) operates best at neutral pH, but PmHS2 (solid line, squares) works faster at acidic pH.

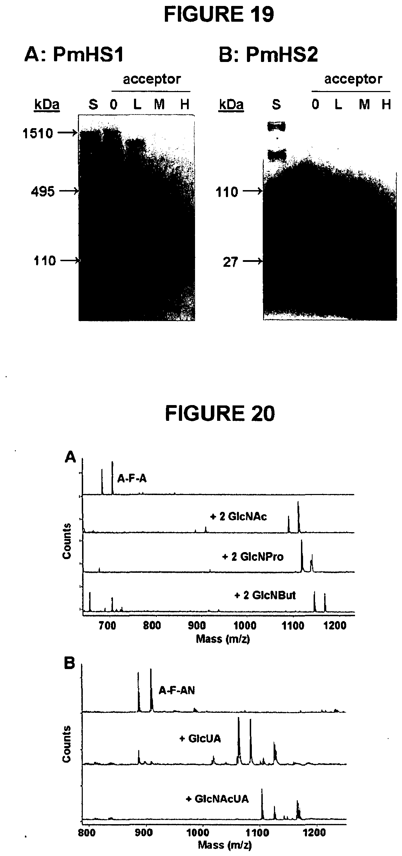

FIG. 19 is an agarose gel analysis of monodisperse heparosan polymers. Increasing amounts of heparosan oligosaccharide (n = 2, 3) acceptor (lanes: 0, none; Low, 0.23 nM; Medium, 2.3 nM; High, 22 nM final conc.) were added to 40 µl reactions containing 5 mM UDP-GIcUA, 5 mM UDP-GIcNAc and 13 µg of heparosan synthase catalyst. Polymer (20 µl portion) was analyzed by agarose gel electrophoresis with Stains-All detection. Panel A: PmHS1, 1.2% gel (S, Select-HA^{™} LoLadder and HiLadder). Panel B: PmHS2, 3% gel (S, Select-HA^{™} LoLadder). All polymers were sensitive to heparin lyase III (not shown). The average molecular masses were determined by SEC-MALLS. PmHS1 forms products with a narrow size distribution (polydispersity *M_{w}*/*Mₙ* = 1.06 to 1.18; for reference, the value of an ideal monodisperse polymer is 1) and may be readily stoichiometrically controlled (as indicated by the three different size bands of 800 kDa, 380 kDa, and 100 kDa (L, M and H lanes, respectively)). On the other hand, PmHS2 in the presence of acceptor makes somewhat more polydisperse samples (*M_{w}*/*Mₙ* = 1.1 to 1.63) with lower molecular weight (28 kDa, 24 kDa and 8 kDa(L, M and H lanes, respectively)) and it is more difficult to control of the final polymer size.
FIG. 20 depicts mass spectral analyses of PmHS2-catalyzed single sugar addition of UDP-sugar analogs. The usage of UDP-substrates was detected by the formation of the target compound with the appropriate negative ion molecular mass by MALDI-ToF MS. In each spectrum, the larger molecular weight peak (+ 22 Da) corresponds to the addition of sodium instead of a proton to the carboxylate. Panel A: PmHS2 (∼1-2 µg, 8 µl reaction, 30°C, -6-12 hrs) catalyzed the transfer of monosaccharide from various UDP-hexosamines (UDP-GIcNAc, UDP-GIcNPro or UDP-GIcNBut; -1-3 mM final) to a synthetic GIcUA-terminated acceptor, A-F-A (∼0.6 mM; predicted 683.13 Da, observed 683.13 Da) to form longer molecules (A-F-A+2 GlcNAc product, predicted 1089.29 Da, observed 1089.12 Da; A-F-A+2 GlcNPro product, predicted 1117.32 Da, observed 1117.88 Da; A-F- A+2 GlcNBut product, predicted 1145.35 Da, observed 1145.19 Da). Panel B: PmHS2 was tested with UDP-uronic acids (UDP-GIcUA or UDP-GIcNAcUA) and a synthetic GlcNAc- terminated acceptor, A-F-AN (predicted 886.21 Da, observed 886.09 Da), using the same conditions described above (A-F-AN+GlcUA product, predicted 1062.24 Da, observed 1062.03 Da; A-F-AN+2 GlcNAcUA product, predicted 1103.25 Da, observed 1103.10 Da). PmHS2 can mis-incorporate a variety of unnatural analogs.

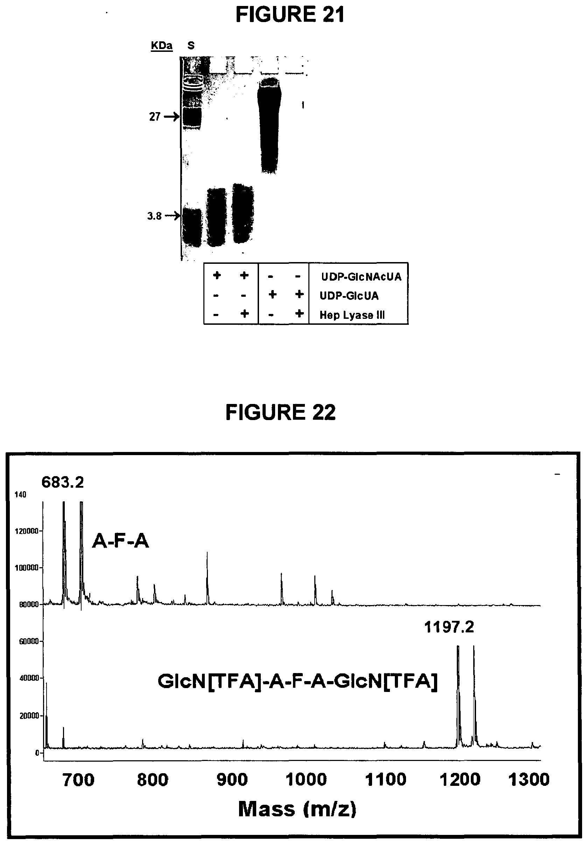

FIG. 21 depicts heparin lyase challenge of native and analog polymers. Two different polymers were synthesized with PmHS2 using UDP-GIcNAc and one of the indicated UDP-uronic acids (either UDP-GIcNAcUA analog or natural UDP-GIcUA). Half of the polymer sample was subjected to heparin lyase III treatment overnight before analysis on a 15% polyacrylamide gel (S, Select-HA^{™} LoLadder and nanoHA₁₀₋₂₀^{™} ladder; key sizes denoted in kDa). The GlcNAcUA-containing polymer was resistant to digestion while the native heparosan was totally degraded.
FIG. 22 depicts mass spectral analyses of PmHS2 -catalyzed single sugar addition of UDP-GIcN[TFA]. PmHS2 (~1-2 µg, 8 µl reaction, 30°C, -6-12 hrs) catalyzed the addition of GlcNTFA to the nonreducing termini of a GIcUA-terminated synthetic glycoside acceptor, A-FA (∼0.6 mM) (Eq.3). This was detected by MALDI-ToF MS and is evident by the formation of a peak with the expected larger mass (predicted exact mass 1197.18; observed mass 1197.21). The same type of result was observed forPmHAS.

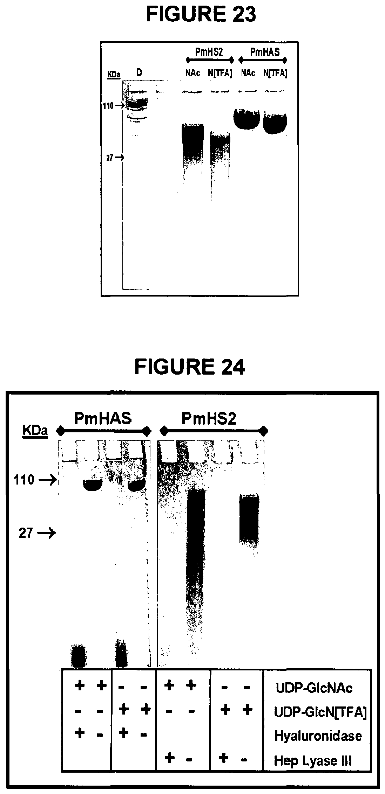

FIG. 23 depicts PAGE analyses of GIcN[TFA] containg polymers synthesized by PmHS2 and PmHAS. PmHS2 or PmHAS (-12 and 100 µg) respectively, were incubated with 25 mM UDP-GIcUA and either UDP-GlcNAc (NAc) or UDP-GIcN[TFA] (N[TFA]) at 30°C, overnight. Reactions were run on polyacrylamide gels (12%) and polymers were detected by Alcian Blue stain. Natural and unnatural polymers were synthesized by the *Pasteurella* enzymes with approximately equal sizes and yields. (D; DNA standard; the position of the HA standards 110 and 27 kDa are depicted with arrows).
FIG. 24 depicts lyase challenge of Natural and GIcN[TFA] containing polymers. Two different polymers were synthesized with PmHS2 or PmHAS using UDP-GIcUA and one of the indicated UDP-hexosamine sugars (either UDP-GIcN[TFA] analog or natural UDP-GIcNAc). Half of the polymer sample was subjected to hyaluronidase or heparosan lyase III treatment. Key sizes denoted in kDa. The GIcN[TFA]-containing polymers were not resistant to digestion.

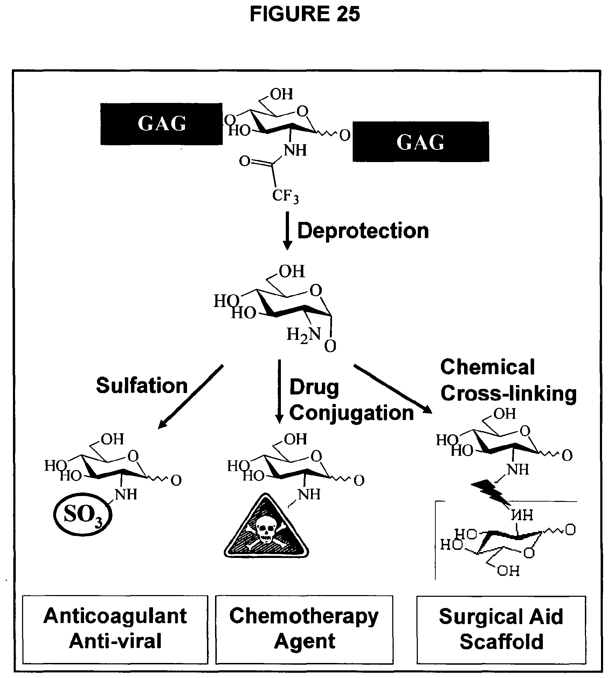

FIG. 25 is a diagram of GIcN[TFA] deprotection and potential medical applications. The GIcN[TFA] sugar can be added to any position within a polymer or oligosaccharide. The TFA group on the hexoasmine sugar can be deprotected with base treatment. This produces a primary amine that is potentially the site for N-sulfation, coupling to drugs and cross-linking site to form a gel; these applications are examples, and other chemistries and theraputics may also be employed.

### DETAILED DESCRIPTION OF THE INVENTION

The invention provides methods for enzymatically producing substantially monodisperse glycosaminoglycan polymers comprising providing at least one functional acceptor and at least one recombinant heparosan synthase capable of elongating the functional acceptor in a controlled and/or repetitive fashion to form extended glycosaminoglycan-like molecules, wherein the recombinant heparosan synthase has an amino acid sequence as set forth in SEQ ID NO: 6 or an amino acid sequence at least 98% identical to the sequence of SEQ ID N0:66, wherein the functional acceptor comprises at least two sugar units of uronic acid and/or hexosamine, wherein at least one UDP-sugar is added in a stoichiometric ratio to the functional acceptor such that the recombinant heparosan synthase elongates the at least one functional acceptor to provide glycosaminoglycan polymers having a desired size distribution and that are in substantially monodisperse size, wherein a desired size distribution of glycosaminoglycan polymers is obtained by controlling the stoichiometric ratio of UDP-sugar to functional acceptor, wherein the substantially monodisperse extended glycosaminoglycan polymers have (a) a molecular weight in a range of from 3.5 kDa to 0.5 MDa, and a polydispersity value in a range of from 1.0 to 1.1, or (b) a molecular weight in a range of from 0.5 MDa to 4.5 MDa, and a polydispersity value in a range of from 1.0 to 1.5.

Glycosaminoglycans (GAGs) are linear polysaccharides composed of repeating disaccharide units containing a derivative of an amino sugar (either glucosamine or galactosamine). Hyaluronan [HA], chondroitin, and heparan sulfate/heparin contain a uronic acid as the other component of the disaccharide repeat while keratan contains a galactose. The GAGs are summarized in Table I.

**Table I**

| Polymer | Disaccharide Repeat | Post-Polymerization Modifications | |
|---|---|---|---|
| | | Vertebrates | Bacteria |
| Hyaluronan | β3GlcNAc β4GlcUA | none | none |
| Chondroitin | β3GalNAc β4GlcUA | O-sulfated/epimerized | none |
| Heparin/heparan | β4GlcNAc α4GlcUA | O,N-sulfated/epimerized | none |
| Keratan | β4GlcNAc β3Gal | O-sulfated | not reported |

An unnatural glycosaminoglycan (unnatural GAG) would be a composition of matter not normally found in known living vertebrates, animals or microbes; different arrangements or structures of chemical groups are added by the hand of man.

Vertebrates may contain all four types of GAGs, but the polysaccharide chain is often further modified after sugar polymerization. One or more modifications including O-sulfation of certain hydroxyls, deacetylation and subsequent N-sulfation, or epimerization of glucuronic acid to iduronic acid are found in most GAGs except HA. An amazing variety of distinct structures have been reported for chondroitin sulfate and heparan sulfate/heparin even within a single polymer chain. A few clever pathogenic microbes also produce unmodified GAG chains; the bacteria use extracellular polysaccharide coatings as molecular camouflage to avoid host defenses. The chondroitin and heparan sulfate/heparin chains in vertebrates are initially synthesized by elongation of a xylose-containing linkage tetrasaccharide attached to a variety of proteins. Keratan is either O-linked or N-linked to certain proteins depending on the particular molecule. HA and all of the known bacterial GAGs are not part of the classification of proteins known as glycoproteins. All GAGs except HA are found covalently linked to a core protein, and such combination is referred to as a proteoglycan. Glycoproteins are usually much smaller than proteoglycans and only contain from 1-60% carbohydrate by weight in the form of numerous relatively short, branched oligosaccharide chains, whereas a proteoglycan can contain as much as 95% carbohydrate by weight. The core protein in a proteoglycan is also usually a glycoprotein, therefore usually contains other oligosaccharide chains besides the GAGs.

GAGs and their derivatives are currently used in the medical field as ophthalmic and viscoelastic supplements, adhesion surgical aids to prevent post-operative adhesions, catheter and device coatings, and anticoagulants. Other current or promising future applications include anti-cancer medications, tissue engineering matrices, immune and neural cell modulators, anti-virals, proliferation modulators, and drug targeting agents.

Complex carbohydrates, such as GAGs, are information rich molecules. A major purpose of the sugars that make up GAGs is to allow communication between cells and extracellular components of multicellular organisms. Typically, certain proteins bind to particular sugar chains in a very selective fashion. A protein may simply adhere to the sugar, but quite often the protein's intrinsic activity may be altered and/or the protein transmits a signal to the cell to modulate its behavior. For example, in the blood coagulation cascade, heparin binding to inhibitory proteins helps shuts down the clotting response. In another case, HA binds to cells via the CD44 receptor that stimulates the cells to migrate and to proliferate. Even though long GAG polymers (i.e., >10² Da) are found naturally in the body, typically the protein's binding site interacts with a stretch of 4 to 10 monosaccharides. Therefore, oligosaccharides can be used to either (a) substitute for the polymer, or (b) to inhibit the polymer's action depending on the particular system.

HA polysaccharide plays structural roles in the eye, skin, and joint synovium. Large HA polymers (~10⁶ Da) also stimulate cell motility and proliferation. On the other hand, shorter HA polymers (~10⁴ Da) often have the opposite effect. HA-oligosaccharides composed of 10 to 14 sugars [HA₁₀₋₁₄] have promise for inhibition of cancer cell growth and metastasis. In an *in vivo* assay, mice injected with various invasive and virulent tumor cell lines (melanoma, glioma, carcinomas from lung, breast and ovary) develop a number of large tumors and die within weeks. Treatment with HA oligosaccharides greatly reduced the number and the size of tumors. Metastasis, the escape of cancer cells throughout the body, is one of the biggest fears of both the ailing patient and the physician. HA or HA-like oligosaccharides appear to serve as a supplemental treatment to inhibit cancer growth and metatasis.

The preliminary mode of action of the HA-oligosaccharide sugars is thought to be mediated by binding or interacting with one of several important HA-binding proteins (probably CD44 or RHAM) in the mammalian body. One proposed scenario for the anticancer action of HA-oligosaccharides is that multiple CD44 protein molecules in a cancer cell can bind simultaneously to a long HA polymer. This multivalent HA binding causes CD44 activation (perhaps mediated by dimerization or a receptor patching event) that triggers cancer cell activation and migration. However, if the cancer cell is flooded with small HA-oligosaccharides, then each CD44 molecule individually binds a different HA molecule in a monovalent manner such that no dimerization/patching event occurs. Thus no activation signal is transmitted to the cell. Currently, it is believed that the optimal HA-sugar size is 10 to 14 sugars. Although this size may be based more upon the size of HA currently available for testing rather than biological functionality - i.e., now that HA molecules and HA-like derivatives <10 sugars are available , the optimal HA size or oligosaccharide composition may be found to be different.

It has also been shown that treatment with certain anti-CD44 antibodies or CD44-antisense nucleic acid prevents the growth and metastasis of cancer cells in a fashion similar to HA-oligosaccharides; in comparison to the sugars, however, these protein-based and nucleic acid-based reagents are somewhat difficult to deliver in the body and/or may have long-term negative effects. A very desirable attribute of HA-oligosaccharides for therapeutics is that these sugar molecules are natural by-products that can occur in small amounts in the healthy human body during the degradation of HA polymer; no untoward innate toxicity, antigenicity, or allergenic concerns are obvious.

Other emerging areas for the potential therapeutic use of HA oligosaccharides are the stimulation of blood vessel formation and the stimulation of dendritic cell maturation. Enhancement of wound-healing and resupplying cardiac oxygenation may be additional applications that harness the ability of HA oligosaccharides to cause endothelial cells to form tubes and sprout new vessels. Dendritic cells possess adjuvant activity in stimulating specific CD4 and CD8 T cell responses. Therefore, dendritic cells are targets in vaccine development strategies for the prevention and treatment of infections, allograft reactions, allergic and autoimmune diseases, and cancer.

Heparin interacts with many proteins in the body, but two extremely interesting classes are coagulation cascade proteins and growth factors. Antithrombin III [ATIII] and certain other hemostasis proteins are 100,000-fold more potent inhibitors of blood clotting when complexed with heparin. Indeed, heparin is so potent it must be used in a hospital setting and requires careful monitoring in order to avoid hemorrhage. Newer, processed lower molecular weight forms of heparin are safer, but this material is still a complex mixture. It has been shown that a particular pentasaccharide (5 sugars long) found in heparin is responsible for the ATIII-anticoagulant effect. But since heparin is a very heterogeneous polymer, it is difficult to isolate the pentasaccharide (5 sugars long) in a pure state. The pentasaccharide can also be prepared in a conventional chemical synthesis involving -50 to 60 steps. However, altering the synthesis or preparing an assortment of analogs in parallel is not always feasible - either chemically orfinancially.

Many growth factors, including VEGF (vascular endothelial growth factor), HBEGF (heparin-binding epidermal growth factor), and FGF (fibroblast growth factor), bind to cells by interacting simultaneously with the growth factor receptor and a cell-surface heparin proteoglycan; without the heparin moiety, the potency of the growth factor plummets. Cell proliferation is modulated in part by heparin; therefore, diseases such as cancer and atherosclerosis are potential targets. Abnormal or unwanted proliferation would be curtailed if the growth factor was prevented from stimulating target disease-state cells by interacting with a heparin-like oligosaccharide analog instead of a surface-bound receptor. Alternatively, in certain cases, the heparin oligosaccharides alone have been shown to have stimulatory effects.

Chondroitin is the most abundant GAG in the human body, but all of its specific biological roles are not yet clear. Phenomenon such as neural cell outgrowth appears to be modulated by chondroitin. Both stimulatory and inhibitory effects have been noted depending on the chondroitin form and the cell type. Therefore, chondroitin or similar molecules are of utility in re-wiring synaptic connections after degenerative diseases (e.g., Alzheimer's) or paralytic trauma. The epimerized form of chondroitin (GlcUA converted to the C5 isomer, iduronic acid or IdoUA), dermatan, selectively inhibits certain coagulation proteins such as heparin cofactor II. By modulating this protein in the coagulation pathway instead of ATIII, dermatan appears to allow for a larger safety margin than heparin treatment for reduction of thrombi or clots that provoke strokes and heartattacks.

Many details of GAG/protein interactions are not yet clear due to (a) the heterogeneity of GAGs (in part due to their biosynthesis pathway), and (b) the difficulty in analyzing long polysaccharides and membrane receptor proteins at the molecular level. Fortunately, many short oligosaccharides have biological activities that serve to assist research pursuits as well as to treat disease in the near future. Conventional chemical synthesis of short GAG oligosaccharides is possible, but the list of roadblocks includes: (i) difficult multi-step syntheses that employ toxic catalysts, (ii) very low yield or high failure rates with products longer than ~6 monosaccharides, (iii) imperfect control of stereoselectivity (e.g., wrong anomer) and regioselectivity (e.g., wrong attachment site), and (iv) the possibility for residual protection groups (non-carbohydrate moieties) in the final product.

Chemoenzymatic synthesis, however, employing catalytic glycosyltransferases with exquisite control and superb efficiency is currently being developed by several universities and companies. A major obstacle is the production of useful catalyst because the vast majority of glycosyltransferases are rare membrane proteins that are not particularly robust.

All of the known HA, chondroitin and heparosan/heparan sulfate/heparin glycosyltransferase enzymes that synthesize the alternating sugar repeat backbones in microbes and in vertebrates utilize UDP- sugar precursors and divalent metal cofactors (e.g., magnesium, cobalt, and/or manganese ion) near neutral pH according to the overall reaction:

nUDP-GIcUA + nUDP-HexNAc D 2nUDP + [GlcUA-HexNAc]ₙ

where HexNAc = GlcNAc or GalNAc. Depending on the specific GAG and the particular organism or tissue examined, and the degree of polymerization, n, ranges from about 25 to about 10,000. Smaller molecules may be made *in vitro,* as desired. If the GAG is polymerized by a single polypeptide, the enzyme is called a synthase or co-polymerase.

The present inventors have discovered four new dual-action enzyme catalysts from distinct isolates of the Gram-negative bacterium *Pasteurella multocida* using various molecular biology strategies. *P. multocida* infects fowl, swine, and cattle as well as many wildlife species. The enzymes are: a HA synthase, or (pmHAS); a chondroitin synthase, or (pmCS); and two heparosan synthases, or (pmHS1 and PmHS2). To date, no keratan synthase from any source has been identified or reported in the literature.

In U.S. Serial No. 10/217,613, filed August 12, 2002, the molecular directionality of pmHAS synthesis was disclosed and claimed. pmHAS is unique in comparison to all other existing HA synthases of *Streptococcus* bacteria, humans and an algal virus. Specifically, recombinant pmHAS can elongate exogeneously-supplied short HA chains (e.g., 2-4 sugars) into longer HA chains (e.g., 3 to 150 sugars). The pmHAS synthase has been shown to add monosaccharides one at a time in a step-wise fashion to the growing chain. The pmHAS enzyme's exquisite sugar transfer specificity results in the repeating sugar backbone of the GAG chain. The pmCS enzyme, which is about 90% identical at the amino acid level to pmHAS, performs the same synthesis reactions but transfers GalNAc instead of GlcNAc. The pmCS enzyme was described and enabled in U.S. Serial No. 09/842,484. The pmHS1 and PmHS2 enzymes are not very similar at the amino acid level to pmHAS, but perform the similar synthesis reactions; the composition of sugars is identical but the linkages differ because heparosan is (α-4GlcUA-β-4GlcNAc). The pmHS1 and PmHS2 enzymes were described and enabled in copending U.S. Serial No. 10/142,143.

The explanation for the step-wise addition of sugars to the GAG chain during biosynthesis was determined by analyzing mutants of the pmHAS enzyme. pmHAS possesses two independent catalytic sites in one polypeptide. Mutants were created that transferred only GlcUA, and distinct mutants were also created that transferred only GlcNAc. These mutants cannot polymerize HA chains individually, but if the two types of mutants are mixed together in the same reaction with an acceptor molecule, then polymerization was rescued. The chondroitin synthase, pmCS, has a similar sequence and similar two-domain structure. The heparosan synthases, pmHS1 and PmHS2, also contain regions for the two active sites. Single action mutants have also been created for the chondroitin synthase, pmCS, and are described hereinafter in detail.

The naturally occurring *Pasteurella* GAG synthases are very specific glycosyltransferases with respect to the sugar transfer reaction; only the correct monosaccharide from the authentic UDP-sugar is added onto acceptors. The epimers or other closely structurally related precursor molecules (e.g., UDP-glucose) are usually not utilized. The GAG synthases do, however, utilize certain heterologous acceptor sugars. For example, pmHAS will elongate short chondroitin acceptors with long HA chains. pmHS1 will also add long heparosan chains onto HA acceptor oligosaccharides as well as heparin oligosaccharides (see hereinbelow).

It has also been determined that the recombinant pmHAS, pmHS1, pmHS2, and pmCS synthases add sugars to the nonreducing end of a growing polymer chain. The correct monosaccharides are added sequentially in a stepwise fashion to the nascent chain or a suitable exogenous oligosaccharide or polysaccharide acceptor molecule. The pmHAS sequence, however, is significantly different from the other known HA synthases. There appears to be only two short potential sequence motifs ([D/N]DGS[S/T], SEQ ID NO:68; DSD[D/T]Y, SEQ ID NO:69) in common between pmHAS (Class II) and the Group A HAS spHAS (Class I). Instead, a portion of the central region of the pmHAS is more homologous to the amino termini of other bacterial glycosyltransferases that produce different capsular polysaccharides or lipopolysaccharides. Furthermore, pmHAS is about twice as long as any other HAS enzyme.

When the pmHAS is given long elongation reaction times, HA polymers of at least 400 sugars long are formed. Unlike the Class I HA synthases, recombinant versions of pmHAS and pmCS produced in certain foreign hosts also have the ability to extend exogenously supplied HA or chondroitin oligosaccharides with long HA and chondroitin polymers *in vitro,* respectively. The recombinant pmHS1 and pmHS2 enzymes produced in a foreign host have the ability to extend HA, chondroitin, or heparin oligosaccharides with long heparosan chains *in vitro.* See e.g., U.S. Serial No. 10/195,908, filed July 15, 2002. If recombinant versions of pmHAS or pmCS or pmHS1 or pmHS2 are supplied with functional acceptor oligosaccharides, total HA, chondroitin and heparin biosynthesis is increased up to 50-fold over reactions without the exogenous oligosaccharide. The native versions of the pmHAS, pmCS, pmHS1, and PmHS2 enzymes isolated from *P. multocida* do not perform such elongation reactions with exogenous acceptor (or perform with very low efficiency) due to the presence of a nascent HA, chondroitin, or heparin chain in the natural host. The nature of the polymer retention mechanism of the pmHAS, pmCS, pmHS1, and PmHS2 polypeptide might be the causative factor for this activity: i.e. a HA- or chondroitin- or heparin-binding site may exist that holds onto the HA or chondroitin or heparin chain during polymerization. Small HA or chondroitin or heparin oligosaccharides supplied by the hand of man are also capable of occupying this site of the recombinant enzyme and thereafter be extended into longer polysaccharide chains.

Most membrane proteins are relatively difficult to study due to their insolubility in aqueous solution, and the native HASs, CSs, HSs, and PmHS2s are no exception. The HAS enzyme from Group A and C *Streptococcus* bacteria has been detergent-solubilized and purified in an active state in small quantities. Once isolated in a relatively pure state, the streptococcal enzyme has very limited stability. A soluble recombinant form of the HAS enzyme from *P. multocida* called pmHAS¹⁻⁷⁰³ comprises residues 1-703 of the 972 residues of the native pmHAS enzyme. pmHAS¹⁻⁷⁰³ can be mass-produced in *E. coli* and purified by chromatography. The pmHAS¹⁻⁷⁰³ enzyme retains the ability of the parent enzyme to add onto either a long HA polymer, a short HA primer, a long chondroitin polymer, a short chondroitin primer, a short chondroitin polymer, as well as other exogenous acceptors. The chondroitin chain may also be sulfated. Furthermore, the purified pmHAS¹⁻⁷⁰³ enzyme is stable in an optimized buffer for days on ice and for hours at normal reaction temperatures. One formulation of the optimal buffer consists of 1M ethylene glycol, 0.1 - 0.2 M ammonium sulfate, 50mM Tris, pH 7.2, and protease inhibitors which also allow the stability and specificity at typical reaction conditions for sugar transfer. For the reaction UDP-sugars and divalent manganese (10-20 mM) are added. pmHAS¹⁻⁷⁰³ will also add a HA polymer onto plastic beads with an immobilized short HA primer or any other substrate capable of having an acceptor molecule or acceptor group thereon.

Full-length, native sequence PmHS1 or PmHS2 can be converted into higher yield, soluble proteins that are purifiable by the addition of fusion protein partners, such as, but not limited to, maltose-binding protein (MBP).

pmCS, pmHAS, pmHS1, and PmHS2 possess two separate glycosyltransferase sites. Protein truncation studies demonstrated that residues 1-117 of pmHAS can be deleted without affecting catalytic activity; similar truncation of the homologous pmCS, pmHS1, and PmHS2 enzymes may also be preferred. The carboxyl-terminal boundary of the GlcUA-Transferase of pmHAS resides within residues 686-703 and within residues 686- 704 of pmCS. These sites each contain a DGS and DXD motif; all aspartate residues of these motifs are essential for HA synthase activity. D196, D247 and D249 mutants possessed only GlcUA-transferase activity while D477, D527 and D529 mutants possessed only GlcNAc-transferase activity. These results further confirm our previous assignment of the active sites within the synthase polypeptide. The WGGED sequence motif appears to be involved in GlcNAc-transferase activity because E396 mutants and D370 mutants possessed only GlcUA-transferase activity. The highly homologous (90% identical) pmCS can also be mutated in the same fashion. For example, mutating the homologous DXD motif in the GlcUA site of pmCS results in an enzyme with only GalNAc-transferase activity.

Type F *P.multocida* synthesizes an unsulfated chondroitin (β3N- acetylgalactosamine [GalNAc]-β4GlcUA) capsule. Domain swapping between pmHAS and the homologous chondroitin synthase, pmCS, has been performed. A chimeric or hybrid enzyme consisting of residues 1-427 of pmHAS and residues 421-704 of pmCS was an active HA synthase. On the other hand, the converse chimeric or hybrid enzyme consisting of residues 1-420 of pmCS and residues 428-703 of pmHAS was an active chondroitin synthase. Overall, these findings support the model of two independent transferase sites within a single polypeptide as well as further delineate the site boundaries of both enzymes. The hexosamine-transferase site resides in the N-terminal domain while the GlcUA- transferase site resides in the COOH-terminal domain of these GAG synthases.Thus, certain units or regions of the GAG synthase sequences are able to function (with novel or typical catalytic activity) in various unnatural or new combinations assembled by the hand of man.

Polysaccharides, especially those of the glycosaminoglycan class, serve numerous roles in the body as structural elements and signaling molecules. By grafting or making hybrid molecules composed of more than one polymer backbone, it is possible to meld distinct physical and biological properties into a single molecule without resorting to unnatural chemical reactions or residues. The method of the present invention incorporates the propensity of certain recombinant enzymes, when prepared in a virgin state, to utilize various acceptor molecules as the seed for further polymer growth: naturally occurring forms of the enzyme or existing living wild- type host organisms do not display this ability. Thus, the method of the present invention can result in (a) the production of hybrid oligosaccharides or polysaccharides and (b) the formation of polysaccharide coatings. Such hybrid polymers can serve as "molecular glue" -- i.e., when two cell types or other biomaterials interact with each half of a hybrid molecule, then each of the two phases are bridged.

In addition, adding new chemical groups and thus forming unnatural glycosaminoglycan polymers, may facilitate coupling to other molecules or surfaces, even cells.

Such polysaccharide coatings are useful for integrating a foreign object within a surrounding tissue matrix. For example, a prosthetic device is more firmly attached to the body when the device is coated with a naturally adhesive polysaccharide. Additionally, the device's artificial components could be masked by the biocompatible coating to reduce immunoreactivity or inflammation.

Recombinant pmHAS, pmCS, pmHS1, and PmHS2 elongate exogenous functional oligosaccharide acceptors to form long or short polymers *in vitro*; thus far no other Class I HA synthase has displayed this capability. The directionality of synthesis was established definitively by testing the ability of pmHAS and pmCS and pmHS1 and PmHS2 to elongate defined oligosaccharide derivatives. The non-reducing end sugar addition allows the reducing end to be modified for other purposes; the addition of GAG chains to small molecules, polymers, or surfaces is thus readily performed. Analysis of the initial stages of synthesis demonstrated that pmHAS and pmCS and pmHS1 and PmHS2 added single monosaccharide units sequentially. Apparently the fidelity of the individual sugar transfer reactions is sufficient to generate the authentic repeating structure of HA or chondroitin or heparin. Therefore, simultaneous addition of disaccharide block units is not required as hypothesized in some recent models of polysaccharide biosynthesis. pmHAS and pmCS and pmHS1 and PmHS2 appear distinct from most other known HA and chondroitin and heparin synthases based on differences in sequence, topology in the membrane, and/or putative reaction mechanism.

As mentioned previously, pmHAS, the 972-residue membrane-associated hyaluronan synthase, catalyzes the transfer of both GlcNAc and GlcUA to form an HA polymer. In order to define the catalytic and membrane-associated domains, pmHAS and pmCS mutants have been analyzed. pmHAS¹⁻⁷⁰³ is a soluble, active HA synthase suggesting that the carboxyl-terminus is involved in membrane association of the native enzyme. pmHAS¹⁻⁶⁵⁰ is inactive as a HA synthase, but retains GIcNAc-transferase activity. Within the pmHAS sequence, there is a duplicated domain containing a short motif, DGS or Asp-Gly-Ser, that is conserved among many glycosyltransferases. Changing this aspartate in either domain to asparagine, glutamate, or lysine reduced the HA synthase activity to low levels. The mutants substituted at residue 196 possessed GIcUA-transferase activity while those substituted at residue 477 possessed GlcNAc-transferase activity. The Michaelis constants of the functional transferase activity of the various mutants, a measure of the apparent affinity of the enzymes for the precursors, were similar to wild-type values. Furthermore, mixing D196N and D477K mutant proteins in the same reaction allowed HA polymerization at levels similar to the wild-type enzyme. These results provide the first direct evidence that the synthase polypeptide utilizes two separate glycosyltransferase sites. Likewise, pmCS mutants were made and tested having the same functionality and sequence similarity to the mutants created for pmHAS.

*Pasteurella multocida* Type F, the minor fowl cholera pathogen, produces an extracellular polysaccharide capsule that is a putative virulence factor. As outlined in U.S. Serial No. 09/842,484, filed April 25, 2002, and entitled Chondroitin Synthase Gene and Methods of Making and Using Same, the contents of which are hereby expressly incorporated herein in their entirety, the capsule of *Pasteurella multocida* Type F was removed by treating microbes with chondroitin AC lyase. It was found by acid hydrolysis that the polysaccharide contained galactosamine and glucuronic acid. A Type F polysaccharide synthase was molecularly cloned and its enzymatic activity was characterized. The 965-residue enzyme, called pmCS, is 90% identical at the nucleotide and the amino acid level to the hyaluronan synthase, pmHAS, from *P. multocida* Type A. A recombinant *Escherichia* coli-derived, truncated, soluble version of pmCS (residues 1-704) was shown to catalyze the repetitive addition of sugars from UDP-GalNAc and UDP-GlcUA to chondroitin oligosaccharide acceptors *in vitro.* Other structurally related sugar nucleotide precursors did not substitute in the elongation reaction. Polymer molecules composed of ~10³ sugar residues were produced as measured by gel filtration chromatography. The polysaccharide synthesized *in vitro* was sensitive to the action of chondroitin AC lyase but resistant to the action of hyaluronan lyase. This was the first report identifying a glycosyltransferase that forms a polysaccharide composed of chondroitin disaccharide repeats, [β(1,4)GlcUA-β(1,3)GalNAc]ₙ. In analogy to known hyaluronan synthases, a single polypeptide species, pmCS, possesses both transferase activities. The heparin synthases, pmHS1 and PmHS2, from *P. multocida,* also are a single polypeptide species that possess both transferase activities to catalyze heparin/heparosan.

Promising initial target polymers for a variety of therapeutic uses are glycosaminoglycan chains composed of about 1 kDa to about 4 MDa (kDa is defined as 10³ Da, whereas MDa is defined as 10⁶ Da). The two current competing state-of-the-art techniques for creating the desired smaller size glycosaminoglycan [GAG] polymers are extremely limited and will not allow the medical potential of the sugars to be achieved. Small GAG molecules are presently made either by: (1) partially depolymerizing costly large polymers with degradative enzymes or with chemical means (e.g., heat, acid, sonication), or (2) highly demanding organic chemistry-based carbohydrate synthesis. The former method is difficult to control, inefficient, costly, and is in a relatively stagnant development stage. For example, the enzyme wants to degrade the polymer to the 2 or 4 sugar end stage product, but this sugar is inactive for many therapeutic treatments. The use of acid hydrolysis also removes a fraction of the acetyl groups from the GlcNAc or GalNAc groups thereby introducing a positive charge into an otherwise anionic molecule. The latter method, chemical synthesis, involves steps with low to moderate repetitive yield and has never been reported for a HA-oligosaccharide longer than 6 to 8 sugars in length. Also racemization (e.g., production of the wrong isomer) during chemical synthesis creates inactive or harmful molecules; the inclusion of the wrong isomer in a therapeutic preparation in the past has had tragic consequences as evidenced by the birth defects spawned by the drug Thalidomide. As sugars contain many similar reactive hydroxyl groups, in order to effect proper coupling between two sugars in a chemical synthesis, most hydroxyl groups must be blocked or protected. At the conclusion of the reaction, all of the protecting groups must be removed, but this process is not perfect; as a result, a fraction of the product molecules retain these unnatural moieties.

The partial depolymerization method only yields fragments of the original GAG polymer and is essentially useless for creating novel sugars beyond simple derivatizations (e.g., esterifying some fraction of GlcUA residues in an indiscriminate fashion). Chemical synthesis may suffice in theory to make novel sugars, but the strategy needs to be customized for adding a new sugar, plus the problems with side-reactions/isomerization and the ultimate oligosaccharide size still pose the same trouble as described earlier. Another distinct method using the degradative enzymes to generate small molecules by running in reverse on mixtures of two polymers (e.g., HA and chondroitin) has some potential for novel GAG polysaccharide synthesis. See e.g. J Biochem (Tokyo). 2000 Apr;127(4):695-702, Chimeric glycosaminoglycan oligosaccharides synthesized by enzymatic reconstruction and their use in substrate specificity determination of Streptococcus hyaluronidase, Takagaki K, Munakata H, Majima M, Kakizaki I, Endo M.; and J Biol Chem. 1995 Feb 24;270(8):3741-7, Enzymic reconstruction of glycosaminoglycan oligosaccharide chains using the transglycosylation reaction of bovine testicular hyaluronidase, Saitoh H, Takagaki K, Majima M, Nakamura T, Matsuki A, Kasai M, Narita H, Endo M. However, this technology can make only a very limited scope of products with a block pattern (no single or specifically spaced substitutions possible) using slow reactions that cannot easily be customized or controlled. No other technology is as versatile as the presently claimed and disclosed biocatalytic system with respect to flexibility of final polysaccharide structure in the about 1 kDa to about 4 MDa size range. Novel, designer molecules can be prepared with minimal re-tooling by use of the appropriate hyaluronic acid or chondroitin or heparin enzyme catalysts and substrates.

The size of the HA polysaccharide dictates its biological effect in many cellular and tissue systems based on many reports in the literature. However, no source of very defined, uniform HA polymers with sizes greater than 2-5 kDa is currently available. This situation is complicated by the observation that long and short HA polymers appear to have antagonistic or inverse effects on some biological systems. Therefore, HA preparations containing a mixture of both size populations may yield contradictory or paradoxical results..

The methods for enzymatically producing defined substantially monodisperse glycosaminoglycan polymers in accordance with the present invention involves providing at least one functional acceptor, wherein the functional acceptor comprises at least two sugar units of uronic acid and/or hexosamine and at least one recombinant heparosan transferase capable of elongating the functional acceptor in a controlled and/or repetitive fashion to form extended glycosaminoglycan-like molecules. At least one UDP-sugar (such as but not limited to, UDP-GlcUA, UDP-GalUA UDP-GlcNAc, UDP-Glc, UDP-GalNAc, UDP-GlcN, UDP-GaIN), is added in a stoichiometric ratio to the functional acceptor such that the recombinant heparosan transferase elongates the at least one functional acceptor to provide glycosaminoglycan polymers having a desired size distribution and that are substantially monodisperse in size wherein a desired size distribution of glycosaminoglycan polymers is obtained by controlling the stoichiometric ratio of UDP-sugar to functional acceptor and wherein the substantially monodisperse extended glycosaminoglycan polymers have (a) a molecular weight in a range of from 3.5 kDa to 0.5 MDa, and a polydispersity value in a range of from 1.0 to 1.1, or (b) a molecular weight in a range of from 0.5 MDa to 4.5 MDa, and a polydispersity value in a range of from 1.0 to 1.5. Such desired size distribution is obtained by controlling the stroichiometric ratio of UDP-sugar to functional acceptor.

The term "substantially monodisperse in size" as used herein will be understood to refer to defined glycoasminoglycan polymers that have a very narrow size distribution. For example, substantially monodisperse glycosaminoglycan polymers having a molecular weight in a range of from about 3.5 kDa to about 0.5 MDa will have a polydispersity value (i.e., Mw/Mn, where Mw is the average molecular weight and Mn is the number average molecular weight) in a range of from about 1.0 to about 1.1, and preferably in a range from about 1.0 to about 1.05. In yet another example, substantially monodisperse glycosaminoglycan polymers having a molecular weight in a range of from about 0.5 MDa to about 4.5 MDa will have a polydispersity value in a range of from about 1.0 to about 1.5, and preferably in a range from about 1.0 to about 1.2.

The functional acceptor utilized in accordance with the present invention has at least two sugar units of uronic acid and/orhexosamine, wherein the uronic acid may be GlcUA, IdoUA (iduronic acid), GalUA, and structural variants or derivatives thereof; and the hexosamine may be GlcNAc, GalNAc, GlcN, GaIN, and structural variants or derivatives thereof.

In one embodiment, the functional acceptor may be an HA oligosaccharide, polysaccharide or polymer; a chondroitin oligosaccharide, polysaccharide or polymer; a chondroitin sulfate oligosaccharide, polysaccharide or polymer; a heparosan oligosaccharide, polysaccharide or polymer; a heparin oligosaccharide, polysaccharide, or polymer; a heparin oligosaccharide, polysaccharide or polymer; a heparosan-like oligosaccharide, polysaccharide or polymer; or a sulfated or modified oligosaccharide, polysaccharide or polymer. In another embodiment, the functional acceptor may be an extended acceptor such as HA chains, chondroitin chains, heparosan chains, mixed glycosaminoglycan chains, analog containing chains or any combination thereof.

Another functional acceptor class that may be utilized in accordance with the method of the present invention includes synthetic glycosides (i.e., sugars that have a non-sugar component at the reducing end) or similar synthetic carbohydrates. The synthetic portion substitutes for one of the natural sugar units; these molecules are less expensive and can possess useful groups.

The functional acceptor utilized in a method in accordance with the present invention may further comprise a moiety selected from the group consisting of a fluorescent tag, a radioactive tag or therapeutic, an affinity tag, a detection probe, a medicant, a biologically active agent, a therapeutic agent, and combinations thereof. The UDP-sugar provided in accordance with the present invention may be radioactive or nuclear magnetic resonance-active.

The recombinant heparosan synthase utilized in a method in accordance with the present invention has an amino acid sequence as set forth in SEQ ID NO: 6 or or an amino acid sequence that is at least 98% identical to SEQ ID NO: 66. In some embodiments, the recombinan theparosan synthase has an amino acid sequence as set forth in SEQ ID NO: 66

In one embodiment of the present invention, methods of enzymatically producing glycosaminoglycan polymers having unnatural structures are provided. The methods include providing at least one functional acceptor as described above, providing at least one recombinant heparosan synthase as described above, and providing at least one UDP-sugar analog, wherein the at least one UDP-sugar analog is not found in mammals in a native state. The at least one recombinant glycosaminoglycan transferase then elongates the at least one functional acceptor to provide glycosaminoglycan polymers having the sugar analog incorporated therein, thereby providing glycosaminoglycan polymers having an unnatural structure.

In one embodiment, the at least one UDP-sugar analog is selected from the group consisting of UDP-GlcN, UDP-GlcNAcUA, UDP-GlcNAcNAc, UDP-GlcdiNAcUA, UDP-GlcN[TFA], UDP-GlcNBut, UDP-GlcNPro, UDP-6-F-6-deoxyGlcNAc, UDP-2-F-2-deoxyGlcUA, and combinations thereof. The at least one UDP-sugar analog may also further comprise a moiety selected from the group consisting of a fluorescent tag, a radioactive tag or therapeutic, an affinity tag, a detection probe, a medicant, a biologically active agent, a therapeutic agent, and combinations thereof.

As used herein, the term "nucleic acid segment" and "DNA segment" are used interchangeably and refer to a DNA molecule which has been isolated free of total genomic DNA of a particular species. Therefore, a "purified" DNA or nucleic acid segment as used herein, refers to a DNA segment which contains a Hyaluronate Synthase ("HAS") coding sequence or Chondroitin Synthase (CS) coding sequence or Heparin/Heparosan Synthase (HS) coding sequence yet is isolated away from, or purified free from, unrelated genomic DNA, for example, total *Pasteurella multocida.* Included within the term "DNA segment", are DNA segments and smaller fragments of such segments, and also recombinant vectors, including, for example, plasmids, cosmids, phage, viruses, and the like.

Similarly, a DNA segment comprising an isolated or purified *pmHAS or pmCS or pmHS1 or PmHS2* gene refers to a DNA segment including HAS or CS or HS coding sequences isolated substantially away from other naturally occurring genes or protein encoding sequences. In this respect, the term "gene" is used for simplicity to refer to a functional protein-, polypeptide- or peptide- encoding unit. As will be understood by those in the art, this functional term includes genomic sequences, cDNA sequences or combinations thereof. "Isolated substantially away from other coding sequences" means that the gene of interest, in this case *pmHAS or pmCS or pmHS1 or PmHS2* forms the significant part of the coding region of the DNA segment, and that the DNA segment does not contain other non-relevant large portions of naturally-occurring coding DNA, such as large chromosomal fragments or other functional genes or DNA coding regions. Of course, this refers to the DNA segment as originally isolated, and does not exclude genes or coding regions later added to, or intentionally left in, the segment by the hand of man.

Due to certain advantages associated with the use of prokaryotic sources, one will likely realize the most advantages upon isolation of the HAS or CS or HS gene from the prokaryote *P. multocida.* One such advantage is that, typically, eukaryotic genes may require significant post-transcriptional modifications that can only be achieved in a eukaryotic host. This will tend to limit the applicability of any eukaryotic HAS or CS or HS gene that is obtained. Moreover, those of ordinary skill in the art will likely realize additional advantages in terms of time and ease of genetic manipulation where a prokaryotic enzyme gene is sought to be employed. These additional advantages include (a) the ease of isolation of a prokaryotic gene because of the relatively small size of the genome and, therefore, the reduced amount of screening of the corresponding genomic library, and (b) the ease of manipulation because the overall size of the coding region of a prokaryotic gene is significantly smaller due to the absence of introns. Furthermore, if the product of the pmHAS or pmCS or pmHS1 or PmHS2 gene (i.e., the enzyme) requires posttranslational modifications, these would best be achieved in a similar prokaryotic cellular environment (host) from which the gene was derived.

DNA sequences may further include genetic control regions which allow the expression of the sequence in a selected recombinant host. The genetic control region may be native to the cell from which the gene was isolated, or may be native to the recombinant host cell, or may be an exaggerous segment that is compatible with and recognized by the transcriptional machinery of the selected recbominant host cell. Of course, the nature of the control region employed will generally vary depending on the particular use (e.g., cloning host) envisioned.

Nucleic acid segments having HAS or CS or HS activity may be isolated by the methods described herein. The term "a sequence essentially as set forth in SEQ ID NO:X means that the sequence substantially corresponds to a portion of SEQ ID NO:X and has relatively few amino acids or codons encoding amino acids which are not identical to, or a biologically functional equivalent of, the amino acids or codons encoding amino acids of SEQ ID NO:X. The term "biologically functional equivalent" is well understood in the art and is further defined in detail herein, as a gene having a sequence essentially as set forth in SEQ ID NO:X, and that is associated with the ability of prokaryotes to produce HA or a hyaluronic acid or chondroitin or heparin polymer *in vitro* or *in vivo.* In the above examples X refers to either SEQ ID NO:1, 2, 3, 4, 5, 6, 7, 8, 9, 65, 66 or 67 or any additional sequences set forth herein, such as the truncated or mutated versions of pmHAS¹⁻⁷⁰³ that are contained generally in SEQ ID NOS:10-48.

The art is replete with examples of practitioner's ability to make structural changes to a nucleic acid segment (i.e. encoding conserved or semi-conserved amino acid substitutions) and still preserve its enzymatic or functional activity when expressed. See for special example of literature attesting to such: (1) Risler et al. Amino Acid Substitutions in Structurally Related Proteins. A Pattern Recognition Approach. J. Mol. Biol. 204:1019-1029 (1988) [... according to the observed exchangeability of amino acid side chains, only four groups could be delineated; (i) Ile and Val; (ii) Leu and Met, (iii) Lys, Arg, and Gin, and (iv) Tyr and Phe. ]; (2) Niefind et al. Amino Acid Similarity Coefficients for Protein Modeling and Sequence Alignment Derived from Main-Chain Folding Anoles. J. Mol. Biol. 219:481-497 (1991) [similarity parameters allow amino acid substitutions to be designed]; and (3) Overington et al. Environment-Specific Amino Acid Substitution Tables: Tertiary Templates and Prediction of Protein Folds, Protein Science 1:216-226 (1992) [Analysis of the pattern of observed substitutions as a function of local environment shows that there are distinct patterns... Compatible changes can be made.]

It is widely recognized that a pair of distinct enzymes with even 30, 50 or 70% identity or similarity at the active site (of functional regions) thereof can possess the same catalytic activity. As most of the protein sequence is a scaffold for the active site, it is not required that all regions of the enzymes be exactly the same between functional enzyme homologs or analogs. In addition, some extra (non-catalytic) sequences may also be present, thus lowering the total protein similarity levels. Thus, functional regions (and not entire sequences) should be the basis for similarity comparisons between two enzymes.

These references and countless others, indicate that one of ordinary skill in the art, given a nucleic acid sequence or an amino acid, could make substitutions and changes to the nucleic acid sequence without changing its functionality (specific examples of such changes are given hereinafter and are generally set forth in SEQ ID NOS:10-48). Also, a substituted nucleic acid segment may be highly identical and retain its enzymatic activity with regard to its unadulterated parent, and yet still fail to hybridize thereto. Additionally, the present application discloses 4 enzymes and numerous mutants of these enzymes that still retain at least 50% of the enzymatic activity of the unmutated parent enzyme - i.e., ½ of the dual action transferase activity of the unadulterated parent. As such, variations of the sequences and enzymes that fall within the above-defined functional limitations have been disclosed and enabled. One of ordinary skill in the art, given the present specification, would be able to identify, isolate, create, and test DNA sequences and/or enzymes that produce natural or chimeric or hybrid GAG molecules.

One of ordinary skill in the art would appreciate that substitutions can be made to the pmHAS or pmCS or pmHS1 or PmHS2 nucleic acid segments listed in SEQ ID NO:1, 3, 5, 7, 65 and 67, respectively. Indeed, such changes have been made and are described hereinafter with respect to the mutants produced. Standardized and accepted functionally equivalent amino acid substitutions are presented in Table II.

**TABLE II**

| **Amino Acid Group** | **Conservative and Semi-Conservative Substitutions** |
|---|---|
| NonPolar R Groups | Alanine, Valine, Leucine, Isoleucine, Proline, Methionine, Phenylalanine, Tryptophan |
| Polar, but uncharged, R Groups | Glycine, Serine, Threonine, Cysteine, Asparagine, Glutamine |
| Negatively Charged R Groups | Aspartic Acid, Glutamic Acid |
| Positively Charged R Groups | Lysine, Arginine, Histidine |

Thus, it will be appreciated by those of skill in the art that other means may be used to obtain the HAS or CS or HS gene or cDNA, in light of the present disclosure. For example, polymerase chain reaction or RT-PCR produced DNA fragments may be obtained which contain full complements of genes or cDNAs from a number of sources, including other strains of *Pasteurella* or from a prokaryot with similar glycosyltransferases or from eukaryotic sources, such as cDNA libraries. Virtually any molecular cloning approach may be employed for the generation of DNA fragments. Thus, the only limitation generally on the particular method employed for DNA isolation is that the isolated nucleic acids should encode a biologically functional equivalent HAS or CS or HS.

Once the DNA has been isolated, it is ligated together with a selected vector. Virtually any cloning vector can be employed. Typical useful vectors include plasmids and phages for use in prokaryotic organisms and even viral vectors for use in eukaryotic organisms. Examples include pKK223-3, pMAL-c, pSA3, recombinant lambda, SV40, polyoma, adenovirus, bovine papilloma virus and retroviruses. However, it is believed that particular advantages will ultimately be realized where vectors capable of replication in both biotechnologically useful Gram-positive or Gram-negative bacteria (e.g., *Bacillus*, *Lactococcus*, or *E. coli*) are employed.

Vectors such as these, exemplified by the pSA3 vector of Dao and Ferretti or the pAT19 vector of Trieu-Cuot, et al., allow one to perform clonal colony selection in an easily manipulated host such as *E. coli,* followed by subsequent transfer back into a food grade *Lactococcus* or *Bacillus* strain for production of hyaluronan or chondroitin or heparin polymer. A recombinant vector can be employed to make the functional GAG synthase for *in vitro* use. These are benign and well studied organisms used in the production of certain foods and biotechnology products and are recognized as GRAS (generally recognized as safe) organisms. These are advantageous in that one can augment the *Lactococcus* or *Bacillus* strain's ability to synthesize HA or chondroitin or heparin through gene dosaging (i.e., providing extra copies of the HAS or CS or HS gene by amplification) and/or inclusion of additional genes to increase the availability of HA or chondroitin or heparin precursors. The inherent ability of a bacterium to synthesize HA or chondroitin or heparin can also be augmented through the formation of extra copies, or amplification, of the plasmid that carries the HAS or CS or HS gene. This amplification can account for up to a 10-fold increase in plasmid copy number and, therefore, the HAS or CS or HS gene copy number.

Another procedure to further augment HAS or CS or HS gene copy number is the insertion of multiple copies of the gene into the plasmid. Another technique would include integrating at least one copy of the HAS or CS or HS gene into chromosomal DNA. This extra amplification would be especially feasible, since the bacterial HAS or CS or HS gene size is small. In some scenarios, the chromosomal DNA-ligated vector is employed to transfect the host that is selected for clonal screening purposes such as *E. coli,* through the use of a vector that is capable of expressing the inserted DNA in the chosen host.

It will also be understood that amino acid and nucleic acid sequences may include additional residues, such as additional N- or C-terminal amino acids or 5' or 3' nucleic acid sequences, and yet still be essentially as set forth in one of the sequences disclosed herein, so long as the sequence meets the criteria set forth above, including the maintenance of biological protein activity where protein expression and enzyme activity is concerned. The addition of terminal sequences particularly applies to nucleic acid sequences which may, for example, include various non-coding sequences flanking either of the 5' or 3' portions of the coding region or may include various internal sequences, which are known to occur within genes. Furthermore, residues may be removed from the N- or C- terminal amino acids and yet still be essentially as set forth in one of the sequences disclosed herein, so long as the sequence meets the criteria set forth above, as well.

Traditionally, chemical or physical treatments of polysaccharides were required to join two dissimilar materials. For example, a reactive nucleophile group of one polymer or surface was exposed to an activated acceptor group of the other material.Two main problems exist with this approach, however. First, the control of the chemical reaction cannot be refined, and differences in temperature and level of activation often result in a distribution of several final products that vary from lot to lot preparation. For instance, several chains may be cross-linked in a few random, ill-defined areas, and the resulting sample is not homogenous. Second, the use of chemical reactions to join molecules often leaves an unnatural or nonbiological residue at the junction of biomaterials. For example, the use of an amine and an activated carboxyl group would result in an amide linkage. This inappropriate residue buried in a carbohydrate may pose problems with biological systems such as the subsequent production of degradation products which accumulate to toxic levels or the triggering of an immune response.

The terms "unnatural sugar" and "sugar analog" are used herein interchangeably, and will be understood to refer to a sugar analog that is not found in mammals in a native state, that is, a sugar analog that is produced by the hand of man. This sugar unit may be a component of a precursor UDP-sugar, or an acceptor, or the monosaccharide itself.

### Use of pmHAS for Polymer Grafting and Polysaccharide Production

Most polysaccharide polymers must be of a certain length before their physical or biological properties become apparent. Often the polysaccharide must comprise at least 20-100 sugar units. Certain enzymes that react with exogenous polymers have been previously available, but typically add only one sugar unit. The unique enzymes described in the context of the present invention, (e.g., pmHS1, and PmHS2) can form polymers of at least 100-400 sugar units in length. Thus, one embodiment of the presently claimed and disclosed invention, results in long, defined linear polymers composed of only natural glycosidic linkages. In addition, the presently claimed and disclosed invention also includes the addition of 1 or 2 sugars, as well as the addition of 2-100sugars.

The four known glycosaminoglycan synthesizing enzymes from *Pasteurella multocida* bacteria normally make polymers similar to or identical to vertebrate polymers. These bacteria employ the polysaccharide, either HA (Type A bacteria), chondroitin (Type F bacteria), or heparosan (unsulfated, unepimerized heparin; Type D bacteria) as an extracellular coating to serve as molecular camouflage. Native enzymes normally make polymer chains of a single type of sugar repeat. If a recombinant HAS or CS or HS enzyme is employed, however, the enzyme can be forced to work on an exogenous functional acceptor molecule. For instance, the recombinant enzyme may be incubated with a polymer acceptor, and the recombinant enzyme will then elongate the acceptor with UDP-sugar precursors. The known native enzymes do not perform this reaction since they already contain a growing polymer chain that was formed in the living cell; enzyme preparations from native cells typically retain the polymer following isolation.

pmHAS (SEQ ID NO:2), a 972 amino acid residue protein from *Pasteurella multocida,* is made in a functional state in recombinant *Escherichia coli.* The *pmHAS* gene is given in SEQ ID NO:1. Other functional derivatives of pmHAS, for example an enzyme called pmHAS¹⁻⁷⁰³ (SEQ ID NO:9) and the *pmHAS¹⁻⁷⁰³* gene (SEQ ID NO:67), have been produced which are soluble. The soluble form can be prepared in larger quantities and in a purer state than the naturally occurring full-length enzyme. The preferred *E. coli* strains do not have an UDP-GIc dehydrogenase and therefore the recombinant enzyme does not make HA chain in the foreign host. Therefore, the enzyme is in a virgin state since the empty acceptor site can be occupied with foreign polymers. For example, the recombinant enzyme may be incubated in a mixture comprising from about 10 to about 50mM Tris pH 7.2, from 0.5 to about 20 mM MnCl₂, from about 0.1 to about 30 mM UDP-GIcUA, from about 0.1 to about 30 mM UDP-GIcNAc, and a suitable acceptor at about 20-37° for from about 1 to about 600 minutes. Suitable acceptors can be any functional acceptor, such as a glycosaminoglycan acceptor or sugar acceptor, for example, but not by limitation, short HA chains (two or more sugar units such as HA₄) or short chondroitin sulfate chains (5 sugar units) or long chondroitin sulfate chains (~10² sugar units). In the case of the latter acceptors, pmHAS (or its derivatives), then elongates the foreign acceptors (i.e., long or short chondroitin polymers, plus or minus sulfation) at their nonreducing termini with authentic HA chains. The length of the HA chain added onto the acceptor is controlled by altering the concentration of UDP-sugars (thus changing the stoichiometry of UDP-sugar to acceptor) and/or the reaction time. Immobilized acceptors, such as beads or other solid objects with bound acceptor oligosaccharides, can also be extended by the pmHAS enzyme using UDP-sugars. In this manner, the pmHAS enzyme (or its derivatives) can be used to attach polysaccharide chains to any suitable acceptormolecule.

Type A *P. multocida* produces HA capsule [GIcUA-GIcNAc repeats] and possesses the pmHAS enzyme. On the other hand, Type F *P. multocida* produces a chondroitin or chondroitin-like polymer capsule [GIcUA-GaINAc repeats]. The DNA encoding an open reading frame (GenBank accession #AF195517) that is 90% identical to pmHAS at the protein level has been cloned; this enzyme is called PmCS, the *P. multocida* chondroitin synthase. The amino acid sequence of pmCS is set forth in SEQ ID NO:4 and the *pmCS* gene sequence is set forth in SEQ ID NO:3. As the PmCS enzyme's sequence is so similar to pmHAS, one of ordinary skill in the art, given the present specification, is able to manipulate the pmCS in the same manner as that for pmHAS and any manipulation that is successful with regard to the pmHAS would be performable with the pmCS, with the exception that chondroitin chains would be grafted instead of HA. Either HA or chondroitin chains can serve as acceptors for pmCS as both acceptors serve well for pmHAS. The PmHS1 and PmHS2 enzymes possess some similarities to these other GAG synthases, but elongate with heparosan, a distinct polymer, when supplied with UDP-GIcNAc and UDP-GlcUA. If the enzymes are supplied with analog sugar precursors, novel unnatural GAG extensions result.

Such hybrid polysaccharide materials composed of HA, chondroitin and/or heparin within a single polymer chain cannot be formed in a controlled fashion (targeted size and monodisperse) especially with regard to medium to large size polymers (i.e., greater than 2 to 5KDa) by any other existing process without (1) leaving unnatural residues, and/or (2) producing undesirable crosslinking reactions. The testicular hyaluronidase method gives a variety of small products derived from quasi-random linkage of GAGs, HA and chondroitin. Very large polymers are not major or significant products. The chimeric or hybrid polysaccharide materials can serve as biocompatible molecular glue for cell/cell interactions in artificial tissues or organs and the HA/chondroitin/heparin hybrid mimics natural proteoglycans that normally contain an additional protein intermediate between polymer chains. A recombinant HA/chondroitin/heparin chimeric or hybrid polysaccharide, devoid of such an intermediary protein, is desirous since molecules from animal sources are potentially immunogenic -- the chimeric or hybrid polysaccharide, however, would not appear as foreign to the host, thus no immune response is generated. Also, the recombinant polymers can be made free of adventitious agents (e.g., prions, viruses etc.). In addition, the molecules are not degraded by proteases.

An intrinsic and essential feature of polysaccharide synthesis is the repetitive addition of sugar monomer units to the growing polymer. The glycosyltransferase remains in association with the nascent chain. This feature is particularly relevant for HA biosynthesis as the HA polysaccharide product, in all known cases, is transported out of the cell; if the polymer was released, then the HAS would not have another chance to elongate that particular molecule. Four possible mechanisms for maintaining the growing polymer chain at the active site of the enzyme are immediately obvious. First, the enzyme possesses a carbohydrate polymer binding pocket or cleft. Second, the nascent chain is covalently attached to the enzyme during its synthesis. Third, the enzyme binds to the nucleotide base or the lipid moiety of the precursor while the nascent polymer chain is still covalently attached. Fourth, other molecules help retain the chain.

The HAS activity of the native pmHAS enzyme found in *P. multocida* membrane preparations is not stimulated by the addition of HA oligosaccharides; theoretically, the endogenous nascent HA chain initiated *in vivo* renders the exogenously supplied acceptor unnecessary. However, recombinant pmHAS produced in an *E. coli* strain that lacks the UDP-GIcUA precursor, and thus lacks a nascent HA chain, is able to bind and to elongate exogenous HA oligosaccharides. As mentioned above, there are four likely means for a nascent HA chain to be held at or near the active site. In the case of pmHAS, it appears that a HA-binding site exists near or at the sugar transferase catalytic site. In general, PmCS, PmHS1 and PmHS2 should have similar architectures for theirsubstrates.

Defined oligosaccharides that vary in size and composition are used to discern the nature of the interaction between pmHAS and the sugar chain. For example, it appears that the putative HA-polymer binding pocket of pmHAS will bind and elongate at least an intact HA disaccharide with increased efficiency occurring when a trisaccharide is used (reduced tetramer or a synthetic trisaccharide). Synthetic mimics (i.e., glycosides) of disaccharides or trisaccharides are also functional acceptors. Oligosaccharide binding to pmHAS appears to be somewhat selective because the heparosan pentamer, which only differs in the glycosidic linkages from HA-derived oligosaccharides, does not serve as an acceptor. However, chondroitin [GIcUA-GaINAc repeat] does serve as an acceptor for pmHAS.

To date, no other HA synthase besides pmHAS has been shown to utilize an exogenous acceptor or primer sugar. In an early study of a cell-free HA synthesis system, preparations of native Group A *Streptococcal* HAS were neither inhibited nor stimulated by the addition of various HA oligosaccharides including the HA tetramer derived from testicular hyaluronidase digests. These membrane preparations were isolated from cultures that were producing copious amounts of HA polysaccharide. The cells were hyaluronidase-treated to facilitate handling. Therefore, it is quite likely that the native streptococcal enzyme was isolated with a small nascent HA chain attached to or bound to the protein much as suspected in the case of the native pmHAS. Theoretically, the existing nascent chain formed *in vivo* would block the entry and subsequent utilization of an exogenous acceptor by the isolated enzyme *in vitro.* With the advent of molecularly cloned HAS genes, it is possible to prepare virgin enzymes lacking a nascent HA chain if the proper host is utilized for expression. In these tests, recombinant yeast with spHAS did not use HA acceptors proving that the Class I enzyme intrinsically cannot elongate such acceptors.

Both heparin and chondroitin, in mammalian systems, are synthesized by the addition of sugar units to the nonreducing end of the polymer chain. In humans and animals *in vivo,* the glycosyltransferases initiate chain elongation on at least primer monosaccharides [more preferably tetrasaccharides such as xylose-galactose-galactose-GIcUA] that are attached to serine residues of proteoglycan core molecules. *In vitro,* enzyme extracts transfer a single sugar to exogenously added heparin or chondroitin oligosaccharides; unfortunately, the subsequent sugar of the disaccharide unit is usually not added and processive elongation to longer polymers does not occur. Therefore it is likely that some component is altered or missing in the *in vitro* system. In the case of heparin biosynthesis, it appears that a complexe of EXT 1 and 2 enzymes transfers both GlcUA and GlcNAc sugars to the glycosaminoglycan chain.

Recent work with the *E. coli* K5 KfiA and KfiC enzymes, which polymerize heparosan, indicates that a pair of proteins can transfer both sugars to the nonreducing end of acceptor molecules *in vitro.* Extensive processive elongation, however, was not demonstrated in these experiments; crude cell lysates transferred a single sugar to long defined even- or odd-numbered oligosaccharides.

Recombinant pmHAS adds single monosaccharides in a sequential fashionto the nonreducing termini of the nascent HA chain; elongation of HA polymers containing hundreds of sugars has been demonstrated *in vitro.* The simultaneous formation of the disaccharide repeat unit is not necessary for generating the alternating structure of the HA molecule. The intrinsic specificity and fidelity of each half-reaction (e.g., GlcUA added to a GlcNAc residue or vice versa) apparently is sufficient to synthesize authentic HA chains.

A great technical benefit resulting from the alternating disaccharide structure of HA is that the reaction can be dissected by controlling the availability of UDP-sugar nucleotides. By omitting or supplying precursors in a reaction mixture, the glycosyltransferase may be stopped and started at different stages of synthesis of the heteropolysaccharide. In contrast, there is no facile way to control in a step-wise fashion the glycosyltransferase enzymes that produce important homopolysaccharides such as chitin, cellulose, starch, and glycogen.This control also is possible for a targeted synthesis of GAGs with natural and/or unnatural sugars.

An alternative method for controlling polymerization has been accomplished by creating mutants that only add one sugar linkage onto a short HA oligosaccharide. For example, pmHAS¹⁻⁶⁵⁰ (SEQ. ID NO:10) can only add single GlcNAc sugars onto the non-reducing end (i.e., HA tetrasaccharide [GIcNAc-GIcUA-GIcNAc-GIcUA]) of an acceptor (i.e., forms the HA pentamer). On the other hand, a mutant has been created and called pmHAS¹⁻⁷⁰³-D477N (SEQ. ID NO:11) [pmHAS residues 1-703 with an asparagine substituted for the asparatate at position 477], that transfers only a single GlcNAc residue onto the non-reducing terminal GIcUa group of the short HA oligosaccharide. If extracts of two such single-action point mutants (e.g. D477N, SEQ ID NO:11 and D196N [i.e., pmHAS residues 1-703 with an asparagine sustituted for the aspartate at position 196], SEQ ID NO:12) are mixed together with an acceptor in the presence of UDP-GIcNAc and UDP-GIcUA, then significant polymerization is achieved. It is also obvious that by carrying out the steps of GlcNAc or GlcUA transfer separately and sequentially, almost any HA chain length is possible. The same is also true with regard to PmCS either alone or in combination with pmHAS as well as pmHS1 (potential sites described in Kane et al., 2006) or PmHS2 either alone or in combination with pmCS and pmHAS, individually or as a group.

### pmHS1 and PmHS2 Identification and Molecular Cloning

As stated hereinabove, *Pasteurella multocida* Type D, a causative agent of atrophic rhinitis in swine and pasteurellosis in other domestic animals, produces an extracellular polysaccharide capsule that is a putative virulence factor. It has been reported that the capsule of Type D was removed by treating microbes with heparin lyase III. A 617- residue enzyme, pmHS1 (SEQ ID NOS:5 and 66), and a 651-residue enzyme, PmHS2 (SEQ ID NO:8), which are both authentic heparosan (unsulfated, unepimerized heparin) synthase enzymes have been molecularly cloned and are presently claimed and disclosed in copending U.S. Application Serial No. 10/142,143. Recombinant *Escherichia coli*-derived pmHS1 or PmHS2 catalyzes the polymerization of the monosaccharides from UDP-GlcNAc and UDP-GlcUA. Other structurally related sugar nucleotides do not substitute. Synthase activity was stimulated about 7- to 25-fold by the addition of an exogenous polymer acceptor. Molecules composed of -500 to 3,000 sugar residues were produced *in vitro.* The polysaccharide was sensitive to the action of heparin lyase III but resistant to hyaluronan lyase. The sequence of pmHS1 enzyme is not very similar to the vertebrate heparin/heparan sulfate glycosyltransferases, EXT1/2 (SEQ ID NOS:61/62), or to other *Pasteurella* glycosaminoglycan synthases that produce hyaluronan or chondroitin. Certain motifs do exist however, between the pmHS1, pmHS2, and KfiA (SEQ ID NO:59) and KfiC (SEQ ID NO:60) thereby leading to deduced amino acid motifs that are conserved throughout this class of GAG synthases for the production of heparin/heparosan. The pmHS1 and PmHS2 enzymes are the first microbial dual-action glycosyltransferase to be described that form a polysaccharide composed of β4GlcUA-α4GlcNAc disaccharide repeats. In contrast, heparosan biosynthesis in *E. coli* K5 requires at least two separate polypeptides, KfiA and KfiC, to catalyze the same polymerization reaction.

*Molecular Cloning of the Type D P. multocida Heparosan Synthase* - A PCR product which contained a portion of the Type D UDP-glucose dehydrogenase gene was used as a hybridization probe to obtain the rest of the Type D P. *multocida* capsular locus from a lambda library. We found a functional heparosan synthase, which we named pmHS1, in several distinct Type D strains from different host organisms isolated around the world (i.e., A2 clone SEQ ID NOS:5 and 6; bioclone SEQ ID NOS:65 and 66). In every case, an open reading frame of 617 residues with very similar amino acid sequence (98-99% identical) was obtained. In the latter stages of our experiments, another group deposited a sequence from the capsular locus of a Type D organism in GenBank ¹⁵. In their annotation, the carboxyl terminus of the pmHS1 homolog is truncated and mutated to form a 501-residue protein that was called DcbF (GenBank Accession Number AAK17905) (SEQ ID NOS:57 and 58). No functional role for the protein except glycosyltransferase was described and no activity experiments were performed. As described herein, membranes or cell lysates prepared from *E. coli* with the recombinant *dcbF* gene do not possess heparosan synthase activity. The gene annotated as *DcbF* (SEQ ID NO:58) is truncated at the carboxyl terminus in comparison to the presently claimed and described P. *multocida* HS clones. The truncated (T) or the full-length (FL) open reading frames of DcbF were cloned into the expression system pETBlue-1 vector, as described hereinabove. Membranes isolated from the same host strain, *E. coli* Tuner with the various recombinant plasmids were tested in HS assays with both radiolabeled UDP-sugars. The results of these experiments are summarized in Table III.

**TABLE III**

| **Clone** | **[14C]GlcUA Incorp. (dpm)** | **[3H]GlcNAc Incorp. (dpm)** |
|---|---|---|
| Negative Control | 160 | 40 |
| B1(FL) | 710(^{∗}) | 1040(^{∗}) |
| 012(T) | 40 | 265 |
| 013(T) | 70 | 1610 |
| 019(T) | 55 | 1105 |
| N2(T) | 70 | 1910 |
| N4(T) | 70 | 880 |
| N5(T) | 80 | 650 |

Five-fold less FL enzyme than T enzymes were tested in these parallel assays. At most, only a single GlcNAc sugar is added to the exogenously supplied acceptor in the truncated enzymes (T). Full-length HS from Type D *P. multocida,* however, adds both sugars (*) to the nascent chain. Thus, the previously annotated and deposited *DcbF* gene is *not* a functional heparosan synthase.

Another deduced gene was recently uncovered by the University of Minnesota in their Type A *P. multocida* genome project, originally (and erronenously) called "PgIA", but now correctly re-named *PmHS2* (GenBank Accession Number AAK02498), encoding 651 amino acids that are similar to pmHS1 (73% identical in the major overlapping region). However, the PmHS2 gene (SEQ ID NO:7) is not located in the putative capsule locus. This group made no annotation of the function of PmHS2. Our studies show that this PmHS2 protein (SEQ ID NO:8) also polymerizes GlcUA and GlcNAc residues to form heparosan. We also found that a Type D strain and a Type F strain also appear to contain a homologous *PmHS2* gene as shown by PCR and activity analysis.

As mentioned before, during the pmHS1 cloning project in the present inventor(s)' laboratory, investigators at the University of Minnesota published the complete genome of a *Pasteurella multocida* isolate. The fragments of the presently claimed and disclosed *pmHS1* gene were utilized as the query in a BLAST search against this *P. multocida* genome. A gene annotated as *pg*/*A,* but with no ascribed, predicted or demonstrated function was found to be very similar to the *pmHS1* gene. The *pglA* gene is not in the main capsule locus found by either the DeAngelis or the Adler groups. The *pglA* open reading frame was obtained from two different encapsulated strains: Type A (P-1059 from a turkey - this strain is not the same as the Univ. of Minnesota strain - clones denoted as "A") and Type D (P-3881 from a cow - clones denoted as "D"). The *pmHS2* gene was amplified from chromosomal templates prepared by method of Pitcher et al (Letters in Applied Microbiology, 1989). PCR with *Taq* polymerase (18 cycles) using custom flanking oligonucleotide primers that correspond to the region of the start codon and the stop codon of *pmHS2.* An appropriate size amplicon corresponding to the *pmHS2* gene was found in both Type A and D strains; this result was rather unexpected if one considers that the capsular compositions are HA and N-acetylheparosan polysaccharides, for Type A and Type D strains, respectively. The resulting -1.9 kilobase PCR amplicons were ligated into an expression vector, pETBlue-1 (Novagen), transformed into the cloning host, *E. coli* Novablue (Novagen), and selected on LB carbenicillin and tetracycline plates at 30°. The colonies were screened for the presence of insert in the proper orientation by PCR with a combination of vector and insert primers. Clones were streak isolated, small cultures were grown, and preparations of the plasmid DNA were made. The plasmids were transformed into the expression host, *E. coli* Tuner (Novagen), and selected on LB with carbenicillin and chloramphenicol.

After streak isolation, small cultures were grown at 30D° as the starting inoculum (1:100) for larger cultures (50 ml) for protein expression and activity assay. These cultures were grown in the same LB supplemented with 1% casein amino acids and trace element solution with vigorous shaking (250 rpm) at 30D°. The cells were grown to mid-logarithmic phase (2.5 hours), induced with 0.5 mm IPTG, and grown for 4.5 hours. Cells were collected by centrifugation and frozen at -80D° overnight. The membrane preparations were isolated by cold lysozyme/ultrasonication method of DeAngelis et. al. (J. Biol. Chem.,1998; pmHAS isolation the contents of which are expressly incorporated herein in their entirety) except that 0.1 mM mercaptoethanol was used as the reducing agent. The membranes were assayed for radioactive sugar incorporation and descending paper chromatography (according to the methodology of DeAngelis and Padget-McCue, J. Biol. Chem., 2000, the contents of which are expressly incorporated herein in their entirety). Later improvements on the PmHS catalysts included fusion to maltose-binding protein and growth in an *E. coli* strain that readily lyses.

In general, a mixture with membranes, 50 mM Tris, pH 7.2, 10mM MgCl₂, 10 mM MnCl₂, 0.4mM UDP-[³H]GlcNAc, 0.2 mM UDP-[¹⁴C]GlcUA, and heparin oligosaccharide acceptor (2 µg uronic acid) were incubated at 30° for 2.5 hours before analysis by paper chromatography. As expected for a polysaccharide synthase, both sugars were incorporated into polymer (Table IV). Negative controls using membranes from a plasmid with an irrelevant control insert did not show incorporation. Therefore, PmHS2 is a dual-action synthase capable of sugar biosynthesis as shown by functional expression of activity of one recombinant gene in a foreign host that normally does not make GlcUA/GlcNAc polymers. The relaxed specificity of UDP-sugar incorporation of PmHS2 should be of use for the design and production of new polymers with altered characteristics.

**TABLE IV**

| *In vitro* incorporation of sugar by membranes containing recombinant pmHS2 | | |
|---|---|---|
| **CLONE** | **[³H]GlcNAc (dpm)** | **[¹⁴C]GlcUA (dpm)** |
| PmHS2-A2 | 50,400 | 54,900 |
| PmHS2-A4 | 39,100 | 41,000 |
| PmHS2-D4 | 32,500 | 34,200 |
| PmHS2-D7 | 44,800 | 46,600 |

The typical background for negative controls is less than 200 dpm incorporation. Type A and Type D isolates have the PmHS2, a synthase that incorporates both GlcUA and GlcNAc sugars. (A=Type A; D = Type D; # = independent clone number). Table V shows PmHS2 Sugar Specificity test results. The experiments summarized in Table V are similar to the experiments summarized in Table IV (with less enzyme) *except* that other UDP-sugars that are not normally found in heparin or heparosan were also tested (note - 60 minute incubation times, 50 µl reactions). The Type A and the Type D enzymes behave in a similar fashion with relaxed sugar specificity in this test. The PmHS2 system can add a glucose instead of a GlcNAc sugar. The ability to co- polymerize the sugars that compose the authentic heparin backbone were tested by performing two parallel reactions:

UDP-[¹⁴C]GlcUA + various combinations of 2^{nd} UDP-sugars.

UDP-[³H]GlcNAc + various combinations of 2^{nd} UDP-sugars.

*P. multocida* Type F-derived recombinant pmHS2 is thus also a heparosan synthase. As shown in the following Table VII, the Type F PmHS2 can incorporate the authentic heparin sugars.

The pmHS2 homolog of *P. multocida* Type F strain P-4218 was amplified with flanking primers as described for the Type A and D strains. The ORF was subcloned into the pETBlue-1 system in *E. coli* Tuner cells for use as a source of membrane preparations as described. Three independent clones (F 3, 4, 18) were assayed under standard HS assay measuring radiolabeled sugar incorporation with paper chromatography. A negative control, membranes from "Blank" vector and a positive control, the Type D pmHS2 clone D7, were tested in parallel. Reactions plus/minus the Type D polymer acceptor were assayed.

**TABLE V**

| Panel I. Type A PmHS2-A2 | |
|---|---|
| 2^{nd} Sugar | [³H]GlcNAc Incorporated into Polymer (dpm) |
| None | 450 |
| UDP-GlcUA | 12,900 |
| UDP-GalUA | 400 |
| UDP-Glc | 430 |

| 2^{nd} Sugar | [¹⁴C]GlcUA Incorporated into Polymer (dpm) |
|---|---|
| None | 60 |
| UDP-GlcNAc | 7,700 |
| UDP-GalNAc | 60 |
| UDP-Glc | 985 |

| Panel II. Type D PmHS2-D7 | |
|---|---|
| 2^{nd} Sugar | [³H]GlcNAc Incorporated into Polymer (dpm) |
| None | 570 |
| UDP-GlcUA | 13,500 |
| UDP-GalUA | 530 |
| UDP-Glc | 500 |

| 2^{nd} Sugar | [¹⁴C]GlcUA Incorporated into Polymer (dpm) |
|---|---|
| None | 60 |
| UDP-GlcNAc | 6,500 |
| UDP-GalNAc | 40 |
| UDP-Glc | 660 |

**Table VI. Acceptor Usage of PmHS2 from Types A and D**

| The Type A and the Type D clones were tested for stimulation by addition of the Type D polysaccharide acceptor (described hereinbefore with respect to pmHS1). Weaker stimulation of activity by acceptor on pmHS2 was observed in comparison to pmHS1 (comparison is not shown here). | | |
|---|---|---|
| **[¹⁴C-GlcUA] incorporation** | | |
| **Clone** | **Acceptor** | **NO Acceptor** |
| A2 | 1560 | 1210 |
| D7 | 1240 | 1080 |

**Table VII**

| **Activity of pmHS2 from Type F** | | | |
|---|---|---|---|
| **Membranes** | **Acceptor** | **³H-GlcNAc (dpm)** | **¹⁴C-GlcUA (dpm)** |
| Blank | 0 | 8 | 8 |
| PmHS2 F 3 | + | 7100 | 3100 |
| PmHS2 F 4 | 0 | 6100 | 3800 |
| PmHS2 F 4 | + | 11000 | 6400 |
| PmHS2 F 18 | 0 | 20000 | 10000 |
| PmHS2 F 18 | + | 23000 | 12000 |
| PmHS2 D 7 | 0 | 36000 | 17000 |

The next best heterologous matches for the pmHS1 enzyme in the Genbank database are KfiA and KfiC proteins from *E. coli* K5; these two proteins work together to make the heparosan polymer. There is a good overall alignment of the enzyme sequences if smaller portions of pmHS1 ORF are aligned separately with KfiA (pmHS12, SEQ ID NO:59)and KfiC (pmHS11, SEQ ID NO:60). The MULTALIN alignment program (Corpet, 1988) identified regions that were very similar. Some of the most notable sequence similarities occur in the regions containing variants of the DXD amino acid sequence motif. Indeed, the first 1-360 residues of pmHS1 align with an approximate 38% identity to the *E*. *coli* KfiC, a single action GlcUA-transferase, while the 361-617 residues of pmHS12 align with an approximate 31% identity to the *E. coli* KfiA, a GlcNAc-transferase. Thus, the pmHS1 is a naturally occurring fusion of two different glycosyltransferase domains. The pmHS1 is a dual action enzyme that alone makes heparin/heparosan polymers because both sugar transferase sites exist in one polypeptide enzyme.

The amino acid sequence of the heparosan synthase, pmHS1, however, is very different from other *Pasteurella* GAG synthases, pmHAS and pmCS. The pmHAS and pmHS1 enzymes both perform the task of polymerizing the identical monosaccharides; HA and heparin only differ with respect to their linkages. The creation of different anomeric linkages probably requires very distinct active sites due to the disparity between a retaining (to form α-linkages) and an inverting (to form β-linkages) transfer mechanism. The putative dual-action vertebrate heparin synthases, EXT1 (SEQ ID NO:61) and EXT2 (SEQ ID NO:62), also appear to have two transferase domains, but the amino acid sequences are not similar to pmHS1. Thus, by aligning pmHS2, pmHS1 (B10 and A2 clones), KfiA, or KfiC, deduced amino acid sequence motifs have been identified. Such motifs are listed below.

Comparisons of the two known sets of heparin/heparosan biosynthesis enzymes from the *E. coli* K5 *Kfi* locus, the PmHS2 enzyme, and the pmHS1 from Type D capsular locus, allows for the initial assessment and bioinformatic prediction of new enzymes based on the amino acid sequence data. The closer the match (%identity) in a single polypeptide for the two sequence motifs described hereinafter (corresponding to the critical elements of the GlcUA-transferase and the GlcNAc-transferase), the higher the probability that the query enzyme is a new heparin/heparosan synthase (a single dual-action enzyme). The closer the match (%identity) in two polypeptides (especially if encoded in the same operon or transcriptional unit) for the two sequence motifs, the higher the probability that the query enzymes are a pair of single-action glycosyltransferases. Thus, one of ordinary skill in the art would appreciate that given the following motifs, one would be able to ascertain and ascribe a probable heparin synthase function to a newly discovered enzyme and then test this ascribed function in a manner to confirm the enzymatic activity. Thus, single dual-action enzymes possessing enzymatic activity to produce heparin/heparosan and having at least one of the two disclosed motifs are contemplated.
**Motif I: (SEQ ID NO:63)**
**Motif II: (SEQ ID NO:64)**
   (K/R)DXGKFIX12-17DDDI(R/I)YPXDYX3MX40-50 VNXLGTGTV

Motif I corresponds to the GlcUA transferase portion of the enzyme, while Motif II corresponds to the GlcNAc transferase portion of the enzyme. With respect to the motifs:
X = any residue
*parentheses* enclose a subset of potential residues [separated by a *slash*] that may be at a particular position (e.g., - (K/R) indicates that either K or R may be found at the position - i.e., there are semiconserved residues at thatposition.

The *consensus* X spacing is shown with the number of residues in *subscript* (e.g., X12-17), but there are weaker constraints on these particular residues, thus spacing may be longer or shorter. Conserved residues may be slightly different in a few places especially if a chemically similar amino acid is substituted (*e*.*g*., K for a R, or E for a D). Overall, at the 90% match level, the confidence in this predictive method is very high, but even a 70-50% match level without excessive gap introduction (*e*.*g*., altered spacing between conserved residues) or rearrangements (miss-positioning with respect to order of appearance in the amino to carboxyl direction) would also be considered to be within the scope of these motifs. One of ordinary skill in the art, given the present specification, general knowledge of the art, as well as the extensive literature of sequence similarity and sequence statistics (*e*.*g*., the BLAST information website at www.ncbi.nlm.mih.gov), would appreciate the ability of a practitioner to identify potential new heparin/heparosan synthases based upon sequence similarity or adherence to the motifs presented herein and thereafter test for functionality by means of heterozologous expression, to name but one example.

### pmHS1 and PmHS2 Polymer Grafting and Use of Chimeric or Hybrid or Mutant Transferases

As mentioned hereinabove, it was first discovered and disclosed that pmHAS-catalyzed synthesis *in vitro* was unique in comparison to all other existing HA synthases of *Streptococcus,* bacteria, humans or an algal virus. Specifically, recombinant pmHAS can elongate exogenously supplied functional acceptors (described herein) into longer glycosaminoglycans. The pmHAS synthase adds monosaccharides one at a time in a step-wise fashion to the growing chain. The pmHAS exquisite sugar transfer specificity results in the repeating sugar backbone of the GAG chain. The pmCS enzyme, which is 90% identical at the amino acid level to pmHAS, performs the same synthesis reactions but incorporates GalNAc instead of GlcNAc. The pmHS1 and PmHS2 enzymes can also add heparosan chains onto exogenous supplied functional acceptors such as long or short heparosan polymers.

The *Pasteurella* GAG synthases (pmHAS, pmCS, pmHS1 and PmHS2) are very specific glycosyltransferases with respect to the sugar transfer reaction: usually only the authentic sugar is added onto acceptors. The epimers or closely structurally related molecules (*e*.*g*., UDP-glucose) are not utilized. However, these GAG synthases from *Pasteurella* do utilize heterologous acceptor sugars. For example, pmHAS elongates short chondroitin acceptors with HA chains. Additionally, pmHS1 adds heparosan chains onto HA acceptor oligosaccharides. Thus, a diverse range of hybrid of chimeric or hybrid GAG oligosaccharides can be made with the disclosed GAG synthases (i.e., pmHAS, pmCS, pmHS1, and PmHS2). The chemoenzymatic methodology can be used in either a liquid-phase synthesis of soluble, free sugars or in a solid-phase synthesis to build sugars on surfaces (as disclosed hereinafter).

Synthase activity assays (2.5 hours, 30°) with subsequent paper chromatography separations and liquid scintillation counting of the origin zone. Typical reaction buffer (Tris & Mn ion; DeAngelis & White 2001) contained both radioactive UDP-GlcNAc and UDP-GlcUA and various acceptor sugars (as noted in table). Unless noted, the HA was from testicular Haase digestions (Leech means leech HAase). Hep2 or Hep2 are synthetic heparosan disaccharide or trisaccharide analogs, respectively (Haller & Boons, 2001). Recombinant *E*. *coli* derived membranes from cell with plasmids containing pmHS1 gene or no insert (vector). With no membranes and no acceptor sugar, the background was 70 and 35 dpm, respectively.

Thus, chimeric or hybrid GAGS can be made using the *Pasteurella* GAG synthases. As shown in Table VIII, synthetic di- and tri-saccharides of heparosan, and HA can be elongated. Naturally derived HA tetramers can also be elongated. The reducing end is not required to be in a free state (aglycons are not a problem), therefore, the reducing end can serve as the tether site onto a surface, drug, or other synthetic or natural molecule. Exemplary compounds that can be made using the *Pasteurella* GAGs include, but are not limited to:

| | | | | |
|---|---|---|---|---|
| HA-C | CS-HA C-HA | | HA-HP C-HP | HA-C-HA |
| CS-HA-C | C-HA-C | HA-C-HP | CS-HA-HP | C-HA-HP |

and so forth, and one of ordinary skill in the art given this specification would appreciate and be able to construct any number of chimeric or hybrid GAG molecules using the *Pasteurella* GAG synthases disclosed and claimed herein. With respect to the above-referenced chimeric or hybrid GAGs, HA=hyaluronan; C=chondroitin; CS=chondroitin sulfate; and HP=heparosan or heparin like molecules.

**TABLE VIII**

| **Acceptor Sugar Usage of pmHS1 Test** | | | | | |
|---|---|---|---|---|---|
| | **PmHS1** | | **Vector** | | |
| **Acceptor Sugar** | **³H-GlcNAc** | **¹⁴C-GlcUA** | **³H-GlcNAc** | | **¹⁴C-GlcUA** |
| | **(dpm)** | | **(dpm)** | | |
| None | 690 | 580 | 55 | 60 | |
| Type D (0.38µg) sonicated | 4400 | 4500 | 80 | 60 | |
| Heparin (10 µg) porcine | 570 | 560 | 50 | 65 | |
| HA4 (12.5 µg) | 5900 | 6500 | 85 | 65 | |
| HA4 (0.5 µg) | 2200 | 2600 | 60 | 75 | |
| HA4-10 (25 µg) | 7400 | 6900 | 75 | 70 | |
| HA4-10 (1 µg) | 2300 | 2200 | 120 | 70 | |
| HA4 leech (12.5 µg) | 880 | 670 | 45 | 85 | |
| HA8-14 leech (25 µg) | 1100 | 1000 | 70 | 90 | |
| Hep2 (1 µg) | 1800 | 1700 | 70 | 95 | |
| Hep3 (25 µg) | 5800 | 5600 | 55 | 75 | |
| Hep3 (1 µg) | 9700 | 10000 | 45 | 90 | |

The C-terminal halves of pmHAS and pmCS (the putative GlcUA-transferase) can be switched and the sugar-transfer specificity for GlcNAc and GalNAc is not disturbed. This finding suggested that the hexosamine specificity determinants of the enzymes between GlcNAc- and GlcUA-transfer are located in their amino-terminal halves. To define the critical residues or regions that specify sugar transfer, further domain swapping were performed by PCR-overlap-extension.

Certain chimeric or hybrid constructs, such as pm-EG and pm-IK, are not dual-action enzymes and do not have either pmHAS or pmCS activities. But pm-FH, which possesses pmCS residues 1-258, is an active pmCS, although its remaining part is from pmHAS residues 266-703. When more of the pmCS sequence is replaced by pmHAS sequence as in pm-JL enzyme construct (which possesses pmCS residues 1-214 at the amino-terminal and pmHAS residues 222-703 at the carboxyl-terminal), the enzyme is converted into a catalyst with HAS activity. The conversion of GalNAc-transferring activity into GlcNAc-transferring activity indicated that residues 222-265 of pmHAS and probably the corresponding residues 215-258 of pmCS play critical role in the selectivity between binding and/or transferring of GalNAc and GlcNAcsubstrate.

Site-directed mutagenesis of region HAS222-265/CS215-258: none of the residues tested in this region are sufficient alone to switch the sugar transfer specificity between pmHAS and pmCS. In the above identified regions, there are 14 residues that are different between pmHAS and pmCS. We checked the primary sequences of the predicted chondroitin synthases from several independent type F *Pasteurella multocida* in the region of 215to 258. Based on the comparison of these amino acid sequences, most of the differences between pmHAS and pmCS are conserved among those independent strains (FIG. 1). To identify possible critical individual residues that might be important for the selectivity between GalNAc and GlcNAc substrate, we utilized site-directed mutagenesis to change a single or multiple residues in this region. We used either pmHAS1-703 DNA (for I243-, I243/G244/L245-containing mutants) or pmCS¹⁻⁷⁰⁴ DNA (for Y216-, L220-, or C221-containing mutants) as templates and replaced the target residue(s) with the corresponding one(s) in the other enzyme (FIG. 1). Results from enzymatic assays showed that all pmCS¹⁻⁷⁰⁴ mutants transfer GalNAc instead of GlcNAc and all pmHAS¹⁻⁷⁰³ mutants transfer GlcNAc instead of GalNAc. This finding indicates that none of the residues that we tested here are sufficient alone to switch the sugar transfer specificity between pmHAS and pmCS.

### Domain swapping between pmHAS and pmCS: pmCS¹⁻²¹⁴-HAS²²²⁻²⁶⁵-CS²⁵⁸⁻⁷⁰⁴transfers both GlcNAc and GalNAc and GlcN

Based on the above studies, we hypothesized that additional residues in the 44-residues region were important for the selectivity between GalNAc and GlcNAc transferase. To prove our hypothesis, this region was swapped between pmHAS¹⁻⁷⁰³ and pmCS¹⁻⁷⁰⁴ by PCR-overlap-extension. Pm-EG and pPmF4A (a library clone containing pmCS gene locus) DNAs were used to create pmCS¹⁻²¹⁴-HAS²²²⁻²⁶⁵-CS²⁵⁸⁻⁷⁰⁴. Pm-FH and pPm7A (a D library clone containing pmHAS gene locus) DNAs were used to create pmHAS¹⁻²²¹-CS²¹⁵⁻²⁵⁸-HAS²⁶⁶⁻⁷⁰³ (FIG. 2). PmHAS¹⁻²²¹-CS²¹⁵⁻²⁵⁸-HAS²⁶⁶⁻⁷⁰³ did not express. Interestingly, pmCS¹⁻²¹⁴-HAS²²²⁻²⁶⁵-CS²⁵⁸⁻⁷⁰⁴ could transfer both GlcNAc and GalNAc with preference for UDP-GaINAc as judged by HAS assay and CS assay, supporting our conclusion that this region in pmHAS and pmCS plays a critical role in determination of sugar substrate specificity. We also obtained a pmCS¹⁻²¹⁴-HAS²²²⁻²⁶⁵-CS²⁵⁸⁻⁷⁰⁴ clone that possesses an additional mutation of I243V; this clone lost GlcNAc-transferring activity and was switched back into a chondroitin synthase. This finding suggests that I243 in pmHAS, and probably V236 in pmCS, plays important yet unknown roles in the determination of sugar substrate specificity.

In order to examine whether pmCS¹⁻²¹⁴-HAS²²²⁻²⁶⁵-CS²⁵⁸⁻⁷⁰⁴ could transfer sugars other than GlcNAc and GalNAc, different sugar substrates, including UDP-glucose, UDP-galactose, UDP-mannose, UDP-xylose and UDP-glucosamine (GlcN), along with isotope-labeled GlcUA and HA oligosaccharide acceptor, were included when performing the polymerization assay. The results demonstrated that pmCS¹⁻²¹⁴-HAS²²²⁻²⁶⁵- CS²⁵⁸⁻⁷⁰⁴ will use UDP-GlcNAc, UDP-GalNAc, or UDP-glucosamine Table IX. This observation indicated that although swapping of the small region between pmCS and pmHAS resulted in relaxation of substrate selectivity, the enzyme is not so promiscuous that all UDP-sugars will substitute.

The possibility that the chimeric or hybrid enzyme could synthesize hybrid polymers with a blend of HA- and chondroitin-like sugars was also exploited. Reactions containing ³H-UDP-GalNAc, ¹⁴C-UDP-GlcNAc, UDP-GlcUA and HA acceptor were performed. The ratio of the incorporation of ³H-GalNAc and ¹⁴C-GlcNAc changed according to the UDP-sugar ratio in the reaction mixture included in the reaction. Gel filtration analysis of the polymerization products demonstrated that the molecules contain both ³H and ¹⁴C. The characterization of all the chimeric or hybrid proteins is summarized in FIG. 3. In addition, similar strategies for mutagenesis of PmHS1 and PmHS2 or production of chimeric or hybrid enzymes from portions thereof are expected to produce novel, useful catalysts.

Truncation analysis of pmHAS has identified a carboxyl-terminal region that appears to be responsible for the membrane association of pmHAS. Site-directed mutagenesis studies focused on several conserved motifs indicated that these conserved residues are critical for function. PmHAS and PmCS each contain two separate glycosyltransferase sites (Jing and DeAngelis, 2003). Thus the novel "one polypeptide, two active sites" theory has been confirmed. A 44-residue region of the enzymes has been demonstrated to be critical for sugar-transfer specificity. Based on this discovery, an enzyme that can transfer GalNAc, GlcN, and GlcNAc has been engineered.

**Table IX**

| **Sugar substrate specificity of pmCS¹⁻²¹⁴-HAS²²²⁻²⁶⁵-CS²⁵⁸⁻⁷⁰⁴** Standard polymerization assays were performed in the presence of isotope-labeled GlcUA, HA oligosaccharide acceptor, and one of the following sugar substrates. The sugar incorporation was indicated as the percentage of the incorporation of UDP-GalNAc. PmCS¹⁻²¹⁴-HAS²²²⁻²⁶⁵-CS²⁵⁸⁻⁷⁰⁴ can transfer GalNAc, GlcNAc, and Glucosamine. | |
|---|---|
| substrate sugar | incorporation |
| UDP-GalNAc | 100% |
| UDP-GlcNAc | 28% |
| UDP-Glucosamine | 2% |
| UDP-Galactose | not detectable |
| UDP-Glucose | not detectable |
| UDP-Mannose | not detectable |
| UDP-Xylose | not detectable |

Type A *Pasteurella multocida* produces a hyaluronan [HA] capsule to enhance infection. The 972-residue hyaluronan synthase, pmHAS, polymerizes the linear HA polysaccharide chain composed of GlcNAc and GlcUA. PmHAS possesses two separate glycosyltransferase sites. Protein truncation studies demonstrated that residues 1-117 can be deleted without affecting catalytic activity. The carboxyl-terminal boundary of the GlcUA-transferase resides within residues 686-703. Both sites contain a DXD motif. All four aspartate residues are essential for HA synthase activity. D247 and D249 mutants possessed only GlcUA-transferase activity while D527 and D529 mutants possessed only GlcNAc-transferase activity. These results further confirm our previous assignment of the active sites within the synthase polypeptide. The WGGED sequence motif appears to be involved in GlcNAc-transferase activity because E396 mutants and D370 mutants possessed only GlcUA-transferase activity.

Type F *P. multocida* synthesizes an unsulfated chondroitin GalNAc and GlcUA capsule. Domain swapping between pmHAS and the homologous chondroitin synthase, pmCS, was performed. A chimeric or hybrid enzyme consisting of residues 1-427 of pmHAS and residues 421-704 of pmCS was an active HA synthase. On the other hand, the converse chimeric or hybrid enzyme consisting of residues 1-420 of pmCS and residues 428-703 of pmHAS was an active chondroitin synthase. Overall, these findings support the model of two independent transferase sites within a single polypeptide as well as further delineate the site boundaries.

pmHAS utilizes two separate glycosyltransferase sites to catalyze the transfer of GlcNAc and GlcUA to form the HA polymer. Within the pmHAS sequence, there is a pair of duplicated domains which are similar to the "Domain A" proposed by Saxena. Both domains of pmHAS possess a short sequence motif containing DGS that is conserved among many β-glycosyltransferases. Changing the aspartate in either motif to asparagines, glutamate, or lysine significantly reduced or eliminated the HAS activity. However, the D196 mutants and the D477 mutants maintain high level of GlcUA-transferase and GlcNAc-transferase activity, respectively.

pmCS contains 965 amino acid residues and is about 90% identical to pmHAS. A soluble recombinant *Escherichia* coli-derived pmCS¹⁻⁷⁰⁴ catalyzes the repetitive addition of sugars from UDP-GalNAc and UDP-GlcUA to chondroitin oligosaccharide acceptors *in vitro.*

In order to analyze the contribution of the amino terminal region of pmHAS, various recombinant truncated polypeptides were produced (pmHAS⁴⁶⁻⁷⁰³, pmHAS⁷²⁻⁷⁰³, pmHAS⁹⁶⁻⁷⁰³ and pmHAS¹¹⁸⁻⁷⁰³) in *E. coli.* The truncated versions pmHAS⁴⁶⁻⁷⁰³ and pmHAS⁷²⁻⁷⁰³ were as active as pmHAS¹⁻⁷⁰³, a soluble polypeptide with complete HAS activity. PmHAS⁹⁶⁻⁷⁰³ expressed at a very low level compared with other constructs but was active. PmHAS¹¹⁸⁻⁷⁰³ expressed better than pmHAS⁹⁶⁻⁷⁰³ and still elongated HA chains. Therefore, it is probable that further deletion beyond residue 72 affected the overall folding efficiency of the entire polypeptide. Observation of lower molecular weight degradation bands derived from pmHAS¹¹⁸⁻⁷⁰³ on Western blots also suggests that improper folding occurs to some extent. Overall, these findings suggest that the amino-terminal 117 residues are not required for HA synthase activity.

pmHAS¹⁻⁶⁵⁰ loses its GIcUA-transferase activity. To further delineate the GlcUA-transferase domain within the carboxyl terminal region, two slightly longer mutants, pmHAS¹⁻⁶⁶⁸ and pmHAS¹⁻⁶⁸⁶ were created. Both mutants also could not polymerize HA due to the loss of GlcUA-transferase activity, indicating that the carboxyl-terminal boundary of the GlcUA-transferase resides between residues 686 and 703. Similar analyses of PmHS1 (Kane et al., 2006) suggest that residues -1-77 and ~601-651 are indispensable for catalytic activity (these truncations have been assigned SEQ ID NOS:71 and 70, respectively).

### Monodisperse Glycosaminoglycan Polymer Synthesis

The size of the hyaluronan [HA] polysaccharide dictates its biological effect in many cellular and tissue systems based on many reports in the literature. However, no source of very defined, uniform HA polymers with sizes greater than 2 to 5 kDa is currently available. This situation is complicated by the observation that long and short HA polymers appear to have antagonistic or inverse effects on some biological systems. Therefore, HA preparations containing a mixture of both size populations may yield contradictory or paradoxical results.

The *Pasteurella* bacterial HA synthase enzyme, PmHAS, catalyzes the synthesis of HA polymers utilizing monosaccharides from UDP-sugar precursors *in vivo* and *in vitro.* PmHAS will also elongate exogenously supplied HA oligosaccharide acceptors *in vitro*; in fact, HA oligosaccharides substantially boost the overall incorporation rate. A purified recombinant, PmHAS derivative was employed herein to produce either native composition HA or derivatized HA. The same general behavior was exhibited by PmCS and PmHS1 and PmHS2; the presence of acceptors stimulated polymerization.

HA polymers of a desired size were constructed by controlling stoichiometry (i.e., ratio of precursors and acceptor molecules). The polymerization process is synchronized in the presence of acceptor, thus all polymer products are very similar. In contrast, without the use of an acceptor, the polymer products are polydisperse in size. In the present examples, stoichiometrically controlled synchronized synthesis reactions yielded a variety of HA preparations in the range of -15 kDa to about 1.5 MDa. Each specific size class had a polydispersity value in the range of 1.01 for polymers up to 0.5 MDa or ~1.2 for polymers of -1.5 MDa (1 is the ideal monodisperse size distribution) as assessed by size exclusion chromatography/multi-angle laser light scattering analysis. The selectHA preparations migrate on electrophoretic gels (agarose or polyacrylamide) as very tight bands. Similarly, PmCS and PmHS1 will produce defined monodisperse polymers in reactions with acceptor.

The use of a modified acceptor allows the synthesis of selectHA polymers containing radioactive (e.g., 3H, 1251), fluorescent (e.g., fluorescein, rhodamine), detection (i.e., NMR or X-ray), affinity (e.g., biotin) or medicant tags. In this scheme, each molecule has a single detection agent located at the reducing terminus. Alternatively, the use of radioactive UDP-sugar precursors allows the synthesis of uniformly labeled selectHA polymers with very high specific activities.

Overall, the selectHA reagents should assist in the elucidation of the numerous roles of HA in health and disease due to their monodisperse size distributions and defined compositions. It must be emphasized that unpredicted kinetic properties of the Pasteurella GAG synthases in a recombinant virgin state in the presence of defined, unnatural reaction conditions facilitates targeted size range production of monodisperse polymers that are not synthesizable by previously reported methods.

Affect of HA acceptor on pmHAS-catalyzed polymerization. HA polymerization reactions were performed with purified pmHAS and UDP-sugar precursors under various conditions, and the reaction products were analyzed by agarose gel or acrylamide gel electrophoresis. The size distribution of HA products obtained were observed to be quite different based on the presence or absence of the HA4 acceptor in the reaction (Jing and DeAngelis, 2004). When 30 mM of UDP-sugars were present as well as 0.03 ug/ul of HA4, pmHAS synthesized smaller chains with a narrow size distribution. The Mn determined by MALLS is 551.5kDa and its polydispersity (Mw/Mn) is 1.006 (Jing and DeAngelis, 2004). However, without HA4, pmHAS synthesized a more polydisperse product with the same amount of precursor sugars. The Mn determined by MALLS is 1.53 MDa and its polydispersity (Mw/Mn) is 1.169.

To verify whether pmHAS can utilize HA acceptors of various sizes, parallel assays were set up using the same starting conditions, and at various times additional UDP- sugars were added to the reaction. The result indicated that intermediate products were utilized as starting material for later chain elongation bypmHAS.

Size control of HA. The polymerization by pmHAS in the presence of HA acceptor is a synchronized process, and thus a more defined HA preparation can be obtained with pmHAS. This synchronization is probably due to the difference in rate or efficiency of new chain initiation versus chain elongation as speculated earlier in DeAngelis, 1999 and depicted in Fig. 4 model. The addition of acceptor appears to bypass the slower initiation step; thus all chains are elongated in parallel resulting in a more homogenous final population (Jing and DeAngelis, 2004). A model demonstrating *Pasteurella* synthase reaction synchronization mediated by acceptor usage is shown in Fig. 5.

The synthase enzyme will preferentially add available UDP-sugar precursors to the acceptor termini. If there are many acceptors, thus many termini, then a limited amount of UDP-sugars will be distributed among many molecules and thus result in many short polymer chain extensions. Conversely, if there are few acceptors, thus few termini, then the limited amount of UDP-sugars will be distributed among few molecules and thus result in a few long polymer chain extensions (modeled in FIG. 6). It has previously been observed that chain initiation is the rate-limiting step for pmHAS, and the enzyme prefers to transfer sugars onto existing HA chains when acceptor is included in the reaction. If the polymerization is indeed a synchronized process, then the amount of HA4 should affect the final size of the HA product when the same amount of UDP-sugar is present. To test this speculation, assays were performed with various levels of HA4 with fixed amount of UDP- sugar and pmHAS (Fig. 8A). To determine the size and polydispersity of these HA products, HA polymer sizes were determined by size exclusion chromatography - Multi Angle Laser Light Scattering (SEC-MALLS, Fig. 8B). Using the same strategy HA was generated from 27 kDa to 1.3 MDa with polydispersity ranging from 1.001 to 1.2. Fig. 8 demonstrates the monodispersity of the various HA polymers resulting from reaction synchronization. This range has been extended from 5 kDa to -2.5 MDa.

*In vitro* synthesis of fluorescent HA. The *in vitro* technology for the production of monodisperse glycosaminoglycans also allows the use of modified acceptor to synthesize HA polymers containing various types of foreign moieties. An example is shown using fluorescent HA4 to produce fluorescent monodisperse HA of various sizes (Fig. 9). Similarly, radioactive (e.g., ³H, ¹²⁵I), affinity (e.g., biotin), detection (e.g., probe for NMR or X-ray uses or a reporter enzyme), or medicant tagged glycosaminoglycan polymers are possible with the appropriate modified acceptor.

In addition to the small sugar chains (e.g., tetrasaccharide HA4), larger HA polymers can be used as starting acceptor for pmHAS; the enzyme will elongate existing chains with more sugars. Experiments were performed using 575 kDa HA and 970 kDa HA (synthesized *in vitro* with pmHAS and HA4 as acceptor, using the previously described methods) and a commercially available HA sample (~2 MDa; Genzyme) as acceptors. The results indicate that the existing HA chains were further elongated (FIG. 10). For example, the ~2 MDa starting material in lane 11 was elongated to produce the larger (i.e., slower migrating) material in lane 10. Therefore, a method for creating higher value longer polymers is also described. The length of the final product can be controlled stoichiometrically as shown in lanes 7-9; a lower starting acceptor concentration (lane 7) results in longer chains because the same limited amount of UDP-sugars is consumed, making a few long chains instead of many shorter chains (lane 9). PmHS1 similarly can elongate longer polymer acceptors; for example, a ~50 kDa polymer served as an acceptor (Kane et al., 2006).

The molecular weights of naturally existing HA polymers usually range from hundreds of thousands up to several millions of Daltons. For research requiring smaller HA polymers, enzymatic degradation is usually the first choice. However, this process is not satisfactory because it is time-consuming and the final yield of the targeted HA size fraction is low, and demanding chromatography is required. With these *in vitro* synthesis techniques, HA as small as 10 kDa can be generated with polydispersity around 1.001.

High molecular HAs are commercially available from animal or bacterial sources. Problems with those include possible contaminants leading to immunological responses as well as broad size distribution (Soltes etc., 2002). Polydispersities (Mw/Mn) are commonly higher than 1.5. Conclusions drawing from experimental data during biological research with these HA could be misleading. Thus there exists a need for uniform HA to perform biological study, as agreed by Uebelhart and Williams (1999).

To determine the exact average molecular mass of HA, MALLS is usually the choice. Yet many people have the need to quickly estimate the mass. For this purpose, some groups investigated the correlation of HA migration on agarose gel with DNA (Lee and Cowman, 1994). The drawback of this method is that, first, the HA samples used were not uniform, and second, the migration of HA and DNA on agarose gel changes differently with the change of the concentration of agarose gel. The *in vitro* generated HA of defined size distribution provide excellent series of standards for this purpose (Fig. 11).

In addition to making HA polymers, the relaxed acceptor specificity of pmHAS allows the use of various chondroitin acceptors. This allows the production of monodisperse hybrid GAGs that have utility in medicine including tissue engineering and surgical aids. In particular, new protein-free proteoglycans are now possible that do not have antigenicity or allergenicity concerns compared to animal-derived products.

In FIG. 12, various monodisperse chondroitin sulfate HA hybrid GAGs are created by elongating a variety of chondroitin sulfates (A, B, and C) with pmHAS, thus adding HA chains. Various amounts of HA were added to the preparations (at various times during reaction as noted) by adding more UDP-sugars. For example, lanes 3-6 show hybrids with a constant amount of chondroitin sulfate and increasing HA chain lengths. The starting chondroitin sulfates stain weakly here, and the band position is marked with an arrow. Without the acceptor (lanes 23-26), no such defined bands are seen; after a long period, some HA polymer shows up (lane 26) which results from *de novo* initiation without acceptor.

In FIG. 13, chondroitin sulfate A was elongated with pmHAS, thus adding HA chains. Various amounts of HA were added to the preparations by controlling the level of chondroitin acceptor (thus changing the UDP-sugar/acceptor ratio) as well as adding more UDP-sugars during the reaction. By changing the UDP-sugar/acceptor ratio, stoichiometric control of the hybrid GAG size was demonstrated.

In FIG. 14, pmCS and UDP-GlcUA, UDP-GalNAc were reacted with either a 81 kDa HA acceptor (migration position marked with arrow; lanes 3-7) or no acceptor (lanes 9- 13). Various lengths of chondroitin were added to the HA chains (at longer times with more UDP-sugars producing longer hybrid chains). Without the acceptor, no such defined bands were seen; after a long period, some long pure chondroitin polymer shows up which results from *de novo* initiation without acceptor.

In FIG. 15, Size exclusion (or gel filtration) chromatography analysis coupled with multi-angle laser light scattering detection confirms the monodisperse nature of polymers . In the FIG. 15A, HA (starting MW 81 kDa) extended with chondroitin chains using pmCS (same sample used in Fig. 14, lane #7, overnight [O/N] extension ) was analyzed; the material was 280,000 Mw and polydispersity (Mw/Mn) was
1.003 +/- 0.024. Chondroitin sulfate HA extended with HA chains using pmHAS (same sample used in Fig. 12, lane #23) was analyzed and shown in FIG. 15B; the material was 427,000 Mw and polydispersity (Mw/Mn) was 1.006 +/- 0.024.

In FIG. 16, a 0.7% agarose gel detected with Stains-all compares the monodisperse, 'select HA' to commercially produce HA samples is shown. In lanes 1-3, the mixture of various monodisperse HAs (separate reaction products that were recombined to run all in one lane; sizes from top to bottom of lane: 1.27 MDa, 946 kDa, 575 kDa, 284 kDa, 27 kDa) run as discrete, tight bands. In contrast, in lanes 4-7, the commercially produced HA samples run as polydisperse smears (lane 4, 1.1 MDa; 5, 810 kDa; 6, 587 kDa; 7, 350 kDa). Remarkably, the monodisperse HA bands look almost as narrow as the single-molecule species of DNA present in lane 8 (BIOLINE standard).

Next, it was demonstrated that the catalytic utility of PmHS1 and PmHS2 are very distinct as measured by various criteria, including their ability to produce polymers either with monodisperse size distributions or with unnatural sugar compositions.

The maltose binding protein/heparosan synthase fusion constructs (Sismey- Ragatz et al., 2007) had greatly increased protein expression in comparison to the earlier generation thioredoxin PmHS1 fusion construct (Kane et al., 2006). The MBP also allowed for efficient purification as depicted in FIG. 17; in contrast, the thioredoxin affinity handle was observed to leach substantial amounts of target protein thus thwarting purification attempts (data not shown). Furthermore, both MBP-PmHS constructs possessed increased stability at useful reaction temperatures (e.g., active at pH 7.2 at 30°C for 24hrs).

Previous studies on the efficiency of cognate acceptor utilization by the crude native sequence enzymes suggested that these relatively homologous *Pasteurella* synthases had different catalytic properties; acceptor stimulated PmHS1 sugar incorporation ~7- to 25-fold (by serving as a primer to circumvent the slow initiation step) while PmHS2 is boosted only ~2.5-fold (DeAngelis et al., 2002 and 2004). These levels of acceptor stimulation were also observed for the purified fusion enzymes. In polymerization assays without acceptor, it also appears that purified PmHS2 has ~2-fold higher level of *de novo* initiation of sugar chains compared to purified PmHS1 (∼5.2 versus -2.6 pmoles monosaccharide transferred/min/µg protein). On the other hand, it appears that PmHS1 has an elongation rate which is -3-fold faster than PmHS2 (-76 versus -28 pmoles monosaccharide transferred/min/µg protein).

The pH profiles as determined by polymerization assays were also different; the purified PmHS1 catalyst preferred a neutral pH while purified PmHS2 preferred acidic (pH -4-5) conditions (FIG. 18). This result of differential activity was not expected considering that the protein sequences of PmHS1 and PmHS2 are relatively homologous. Simplistically, based on expected amino acid side-chain pKₐ values it may be likely that one or more histidine residues in PmHS2 (but not present in PmHS1) is protonated at the lower pH, thus gaining a positive charge, making a better contact or providing improved electrostatic steering for a negatively charged substrate (either a heparosan oligosaccharide or a UDP-sugar). Alternatively, one or more glutamate or aspartate residues in PmHS2 (but not PmHS1) are protonated at lower pH, thus neutralized, reducing potential electrostatic repulsion of a negatively charged substrate. It is important to note that even though PmHS2 prefers the acidic pH for maximal activity, this catalyst is not very stable in those conditions. The PmHS2 protein did not demonstrate noticeable additional proteolysis at low pH as assessed by western blotting (not shown), thus the loss of activity must be due to denaturing via an unfolding event.

The size of acceptor oligosaccharide preferred by each of the synthases was also examined. The heparosan tetrasaccharide was about ~150-fold and -8-fold better than the corresponding disaccharide for PmHS1 or for PmHS2, respectively. In addition, the synthetic glycoside, AFA, was also a useful acceptor for PmHS1 and PmHS2. These findings suggest that the size of the active site pockets of the heparosan synthases may be similar to those hypothesized for the acceptor sites of PmHAS; a site that appears to bind 3 or 4 monosaccharides is hypothesized to make contact with the nascent HA chain. Simpler glycosides are also good acceptors for PmHS1 or PmHS2.

**Monodisperse Heparosan** - Synchronized polymerization reactions should result in monodisperse heparosan polymers as previously observed for PmHAS, as described herein. The formation of heparosan with narrow size distribution is dependent on the ability of the glycosyltransferase to be primed by acceptors (thus avoiding a slow *de novo* initiation event yielding out of step elongation events) and efficiently transfer monosaccharides from UDP-sugars (Fig. 4-6). It is likely that PmHS1 catalyzes the synthesis of higher molecular weight monodisperse polymer when compared to PmHS2 due to its better ability to utilize and rapidly elongate exogenously supplied acceptors. As determined by agarose gel and SEC-MALLS analyses (FIG. 19), PmHS1 produced various sizes of monodisperse high molecular weight heparosan (∼70% average yield based on starting UDP-sugars) while under identical conditions PmHS2 did not. Such monodisperse heparosan polymer may serve as the starting material for the creation of defined molecules that are more predictable with respect to biological responses and potency.

### Production of Glycosaminoglycan Polymers with Unnatural Structures

The testing of a variety of different UDP-sugar donor substrates is a means to determine the tolerance of the synthase active sites for a variety of donor and acceptor functional groups. Characterizing donor preference is obvious, but in the case of a polymer with repeating saccharide units, once a sugar is added onto the non-reducing terminus of the nascent chain, the unnatural sugar then serves as an acceptor substrate. Therefore, a successful analog must be able to play multiple roles to produce a polysaccharide chain.

MALDI-ToF MS analyses and radiolabeled sugar incorporation assays revealed that PmHS2 has the ability to catalyze the incorporation of several unnatural donor sugar analogs while PmHS1 appears much more strict (Table X, FIG. 20). The GlcNAc-transferase site of PmHS2 will accept different acyl chain lengths at the C2 position as long as the amine is acylated, but does not appear to tolerate substitution at the C3 or C5 positions. Interestingly, UDP-GlcNPro, the UDP-GlcNAc analog with an extra methylene group in the acyl chain, is preferred by both enzymes more than the authentic substrate; perhaps a hydrophobic pocket is responsible for this catalyst/substrate contact. However, this pocket must have limited dimensions because UDP-GlcNBut, a molecule with two more additional methylene units than the authentic donor, is a worse substrate. Other hydrophobic moieties with different structures are expected to serve as donors as well. By analogy, a hydrophobic pocket on glycosaminoglycan binding proteins or receptors such as the HA-binding site of TSG-6 (Blundell et al., 2005) may bind with higher affinity to polymers containing hexosamines with longer acyl chains, thus new sugar ligand derivatives with more potent inhibition or signaling effects may be possible.

**TABLE X: Donor Substrate Usage by PmHS1 and by PmHS2.**

| Each UDP-sugar analog GlcUA by radiolabeled authentic UDP-sugar and a potential UDP-GlcNAc combination of both analogs are presented **n.a.**, not applicable. All with mass spectrometry efficiency. Overall, have more restricted was tested for its ability to sugar polymerization assays the appropriate second UDP-s substitute) were co-incubated authentic donors, UDP-GlcNAc a as **+++** = > 200 %, **++** = positive compounds were verifi (FIG. 20) except for PmHS2 can mis-incorporate several donor usage. substitute for UDP-GlcNAc or UDP-and paper chromatography. An ugar analog (*e.g.,* UDP-GlcUA and with enzyme. The rates for the nd UDP-GlcUA, are set to 100%; 100-11%, **+** = 10-1%, **-** = <∼0.2%, ed by single sugar addition assays UDP-GlcdiNAcUA due to low transfer analogs, but PmHS1 appears to | | | | |
|---|---|---|---|---|
| | **PmHS1** | | **PmHS2** | |
| ***UDP-Sugar Analog*** | **substitutes for:** | | **substitutes for:** | |
| | **UDP-GlcUA?** | **UDP-GlcNAc?** | **UDP-GlcUA?** | **UDP-GlcNAc?** |
| UDP-Glc**N** | **n.a.** | - | **n.a.** | - |
| UDP-GlcNAc**UA** | - | - | **+** | - |
| UDP-Glc**diNAc** | **n.a.** | - | **n.a.** | - |
| UDP-Glc**diNAcUA** | - | - | **+** | - |
| UDP-GlcN**But** | **n.a.** | - | **n.a.** | **+** |
| UDP-GlcN**Pro** | **n.a.** | **+++** | **n.a.** | **+++** |

The GlcUA-transferase site of PmHS2, but not PmHS1, is tolerant of extra chemical groups at the C2 or C3 positions (Table X). The UDP-GlcNAcUA analog possesses within a single pyranose unit both the C6 carboxylate and the C2 acetylated amide groups (normally found separately on two adjacent pyranose units in native heparosan). It was observed that the PmHS2 enzyme only utilizes this analog to substitute for the uronic acid unit of the disaccharide repeat, and not the hexosamine (Table X). At this time, it is difficult to predict if this analog fails as a hexosamine because it is a poor donor and/or a poor acceptor. Preparative syntheses employing PmHS2 catalyst with no acceptor gave average polymer yields of ~60% or ~22% for authentic heparosan versus unnatural GlcNAcUA-containing heparosan, respectively. Higher concentrations of UDP-sugar precursor helped compensate for the slower incorporation rates of some unnatural analogs.

The relaxed specificity of PmHS2 could be due to different active site geometry or different surrounding residues than PmHS1, which facilitates the favorable binding interactions and/or avoids certain hindrances (e.g., steric, electrostatic) with the analogs. Overall, these results help to elucidate the nature of the synthase active site without an experimentally determined three-dimensional enzyme structure.

From previous work and here with purified PmHS1 and PmHS2, the isomeric state of the C4 hydroxyl of the UDP-sugar precursors appears to be critical for these synthases because the C4 epimers of the authentic substrates, UDP-GalNAc and UDP-GalUA, are not functional analogs in the polymerization assay (not shown). This observed stringency is probably due to the importance of the hydroxyls forming the glycosidic linkages of the heparosan chain, (-GlcUA-β1,4-GlcNAc-α1,4-), residing in the correct orientation for catalytic residues to couple the saccharide units.

The evolutionary history of PmHS1 and PmHS2 is not yet known. Two opposing hypotheses are possible: (I) the "traditional" scenario where a gene encoding a substrate selective PmHS1 progenitor was duplicated and the resulting PmHS2 ancestor, unfettered from its normal duty of making heparosan, became less specific for a potential hitherto unknown function, or (II) a more recently recognized scenario (Jensen, 1976) where a gene encoding a nonspecific PmHS2 progenitor was duplicated resulting in a PmHS1 ancestor that became more substrate specific in order to make heparosan. As PmHS2 (but not PmHS1) occurs in many Type A and Type F strains (HA or chondroitin capsule producers, respectively), model II may be more likely. More DNA sequence information from other isolates and species may be required to establish whether PmHS1 or PmHS2 was the primordial enzyme. Pathogenic bacteria are under extreme selective pressure from host defenses thus the potential to alter capsule composition and maintain virulence is a valuable asset.

The promiscuity of PmHS2 makes it a useful catalyst for preparing glycosaminoglycan polymer analogs with new biological or chemical properties. For example, unnatural polymers containing the GlcNAcUA monomer are not digested by heparin lyase III, an enzyme known to digest most other heparinoids (FIG. 21). Depending on the substitutions, similar heparinoids may have a slower turnover rate potentially making it a longer acting therapeutic. These new polymers should also prove to be very useful in the pursuit of understanding the structure/function relationships of the polymer and the interaction of heparinoids with various binding proteins including receptors, growth factors, and coagulation factors.

Single sugar addition and polymerization assays confirmed that both PmHAS and PmHS2 would utilize the UDP-GlcN[TFA] (UDP-N-(trifluoroacetyl)glucosamine) as a hexosamine donor substitute (FIGS. 22 and 23). In contrast, PmHS1, recombinant *Xenopus* HAS and recombinant *Streptococcus* HAS did not effectively transfer UDP-GlcN[TFA] (>0.01% for PmHS1, ~1.0% for *Xenopus* and streptococcal HAS). This data indicates again that PmHS2 has relaxed specificity at the C-2 position in the GlcNAc transferase site and reveals for the first time that PmHAS will also tolerate unnatural groups at the C-2 position. The relative efficiency of catalyst to transfer UDP-GlcN[TFA] was assessed by radiolabel incorporation and PAGE.

GAGs mediate many of their biological effects via interactions with proteins. Here, the binding properties and the sensitivity to degradation enzymes of the unnatural GlcN[TFA] polymer in comparison to the natural HA polymer were tested. One of the major HA binding proteins is CD44, a glycoprotein expressed on most cell surfaces and facilitates many signaling events as well as the cellular intake of HA. Only a portion of the CD44 protein (residues 1-199) was used in this study; this domain contains the link module which facilitates HA binding. HABP or aggrecan also specifically binds to HA and is a huge proteoglycan complex found in cartilage. Aggrecan is composed of three gobular domains and two extended regions, but in this assay, only the link module in the gobular 1 domain (HA binding region) was tested for binding. ELISA binding assays indicated that the GlcN[TFA] polymer binds at least 100-fold more weakly to both HA binding proteins, HABP and CD44, compared to HA polymer of similar size. Unlike PmHAS, which will bind and extend a GlcN[TFA] polymer, these HA binding proteins will not tolerate a TFA group at the C-2 position. Digestion experiments using Heparin Lyase III and hyaluronidase determined that the GlcN[TFA]-containing GAG polymers are susceptible to degradation by the appropriate enzyme and thus, recognized by these distinct degradative enzymes (FIG. 24).

Removal of natural acetyl groups from GAGs usually requires extreme conditions (hydrazine at 100°C for hours) that often results in cleavage and sugar ring perturbations. The use of the TFA group provides a specific method for N-deacetylation under milder basic conditions. The electronegative fluorine atoms remove electron density from the amide bond thus weakening it. The deprotected amine can hypothetically be conjugated very effectively with any amine-reactive moiety or chemically cross linked to form a gel (FIG. 25). Furthermore, the amino group is the natural target for the N-sulfation enzyme, which will help to facilitate the bioenzymatic production of heparin.

**TABLE XI: Unnatural UDP-Sugar Substrate Utilization**

| **UDP-Sugar** | **PmHAS** |
|---|---|
| **U-GlcN** | N |
| **U-GlcNAcUA** | N |
| **U-GlcNAcNAc** | N |
| **U-GlcdiNAcUA** | N |
| **U-GlcN[TFA]** | Y** (0.6%) |
| **U-GlcNBut** | Y** (2%) |
| **U-GlcNPro** | Y** (100%) |

| | |
|---|---|
| **Acts like GlcNAc | |

**Table XII**

| | **PmHS1 Substitutes for:** | | **PmHS2 Substitutes for?** | | **PmHAS Substitutes for?** | |
|---|---|---|---|---|---|---|
| **UDP-Sugar analogs** | **UDP-GlcUA?** | **UDP-GlcNAc?** | **UDP-GlcUA?** | **UDP-GlcNAc?** | **UDP-GlcUA?** | **UDP-GlcNAc?** |
| UDP-2-deoxy-2-fluoro-GlcUA | + | n.a. | + | n.a. | + | n.a. |
| UDP-2-deoxy-6-fluoro-GlcNAc | n.a. | +++ | n.a. | +++ | n.a. | + |
| UDP-6,6'-difluoro-GlcNAc | n.a. | + | n.a. | + | n.a. | - |

Other UDP-sugar donors that substitute for the authentic uronic acid or hexosamine also are incorporated by PmHAS, PmHS1 or PmHS2 (see Tables X-XII). For example, the resulting GAG-like polymers with fluorine-containing analogs (Table XII) will differ in chemical properties and biological activities.

There are a multitude of roles for GAGs in the body. Some cellular or molecular systems will recognize and/or metabolize the GAG-analogs in a different fashion than the natural sugars or other related analogs; this avenue allows targeting or selectivity in a therapeutic treatment.

For the analogs in the Table XII, another biomedical application is the preparation of new NMR (nuclear magnetic resonance) or MRI (magnetic resonance imaging) probes. The fluorine atom, ¹⁹F, is a very useful since it has good spectral properties and the normal animal or human body contains very little of this atom. GAGs with ¹⁹F will be readily tracked in the body; if a tissue or cell binds and/or internalizes the probe, it may be detected by this non-invasive procedure. For example, a diseased or cancerous cell that preferentially binds the probe will have more ¹⁹F signal. Likewise, the use of other NMR-active tags will be similarly possible when incorporated into the GAG chain. ¹⁸F, the positron emitting radioactive atom, could also be used in a similar fashion, and is tracked using a PET scanner.

### Biomaterials and Methods of Making Same

Biomaterials also play a pivotal role in the field of tissue engineering. Biomimetic synthetic polymers have been created to elicit specific cellular functions and to direct cell-cell interactions both in implants that are initially cell-free, which may serve as matrices to conduct tissue regeneration, and in implants to support cell transplantation. Biomimetic approaches have been based on polymers endowed with bioadhesive receptor-binding peptides and mono- and oligosaccharides. These materials have been patterned in two- and three-dimensions to generate model multicellular tissue architectures, and this approach may be useful in future efforts to generate complex organizations of multiple cell types. Natural polymers have also played an important role in these efforts, and recombinant polymers that combine the beneficial aspects of natural polymers with many of the desirable features of synthetic polymers have been designed and produced. Biomaterials have been employed to conduct and accelerate otherwise naturally occurring phenomena, such as tissue regeneration in wound healing in the otherwise healthy subject; to induce cellular responses that might not be normally present, such as healing in a diseased subject or the generation of a new vascular bed to receive a subsequent cell transplant; and to block natural phenomena, such as the immune rejection of cell transplants from other species or the transmission of growth factor signals that stimulate scarformation.

Approximately 10 years ago, the concept of bioadhesion was introduced into the pharmaceutical literature and has since stimulated much research and development both in academia and in industry. The first generation of bioadhesive drug delivery systems (BBDS) were based on so-called mucoadhesive polymers, i.e., natural or synthetic macromolecules, often already well accepted and used as pharmaceutical excipients for other purposes, which show the remarkable ability to 'stick' to humid or wet mucosal tissue surfaces. While these novel dosage forms were mainly expected to allow for a possible prolongation, better localization or intensified contact to mucosal tissue surfaces, it had to be realized that these goals were often not so easily accomplished, at least not by means of such relatively straightforward technology. However, although not always convincing as a glue, some of the mucoadhesive polymers were found to display other, possibly even more important biological activities, namely to inhibit proteolytic enzymes and/or to modulate the permeability of usually tight epithelial tissue barriers. Such features were found to be particularly useful in the context of peptide and protein drug delivery.

The primary goal of bioadhesive controlled drug delivery is to localize a delivery device within the body to enhance the drug absorption process in a site-specific manner. Bioadhesion is affected by the synergistic action of the biological environment, the properties of the polymeric controlled release device, and the presence of the drug itself. The delivery site and the device design are dictated by the drug's molecular structure and its pharmacological behavior.

For example, sutures or bandages made of heparosan-chains grafted on the surface or throughout the material in combination with the fibrinogen glue. The immobilized heparosan does not diffuse away as in current formulations, but rather remains at the wound site.

Organic materials have also been postulated for use as bioadhesives. Bioadhesive lattices of water-swollen poly(acrylic acid) nano-and microparticles have been synthesized using an inverse (W/O) emulsion polymerization method. They are stabilized by a co-emulsifier system consisting of SPAN^{™} 80 and TWEEN^{™} 80 dispersed in aliphatic hydrocarbons. The initial polymerization medium contains emulsion droplets and inverse micelles which solubilize a part of the monomer solution. The polymerization is then initiated by free radicals, and particle dispersions with a narrow size distribution are obtained. The particle size is dependent on the type of radical initiator used. With water-soluble initiators, for example ammonium persulfate, microparticles are obtained in the size range of 1 to 10 micrometer, indicating that these microparticles originate from the emulsion droplets since the droplet sizes of the W/O emulsion show similar distribution. When lipophilic radical initiators, such as azobis-isobutyronitrile, are used, almost exclusively nanoparticles are generated with diameters in the range of 80 to 150 nm, due to the limited solubility of oligomeric poly(acrylic acid) chains in the lipophilic continuous phase. These poly(acrylic acid) micro- and nanoparticles yielded excellent bioadhesive properties in an in-vitro assay and may, therefore, be suitable for the encapsulation of peptides and other hydrophilic drugs..

HA, heparosan or chondroitin chains would be the natural substitute for poly(acrylic-acid) based materials. These GAGs are negatively- charged polymers as is poly(acrylic-acid), but glycosaminoglycans are naturally occurring molecules in the vertebrate body and would not invoke an immune response like a poly(acrylic-acid) material.

The interest in realizing 'true' bioadhesion continues: instead of mucoadhesive polymers, plant or bacterial lectins, i.e., adhesion molecules which specifically bind to sugar moieties of the epithelial cell membrane are now widely being investigated as drug delivery adjuvants. These second-generation bioadhesives not only provide for cellular binding, but also for subsequent endo- and transcytosis. This makes the novel, specifically bioadhesive molecules particularly interesting for the controlled delivery of DNA/RNA molecules in the context of antisense or gene therapy.

For the efficient delivery of peptides, proteins, and other biopharmaceuticals by nonparenteral routes, in particular via the gastrointestinal, or Gl, tract, novel concepts are needed to overcome significant enzymatic and diffusional barriers. In this context, bioadhesion technologies offer some new perspectives. The original idea of oral bioadhesive drug delivery systems was to prolong and/or to intensify the contact between controlled-release dosage forms and the stomach or gut mucosa. However, the results obtained during the past decade using existing pharmaceutical polymers for such purposes were rather disappointing. The encountered difficulties were mainly related to the physiological peculiarities of Gl mucus. Nevertheless, research in this area has also shed new light on the potential of mucoadhesive polymers. First, one important class of mucoadhesive polymers, poly(acrylic acid), could be identified as a potent inhibitor of proteolytic enzymes. Second, there is increasing evidence that the interaction between various types of bio(muco)adhesive polymers and epithelial cells has direct influence on the permeability of mucosal epithelia. Rather than being just adhesives, mucoadhesive polymers may therefore be considered as a novel class of multifunctional macromolecules with a number of desirable properties for their use as biologically active drug delivery adjuvants.

In order to overcome the problems related to Gl mucus and to allow longer lasting fixation within the Gl lumen, bioadhesion probably may be better achieved using specific bioadhesive molecules. Ideally, these bind to surface structures of the epithelial cells themselves rather than to mucus by receptor-ligand-like interactions. Such compounds possibly can be found in the future among plant lectins, novel synthetic polymers, and bacterial or viral adhesion/invasion factors. Apart from the plain fixation of drug carriers within the Gl lumen, direct bioadhesive contact to the apical cell membrane possibly can be used to induce active transport processes by membrane-derived vesicles (endo- and transcytosis). The nonspecific interaction between epithelia and some mucoadhesive polymers induces a temporary loosening of the tight intercellular junctions, which is suitable for the rapid absorption of smaller peptide drugs along the paracellular pathway. In contrast, specific endo- and transcytosis may ultimately allow the selectively enhanced transport of very large bioactive molecules (polypeptides, polysaccharides, or polynucleotides) or drug carriers across tight clusters of polarized epi- or endothelial cells, whereas the formidable barrier function of such tissues against all other solutes remains intact.

Bioadhesive systems are presently playing a major role in the medical and biological fields because of their ability to maintain a dosage form at a precise body-site for a prolonged period of time over which the active principle is progressively released. Additional uses for bioadhesives include: bioadhesives/mucoadhesives in drug delivery to the gastrointestinal tract; nanoparticles as a gastroadhesive drug delivery system; mucoadhesive buccal patches for peptide delivery; bioadhesive dosage forms for buccal/gingival administration; semisolid dosage forms as buccal bioadhesives; bioadhesive dosage forms for nasal administration; ocular bioadhesive delivery systems; nanoparticles as bioadhesive ocular drug delivery systems; and bioadhesive dosage forms for vaginal and intrauterine applications.

The bioadhesive may also contain liposomes. Liposomes are unilamellar or multilamellar lipid vesicles which entrap a significant fraction of aqueous solution. The vesicular microreservoirs of liposomes can contain a variety of water-soluble materials, which are thus suspended within the emulsion. The preparation of liposomes and the variety of uses of liposomes in biological systems has been disclosed in U.S. Patent Nos. 4,708,861; 4,224,179; and 4,235,871. Liposomes are generally formed by mixing long chain carboxylic acids, amines, and cholesterol, as well as phospholipids, in aqueous buffers. The organic components spontaneously form multilamellar bilayer structures called liposomes. Depending on their composition and storage conditions, liposomes exhibit varying stabilities. Liposomes serve as models of cell membranes and also are used as drug delivery systems.

Most attempts to use liposomes as drug delivery vehicles have envisioned liposomes as entities which circulate in blood, to be taken up by certain cells or tissues in which their degradation would slowly release their internal aqueous drug-containing contents. In an effort to aid in their up-take by a given target tissue, some liposomes have been Atailored@ by binding specific antibodies or antigens to the outer surface. Liposomes have also been devised as controlled release systems for the delivery of their contents in vivo. Compositions in which liposomes containing biologically active agents are maintained and immobilized in polymer matrices, such as methylcellulose, collagen and agarose, for sustained release of the liposome contents, are described in U.S. Patent No. 4,708,861 to Popescu et al.

The treating agents useful herein are selected generally from the classes of medicinal agents and cosmetic agents. Substantially any agent of these two classes of materials that is a solid at ambient temperatures may be used. Treating agents that are liquid at ambient temperatures, e.g., nitroglycerine, can be used, but are not preferred because of the difficulties presented in their formulation. The treating agent may be used singly or as a mixture of two or more such agents.

One or more adjuvants may also be included with a treating agent, and when so used, an adjuvant is included in the meaning of the phrase treating agent or medicament. Exemplary of useful adjuvants are chelating agents such as EDTA that bind calcium ions and assist in passage of medicinal agents through the mucosa and into the blood stream. Another illustrative group of adjuvants are the quaternary nitrogen-containing compounds such as benzalkonium chloride that also assist medicinal agents in passing through the mucosa and into the blood stream.

The treating agent is in an amount that is sufficient to prevent, cure and/or treat a condition for a desired period of time for which the composition is to be administered, and such an amount is referred herein as an effective amount. As is well known, particularly in the medicinal arts, effective amounts of medicinal agents vary with the particular agent involved, the condition being treated and the rate at which the composition containing the medicinal agent is eliminated from the body, as well as varying with the animal in which it is being used, and the body weight of that animal. Consequently, effective amounts of treating agents may not be defined for each agent. Thus, an effective amount is that amount which in a composition provides a sufficient amount of the treating agent to provide the requisite activity of treating agent in or on the body of the treated animal for the desired period of time, and is typically less than that amount usually used.

Inasmuch as amounts of particular treating agents in the blood stream that are suitable for treating particular conditions are generally known, as are suitable amounts of treating agents used in cosmetics, it is a relatively easy laboratory task to formulate a series of controlled release compositions containing a range of such treating agent for a particular composition.

The second principle ingredient is a bioadhesive comprising an amount of hyaluronic acid (HA) from pmHAS or chondroitin from PmCS or heparin from pmHS1 or PmHS2. Such a glycosaminoglycan bioadhesive made from a HA or chondroitin or heparin chain directly polymerized onto a molecule with the desired pharmacological property or a HA or chondroitin or heparin chain polymerized onto a matrix or liposome which in turn contains or binds the medicament.

Woodfield et al. (2002) describe that articular cartilage lesions resulting from trauma or degenerative diseases are commonly encountered clinical problems. It is well- established that adult articular cartilage has limited regenerative capacity, and, although numerous treatment protocols are currently employed clinically, few approaches exist that are capable of consistently restoring long-term function to damaged articular cartilage. Tissue engineering strategies that focus on the use of three-dimensional scaffolds for repairing articular cartilage lesions offer many advantages over current treatment strategies. Appropriate design of biodegradable scaffold conduits (either preformed or injectable) allow for the delivery of reparative cells bioactive factors, or gene factors to the defect site in an organized manner. This review seeks to highlight pertinent design considerations and limitations related to the development, material selection, and processing of scaffolds for articular cartilage tissue engineering, evidenced over the last decade. In particular, considerations for novel repair strategies that use scaffolds in combination with controlled release of bioactive factors or gene therapy.

The various substantially monodisperse glycosaminoglycan polymers produced by the methods of the present invention, especially the hybrid or chimeric polymers, are promising materials for incorporation, either directly or indirectly, into a scaffold for cell growth and implantation. In addition, the polymers may be attached to surfaces or devices via acceptor moiety or a direct chain interaction.

Bello et al. (2001) describe that tissue-engineered skin is a significant advance in the field of wound healing and was developed due to limitations associated with the use of autografts. These limitations include the creation of a donor site which is at risk of developing pain, scarring, infection and/or slow healing. A number of products are commercially available and many others are in development. Cultured epidermal autografts can provide permanent coverage of large area from a skin biopsy. However, 3 weeks are needed for graft cultivation. Cultured epidermal allografts are available immediately and no biopsy is necessary. They can be cryopreserved and banked, but are not currently commercially available. A nonliving allogeneic acellular dermal matrix with intact basement membrane complex (Alloderm) is immunologically inert. It prepares the wound bed for grafting allowing improved cultured allograft 'take' and provides an intact basement membrane. A nonliving extracellular matrix of collagen and chondroitin-6-sulfate with silicone backing (Integra) serves to generate neodermis. A collagen and glycosaminoglycan dermal matrix inoculated with autologous fibroblasts and keratinocytes has been investigated but is not commercially available. It requires 3 to 4 weeks for cultivation. Dermagraft consists of living allogeneic dermal fibroblasts grown on degradable scaffold. It has good resistance to tearing. An extracellular matrix generated by allogeneic human dermal fibroblasts (TransCyte) serves as a matrix for neodermis generation. Apligraf is a living allogeneic bilayered construct containing keratinocytes, fibroblasts and bovine type I collagen. It can be used on an outpatient basis and avoids the need for a donor site wound. Another living skin equivalent, composite cultured skin (OrCel), consists of allogeneic fibroblasts and keratinocytes seeded on opposite sides of bilayered matrix of bovine collagen. There are limited clinical data available for this product, but large clinical trials are ongoing. Limited data are also available for 2 types of dressing material derived from pigs: porcine small intestinal submucosa acellular collagen matrix (Oasis) and an acellular xenogeneic collagen matrix (E-Z-Derm). Both products have a long shelf life. Other novel skin substitutes are being investigated. The potential risks and benefits of using tissue-engineered skin need to be further evaluated in clinical trials but it is obvious that they offer a new option for the treatment of wounds.

The various substantially monodisperse glycosaminoglycan polymers produced by the methods of the present invention, especially the hybrid or chimeric polymers, are promising components for tissue engineered organs including skin.

Vlodavsky et al. (1996) disclose that heparan sulfate proteoglycans (HSPGs) are ubiquitous macromolecules associated with the cell surface and extracellular matrix (ECM) of a wide range of cells of vertebrate and invertebrate tissues. The basic HSPG structure consists of a protein core to which several linear heparan sulfate (HS) chains are covalently attached. The polysaccharide chains are typically composed of repeating hexuronic and D-glucosamine disaccharide units that are substituted to a varying extent with N- and O-linked sulfate moieties and N-linked acetyl groups. Beside serving as a scaffold for the attachment of various ECM components (e.g., collagen, laminin, fibronectin), the binding of HS to certain proteins has been suggested to induce a conformational change which may lead to the exposure of novel reactive determinants or conversely stabilize an inert protein configuration. Of particular significance is the interaction of HS with fibroblast growth factors (FGFs), mediating their sequestration, stabilization and high affinity receptor binding and signaling. Cellular responses to FGFs may hence be modulated by metabolic inhibitors of HS synthesis and sulfation, HS-degrading enzymes, and synthetic mimetics of heparin/HS. HS is involved in basic FGF (bFGF) receptor binding and mitogenic activity and its modulation by species of heparin, HS, and synthetic polyanionic 'heparin-mimicking' compounds. The results are discussed in relation to the current thoughts on the dual involvement of low and high affinity receptor sites in the growth promoting and angiogenic activities of bFGF and other heparin-binding growth factors.

The mimetics based on the various substantially monodisperse glycosaminoglycan polymers produced by the methods of the present invention, including the hybrid or chimeric polymers, are promising due to their inherent abilities to interact, trigger, or bind a variety of molecules including cytokines, receptors, and growth factors. These GAG molecules should thus serve as modulators of cell behavior and/or growth via numerous natural pathways in mammals and humans.

livanainen et al. (2003) disclose that dynamic interactions between endothelial cells and components of their surrounding extracellular matrix are necessary for the invasion, migration, and survival of endothelial cells during angiogenesis. These interactions are mediated by matrix receptors that initiate intracellular signaling cascades in response to binding to specific extracellular matrix molecules. The interactions between endothelial cells and their environment are also modulated by enzymes that degrade different matrix components and thus enable endothelial invasion. Recent reports on gene targeting in mice have confirmed the role of two classes of matrix receptors, integrins and cell surface heparan sulfate proteoglycans, and a group of matrix degrading proteolytic enzymes, matrix metalloproteinases, in angiogenesis. The significance of endothelial cell-matrix interactions is further supported by several ongoing clinical trials that analyze the effects of drugs blocking this interaction on angiogenesis-dependent growth of human tumors.

The mimetics based on various substantially monodisperse glycosaminoglycan polymers produced by the methods of the present invention, including the hybrid or chimeric polymers, are promising due to their inherent abilities to intearct, trigger, or bind a variety of molecules including cytokines, receptors, and growth factors. These molecules should thus serve as modulators of cell behavior and/or growth.

Song et al. (2002) teach that glypicans are a family of heparan sulfate proteoglycans that are bound to the cell surface by a glycosyl-phosphatidylinositol anchor. Six members of this family have been identified in mammals. In general, glypicans are highly expressed during development, and their expression pattern suggests that they are involved in morphogenesis. One member of this family, glypican-3, is mutated in the Simpson-Golabi-Behmel syndrome. This syndrome is characterized by overgrowth and various developmental abnormalities that indicate that glypican-3 inhibits proliferation and cell survival in the embryo. It has consequently been proposed that glypicans can regulate the activity of several growth factors that play a critical role in morphogenesis. Defined heparosan derivatives could be used to modulate such activities.

The various substantially monodisperse glycosaminoglycan polymers produced by the methods of the present invention, especially the hybrid or chimeric polymers, are promising materials for incorporation, either directly or indirectly, onto cell surfaces. The polymers may be attached to cell surfaces or devices via acceptor moiety (for example, but not by way of limitation, a lipid conjugate).

The substantially monodisperse glycosaminoglycan polymers of the present invention may also be a starting material for sulfated heparin-based anticoagulants, antivirals, proliferation modulators, etc. Size defined molecules as well as analogs should allow a multitude of therapeutics to be created with potential for enhanced activity and better control.

### MATERIALS AND METHODS

Membrane preparations containing recombinant pmHAS (GenBank AF036004) (SEQ. ID NOS:1 and 2) were isolated from *E. coli* SURE(pPmHAS). Membrane preparations containing native pmHAS were obtained from the *P. multocida* strain P-1059 (ATCC #15742). pmHAS was assayed in 50 mM Tris, pH 7.2, 20 mM MnC1₂, and UDP-sugars (UDP-[¹⁴C]GlcUA, 0.3 µCi/mmol, NEN and UDP-GlcNAc) at 30DC. The reaction products were analyzed by various chromatographic methods as described below. Membrane preparations containing other recombinant HAS enzymes, Group A *streptococcal* HasA or *Xenopus* DG42 produced in the yeast *Saccharomyces cerevisiae*, were prepared.

Uronic acid was quantitated by the carbazole method. Even-numbered HA oligosaccharides [(GlcNAc-GlcUA)ₙ] were generated by degradation of HA (from Group A *Streptococcus*) with either bovine testicular hyaluronidase Type V (n=2-5) or *Streptomyces hyaluroniticus* HA lyase (n=2 or 3) in 30 mM sodium acetate, pH 5.2, at 30D° overnight. The latter enzyme employs an elimination mechanism to cleave the chain resulting in an unsaturated GlcUA residue at the nonreducing terminus of each fragment. For further purification and desalting, some preparations were subjected to gel filtration with P-2 resin (BioRad) in 0.2 M ammonium formate and lyophilization. Odd-numbered HA oligosaccharides [GlcNAc(GlcUA-GlcNAc)ₙ] ending in a GlcNAc residue were prepared by mercuric acetate-treatment of partial HA digests generated by HA lyase (n=2-7). The masses of the HA oligosaccharides were verified by matrix-assisted laser desorption ionization time-of-flight mass spectrometry. Sugars in water were mixed with an equal volume of 5 mg/ml 6-azo-2-thiothymine in 50% acetonitrile/0.1% trifluoroacetic acid, and rapidly air-dried on the target plate. The negative ions produced by pulsed nitrogen laser irradiation were analyzed in linear mode (20 kV acceleration; Perceptive Voyager).

Other oligosaccharides that are structurally similar to HA were also tested in HAS assays. The structure of heparosan pentamer derived from the *E. coli* K5 capsular polysaccharide is α4GlcUA- β4GlcNAc; this carbohydrate has the same composition as HA but the glycosidic linkages between the monosaccharides are different. The chitin-derived oligosaccharides, chitotetraose and chitopentaose, are β4GlcNAc polymers made of 4 or 5 monosaccharides, respectively.

Various oligosaccharides were radiolabeled by reduction with 4 to 6 equivalents of sodium borotritide (20 mM, NEN; 0.2 µCi/mmol) in 15 mM NaOH at 30DC for 2 hrs. ³H-oligosaccharides were desalted on a P-2 column in 0.2 M ammonium formate to remove unincorporated tritium and lyophilized. Some labeled oligosaccharides were further purified preparatively by paper chromatography with Whatman 1 developed in pyridine/ethyl acetate/acetic acid/H₂O (5:5:1:3) before use as an acceptor.

Paper chromatography with Whatman 3M developed in ethanol /1M ammonium acetate, pH 5.5 (65:35) was used to separate high molecular weight HA product (which remains at the origin) from UDP-sugars and small acceptor oligosaccharides. In the conventional HAS assay, radioactive UDP-sugars are polymerized into HA. To obtain the size distribution of the HA polymerization products, some samples were also separated by gel filtration chromatography with Sephacryl S-200 (Pharmacia) columns in 0.2 M NaCl, 5 mM Tris, pH 8. Columns were calibrated with dextran standards. The identity of the polymer products was assessed by sensitivity to specific HA lyase and the requirement for the simultaneous presence of both UDP-sugar precursors during the reaction. Thin layer chromatography [TLC] on high performance silica plates with application zones (Whatman) utilizing butanol/acetic acid/water (1.5:1:1 or 1.25:1:1) development solvent separated ³H-labeled oligosaccharides in reaction mixes. Radioactive molecules were visualized after impregnation with EnHance spray (NEN) and fluorography at ~80°.

Membrane preparations containing recombinant full length pmHAS, pmHAS⁴³⁷⁻⁹⁷², pmHAS ⁴³⁷⁻⁷⁵⁶, pmHAS ¹⁻⁷⁵⁶, pmHAS ¹⁻⁵⁶⁷ and pmHAS ¹⁵²⁻⁷⁵⁶ were isolated from *E. coli* as described. For soluble truncated pmHAS proteins, pmHAS¹⁻⁷⁰³, pmHAS¹⁻⁶⁵⁰, and pmHAS¹⁻⁷⁰³-derived mutants, cells were extracted with B-Per^{™} II Bacterial Protein Extraction Reagent (Pierce) according to the manufacturer's instruction except that the procedure was performed at 7D° in the presence of protease inhibitors. Membrane preparations of *P*. *multocida* P-1059 (ATCC 15742) were made as described.

The size of GAG polymers was analyzed by chromatography on a Phenomenex PolySep-GFC-P 3000, P 4000 or P5000 column (300x7.8 mm) eluted with 0.2 M sodium nitrate at 0.6 ml/min on a Waters 600E system. The column was standardized with various size fluorescent dextrans (580, 50, and 12 kDa). Radioactive components were detected with a LB508 Radioflow Detector (EG & G Berthold) and Zinsser cocktail (1.8 ml/min). In comparison to the full HAS assay using paper chromatography described above, these 3 minute reactions contained twice the UDP-sugar concentrations, 0.06 µCi UDP-[¹⁴C]GlcUA, and 0.25 µg even-numbered GAG oligosaccharide. Also, addition of ethylenediamine tetracetic acid (final conc. 22 mM) and boiling (2 min) was employed to terminate the reactions instead of addition of SDS.

A lambda library of Sau3A partially digested Type F *P. multocida* P-4679 DNA (~4-9 kb average length insert) was made using the BamHI-cleaved Zap Express vector system (Stratagene). The plaque lifts were screened by hybridization (5× SSC, 50°C; 16 hrs) with the digoxigenin-labeled probe using the manufacturer guidelines for colorimetric development. *E. coli* XLI-Blue MRF was co-infected with the purified, individual positive lambda clones and ExAssist helper phage to yield phagemids. The resulting phagemids were transfected into *E. coli* XLOLR cells to recover the plasmids. Sequence analysis of the plasmids revealed a novel open reading frame, which was called pmCS, with high homology to pmHAS. This same method was utilized to identify a novel open reading frame, which was called pmHS1 (DNA sequence facilities at Oklahoma State University and University of Oklahoma HSC). The ORF was amplified and sequenced from several highly encapsulated isolates (see hereinbelow); very similar sequences wereobtained.

In previous studies with pmHAS, it was found that a functional, soluble enzyme would be created if a portion of the carboxyl terminus was truncated by molecular genetic means. Therefore, a portion of the pmCS ORF (residues 1-704) in the insert of one of the excised lambda clones, pPmF4A, was amplified by 20 cycles of PCR with Taq polymerase. The sense primer corresponded to the sequence at the deduced amino terminus of the ORF and the antisense primer encoded the new carboxyl terminus followed by an artificial stop codon. The resulting PCR product was purified and concentrated using GeneClean. This insert was cloned using the pETBlue-1 Acceptor system (Novagen) according to the manufacturer's instructions. The Taq-generated single A overhang is used to facilitate the cloning of the open reading frame downstream of the T7 promoter and the ribosome binding site of the vector. The ligated products were transformed into *E. coli* NovaBlue and plated on LB carbenicillin (50 µg/ml) under conditions for blue/white screening. White or light blue colonies were analyzed by restriction digestion. A clone containing a plasmid with the desired truncated ORF, pPm-CS¹⁻⁷⁰⁴, was transformed into *E. coli* Tuner, the T7 RNA polymerase-containing expression host, and maintained on LB media with carbenicillin and chloramphenicol (34 µg/ml) at 30°DC. Log phase cultures were induced with β-isopropylthiogalactoside (0.2 mM final) for 5 hrs. The cells were harvested by centrifugation, frozen, and extracted for 20 min with a mild detergent (bPer II reagent, Pierce) at 7D° in the presence of a broad-range protease inhibitor cocktail. The cells were removed by centrifugation and the soluble extract was used as the source of CS enzyme for *in vitro* assays.

Truncated polypeptides were generated by amplifying the pPm7A insert by 13 cycles of PCR with Taq polymerase (Fisher) and synthetic oligonucleotide primers corresponding to various portions of the pmHAS open reading frame. Except for the construction of pmHAS¹⁻⁶⁸⁶ and pmHAS¹⁻⁶⁶⁸, the primers contained EcoRI and Pstl restriction sites to facilitate cloning into the expression plasmid pKK223-3 (tac promoter; Pharmacia). The resulting recombinant constructs were transformed into *E. coli* TOP 10F cells (Invitrogen) and maintained on Luria-Bertani media with ampicillin selection. The DNA encoding pmHAS¹⁻⁶⁸⁶ and pmHAS¹⁻⁶⁶⁸ were cloned into pETBlue-1 plasmid and expressed in Tuner (DE3)pLacl cells (Novagen) according to manufacturing instructions; these cells were maintained on Luria-Bertani media with carbenicillin and chloramphenicol selection.

Point mutations were made using the QuickChange site-directed mutagenesis method (Stratagene) with the plasmid pKK223/pmHAS¹⁻⁷⁰³ DNA as template. The sequences of the mutant open reading frames were verified by automated DNA sequencing (Oklahoma State University Recombinant DNA/Protein Resource Facility).

Recombinant *E. coli* were grown in Luria-Bertani media with drug selection until OD₆₀₀ was 0.3-0.6 when cells were induced with 0.5 mM isopropyl-1-thio-(β-D-galactoside. Cells were harvested 5 hours after induction. For soluble truncated proteins and pmHAS¹⁻⁷⁰³-derived mutants expressed in *E. coli* TOP10F' cell, cells were extracted with B-Per^{™} II Bacterial Protein Extraction Reagent (an octylthioglucoside-based solution; Pierce) according to the manufacturer's instruction except that the procedure was performed at 7° in the presence of protease inhibitors. For proteins expressed in Tuner(DE3)pLacl, lysis by ultrasonication followed by subcellular fractionation was performed and the supernatant after centrifugation at 100,000 × g was used.

Five assays were designed to detect either (a) the polymerization of long HA chains, (b) the addition of a single GlcNAc to a GlcUA-terminated HA oligosaccharide acceptor, (c) the addition of a single GlcUA to a GlcNAc-terminated HA oligosaccharide acceptor, (d) the polymerization of long chondroitin chains, or (e) the addition of a single GalNAc to a GlcUA-terminated HA oligosaccharide acceptor. The first three assays were described hereinabove. For the chondroitin synthase assay, the same conditions as the HA synthase assay were used except that the other hexosamine precursor, UDP-GalNAc, was employed and there is no ammonium sulfate or ethylene glycol in the assay system. GalNAc-transferase activity was assayed under the same conditions as the GlcNAc-transferase assay except that 0.3 mM UDP-[³H]GalNAc (0.2 µCi; NEN) was used instead of UDP-[³H]GlcNAc. Reactions were terminated by the addition of SDS to 2% (w/v). The reaction products were separated from substrates by descending paper (Whatman 3M) chromatography with ethanol/1 M ammonium acetate, pH 5.5, development solvent (65:35 for the HAS, chondroitin synthase, and GlcUA-transferase assays; 75:25 for GlcNAc-transferase and GalNAc-transferase assay). All assays were adjusted to be linear with regard to incubation time and to protein concentration. Radiolabeled products were quantitated by liquid scintillation counting (Biosafe II, Research Products International).

The pmHAS polypeptides in membranes and extracts were analyzed using standard 8% polyacrylamide SDS gels and Western blotting utilizing a monospecific antibody directed against a synthetic peptide corresponding to residues 526 to 543 of pmHAS (acetyl-LDSDDYLEPDAVELCLKE-amide) as described hereinabove.

The DNA encoding different segments of pmHAS-D or pmCS were generated by amplifying the pPm7A insert or pPmF4A insert, respectively, by 15 cycles of PCR with Taq polymerase (Fisher) and synthetic oligonucleotide primers corresponding to various portions of the pmHAS-D or pmCS open reading frame. Each internal primer contained overlaps with the other segment to allow joining of the two desired segments. The forward and reverse primers for pmHAS residue 1-427 (A segment) were P1 = 5'-ATGAACACATTATCACAAGCAATAAAAGC-3' (SEQ ID NO:49) and P2 = 5'-GCGAATCTTCTATTGGTAAAAGYTTTC-3' (SEQ ID NO:50) (Y=C/T), respectively. The forward and reverse primers for pmCS residue 421-704 (C segment) were P3 = 5'-CTTTTACCAATAGAAGATTCGCATAT-3' (SEQ ID NO:51) and P4 = 5'-GAAGACGTCTTAGGCATCTTTATTCTGAATGAG-3' (SEQ ID NO:52), respectively. The forward and reverse primers for pmCS residue 1-420 (D segment) were P1 and P2. The forward and reverse primers for pmHAS residue 428-703 (B segment) were P3 and P5 = 5'-GGGAATTCTGCAGTTAAATATCTTTTAAGATATCAATCTCTTC-3' (SEQ ID NO:53), respectively. The chimeric or hybrid synthases were created by 15 cycles of PCR with the gel-purified (GeneClean; Bio101) segments and outer primers (pm-AC used A and C segments with primer P1 and P4; pm-BD used B and D segments with primer P1 and P5). The purified PCR products were cloned into pETBlue-1 vector and the chimeric or hybrid proteins were expressed in Tuner(DE3)pLacl cells (Novagen). The complete open reading frames of multiple clones of both constructs were sequenced. A pmAC construct that was perfect, was found but both of the two pmBD constructs that we had sequenced completely had secondary undesired mutations (#1, E695 and I697F; #2, I302V). However, these mutations were in different locations and the enzyme transferase activities were identical. Several other pmBD clones have the identical phenotype but their complete sequences were not determined.

*Analysis of Genomic DNA and Isolation of Capsule Biosynthesis Locus DNA -* Preliminary data from Southern blot analysis using pmHAS-based hybridization probes ⁽¹²⁾ suggested that the Type A synthase and the putative Type D synthase were not very similar at the DNA level. However, PCR suggested that the UDP-glucose dehydrogenase genes, which encode an enzyme that produces the UDP-GlcUA precursor required for both HA and heparin biosynthesis, were very homologous. In most encapsulated bacteria, the precursor-forming enzymes and the transferases are located in the same operon. To make a hybridization probe predicted to detect the capsule locus, Type D chromosomal DNA served as a template in PCR reactions utilizing degenerate oligonucleotide primers (sense: GARTTYBTIMRIGARGGIAARGCIYTITAYGAY (SEQ ID NO:54); antisense: RCARTAICCICCRTAICCRAAISWXGGRTTRTTRTARTG (SEQ ID NO:55), where I = inosine; R = A or G; S = C or G; W = A or T; Y = C or T) corresponding to a conserved central region in many known UDP-glucose dehydrogenase genes. The ~0.3-kb amplicon was generated using Taq DNA polymerase (Fisher), gel-purified, and labeled with digoxigenin (High Prime system, BoehringerMannheim).

*Expression of Recombinant P. multocida Heparosan Synthase.* The pmHS1 ORF (617 amino acids) was amplified from the various Type D genomic DNA template by 18 cycles of PCR with *Taq* polymerase. For constructing the full-length enzyme, the sense primer (ATGAGCTTATTTAAACGTGCTACTGAGC - SEQ ID NO:54) corresponded to the sequence at the deduced amino terminus of the ORF and the antisense primer (TTTACTCGTTATAAAAAGATAAACACGGAATAAG - SEQ ID NO:55) encoded the carboxyl terminus including the stop codon. In addition, a truncated version of pmHS1 was produced by PCR with the same sense primer but a different antisense primer (TATATTTACAGCAGTATCATTTTCTAAAGG -- SEQ ID NO:56) to yield a predicted 501-residue protein, DcbF (SEQ ID NO:57) (GenBank Accession Number AAK17905); this variant corresponds to residues 1- 497 of pmHS1 followed by the residues TFRK. The current optimal constructs are fusions to the maltose-binding protein (pMAL-c vector, New England Biolabs; Sismey-Ragatz et al., 2007).

Construction and Purification of Recombinant Maltose Binding Fusion Construct Proteins - PmHS1 and PmHS2 were both expressed as a carboxyl terminal fusion to maltose binding protein (MBP) using the pMAL-c2X vector (New England BioLabs, Beverly, MA). Polymerase chain reaction was employed to subclone the open reading frames from our previous pETBlue-1 constructs (DeAngelis & White, 2002 & 2004). For PmHS1, new unique flanking restriction sites (*Bam*HI and *Hind*III) were added with the primers used for amplification (15 cycles: 94°C, 30 sec; 52°C, 30 sec; 72°C, 2 min) with *Taq* DNA polymerase. For PmHS2, restriction sites (*Bam*HI and *Pst*I) were added with the primers used for amplification (21 cycles: 94°C, 30 sec; 52°C, 30 sec; 72°C, 3.5 min) with *Pfu* DNA polymerase (Stratagene, La Jolla, CA). The amplicons were gel purified, restriction digested with both appropriate enzymes (Promega, Madison, WI), and ligated to similarly double-cut pMAL-c2X plasmid. *E. coli* ONE SHOT^{®} Top 10F' (Invitrogen, Carlsbad, CA) was used for the initial transformation on LB ampicillin (100 µg/ml) plates and grown at 30°C. To facilitate extracting the enzymes, the expression host *E. coli* XJa (Zymo Research, Orange, CA), which encodes a phage lysin enzyme, was employed allowing for simple freeze/thaw lysis. Cultures were grown in Superior Broth (AthenaES, Baltimore, MD) at 30°C with ampicillin (100 µg/ml), and L-arabinose (3.25 mM; to induce the lysin enzyme). At mid-log phase, isopropyl β-D-1-thiogalactopyranoside (IPTG) (0.2 mM final) was added to induce fusion protein production. One hour after induction, the cultures were supplemented with fructose (12.8 mM final) and grown for ~ 5-12 hours before harvesting by centrifugation at 4°C. The bacteria were resuspended in 20 mM Tris, pH 7.2, and protease inhibitors (p-[4-2-aminoethyl] benzenesulfonyl fluoride hydrochloride, pepstatin, benzamidine, N-[N-(L-3-trans-carboxyoxirane-2-carbonyl)-L-leucyl]-agmatine, leupeptin) on ice, frozen and thawed twice allowing lysin to degrade the cell walls. The lysates was clarified by centrifugation. The synthases were affinity purified via the MBP unit using amylose resin (New England BioLabs). After washing extensively with column buffer (20 mM Tris, pH 7.2, 200 mM NaCl, 1 mM EDTA), the protein was eluted in column buffer containing 10 mM maltose.

Polymerization Assays - Radiolabeled sugar incorporation assays using UDP-[³H]GlcUA or UDP-[³H]GlcNAc (Perkin Elmer NEN, Boston, MA) were employed to monitor substrate usage and pH dependence. Polymerization reactions (40 µl) typically contained 50 mM Tris, pH 7.2, 1 mM MnCl₂, 0.5-1.0 mM UDP-GlcNAc, 0.5-1 mM UDP-GlcUA, 0.1 mCi UDP-[³H]sugar and ~4-5 mg of enzyme (unless noted). The reactions were typically incubated at 30°C for 15 to 30 min then stopped with SDS (2% final). Descending paper chromatography was used to separate unincorporated UDP sugars from the polymer product (i.e. sugars longer than ~ 14 sugars). Three separate experiments were completed for each data set and each assay was verified to be in the linear range with respect to time and enzyme concentration. Less than 5% of the UDP-sugar was consumed. For acceptor usage tests, a "no acceptor" control was performed for all assays in order to determine the *de novo* initiation heparosan synthesis for each enzyme; this value was subtracted from the value attained in parallel assays with an acceptor. For initial tests querying the ability of a synthase to mis-incorporate a non-authentic UDP-sugar donor, the test compound was used at ~ 0.5-1.5 mM in the presence of carrier-free UDP-[³H]GlcUA or UDP-[³H]GlcNAc (0.1 µCi) without acceptor for 12-48 hours. Subsequent assays to obtain relative kinetic rates employed radiolabeled authentic UDP-sugar diluted to 0.6 mM for 90 or 180 min.

Monodisperse Heparosan Synthesis ― A mixture of heparosan tetrasaccharide and hexasaccharide (~1:4 by mass, respectively) acceptors was used to prime synthesis of narrow size distribution heparosan polymers. The length of the chain was controlled by altering the stoichiometry of the UDP-sugar to acceptor as for the non-homologous PmHAS, the *Pasteurella* HA synthase. These reactions typically contained 5-25 mM UDP-sugars in the same reaction buffer used for polymerization assays and were incubated overnight at 30°C.

Single Sugar Addition Assays - Tests were performed under the same buffer conditions as polymerization assays, but contained only a single UDP-sugar substrate (~1-3 mM final) and an appropriately terminated acceptor (e.g., heparosan oligosaccharide, A-F-A or A-F-A-N). The incorporation of sugar from the donor substrate was detected by the increase in mass to the appropriate predicted molecular weight product by MALDI-ToF MS.

Sugar Analysis Techniques - The sizes of the heparosan polymers were analyzed with agarose gels (1.2-3%, 1X TAE buffer, 30 V for 5-6 hours) and Stains-All (1-ethyl-2-[3-(1-ethylnaphtho[1,2-d]thiazolin-2-ylidene)-2-methylpropenyl]naphtho-[1,2-d]thiazolium bromide) detection or with polyacrylamide gels (15%, 29:1 monomer:bis, 1X TBE, 250 V for 30 min) and Alcian Blue staining. The size distribution of the polymers was determined by high performance size exclusion chromatography-multi angle laser light scattering.

The HA4 molecule was converted into a fluorescent derivative in two steps. First, reductive amination of HA4 with cyanoborohydride and excess diaminobutane in 0.1 M borate buffer, pH 8.5, was used to make an amino-HA4 derivative that was purified by gel filtration on P2 resin. Second, the amino- HA4 was derivatized with the N-hydroxysuccinimide ester of Oregon green 488 (Molecular Probes) and the Fluor-HA4 was purified by preparative normal-phase thin layer chromatography (silica developed with 2:1:1 n-butanol/acetic acid/water).

Heparosan oligosaccharide acceptor preparation. A ~55 kDa heparosan polysaccharide acceptor was used as a positive control and a normalization factor for many experiments. Heparosan oligosaccharides (GlcUA-GlcNAc)*ₙ*-(GlcUA-anhydromannitol), *n* = 1, 2 or 3) were prepared by partial deacetylation with base, nitrous acid hydrolysis, and reduction; these polymers contain intact non-reducing termini, but an anhydromannitol group at the reducing end. The fragments were purified by gel filtration on a P2 column (BioRad, Hercules, CA) in 0.2 M ammonium formate, followed by normal phase thin layer chromatography (TLC) on silica plates (Whatman, Clifton, NJ) with *n*-butanol/acetic acid/water (1:1:1). The bands were detected by staining of side lanes with napthoresorcinol. The size and purity of oligosaccharides were verified by matrix assisted laser desorption ionization time of flight mass spectrometry (MALDI-ToF MS). Fluorescein di-β-D-glucuronide (A-F-A; Molecular Probes, Eugene, OR), a commercially available synthetic acceptor that mimics a glycosaminoglycan trisaccharide that was identified in previous work with the *Pasteurella* HA synthase (Williams et al, 2006), was used as the starting material to prepare the A-F-A-N (GlcUA-F-GlcUA-GlcNAc) and the N-A-F-A-N (GlcNAc-GlcUA-F-GlcUA-GlcNAc) acceptors using UDP-GlcNAc with PmHS2. These longer acceptors were purified by TLC (silica plates and *n*-butanol/acetic acid/water, 2:1:1).

Catalyst preparation and in vitro synthesis. The catalyst, pmHAS1-703 or pmCS1-704, are soluble purified *E. coli*-derived recombinant protein. The enzyme in the octylthioglucoside extracts of the cell paste was purified by chromatography on Toyopearl Red AF resin (Tosoh) using salt elution (50 mM HEPES, pH 7.2, 1 M ethylene glycol with 0 to 1.5 M NaCl gradient in 1 hour). The fractions containing the synthase protein (~90% pure by SDS-PAGE/Coomassie-staining) were concentrated by ultrafiltration and exchanged into reaction buffer.

Analysis of *in vitro* synthesized Glycosaminoglycans (GAGs). GAGs were analyzed on agarose gels as described in Lee and Cowman. In brief, agarose gels (0.7- 1.2%) in 1x TAE buffer were run at 40V. Gels are stained with Stains-All dye (0.005% w/v in ethanol) overnight and destained with water. GAG was analyzed on acrylamide gels (15- 20%) as described in Ikegami-Kawai and Takahashi. To purify GAGs, the synthase was removed by choloroform extraction or thin layer chromatography, and GAGs were precipitated as described above or with three volumes of ethanol followed by redissolving in water. Alternatively, the unincorporated precursor sugars were removed by ultrafiltration with Microcon units (Millipore). The concentration was determined by carbazole assay (ref) and a glucuronic acid standard.

Gel filtration/multi-angle laser light scattering analysis was used to determine the absolute molecular weights. Polymers were separated on tandem Toso Biosep TSK-GEL columns (6000PWXL followed by 4000PWXL; each 7.8 mm'30 cm; Japan or equivalent) eluted in 50 mM sodium phosphate, 150 mM NaCl, pH 7 at 0.5 mL/min. The eluant flowed through an

**TABLE XIII.**

| **Mutation/Truncation** | **SEQ ID NO:** |
|---|---|
| pmHAS¹⁻⁶⁵⁰ | 10 |
| pmHAS¹⁻⁷⁰³ D477N | 11 |
| pmHAS¹⁻⁷⁰³ D196N | 12 |
| pmHAS⁴³⁷⁻⁹⁷² | 13 |
| pmHAS⁴³⁷⁻⁷⁵⁶ | 14 |
| pmHAS¹⁵²⁻⁷⁵⁶ | 15 |
| pmHAS¹⁻⁷⁰³ D196E | 16 |
| pmHAS¹⁻⁷⁰³ D196K | 17 |
| pmHAS¹⁻⁷⁰³ D477E | 18 |
| pmHAS¹⁻⁷⁰³ D477K | 19 |
| pmHAS¹⁻⁷⁵⁶ | 20 |
| pmHAS¹⁻⁵⁶⁷ | 21 |
| pmHAS¹⁻⁷⁰⁴ | 22 |
| pmHAS⁴⁶⁻⁷⁰³ | 23 |
| pmHAS⁷²⁻⁷⁰³ | 24 |
| pmHAS⁹⁶⁻⁷⁰³ | 25 |
| pmHAS¹¹⁸⁻⁷⁰³ | 26 |
| pmHAS¹⁻⁶⁶⁸ | 27 |
| pmHAS¹⁻⁶⁸⁶ | 28 |
| pmHAS¹⁻⁷⁰³ D247E | 29 |
| pmHAS¹⁻⁷⁰³ D247N | 30 |
| pmHAS¹⁻⁷⁰³ D247K | 31 |
| pmHAS¹⁻⁷⁰³ D249E | 32 |
| pmHAS¹⁻⁷⁰³ D249N | 33 |
| pmHAS¹⁻⁷⁰³ D249K | 34 |
| pmHAS¹⁻⁷⁰³ D527N | 35 |
| pmHAS¹⁻⁷⁰³ D527E | 36 |
| pmHAS¹⁻⁷⁰³ D527K | 37 |
| pmHAS¹⁻⁷⁰³ D529E | 38 |
| pmHAS¹⁻⁷⁰³ D529N | 39 |
| pmHAS¹⁻⁷⁰³ D529K | 40 |
| pmHAS¹⁻⁷⁰³ E369D | 41 |
| pmHAS¹⁻⁷⁰³ E369Q | 42 |
| pmHAS¹⁻⁷⁰³ E369H | 43 |
| pmHAS¹⁻⁷⁰³ D370E | 44 |
| pmHAS¹⁻⁷⁰³ D370N | 45 |
| pmHAS¹⁻⁷⁰³ D370K | 46 |

Optilab DSP interferometric refractometer and then a Dawn DSF laser photometer (632.8 nm; Wyatt Technology, Santa Barbara, CA) in the multi-angle mode. The manufacturer's software package was used to determine the absolute average molecular weight using a dn/dC coefficient of 0.153.

The pmHAS mutants and truncations have been described previously in parent application US Patent No. 7,223,751, issued to DeAngelis et al. on May 29, 2007. For ease of reference, Table XIII is provided; such Table lists each mutant/truncation and its corresponding SEQ ID NO.

### REFERENCES

Asplund, T., J. Brinck, M. Suzuki, M.J. Briskin, and P. Heldin. (1998) Biochim. Biophys. Acta. 1380, 377-388.
Ausubel, F.M., R. Brent, R.E. Kingston, D.D. Moore, J.G. Seidman, J.A. Smith, and K. Struhl. (1995) Short Protocols in Molecular Biology, 3rd Ed., John Wiley & Sons Inc., New York.
Bello, YM, Falabella, AF, and Eaglstein, WH. (2001) Am J Clin Dermatol, 2:305-313.
Bernfield, M., Gotte, M., Park, P.W., Reizes, O., Fitzgerald, M.L. Licecum, J. and Zako, M. (1999) Annu Rev Biochem 68, 729-777
Bertram, J., M. Stratz, and P. Durre. J. Bact. (1991); 173: 443-8.
Bitter, T. and H.M. Muir., (1962) Anal. Biochem. 4, 330-334.
Blundell, C.D., Almond, A., Mahoney, D.J., DeAngelis, P.L., Cambell, I.D. and Day, A.J., (2005) J. Biol Chem 280, 18189-18201
Bradford, M.M., (1976) Anal. Biochem. 72, 248-254.
Breton, C. and A. Imberty. (1999) Curr. Opin. Struc. Biol. 9, 563-571.
Busch, C., F. Hofmann, J. Selzer, S. Munro, S. Jeckel, and K. Aktories. (1998) J. Biol. Chem., 273, 19566-19572.
Busse, M. and M. Kusche-Gullberg, J. Biol Chem, 2003. 278(42): p. 41333-7
Campbell, R.E., S.C. Mosimann, I. van de Rijn, M.E. Tanner, and N.C.J. Strynadke. (2000) Biochemistry 39, 7012-7023.
Capila, I. and Linhardt, R.J. (2002) Angew Chem Int Ed Engl 41, 319-412
Carter, G.R. and E. Annau. (1953) Am. J. Vet. Res. 14, 475-478.
Charnock, S.J. and G.J. Davies. (1999) Biochemistry, 38, 6380-6385.
Chen, M., Bridges, A. and Liu, J. (2006) Biochemistry 45, 12358-12365
Chen, WY. and Abstangelo G. (1999) Wound Repair Regen, 7,79-89*.*
Chung, J.Y., I. Wilkie, J.D. Boyce, K.M. Townsend, A.J. Frost, M. Ghoddusi, and B. Adler. (2001) Infect. Immun., 69, 2487-2492.
Corpet, F. (1998) Nucleic Acids Res. 16, 10881-10890.
Crater, D.L., and I. van de Rijn. (1995) J. Biol. Chem. 270, 18452-18458. DeAngelis, P.L., Oatman, L.C. and Gay, D.F. (2003) J. Biol Chem 278, 35199-35203
DeAngelis, P.L., J. Papaconstantinou, and P.H. Weigel. J. Biol. Chem., 268, 14568-14571, 1993.
DeAngelis, P.L., J. Papaconstantinou, and P.H. Weigel. J.Biol.Chem., 268, 19181-19184, 1993.
DeAngelis, P.L. and P.H. Weigel. Biochemistry, 33, 9033-9039, 1994.
DeAngelis, P.L., W. Jing, M.V. Graves, D.E. Burbank, and J.L. Van Etten. Science, 278, 1800-1803, 1997.
DeAngelis, P.L. Cell. Mol. Life Sci., 56, 670-682, 1999.
DeAngelis, P.L. Glycobiology. 12(1):9R-16R. Review. 2002.
DeAngelis, P.L., and C.L. White. J.Biol.Chem. 277(9):7209-13, 2002.
DeAngelis, P.L. and White, C.L. (2004) J.Bacteriol 186, 8529-8532
DeAngelis, P.L. Anal. Biochem. 284(1):167-9, 2000.
DeAngelis, P.L. and A.J. Padgett-McCue. J.Biol.Chem. 275(31):24124-9, 2000.
DeAngelis, P.L. J.Biol.Chem. 274(37);26557-62, 1999.
DeAngelis P.L. Microb. Pathog. 24(4):203-9, 1998.
DeAngelis, P.L., W. Jing, R.R. Drake, and A.M. Achyuthan. J.Biol.Chem. 273(14):8454-8, 1998.
DeAngelis, P.L., W. Jing, M.V. Graves, D.E. Burbank, and J.L. Van Etten. Science. 278(5344):1800-3, 1997.
DeAngelis, P.L., and A.M. Achyuthan. J.Biol.Chem. 271(39):23657-60, 1996.
DeAngelis, P.L. Biochemistry. 35(30):9768-71,1996.
DeAngelis, P.L., Oatman, L.C. and Gay, D.F. (2003) J. Biol. Chem., 278.
DeAngelis, P.L. (2002) Anatomical Record , 206, 317-326
DeLuca, S. and J.E. Silbert. (1968) J. Biol. Chem. 243, 2725-2729.
Doughtery, B.A., and I. van de Rijn. (1994) J. Biol. Chem., 269, 169-175.
Drake, C.R., I.S. Roberts, B. Jann, K. Jann, and G.J. Boulnois (1990) FEMS Microbiol. Lett., 54, 227-230.
Duncan, G., C. McCormick, and F. Tufaro. (2001) J. Clin. Invest., 108, 511-516.
Duncan, M.B., Liu, M., Fox, C. and Liu, J., (2006) Biochem Biophys Res Commun 339(4), 1232-1237
Esko, J.D. and U. Lindahl. (2001) J. Clin. Invest. 108, 169-173.
Finke, A., D. Bronne, A.V. Nikolaev, B. Jann, and K. Jann. (1991) J. Bacteriol., 173, 4088-4094.
Gastinel, L.N., C. Cambillau, and Y. Bourne. (1999) EMBO J. 18, 3546-3557.
Gastinel, L.N., C. Bignon, A.K. Misra, O. Hindsgaul, J.H. Shaper, and D.H. Joziasse. (2001) EMBO J. 20, 638-649.
Gherezghiher, T., M.C. Koss, R.E. Nordquist, and C.P. Wilkinson. (1987) J. Chromatogr. 413, 9-15.
Gietz, R.D., R.H. Schiestl, A.R. Willems, and R.A. Woods. (1995) Yeast 11, 355-360.
Griffiths, G., N.J. Cook, E. Gottfridson, T. Lind, K. Lidholt, and I.S. Roberts. J. Biol. Chem., 273, 11752-11757, 1998.
Hagopian, A. and E.H. Eylar. Arch. Biochim. Biophys., 128, 422-433.
Hall, N.A. and A.D. Patrick. (1989) Anal. Biochem. 178, 378-384.
Hansen, L.M. and D.C. Hirch. (1989) Vet. Microbiol. 21, 177-184.
Hardingham, T.E. and A.J. Fosang. (1992) FASEB J. 6, 861-870.
Harmon, B.G., J. Glisson, K.S. Latimer, W.L. Stephens, and J.C. Nunnally. (1991) Am. J. Vet. Res. 52, 1507-1511.
Hascall, V.C. and G.K. Hascall. (1981) in Cell Biology of Extracellular Matrix (Hay, E.D., ed) pp. 39-78, Plenum Publishing Corp. New York.
Heldermon, C., P.L. DeAngelis, and P.H. Weigel. (2001) J. Biol. Chem., 276, 2037-2046.
Hempel, J., J. Perozich, H. Romavacek, A. Hinich, I. Kuo, and D.S. Feingold. (1994) Protein Sci. 3, 1074-1080.
Hodson, N., G. Griffiths, N. Cook, M. Pourhossein, E. Gottfridson, T. Lind, K. Lindholt, and I.S. Roberts. (2000) J. Biol. Chem., 275, 27311-27315.
Hofmann, K. and W. Stoffel. (1993) Biol. Chem. Hoppe-Seyler 347, 166 (abstr.)
livanainen, E., Kahari, VM., Heino, J., and Elenius, K. (2003) Microsc Res Tech, 60:13-22.
Ikegami-Kawai, M. and Takahashi, T. (2002) Analytical Biochem, 311(2), 157-165.
Itano, N., T. Sawai, M. Yoshida, P. Lenas, Y. Yamada, M. Imagawa, T. Shinomura, M. Hamaguchi, Y. Yoshida, Y. Ohnuki, S. Miyauchi, A.P. Spicer, J.A. McDonald, and K. Kimata. (1999) J. Biol. Chem. 274(35), 25085-25092.
Jensen, R.A. (1976) Annu Rev Microbiol 30, 409-425
Jing, W. and P.L. DeAngelis. Glycobiology. 10(9):883-9, 2000.
Jing W. and DeAngelis, P.L., (2004) J Biol Chem 279, 42345-42349
Jing, W. and P. L. DeAngelis, Glycobiology, 2003, 13(10): p. 661R-71R.
Kane, T.A., White, C.L. and DeAngelis, P.L. (2006) J. Biol Chem 281, 33192-33197
Kim, B.T., et al., ProcNatl Acad Sci USA, 2001. 98(13): p. 7176-81.
Kitagawa, H., T. Uyama, and K. Sugahara. (2001) J. Biol. Chem., 276, 38721-38726.
Kitagawa, H., et al., J. Biol Chem, 2007. 282(11): p. 8533- 44.
Knudson, C.B. and W. Knudson (1993) FASEB. J. 7, 1233-1241.
Koyama, M., W. Helbert, T. Imai, J. Sugiyama, and B. Henrissat. (1997) Proc. Natl. Acad. Sci. U.S.A. 94, 9091-9095.
Kroll, J.S., B. Loynds, L.N. Brophy, and E.R. Moxon. (1990) Mol. Microbiol. 4, 1853-1862.
Kumari, K. and P.H. Weigel. (1997) J. Biol. Chem., 272, 32539-32546.
Laurent, T.C., and J.R.E. Fraser. (1992) FASEB J. 6, 2397-2404.
Lee, C.J. (1987) Mol. Immunol., 24, 1005-1019.
Lee, HG and Cowman, MK (1994) Analytical Biochem. 219(2), 278-287.
Li, J., D.M. Rancour, M.L. Allende, C.A. Worth, D.S. Darling, J.B. Gilbert, A.K. Menon and W.W. Young Jr. (2001) Glycobiology, 11, 217-229.
Lidholt, K. (1997) Biochem. Soc. Trans. 25, 866-870.
Lidholt, K. and M. Fjelstad. (1997) J. Biol. Chem. 272, 2682-2687.
Lidholt, K. and U. Lindahl. (1992) Biochem J. 287, 21-29.
Lindahl, U. and M. Hook. (1978) Annu. Rev. Biochem. 47, 385-417.
Lind, T., U. Lindahl, and K. Lidholt. (1993) J. Biol. Chem. 268, 20705-20708.
Lind, T. et al., (1998) J. Biol. Chem. 273, 11752-11757.
Liu, Z., Zhang, J., Chen, X. and Wang, P.G., (2002) Chembiochem 3(4), 348-355
Ludwigs, U., A. Elgavish, J.D. Esko, E. Meexan, and L. Roden. Biochem.J., 245, 795-804, 1987.
Marks, D.L., M. Dominguez, K. Wu, and R.E. Pagano. (2001) J. Biol. Chem. 276, 26492-26498.
Markovitz, A., J.A. Cifonelli, and A. Dorfman. (1959) J. Biol. Chem. 234, 2343-2350.
May, B.J., Q. Zhang, L. Li, M.L. Paustian, T.S. Whittam, and V. Kapur. (2001) Proc. Natl. Acad. Sci. U.S.A. 98, 3460-3465.
Meyer, M.F., and G. Kreil (1996) Proc. Natl. Acad. Sci. U.S.A. 93, 4543-4547.
Morera, S., A. Imberty, U. Aschke-Sannenborn, P.S. Freemont, J. Janin, and W. Ruger. (1999) J. Mol. Biol. 311, 569-577.
Morera, S. L. Lariviere, J. Kurzeck, U. Aschke-Sannenborn, P.S. Freemont, J. Janin, and W. Ruger. (2001) J. Mol. Biol., 311, 569-577.
Ohya, T. and Y. Kaneko. (1970) Biochim. Biophys. Acta 198, 607-609.
Pedersen, L.C., K. Tsuchida, H. Kitagawa, K. Sugahara, T.A. Darden, and M. Negishi. (2000) J. Biol. Chem., 275, 34580-34585.
Persson, K., H.D. Ly, M. Dieckelmann, W.W. Wakarchuk, S.G. Withers, and N.C.J. Strynadka. (2001) Nat. Struct. Biol. 8, 166-175.
Petit, C., G.P. Rigg, C. Pazzani, A.Smith, V. Sieberth, M. Stevens, G. Boulnois, K. Jann, and I.S. Roberts. Mol. Microbiol., 17, 611-620.
Prehm, P. (1983) Biochem. J. 211, 181-189.
Prehm, P. (1983) Biochem. J. 211, 191-198.
Pummill, P.E., et al., Biochim Biophys Acta, 2007. 1770(2): p. 286-90.
Pummill, P.E., E.S. Kempner and P.L. DeAngelis, J. Biol Chem, 2001. 276(43): p. 39832-5.
Pummill, P.E., and P.L. DeAngelis. J.Biol.Chem. 277(24):21610-6, 2002.
Pummill P.E., A.M. Achyuthan, and P.L. DeAngelis. J.Biol.Chem. 273(9):4976-81, 1998.
Quinn, A.W., and K.P. Sing. (1957) Proc. Soc. Exp. Biol. Med. 95, 290-294.
Radominska, A. and R.R. Drake. (1994) Methods Enzymol. 230, 330-339.
Rahemtulla, F. and S. Lovtrup. (1975) Comp. Biochem. Physiol. 50B, 631-635.
Ramakrishnan, B. and P. Qasba. (2001) J. Mol. Biol. 310, 205-218.
Rejzek, M., Mukhopadhyay, B, Wenzel, C.Q., Lam. J.S. and Field, R.A., (2007) Carbohydr Res 342, 460-466
Rimler, R.B. (1994) Vet. Rec. 134, 191-192.
Rimler, R.B. and K.R. Rhodes. (1987) J. Clin. Microbiol. 25, 615-618.
Rimler, R.B. (1994) Vet. Rec. 134, 191-192.
Rimler, R.B., K.B. Register, T. Magyar, and M.R. Ackermann. (1995) Vet. Microbiol.47, 287-294.
Roberts, I.S. (1996) Annu. Rev. Microbiol. 50, 285-315.
Roberts, I.S., R. Mountford, R. Hodge, K.B. Jann, and G. Boulnois. (1988) J. Bacteriol. 170, 1305-1310.
Roden, L. (1980) in The Biochemistry of Glycoproteins and Proteoglycans (Lennarz, W.J., ed) pp. 267-371, Plenum Publishing Corp. New York.
Rodriguez, M.L, B. Jann, and K. Jann. (1988) Eur. J. Biochem. 177, 117-124.
Rohozinski, J., L.E. Girton, and J.L. Van Etten. Virology 168, 363 (1989).
Rosa, F., T.D. Sargent, M.L. Rebbert, G.S. Michaels, M. Jamrich, H. Grunz, E. Jonas, J.A. Winkles, and I.B. Dawid. (1988) Dev. Biol. 129, 114-123.
Rosner, H., H.D. Grimmecke, Y.A. Knirel, and A.S. Shashkov. (1992) Carbohydr. Res. 223, 329-333.
Sala, R.F., et al., Carbohydr Res. 1998. 306(1-2): p. 127-36.
Sambrook, J., E.F. Fritshc, and T. Maniatis. Molecular Cloning: A Laboratory Manual, 2nd edn. Cold Spring Harbor, NY: Cold Spring Laboratory Press. 1989.
Saxena, I.M., R.M. Brown, M. Fevre, R.A. Geremia, and B. Henrissat. J. Bacteriol., 177, 1419-1424, 1995.
Semino, C.E. and P.W. Robbins. (1995) Proc. Natl. Acad. Sci. U.S.A. 92, 3498-3501.
Semino, C.E., C.A. Specht, A. Raimondi, and P.W. Robbins. (1996) Proc. Natl. Acad. Sci. U.S.A. 93, 4548-4553.
Senay, C., et al., EMBO Rep, 2000. 1(3): p. 286-6.
Soltes, L., Mendichi, R., Lath, D., Mach, M. and Bakos, D. (2002) Biomed. Chromatogr. 16, 459-462.
Song, HH. and Filmus, J. (2002) Biochim Biophys Acta, 1573:241-246.
Spicer, A.P. and J.A. McDonald. (1998), J. Biol. Chem. 273(4), 1923-1932.
Spicer, A.P. and T.K. Nguyen, Biochem Soc Trans, 1999. 27(2): p. 109-15.
Stoolmiller, A.C. and A. Dorfman. (1969) J. Biol. Chem. 244, 236-346.
Sugahara, K. and Kitagawa, H. (2002) IUBMB Life 54, 163-175
Sugahara, K., N.B. Schwartz and A. Dorfman. (1979) J. Biol. Chem. 254, 6252-6261.
Sunthankar, P. I. Pastuszak, A. Rooke, A.D. Elbein, I. van de Rijn, W.M. Canfield, and R.R. Drake. (1998) Anal. Biochem., 258(2): 195-201.
Suzuki, M., et al., Biochem J, 1995. 307 (Pt3): p 817-21.
Svanborg-Eden, C., L. Hagberg, R. Hull, S. Hull, K.E. Magnusson, and L. Tarbouriech, N., S.J. Charnock, and G.J. Davies. (2001) J. Mol. Biol. 314, 655-661.
Taylor, K.A., and J.G. Buchanan-Smith. (1992) Anal. Biochem. 201, 190-196.
Telser, A., H.C. Robinson, and A. Dorfman. (1965) Proc. Natl. Acad. Sci. U.S.A. 54, 912-919.
Tengblad, A. (1980) Biochem. J. 185, 101-105.
Tlapak-Simmons, V.L., E.S. Kempner, B.A. Baggenstoss, and P.H. Weigel. (1998) J. Biol. Chem., 273, 26100-26109.
Tlapak-Simmons, V.L., B.A. Baggenstoss, K. Kumari, C. Heldermon, and P.H. Weigel. (1999) J. Biol. Chem. 274, 4246-4253.
Townsend, K.M., J.D. Boyce, J.Y. Chung, A.J. Frost, and B. Adler. (2001) J. Clin. Microbiol., 39, 924-929.
Tracy, B.S., et al., J. Biol Chem 2007. 282(1): p. 337-44.
Tsuchida, K., T. Lind, H. Kitagawa, U. Lindahl, K. Sugahara, and K. Lindholt. (1999) Eur. J. Biochem. 264, 461-467.
Uebelhart, D. and Williams, JM. (1999) Curr. Opin in Rhematology, 11, 427.
Unligil, U.M. and J.M. Rini. (2000) Curr. Opin. Struct. Biol. 10, 510-517.
Unligil, U.M., S. Zhou, S. Yuwaraj, M. Sarkar, H. Schachter, and J.M. Rini. (2000) EMBO J. 19, 5269-5280.
van de Rijn, I. and R.R. Drake (1992) J. Biol. Chem. 267, 24302-24306.
van de Rijn, I. and R.E. Kessler. (1980) Infect. Immun. 27, 444-448.
Van Etten, J.L., D.E. Burbank, A.M. Schuster, and R.H. Meints, Virology, 140, 135 (1985).
Vann, W.F., M.A. Schmidt, B. Jann, and K. Jann. (1981) Eur. J. Biochem. 116, 359-364.
Varki, A. (1996) Proc. Natl. Acad. Sci. U.S.A. 93, 4523-4525.
Vimr, E.R., W. Aaronson, and R.P. Silver. (1989) J. Bacteriol. 171, 1106-1117.
Vlodavsky, I Miao, HQ, Medalion, B., Danager, P., and Ron, D. (1996) Cancer Metastasis, 15:177-186.
Vrielink, A., W. Ruger, H.P.C. Driessen, and P.S. Freemont. (1994) EMBO J. 15, 3413-3422.
Weigel, P.H., V.C. Hascall, and M. Tammi. J. Biol. Chem., 272, 13997-14000, 1997.
Wessels, M.R., A.E. Moses, J.B. Goldberg, and T.J. DiCesare. (1991) Proc. Natl. Acad. Sci. U.S.A. 88, 8317-8321.
Wiggins, C.A.R., and S. Munro. (1998) Proc. Natl. Acad. Sci. U.S.A. 95, 7945-7950.
Williams, K.J., Halkes, K.M., Kamerling, J.P. and DeAngelis, P.L., (2006) J. Biol Chem 281, 5391-5397
Wilson, K. Preparation of genomic DNA from bacteria. In: Ausbel FM, Brent R, Kingston RE, et al., Eds. Current Protocols in Molecular Biology. New York: Wiley Interscience Publishing, 1987: 2.4.1-2.4.5.
Woodfield, TB, Bezemer, JM, Pieper, JS, van Blitterswijk, CA, and Riesle, J. (2002) Crit Rev Eukaryot Gene Expr, 12:209-236.
Yamada, T., T. Higashiyama, and T. Fukuda, Appl. Environ. Microbiol. 57, 3433 (1991).
Yoshida, M., N. Itano, Y. Yamada, and K. Kimata. J. Biol. Chem., 275, 497-506, 2000.
Zak, B.M., Crawford, B.E. and Esko, J.D. (2002) Biochim Biophys Acta 1573, 346-355

## Claims

1. A method for enzymatically producing substantially monodisperse glycosaminoglycan polymers comprising providing at least one functional acceptor and at least one recombinant heparosan synthase capable of elongating the functional acceptor in a controlled and/or repetitive fashion to form extended glycosaminoglycan-like molecules,
wherein the recombinant heparosan synthase has an amino acid sequence as set forth in SEQ ID NO: 6 or an amino acid sequence at least 98% identical to the sequence of SEQ ID NO:66,
wherein the functional acceptor comprises at least two sugar units of uronic acid and/or hexosamine,
wherein at least one UDP-sugar is added in a stoichiometric ratio to the functional acceptor such that the recombinant heparosan synthase elongates the at least one functional acceptor to provide glycosaminoglycan polymers having a desired size distribution and that are in substantially monodisperse size, wherein a desired size distribution of glycosaminoglycan polymers is obtained by controlling the stoichiometric ratio of UDP-sugar to functional acceptor,
wherein the substantially monodisperse extended glycosaminoglycan polymers have (a) a molecular weight in a range of from 3.5 kDa to 0.5 MDa, and a polydispersity value in a range of from 1.0 to 1.1, or (b) a molecular weight in a range of from 0.5 MDa to 4.5 MDa, and a polydispersity value in a range of from 1.0 to 1.5.

2. The method of claim 1, wherein the substantially monodisperse extended glycosaminoglycan polymers have a molecular weight in a range of from 3.5 kDa to 0.5 MDa, and a polydispersity value in a range of from 1.0 to 1.05.

3. The method of any one of claims 1-2, wherein the substantially monodisperse extended glycosaminoglycan polymers have a molecular weight in a range of from 0.5 MDa to 4.5 MDa, and a polydispersity value in a range of from 1.0 to 1.2.

4. The method of any one of claims 1-3, wherein the functional acceptor comprises a reducing end.

5. The method of claim 4, wherein the reducing end of the functional acceptor serves as a tether site onto a surface or drug.

6. The method of any one of claims 1-5, wherein the functional acceptor is hexosamine comprising a TFA group.

7. The method of claim 6, wherein a free amine is produced by N-deacetylation of the TFA-modified hexosamine under mild basic conditions, optionally wherein the free amine is a primary amine.

8. The method of claim 7, wherein the primary amine is a site for (i) N-sulfation, (ii) coupling to drugs or (iii) cross-linking to form a gel.

9. The method of any one of claims 1-8, wherein the UDP-sugar is selected from the group consisting of UDP-GlcUA, UDP-GalUA, UDP-GlcNAc, UDP-Glc, UDP-GalNAc, UDP-GlcN, UDP-GalN, UDP-GlcNAcUA, UDP-GlcNAcNAc, UDP-GlcdiNAcUA, UDP-GlcN[TFA], UDP-GlcNBut, UDP-GlcNPro, UDP-6-F-6-deoxyGlcNAc, and UDP-2-F-2-deoxyGlcUA.

10. The method of any one of claims 1-9, wherein the at least one UDP-sugar is radioactive or nuclear magnetic resonance-active.

11. The method of any one of claims 1-10, wherein the at least one UDP-sugar comprises a moiety selected from the group consisting of a fluorescent tag, a radioactive tag or therapeutic, an affinity tag, a detection probe, a medicant, a biologically active agent, a therapeutic agent, and combinations thereof.

12. The method of any one of claims 1-11, wherein each of the functional acceptor comprises a moiety selected from the group consisting of a fluorescent tag, a radioactive tag or therapeutic, an affinity tag, a detection probe, a medicant, a biologically active agent, a therapeutic agent, and combinations thereof.

13. The method of any one of claims 1-12, wherein the glycosaminoglycan polymers have an unnatural structure.

14. The method of any one of claims 1-13, wherein the functional acceptor comprises Fluorescein di-β-D-glucuronide (A-F-A).

15. The method of any one of claims 1-14, wherein the recombinant heparosan synthase has an amino acid sequence as set forth in SEQ ID NO:66.

## Patentansprüche

1. Verfahren zur enzymatischen Herstellung von im Wesentlichen monodispersen Glykosaminoglykanpolymeren, umfassend das Bereitstellen mindestens eines funktionellen Akzeptors und mindestens einer rekombinanten Heparosansynthase, die in der Lage ist, den funktionellen Akzeptor auf kontrollierte und/oder wiederholte Weise zu verlängern, um verlängerte Glykosaminoglykan-ähnliche Moleküle auszubilden, wobei die rekombinante Heparosansynthase eine Aminosäuresequenz gemäß SEQ ID NO: 6 oder eine Aminosäuresequenz aufweist, die zu mindestens 98 % identisch mit der Sequenz von SEQ ID NO: 66 ist, wobei der funktionelle Akzeptor mindestens zwei Zuckereinheiten von Uronsäure und/oder Hexosamin umfasst,
wobei mindestens ein UDP-Zucker in einem stöchiometrischen Verhältnis zu dem funktionellen Akzeptor zugegeben wird, sodass die rekombinante Heparosansynthase den mindestens einen funktionellen Akzeptor verlängert, um Glykosaminoglykanpolymere mit einer gewünschten Größenverteilung bereitzustellen, die eine im Wesentlichen monodisperse Größe aufweisen, wobei eine gewünschte Größenverteilung von Glykosaminoglykanpolymeren durch Kontrolle des stöchiometrischen Verhältnisses von UDP-Zucker zu funktionellem Akzeptor erhalten wird,
wobei die im Wesentlichen monodispersen verlängerten Glykosaminoglykanpolymere (a) ein Molekülgewicht im Bereich von 3,5 kDa bis 0,5 MDa und einen Polydispersitätswert im Bereich von 1,0 bis 1,1 aufweisen, oder (b) ein Molekülgewicht im Bereich von 0,5 MDa bis 4,5 MDa und einen Polydispersitätswert im Bereich von 1,0 bis 1,5 aufweisen.

2. Verfahren nach Anspruch 1, wobei die im Wesentlichen monodispersen verlängerten Glykosaminoglykanpolymere ein Molekülgewicht im Bereich von 3,5 kDa bis 0,5 MDa und einen Polydispersitätswert im Bereich von 1,0 bis 1,05 aufweisen.

3. Verfahren nach einem der Ansprüche 1-2, wobei die im Wesentlichen monodispersen verlängerten Glykosaminoglykanpolymere ein Molekülgewicht im Bereich von 0,5 MDa bis 4,5 MDa und einen Polydispersitätswert im Bereich von 1,0 bis 1,2 aufweisen.

4. Verfahren nach einem der Ansprüche 1-3, wobei der funktionelle Akzeptor ein reduzierendes Ende umfasst.

5. Verfahren nach Anspruch 4, wobei das reduzierende Ende des funktionellen Akzeptors als Bindungsstelle auf einer Oberfläche oder einem Arzneimittel dient.

6. Verfahren nach einem der Ansprüche 1-5, wobei der funktionelle Akzeptor Hexosamin ist, das eine TFA-Gruppe umfasst.

7. Verfahren nach Anspruch 6, wobei ein freies Amin durch N-Deacetylierung des TFA-modifizierten Hexosamins unter milden basischen Bedingungen hergestellt wird, optional wobei das freie Amin ein primäres Amin ist.

8. Verfahren nach Anspruch 7, wobei das primäre Amin eine Stelle für (i) N-Sulfatierung, (ii) Kopplung an Arzneimittel oder (iii) Quervernetzung zur Bildung eines Gels ist.

9. Verfahren nach einem der Ansprüche 1-8, wobei der UDP-Zucker ausgewählt ist aus der Gruppe bestehend aus UDP-GlcUA, UDP-GalUA, UDP-GlcNAc, UDP-Glc, UDP-GalNAc, UDP-GlcN, UDP-GalN, UDP-GlcNAcUA, UDP-GlcNAcNAc, UDP-GlcdiNAcUA, UDP-GlcN[TFA], UDP-GlcNBut, UDP-GlcNPro, UDP-6-F-6-deoxyGlcNAc und UDP-2-F-2-deoxyGlcUA.

10. Verfahren nach einem der Ansprüche 1-9, wobei der mindestens eine UDP-Zucker radioaktiv oder kernmagnetresonanzaktiv ist.

11. Verfahren nach einem der Ansprüche 1-10, wobei der mindestens eine UDP-Zucker einen Molekülteil umfasst, ausgewählt aus der Gruppe bestehend aus einem fluoreszierenden Tag, einem radioaktiven Tag oder einem Therapeutikum, einem Affinitäts-Tag, einer Nachweissonde, einem Medikament, einem biologisch aktiven Mittel, einem Wirkstoff sowie Kombinationen daraus.

12. Verfahren nach einem der Ansprüche 1-11, wobei jeder des funktionellen Akzeptors einen Molekülteil umfasst, ausgewählt aus der Gruppe bestehend aus einem fluoreszierenden Tag, einem radioaktiven Tag oder einem Therapeutikum, einem Affinitäts-Tag, einer Nachweissonde, einem Medikament, einem biologisch aktiven Mittel, einem Wirkstoff und Kombinationen daraus.

13. Verfahren nach einem der Ansprüche 1-12, wobei die Glykosaminoglykanpolymere eine unnatürliche Struktur aufweisen.

14. Verfahren nach einem der Ansprüche 1-13, wobei der funktionelle Akzeptor Fluorescein-di-β-D-glucuronid (A-F-A) umfasst.

15. Verfahren nach einem der Ansprüche 1-14, wobei die rekombinante Heparosansynthase eine Aminosäuresequenz aufweist, wie in SEQ ID NO: 66 dargelegt.

## Revendications

1. Procédé pour la production de manière enzymatique de polymères de type glycosaminoglycane sensiblement monodispersés comprenant la mise à disposition d'au moins un accepteur fonctionnel et d'au moins une héparosane synthase recombinante capable d'allonger l'accepteur fonctionnel d'une manière contrôlée et/ou répétitive pour former des molécules de type glycosaminoglycane allongée,
l'héparosane synthase recombinante possédant une séquence d'acides aminés telle que représentée dans SEQ ID NO: 6 ou une séquence d'acides aminés au moins 98 % identique à la séquence de SEQ ID NO: 66,
l'accepteur fonctionnel comprenant au moins deux motifs de sucre d'acide uronique et/ou d'hexosamine,
au moins un UDP-sucre étant ajouté en un rapport stœchiométrique à l'accepteur fonctionnel de telle manière que l'héparosane synthase recombinante allonge l'au moins un accepteur fonctionnel pour fournir des polymères de type glycosaminoglycane possédant une distribution de taille souhaitée et qui sont de taille sensiblement monodispersée, une distribution de taille souhaitée de polymères de types glycosaminoglycane étant obtenue en régulant le rapport stœchiométrique d'UDP-sucre sur accepteur fonctionnel,
les polymères de type glycosaminoglycane allongés sensiblement monodispersés possédant (a) un poids moléculaire dans une plage allant de 3,5 kDa à 0,5 MDa, et un indice de polydispersité dans une plage allant de 1,0 à 1,1, ou (b) un poids moléculaire dans une plage allant de 0,5 MDa à 4,5 MDa, et un indice de polydispersité dans une plage allant de 1,0 à 1,5.

2. Procédé selon la revendication 1, les polymères de type glycosaminoglycane allongés sensiblement monodispersés possédant un poids moléculaire dans une plage allant de 3,5 kDa à 0,5 MDa, et un indice de polydispersité dans une plage allant de 1,0 à 1,05.

3. Procédé selon l'une quelconque des revendications 1 et 2, les polymères de type glycosaminoglycane allongés sensiblement monodispersés possédant un poids moléculaire dans une plage allant de 0,5 MDa à 4,5 MDa, et un indice de polydispersité dans une plage allant de 1,0 à 1,2.

4. Procédé selon l'une quelconque des revendications 1 à 3, l'accepteur fonctionnel comprenant une extrémité réductrice.

5. Procédé selon la revendication 4, l'extrémité réductrice de l'accepteur fonctionnel servant comme site d'attache sur une surface ou un médicament.

6. Procédé selon l'une quelconque des revendications 1 à 5, l'accepteur fonctionnel étant l'hexosamine comprenant un groupe TFA.

7. Procédé selon la revendication 6, une amine libre étant produite par N-désacétylation de l'hexosamine modifiée par TFA dans des conditions basiques douces, éventuellement l'amine libre étant une amine primaire.

8. Procédé selon la revendication 7, l'amine primaire étant un site pour (i) une N-sulfatation, (ii) le couplage à des médicaments ou (iii) une réticulation pour former un gel.

9. Procédé selon l'une quelconque des revendications 1 à 8, l'UDP-sucre étant choisi dans le groupe constitué par UDP-GlcUA, UDP-GalUA, UDP-GlcNAc, UDP-Glc, UDP-GalNAc, UDP-GlcN, UDP-GalN, UDP-GlcNAcUA, UDP-GlcNAcNAc, UDP-GlcdiNAcUA, UDP-GlcN[TFA], UDP-GlcNBut, UDP-GlcNPro, UDP-6-F-6-désoxyGlcNAc, et UDP-2-F-2-désoxyGlcUA.

10. Procédé selon l'une quelconque des revendications 1 à 9, l'au moins un UDP-sucre étant radioactif ou actif en résonance magnétique nucléaire.

11. Procédé selon l'une quelconque des revendications 1 à 10, l'au moins un UDP-sucre comprenant un groupement choisi dans le groupe constitué par un marqueur fluorescent, un marqueur ou composé thérapeutique radioactif, un marqueur d'affinité, une sonde de détection, un médicament, un agent biologiquement actif, un agent thérapeutique, et des combinaisons correspondantes.

12. Procédé selon l'une quelconque des revendications 1 à 11, chacun des accepteurs fonctionnels comprenant un groupement choisi dans le groupe constitué par un marqueur fluorescent, un marqueur ou composé thérapeutique radioactif, un marqueur d'affinité, une sonde de détection, un médicament, un agent biologiquement actif, un agent thérapeutique, et des combinaisons correspondantes.

13. Procédé selon l'une quelconque des revendications 1 à 12, les polymères de types glycosaminoglycane possédant une structure non naturelle.

14. Procédé selon l'une quelconque des revendications 1 à 13, l'accepteur fonctionnel comprenant le di-β-D-glucuronide de fluorescéine (A-FA) .

15. Procédé selon l'une quelconque des revendications 1 à 14, l'héparosane synthase recombinante possédant une séquence d'acides aminés telle que représentée dans SEQ ID NO: 66.
